# EUROPEAN PATENT APPLICATION

(11) **EP 2 075 256 A2**
(43) Date of publication of application: **01.07.2009**
(21) Application number: 09003542.9
(22) Date of filing: 14.01.2003
(51) Int. Cl.: C07K 16/00, C07K 19/00, C07K 16/46, C07K 14/00

(54) **Multispecific binding molecules**

(30) Priority: 14.01.2002 CA 2368708; 11.03.2002 WO PCT/CA02/00317; 13.08.2002 CA 2397169; 19.09.2002 CA 2402930
(62) Divisional of application: 03700256.5
(71) Applicant: Herman, William, Thorn Hill, ON L4J 5V2 (CA)
(72) Inventor: Herman, William, Thorn Hill, ON L4J 5V2 (CA)
(74) Representative: Vossius & Partner

(57) **Abstract**

The invention contemplates a composition containing a multispecific ligand containing at least a first ligand binding moiety and a second ligand binding moiety. The first ligand binding moiety specifically binds with a pre-selected first affinity to at least a first ligand. The first ligand has a first biodistribution. The second ligand binding moiety specifically binds with a pre-selected affinity to at least a second ligand. The second ligand has a second biodistribution. The affinity of first and second ligand binding moieties are selected to bias the biodistribution of the multispecific ligand in favour of a selected location of one or both of the ligands.

## Description

### Field of The Invention

The present invention relates to multispecific ligands, for example a heterofunctional ligand comprising at least first and second binding moieties which have cooperating functional affinities including a multispecific ligand, for example, a bispecific antibody, having at least a first portion which binds to a 'lymphatic vessel associated' antigen/receptor and a second portion having at least one immune-affecting functionality including, without limitation, functions related to antigen presentation, immune signaling, suppression or enhancement of immune tolerance or immune stimulation, or binding to a target molecule, for example a cell surface antigen, receptor etc.

### Background of the Invention

Immunotherapy has gained wide acceptance as a promising measure to address several disease states including autoimmune disease, transplant rejection, infectious disease and cancer. Despite rapid and exciting progress in approaches to treatment, the disease burden attributable to such illnesses has not significantly abated. The complex nature of the normal and pathologic immunologic processes associated with such diseases, coupled with logistical problems in evaluating and implementing methods for immunotherapy in human subjects, continue to be some of the obstacles to successful advances in treatment.

Successful approaches to immunotherapy are predicated on the ability of the immunotherapeutic molecule to be delivered in a therapeutic, sub-toxic dose at the desired therapeutic frequency. In the process of selection of a suitable therapeutic molecule, it is recognized that sub-toxic doses may be insufficient for the desired therapeutic effect, especially where the antibody binds incidentally to cell populations other than the target population. In the case of an injectable preparation and especially an intravenous mode of delivery, in contrast to readily self-administered modes of delivery, the optimal dosing frequency for therapeutic purposes could impose an undesirable burden on the patient and care-giver, assuming that such optimal frequency is to begin with deemed convenient for clinical trials.

Numerous research efforts are underway to identify and test ligands including antibodies, biologic effector ligands (e.g. cytokines, chemokines, growth factors colony stimulating factors) receptor agonists orantagonists etc. which will bind to or otherwise interact with or trigger responses in or towards target entities, including pathogenic organisms, tissue specific cells,diseased cells, immune cells etc. A recent example is a renewed interest to find molecules and methods of triggering an interaction with CD45 (see for example Nature (2001) Vol. 409 p. 349-354). Evaluating the biological effect of interactions with such target ligands is often obfuscated and retarded by the biodistribution of such ligands on cells other than the target population which results in undesired and/or confusing pleiotropic effects.

The present invention facilitates scientific assessment, development, role evaluation, therapeutic evaluation, and delivery, particularly targeted delivery of molecules that exert biologic functions and particularly immune relatedfunctions. In particular, the targeting agents and methods which are the subject of the invention herein facilitate scientific evaluation of the biological effects of a more targeted biodistribution of such targeting agents, by limiting undesired or confusing side effects. In preferred aspects the invention contemplates compositions of matter and methods of delivery, in some cases using ligands that but for the targeting methods herein defined would be ineffective or have a broader effect than is desirable; or similarly, but for the severity of the disease or the absence of other therapeutic alternatives for which such ligands are useful, they would otherwise be inappropriate for therapeutic use. The present invention accommodates evaluation of the biological role and/or effects of such ligands for therapeutic or other scientific purposes using such targeting strategies. In particular, the present invention provides a vehicle to preferentially target, on a sub-population of cells for which there is a cell-associated marker, a receptor or receptor ligand which is present on a more heterogeneous population of cells.

### Summary of The Invention

The invention contemplates a composition containing a multispecific ligand containing at least a first ligand binding moiety and a second ligand binding moiety. The first ligand binding moiety specifically binds with a pre-selected first affinity to at least a first ligand. The first ligand has a first biodistribution. The second ligand binding moiety specifically binds with a pre-selected affinity to at least a second ligand. The second ligand has a second biodistribution. The affinity and/or on-rate of first and second ligand binding moities are selected to bias the biodistribution of the multispecific ligand in favour of a selected location of one or both of the ligands. In one embodiment the first ligand binding moiety has a higher intrinsic affinity and/or a higher on-rate.

The invention contemplates a composition containing a multispecific ligand. The multispecific ligand contains at least a first ligand binding moiety and a second ligand binding moiety. The first ligand binding moiety specifically binds to a first ligand having a first biodistribution. The second ligand binding moiety specifically binds to a second ligand having a second biodistribution. The second biodistribution is different from that of the first biodistribution, and the affinity and/or on-rate of the first and second ligand binding moieties to their respective ligands are different and selected to bias the biodistribution of the multispecific ligand towards the first or second biodistribution.

The invention contemplates further, a composition containing a multispecific ligand. The multispecific ligand contains a first ligand binding moiety and a second ligand' binding moiety. The first ligand binding moiety specifically binds with a pre-selected first affinity to a first ligand. The first ligand has a first biodistribution. The second ligand binding moiety specifically binds with a pre-selected affinity to a second ligand. The second ligand has a second biodistribution. In this embodiment of the multispecific ligand, the affinity and/or on-rate of first and second ligand binding moieties are selected to bias the biodistribution of the multispecific ligand.

The invention further contemplates a composition containing a multispecific ligand. The multispecific ligand specifically binds to a target ligand. The target ligand is specific to a selected sub-population of a heterogeneous cell population. This embodiment of the multispecific ligand contains a first ligand binding moiety and a second ligand binding moiety. The first ligand binding moiety specifically binds to a cell sub-population associated ligand. The second ligand binding moiety binds to the target ligand. In this embodiment, the first ligand binding moiety has an affinity and/or a higher on-rate for the sub-population associated ligand higher than the affinity and/or on-rate of the second ligand binding moiety for the target ligand.

The invention further contemplates a composition containing a bispecific ligand containing a first ligand and a second ligand. The first ligand binds to a first target ligand and the second ligand binds to a second target ligand. In this embodiment of the bispecific ligand, the affinity of the first ligand is selected to enable binding to the first target ligand independently of the ability of the second ligand to bind to the second target ligand. Further, the affinity and/or on-rate of the second ligand is selected to substantially reduce the probability of its binding to the second target ligand without the first ligand binding first or substantially contemporaneously to the first target ligand.

The invention further contemplates a composition containing a bispecific antibody containing a first antibody component and a second antibody component. The first antibody component binds to a first target ligand and the second antibody component binds to a second target ligand. In this embodiment, the affinity and/or on-rate and/or avidity of the first antibody component are selected to enable binding to the first target ligand independently of the ability of the second antibody component to bind to the second target ligand. The avidity or affinity and/or on-rate of the second ligand are selected to substantially reduce the probability of its binding to the second target ligand without the first ligand binding first or substantially contemporaneously to the first target ligand.

The invention further contemplates a multispecific ligand containing a first moiety and a second moiety. The first moiety binds to a first target ligand. The second moiety binds to a second target ligand. The affinity and/or avidity and/or on-rate of the first moiety are selected to enable the first moiety to bind to the first target ligand independently of the ability of the second moiety to bind to the second target ligand. The avidity and/or affinity and/or on-rate of the second moiety are selected to substantially reduce the probability of its binding to the second target ligand without the first moiety, first or substantially contemporaneously, binding to the first target ligand.

The invention further contemplates a multispecific ligand containing a first moiety and a second moiety. The first moiety binds to a first target ligand. The second moiety binds to a second target ligand. The affinity and/or avidity and/or on-rate of the first moiety are selected to enable the first moiety to bind to the first target ligand independently of the ability of the second moiety to bind to the second target ligand. The avidity and/or affinity and/or on-rate of the second moiety are selected to substantially reduce the probability of either moiety binding first or for a sufficient duration or series of durations to its respective target ligand to accomplish a therapeutic function without the other moiety, first or substantially contemporaneously, binding to its respective target ligand.

The invention further contemplates a composition containing a multispecific ligand containing a first moiety and a second moiety. The first moiety binds to a first target ligand. The second moiety binds to a second target ligand. The affinity or avidity or both the affinity and avidity of the first moiety are selected to enable the first moiety to bind to the first target ligand independently of the ability of the second moiety to bind to the second target ligand. The avidity or affinity or both the affinity and avidity of the second moiety are selected to enable the second moiety to bind to the second entity in preference to the first moiety binding to the first entity when both first and second moieties are substantially contemporaneously bound to the respective first and second entities.

The invention contemplates a composition containing a multispecific ligand containing a first moiety, a second moiety and a third ligand binding moiety. The first moiety binds to a first target ligand and the second moiety binds to a second target ligand. In this embodiment, the affinity or avidity or both the affinity and avidity of the first moiety are selected to enable the first moiety to bind to the first target ligand in preference to the second moiety binding to the second entity when both first and second moieties are substantially 'contemporaneously bound to the respective first and second entities, and the avidity or affinity or both the affinity and avidity of the second moiety are selected to enable the third target ligand to bind to the second entity in preference to the second moiety binding to the second entity when both the third target ligand and the second moiety are substantially contemporaneously bound to the second entity.

The invention further contemplates a composition containing an antibody which specifically binds to an epitope on a ligand. The ligand recognized by the antibody exerts a biologic effect by binding to a target site on a target ligand. The epitope bound by the antibody is proximal to the binding site of the ligand for the target ligand, so that binding of the antibody reduces but does not prevent the affinity of the ligand for its target ligand.

The invention further contemplates a composition containing a multispecific ligand containing a first ligand binding moiety and a second moiety. The first ligand binding moiety specifically binds to a lymphatic endothelial cell associated marker. The second moiety contains an independent therapeutic function.

The invention further contemplates a composition containing an immunocytokine containing an anti-idiotypic antibody component and a cytokine component. The anti-idiotypic antibody component recognizes the paratope of an antibody which binds to a lymphatic vessel associated ligand.

The invention further contemplates a composition containing a bispecific antibody containing an anti-idiotypic antibody component and an anti-CD3 antibody or an anti-CD28 antibody component. The anti-idiotypic antibody recognizes the paratope of an antibody which binds specifically to a lymphatic vessel associated ligand.

The invention additionally contemplates physiologically acceptable compositions of the compositions encompassed by the invention.

The invention likewise contemplates methods of use of the compositions encompassed by the invention.

A composition comprising a multispecific ligand comprising at least a first ligand binding moiety which specifically binds to a first ligand having a first biodistribution and a second ligand binding moiety which specifically binds to a second ligand having a second biodistribution different from that of the first ligand, and wherein the affinity of the first and second ligand binding moieties are different and selected to bias the biodistribution of the multispecific ligand.

### Detailed Description of Preferred Embodiments

As exemplified above, the dual "affinity"/"on-rate" based targeting strategy of the invention, may be understood in one aspect, in terms of a strategic allocation of the respective affinity properties of the multispecific ligand to at least one "targeting" function and at least one "effector" function. Accordingly, with respect to some embodiments of the invention, the term "multifunctional" ligand is used interchangeably,

Thus according to one preferred embodiment, at least one of the ligand binding moieties is a "targeting" arm in the sense that it at least preferentially recognizes a marker that is associated with one or more specific target entities eg. cell populations, and the other ligand binding moiety is an "effector" arm which binds with relatively less affinity or functional affinity and/or less quickly to a target ligand which optionally has a more diverse biodistribution. In this case, the biodistribution of the multispecific ligand is biased in favour of the location(s) of both ligands relative to the location(s) of the target ligand so as to limit the big distribution to non-target entities.

Such binding or recognition is preferably understood throughout to be specific, in contrast to non-specific binding.

Accordingly, in some embodiment the invention may be summarized in the following parapgraphs:
1. A multispecific ligand which comprises, at least, a targeting moiety which specifically binds to a first ligand having a first cell surface biodistribution and an effector moiety which binds to a second ligand having a second cell surface biodistribution different from but overlapping that of the first ligand, and wherein the targeting moiety is intrinsically predisposed to bind to the first ligand in preference to the effector moiety binding to the second ligand so as to bias the distribution of the multispecifc ligand to a target cell population in a mammal on which both said first and seconds ligands are bioavailable for recognition by said multispecific ligand, said second ligand characterized in that it is bioavailable for recognition on at least one population of cells other than the target cell population.
2. A multispecific ligand according to paragraph 1, wherein the first ligand is pre-targeted to the surface of the target cell population.
3. A multispecific ligand according to paragraph 1, wherein the first ligand is expressed on the surface of the target cell population.
4. A multispecific ligand according to paragraph 1, 2 or 3 wherein the intrinsic affinity of the targeting moiety exceeds the intrinsic affinity of the effector moiety.
5. A multispecific ligand according to paragraph 1, 2, 3 or 4, wherein the on-rate of the targeting moiety exceeds (is faster) than the on-rate of the effector moiety.
6. A multispecific ligand according to paragraph 1, 2, 3, 4 or 5, wherein the intrinsic off-rate of the targeting moiety is less (slower) than the intrinsic off-rate of the effector moiety.
7. A multispecific ligand according to paragraph 1, 2, 3, 4, 5 or 6, wherein the intrinsic on- rate of the targeting moiety exceeds the intrinsic on-rate of the effector moiety. ¹ For example, the effector moiety may be a coybody, which may be generated for example by a process of mutagenesis in which one or more resides in at least one CDR or FR are mutated or added, preferably residues which do not contribute to the specificity and off-rate of a reference antibody for example an antibody of medium or high affinity and desired specificity, for example as determined by alanine scanning mutangenesis, preferably residues located in a CDR loop which does not contribute to the specificity and off-rate of the antibody and are residues which are most likely to border the target ligand binding site for example as determined by well-known methods of three dimensional modeling or statistical plots of residue which are most variable or correspond to mutational hot-spots (these residues may also be residues in the neighbouring FRs, in any case optionally residues which are not conserved (and preferably therefore also do not positively contribute to the solubility, stability and expression characteristics of the reference antibody).
   ¹ optionally by at least 2, 3, 4, 5, 6, 7, 8, 9, 10, 11, 12, 13, 14, 15, 16, 17, 18, 19, 20, 21, 22, 23, 24, 25, 26, 27, 28, 29, 30-40, 40-50, 50-60, 60-70, 70-80, 80-90, or 90-100 (or more) fold. With respect to each of the foregoing at least 100 increments, the intrinsic off-rate of the targeting moiety is optionally at least 2, 3, 4, 5, 6, 7, 8, 9, 10, 11, 12, 13, 14, 15, 16, 17, 18, 19, 20, 21, 22, 23, 24, 25, 26, 27, 28, 29, 30-40, 40-50, 50-60, 60-70, 70-80, 80-90, or 90-100 (or more) fold less than the intrinsic off-rate of the effector moiety.
8. A multispecific ligand according to paragraph 7, wherein the intrinsic on-rate of the targeting moiety is selected so as to exceed the on-rate of the effector moiety.
9. A multispecific ligand according to paragraph 7, wherein the intrinsic on-rate of the effector moiety is selected so as to be less than the on-rate of the targeting moiety.
10. A multispecific ligand according to paragraph 9, wherein one or both of said effector and targeting moieties, comprises one or both of a VH or VL.
11. A multispecific ligand according to paragraph 9, wherein one or both of said effector and targeting moieties, consists essentially of one or both of a VH or VL.
12. A multispecific ligand according to any of the preceding paragraphs, wherein the first and/or second ligand is expressed predominantly on one or more cell populations of hemapoietic origin.
13. A multispecific ligand according to any of the preceding paragraphs, wherein the first and/or second ligand is expressed predominantly on endothelial cells.
13. A multispecific ligand according to any of the preceding paragraphs, wherein the first and/or second ligand is expressed primarily on a hemapoietic stem cell.
14. A multispecific ligand according to any of the preceding paragraphs, wherein the first and or second ligand is expressed predominantly on cells of hemapoietic origin.
15. A multispecific ligand according to any of the preceding paragraphs, wherein the first and/or second ligand is an ABC transporter protein, a ligand that is over-expressed on tumor cells, an adhesion molecule, a ligand that is expressed exclusively or predominantly on one or more types of leucocytes, one or more sub-populations of T cells or B cells, a cytokine receptor, a growth factor receptor, a molecule or complex comprising a transmembrane protein, or a CD molecule, other than CD59, a receptor is selected from the group consisting of tyrosine kinase type receptors, serine kinase type receptors, heterotrimeric G-protein coupled receptors, receptors bound to tyrosine kinase, TNF family receptors, notch family receptors, guanylate cyclase types, tyrosine phosphatase types, decoy receptors, inhibitory receptors and adhesion receptors (other than CD59) or any cell suface receptor-ligand cooperating with such a receptor.
16. A multispecific ligand according to any of the preceding paragraphs, wherein said second ligand binding moiety specifically recognizes said second ligand.
17. A multispecific ligand according to any of the preceding paragraphs, wherein the sum of bioavailable second ligands on said target cell population is less that the sum of bioavailable second ligands on cells other than said target cell population.
18. A multispecific ligand according to any of the preceding paragraphs, wherein the first ligand is preferentially bioavailable for targeting purposes on said target cell population.
19. A multispecific ligand according to paragraph any of the preceding paragraphs, wherein the first ligand is exclusively bioavailable for targeting purposes on said target cell population.
20. A multispecific ligand according to any of the preceding paragraphs, wherein the multispecific ligand is adapted to bind contemporaneously to said first and second ligands².
   ² This may be assessed for example by determining the extent to which the effector moiety out-competes a test ligand having the same specificity as the effector moiety but a somewhat higher on-rate and/or affinity in virtue of the superior (relative to the effector moiety and test ligand) affinity and /or on-rate of the targeting moiety. The effects of modifying the affinity of each of these three elements in either direction with suitable positive and negative controls may be tested to further refine this assessment. The choice of fist and second ligand can be also be influenced by the likelihood that both would be found within or unassociated with lipid rafts (in some cases this may be more important than others), as determined for example by the length of the transmembrane domain, if any, or in some cases whether the ligand is one which is anchored by GPI, whether the effector moiety is selected to agonize or antagonize a receptor and the functional sensitivity of the
21. A multispecific ligand according to paragraph 20, wherein the surface cell density of the first ligand on the target cell population exceeds the surface cell density of the second ligand on said target cell population.
22. A multispecific ligand according to paragraph 1, wherein the number of bioavailable second ligands in said mammal ligands is greater than the number of bioavailable first ligands and wherein the in-vivo site of action of said effector moiety is predominantly on the target cell population.
23. A multispecific ligand according to paragraph 1, 2, 3, 4 or 5, wherein the cell-surface density of the second ligand on the target cell population generally does not exceed the cell-surface density of the second ligand on a non-target cell population and where the in-vivo site of action of said effector moiety is predominantly on the target cell population.
24. A multispecific ligand according to any of the preceding paragraphs, wherein the first and second ligand binding moieties are small molecules³ that are conjugated to a fullerene or a peptide.
25. A multispecific ligand according to any of the preceding paragraphs, comprising a bispecific antibody in which the Fc portion of the molecule is absent or incapable of finding to an Fc receptor.
26. A multispecific ligand according to paragraph 10, 11 or 25, wherein at one or both of the targeting moiety and effector moieties comprises human sequences, and is non-chimeric or includes at least human FRs.
27. A pharmaceutical composition comprising a multispecific ligand according to any of the preceding paragraphs and a pharmaceutically acceptable carrier.
   cell to a less than complete saturation of the second ligand (as influenced by dose and any related MTDs), especially when antagonist effects are being exerted on a receptor (as opposed to agonist effects which may require less binding events depending on the sensitivity of the cell to such events) etc. Although such predictions may have some influence on the a priori choice of ligands for testing, ultimately empirical in vitro assessments may be necessary in most cases.
   ³ The term small molecule typically connotes small synthetic or semi-synthetic organic molecules which are not macromolecules such as proteins, lipoproteins, glycoproteins, long chain fatty acids etc.

The term "effector" is used to refer to the ability to effect a biological consequence through binding, for example effecting a signal transduction event by activating a receptor, or blocking the target ligand from associating with a complementary ligand, for example blocking a receptor from associating with a complementary ligand (eg. its natural ligand whether soluble or on another cell) and thereby, for example, preventing a signal transduction, or for example in the case of a decoy receptor preventing the biological consequence (eg. protective effect) associated with the function of such receptor, or blocking a ligand from associating with a complementary ligand eg. receptor on another entity eg. a cancer cell, infectious agent or immune cell.

The term targeting moiety means capable of distinguishing at least in part between the target entities (eg. cells according to one embodiment), which target entities are defined at least in part by a first ligand, typically found on the target cell population (and at least some entities eg. cells other than the target population of entities) that bear the second ligand acted on by the effector moiety.

A biased biodistribution is preferably accomplished by the multispecific ligand contemporaneously recognizing both ligands on the same entity eg cell, and may be accomplished by such contemporanous recognition occurring on adjacent entities or by increasing the propensity of the multispecfic ligand to locate in proximity to a target entity in virtue of the relatively high affinity targeting arm. The targeting arm may itself be an effector.

In another embodiment, the biological consequence accomplished by the effector arm is at least minimally retargeting, for example wherein the lesser affinity or functional affinity of the first binding moiety is selected to permit the multispecific ligand to preferentially bind to an adjacent entity, for example, a circulating entity which circulates in proximity to a lymphatic endothelial cell to which the multifunctional ligand is bound with lesser affinity. Again, the relatively high affinity first binding moiety may itself be an effector.

In another embodiment the biological consequence accomplished by the effector arm is minimally cooperative targeting, for example where the biodistributions of at least one of the first and second ligands extends to a diverse population of cells other than target cell population and where binding is only possible or consequential if both ligands are available for contemporaneous binding, in this case due to the affinities of the first and second ligand binding being individually insufficient for effective targeting (eg. insufficient for other than ephemeral binding). In the context of this embodiment of the invention, the "cooperative targeting" is not simply ameliorated by the effector arm, it is predicated and reliant on this arm. One or both ligand binding moieties may exert additional effector properties.

It will also be appreciated that any multispecific ligand of the invention or any component thereof may be fused or conjugated to a separate effector as exemplified below, including toxins, cytokines, adhesion molecules etc.

The ligand binding moiety is preferably an antibody or a sequence or sequences of amino acids etc.which are the natural ligand for the target ligand, for example where the ligand is a cytokine or lymphokine receptor, such as IL-2 receptor, the ligand binding moiety may comprise a sequence of amino acids which is IL-2. The ligand binding moiety may also be a mutated or a newly developed form of the natural ligand (eg. developed through combinatorial libraries) or a natural or synthetic chemical ligand (developed through combinatorial chemistry).

In one aspect, the invention contemplates a composition containing a multispecific ligand containing at least a first ligand binding moiety and a second ligand binding moiety, the first ligand binding moiety specifically binding with a pre-selected first affinity to at least a first ligand, having a first biodistribution and the second ligand binding moiety specifically binding with a pre-selected affinity to at least a second ligand with a second biodistribution and wherein the affinity of first and second ligand binding moieties are selected to bias the biological site of biologic activity of the multispecific ligand; and wherein the first ligand binding moiety preferably binds with high affinity (preferably nanamolar affinity or greater) to a specific cell associated marker (e.g. a CD marker, a marker associated with diseased cells, a a marker associated cells in a particular physiological state (e.g. activated T cells, B cells) etc. (such markers may be associated with a particular class of cell or a subclass thereof (if applicable) or particular subpopulation within the subclass (if applicable), however classified, such as epithelial cells, endothelial cells, immune cells (lymphocytes, memory cells, effector cells) monocytes, Tcells (CD4+, CD8+, CD45RO+), hepatocytes, stem cells, etc.(expand) and wherein said second ligand binding binding moiety binds with relatively low, or medium affinity (preferably 0.1 micromolar or less) to a receptor (eg. chemokine, growth factor, cytokine) involved in cell signaling or a decoy receptor, a cell surface receptor ligand eg. the ligand for such receptor which effects a signal or inhibits a signal (eg. CTLA4), a ligand involved in cell adhesion, a receptor or channel (ion channel) for a molecule involved in cell regulation or homeostasis etc.

The invention contemplates that the difference in affinity will in most cases be an essential element in biasing the location of action of the multispecific ligand to yield an acceptable or desired safety profile and that the high affinity of the first ligand binding moiety for the cell associated marker will be optimized for this purpose insofar as the safety profile of the multispecific ligand dictates maximizing its affinity characteristics. The invention also recognizes that choosing the relatively lower affinity of the second ligand binding moiety may assist in this regard up to a point where its effectiveness to bind to the second ligand is significantly comprimised. In this regard, the invention also contemplates that factors other than the choice of affinityof the first and second ligand binding moieties (and of course the avidity effect resulting from having two ligands on the target cell and only one on the non-target cell) may be taken into consideration or optimized to balance the safety and effectiveness profiles of the multispecific ligand, especially if such careful balance is required.

Examples of such factors, one or more of which can be employed in various combinations, are described hereafter:
1) the selection of cell associated marker, in terms of its cell surface density relative to cell density of the second ligand. The number of first and second ligand can readily be assessed by radiolabelling studies or approximated by flow cytometric methods relative to a standard.The selection of the cell surface marker in this respect will depend on the function of the relatively low affinity binding moiety (whether it causes a signal transduction (directly or indirectly eg. through binding to a receptor (where less emphasis on relative cell density may be warranted)(agonist antibodies are well known in the art and include those described in US 6,342,220, US 6,331,302, US 5,635,177, US 6,099,841 see also Cancer Res 2001 Mar 1;61(5):1846-8 and can be made according to routine screening techniques, especially using antibodies capable of cross-linking receptor components (see references below) including antibodies in which the VH and VL are capable of binding individually to different receptor components), binding to a decoy receptor, binding to an inhibitory receptor etc.) or prevents a signal transduction (directly or indirectly, e.g. binding to a receptor, binding to receptor ligand) and in the final analysis how many binding events per cell are required to cause or prevent the sought-after biological effect. This can be assayed in vitro through well known assay methods established in the art for measuring responses to external stimuli such as cytokines, chemokines, growth factors, colony stimulating factors using various immunostaining techniques including flow cytometry (eg. to measure apoptosis (e.g. annexin V binding assay) signal transduction (e.g. using phosphospecific antibodies that detect phosphorylation of serine, tyrosine, threonine), differential gene expression etc.) depending on the type of effect that is being measured (see for example Biosource Method Booklets at http://www.biosource.com/content/techCornerContent/methodPDFs/index.asp; see also Amersham Bioscience catalogues, and those of other well known suppliers etc.) or via animal studies. For example, some growth factors, lymphokines or molecules/ions required for homeostasis are in more delicate balance and can more easily disrupted. IL-2 depletion will cause apoptosis of activated T cells, which can be measured. For example, it may also be necessary or desirable for the cell specific marker to approximate (preferably the the number of cell specific markers on the target cell population is no less in number than 50% more preferably no less than 90% in number relative to the second ligand - as stated above, which will depend on what degree of causation or prevention of the signaling/interaction will cause the desired biologic effect) or preferably out-number (by greater than 50%, preferably by greater that 100% (greater than two fold), preferably by greater that 200% (greater than 3 fold), by greater than 300% (greater than 4 fold)) the target ligand for the relatively low affinity binding moiety especially, for example, if the goal is to block interaction of a receptor with a high affinity ligand that exerts a biological effect in low concentration (eg. a cytokine).
2) Furthermore, in the latter case the affinity of the first binding moiety will preferably be selected to approximate (preferably no less than one order of magnitude, more preferably no less than 5 fold less, more preferably no less three fold less, more preferably no less two fold less, more preferably no less one fold (100%) less), and will preferably equal or exceed the affinity of the natural ligand.
3) Furthermore, the concentration (in virtue of the choice of administered dose) of the multispecific ligand in the target cell microenvironment may also be selected to exceed that of the natural ligand (MTD permiting).
4) the choice of construct will maximize the steric blocking of the target (IgG or F(ab')² vs diabody). Furthermore, in some mammalian systems (eg. mice) the hinge region is naturally longer and this effect can be mimicked for human antibodies through a hinge extension on the N-terminal side of the hinge region using well known neutral linkers (gly4ser) or a repeat of all or a portion of the natural hinge sequence. This extension will also permit a greater span between first and second ligands to be bridged.
5) the choice of construct will include an Fc portion or partial Fc portion (eg. CH2 or minibody-CH3) or weighted Fc eg. by pegylation (site specific pegylation is well known in the art) or IgG subtype naturally having additional Fc domains (e.g. an IgE) (which Fc if it includes the CH3 is preferably mutated to preclude its binding and/or increase its half life as is known in the art see USP 6,121, 022) so as to maximize the shear effects on the multispecific ligand which will be most consequential in the case of univalent binding in order to minimize the duration of such binding (maximun shear force is also preferred where there is an excess in the total number of bioavailable targets of the second ligand binding moiety relative to the total number of bioavailable targets of first ligand binding moiety(greater number of cells and/or greater number of targets per cell and/or increased bioavallablity of such targets eg. on normal cells relative to cancer cells).
6) Optionally, the multispecific ligand will include a 3^{rd} binding moiety which binds to and neutralizes the natural ligand for the receptor sought to be blocked. Such formats are well known in the art (see for example particularly Schoonjans R et al. A new model for intermediate molecular weight recombinant bispecific and trispecific antibodies by efficient heterodimerization of single chain variable domains through fusion to a Fab-chain. Biomol Eng. 2001 Jun;17(6):193-202. Schoonjans R et al. Fab chains as an efficient heterodimerization scaffold for the production of recombinant bispecific and trispecific antibody derivatives.J Immunol. 2000 Dec 15;165(12):7050-7. Schoonjans R, et al. Efficient heterodimerization of recombinant bi- and trispecific antibodies. Bioseparation. 2000;9(3):179-83. see also French RR. Production of bispecific and trispecific F(ab)2 and F(ab)3 antibody derivatives. Methods Mol Biol. 1998;80:121-34; US Patent Application No. 20020004587; Kortt AA, Dimeric and trimeric antibodies: high avidity scFvs for cancer targeting. Biomol Eng. 2001 Oct 15;18(3):95-108).
7) Optionally, two multispecific ligands each binding to different cell specific makers and each having a second ligand binding moiety which binds to the same second ligand eg. a receptor, optionally to a different polypeptide/component of the receptor, may be employed to achieve the desired biologic effect. One or both may also be trispecific as discussed above. According to another embodiment the multispecifc ligand binds is used to protect a first target cell population in virtue of its high affinity (and/or faster on-rate) first ligand binding moiety from the effects of a therapeutic entity which desirably binds to a second target cell population via the second ligand but also undesirably binds to the first target cell population. Therefore the second ligand binding moiety can be used to selectively block the binding of the therapeutic entity e.g. an interleukin, interferon, immunotoxin, etc. to the first target cell population in virtue of the relatively low affinity second ligand binding moiety. In this case, the multispecfic ligand may also comprise a third ligand binding moiety which binds to the therapeutic entity, particularly where the multispecific ligand is first administered first, optionally an anti-idiotypic binding moiety component where the therapeutic comprises an antibody component. Optionally, the multispecific ligand may also comprise an enzyme cleavage site, wherein the cleavage is effected at the second target cell population in virtue of the particular enzyme constitution in the vicinity of that target cell poplulation (e.g. matrix metalloproteases within a tumor) and results in the inability of the multispecific ligand to block the therapeutic entity from binding to the second target cell population eg. in the case of an antibody the cleavage is in a CDR3 or FR to as to interfere with binding capacity. Such enzyme cleavage sites are well known to those skilled in the art.

Some sample targets are listed immediately below, while others are listed later. Greater targeting using a high affinity (and/or faster on-rate) first ligand binding moiety which binds to a cell associated or specific marker may be imparted to a variety of existing antibodies with suitably diminished affinity including those marketed or in clinical trials or listed below which are the subject of the patent and scientific literature, including those listed in PharmaBusiness June 2002 No.51:

| Category | Functional ligand for low affinity arm (location of ligand) | Cell localizing ligand for high affinity arm | Mode of action | Comments |
|---|---|---|---|---|
| Growth factor blockade | IL-2. (soluble) | CD4 T cells | Growth factor blockade for a specifically for CD4 T cell subset; i.e. selective Immunosuppression | IL-2 required for naïve CD4 or CD8 and memory CD4 T cell responses;. Binding of CD4 or CD8 would also block interaction with antigen presenting cells. |
| | IL-15 | CD8 T cells | Growth factor blockade for a specifically for CD8 T cell subset; i.e. selective immunosuppression | IL-15 needed for memory CD8⁺ T cell responses |
| Chemokine blockade | MCP-1 (soluble) | CD11c (monocyte/macrop hage) | Monocyte/macrophage chemokines; e.g. antl-inflammatory agent | At time of percutaneous coronary intervention (PCl) to limit restenosis; arthritis |
| Cell activation blockade | Thrombin (soluble) | CD31 (endothelial cells) or P-selectin (thrombin activated endothelial cells) | Prevent thrombin binding to thrombin receptors on leukocytes or endothelial cells at endothelial surface | Limit thrombosis and endothelial activation e.g at time of PCT |
| Cell activation blockade | CD80/86 (dendritic antigen presenting cells) | CD83 (dendritic cells) | Block interaction with CD28; immunosuppression | At time of allografting to induce tolerance |
| Inhibitory receptor activation | Fc gamma RII (Mast cells) | Fc epsilon RI (Mast cells) | Enhance association of activating and inhibitory receptors | Rx of acute of allergic disease. Advantages over bispecific Fc fusion reagents because of more specific cell targeting . See Zhu D et al.Nat Med 2002 May;8(5):518-21) |
| inhibitory receptor blockade | CTLA-4 | CD8 | Block CD80/86 interaction with CTLA-4 | Enhance specifically CTL mediated anti-tumor responses without global T cell activation. Anti-CTLA4 abs now in therapeutic trials to enhance tumor immunity |
| Adhesion molecule blockade | VCAM-1 (activated endothelial cell) | CD31 (endothelial cells) or E-selectin (activated endothelium) | Block VLA-4-dependent T cell and monocyte adhesion to VCAM-1 on endothelial cells. | Acute Rx of MS flare-up. Anti-VLA-4 is in trial as Rx for MS. |
| Adhesion molecule blockade | ICAM-1 (activated endothelial cell) | CD31 (endothelial cells) or E-selectin (activated endothelium | Block neutrophil adhesion to activated | Acute Rx to reduce reperfusion injury (myocardial infarction, bowel ischemia/surgery) |

| Category | Functional ligand for low affinity arm (location of ligand) | Cell localizing ligand for high affinity arm | Mode of action | Possible therapeutic uses |
|---|---|---|---|---|
| Cell death ligand blockade | CD95L (Fas-ligand) (T cell) | CD25 (activated T cells) | Block activation induced cell death of T cells; enhance anti-tumor immunity | Both ligands on same cell; CD95L expressed on activated not resting T cells |
| Protect from another therapeutic: e.g. immunotoxins | IFN-_ (soluble) | CD31 | Block IFN-_ toxicity towards endothelial cells during IL-2 therapy for tumors | Cell selective block of IFN_ effects; |
| Protect from another therapeutic: | IFN-_R (endothelial cell) | CD31 (endothelial cell) | Block IFN-_ toxicity towards endothelial cells during IL-2 therapy for tumors | Both ligands on same cell. Cell selective block of IFN_ effects; |
| Inhibitory receptor blockade | TGF-_ | CD4 or CD8 | Block immunosuppressive effects of tumors; enhance anti-tumor immunity | TGF- _ mode of immunosuppression not clear |
| Cell type specific activation | CD3 | CD45RO (memory/effector T cells) | Trivalent ab so CD3 can be cross-linked | Anti-tumor Rx; selectively enhance memory T cells; reduce nonspecific activation of irrelvent T cells |
| Growth factor blockade | IL-15 | CD8 T cells | Growth factor blockade for specifically for CD8 T cell subset i.e. selective immunosuppression | For Rx of allograft rejection; T cell subset specific suppression will limit infectious complications or unintended inhibition of CD4⁺ regulatory T cells |
| Cell activation blockade | CD80/86 (dendritic antigen presenting cells) | CD83 (dendritic cells) | Block interaction with CD28; immunosuppression | At time of allografting to induce tolerance. Targeting to dendritic cells will enhance efficiency/ |
| Adhesion molecule blockade | VCAM-1 (activated endothelial cell | CD31 (endothelial cells) or E-selectin (activated endothelium) | Block VLA-4-dependent T cell and monocyte adhesion to VCAM-1 on endothelial cells.. | Acute Rx of MS flare-up. Anti-VLA-4 is in trial as Rx for MS. |
| Cell death ligand blockade | CD95L (Fas-ligand) (T cell) | CD25 (activated T cells) | Block activation induced cell death of T cells; | Enhance anti-tumor immunity |
| Protect from another therapeutic: | IFN-_R (endothelial cell) | CD31 (endothelial cell) | Cell selective block of IFN_ | Block IFN-_ toxicity towards endothelial cells during IL-2 therapy for tumors without impairing other useful IFN-_ effects |
| Inhibitory receptor activation | Fc gamma RII (Mast cells) | Fc epsilon RI (Mast cells) | Enhance association of activating and inhibitory receptors. | Rx of acute of allergic disease. Advantages over bispecific Fc fusion reagents because of more specific cell targeting . See Zhu D et al.Nat Med 2002 May;8(5):518-21) |

The invention also contemplates that FAS can be selectively blocked on various different types of cells such as pancreas beta cells using markers such as GAD65, IA-2, IA2-B, ICA-12. Type 1 Diabetes is characterized by the destruction of insulin producing Beta Cells in the pancreas. One method in which Beta cells are destroyed is thought to be through apoptosis mediated by CD95 receptors on Beta cells. CD-95 seems to be upregulated in Beta cells of those with Type 1 diabetes (see Ann N Y Acad Sci 2002 Apr 958 297-304; J Clin Immunol 2001 Jan;21 (1):15-8). Similarly, using Tg, TPO ligands as cell associated markers CD95, TRAILR1, TRAILR2 can be blocked on thyroid cells. Hashimoto's Thyroiditis (HT) is characterized by the destruction of thyroid hormone producing cells and therefore hypothyroidism. It has been observed that some of this cell destruction is due to apoptosis. The CD95 receptor which is responsible for apoptosis is up regulated in thyroids affected by HT. Blocking the CD95 receptor by the RLAA may reduce the amount of apoptosis. The HAA can target either Tg or TPO which are unique to thyroid tissue.¹ There are also 2 other receptors suspected to be involved with apoptosis in thyroid cells: TRAILR1 and TRAILR2 (see Nat Rev Immunol 2002 Mar;2(3):195-204)

Fas can also be selectively activated on distinct subsets of disease mediating immune cells associated with autoimmune and inflammatory disorders such as activated T cells, regulatory T cells, CD4+ cells, CD8+ cells etc.

Among other embodiments hereinafter enumerated, the invention is also directed to multifunctional ligands which comprise antibodies which recognize cell type specific markers (hereinbefore or hereinafter exemplified), including those available commercially or published in the art, in any combination with those antibodies (hereinbefore or hereinafter exemplified, including those available commercially or published in the art) that recognize ligands on a broader population of cells including the cell population bearing the cell type specific marker, wherein the instrinsic affinity difference is maintained or adjusted to one, two, three, four, five, six or seven orders of magnitude. The invention contemplates that affinity differences (increases or decreases in affinity) can be readily generated by modifying amino acids that are expected a piori to result in changes in affinity (see for example Chowdhury P. et al. Improving antibody affinity by mimicking somatic hypermutation in vitro (1999) Nature Biotechnology Jun; 17 p.568-572), or by a variety of other well-known high throughput methods that can readily be applied to this task, for example by light chain shuffling CDR grafting, parsimonious mutagenesis, shotgun scanning mutagenesis etc. Methods of affinity maturation are well kwown in the art. The invention also contemplates that two hybrisoma derived antibodies can be digested eg. with pepsin to create F(ab')2 and can be chemically recombined to create bispecific antibodies. Hybridoma fusion technology can also be used to create to tetromas for this purposes. In this regard, the invention contemplates that hybridoma derived IgG antibodies can be used for the relatively high affinity cell targeting moiety and that hybridoma derived IgMs can be used for the relatively low affinity binding arm. The invention also contemplates that the repective high and low affinity arms can be conjugated, fused etcaccording to well known methods to respective complementary ligands such as fas/jun strepavidin/biotin (making it possible to pre-target the high affinity arm independently, particularly where the affinity of the complementary ligands for each other is greater than that of the second ligand binding moiety for its target ligand.

According to one embodiment of the invention, the first ligand binding moiety can bind to a cell specific marker and and said second ligand binding moiety binds to the extrracellular portion of a ligand involved involved in membrane transport across the cell membrane, for example an ion channel, vitamin recepetor etc. to inhibit uptake or export. For example the first ligand binding moiety may bind to a cancer cell specific marker and the second ligand binsing moiety can bind to p-glycoprotein. High affinity tumor specific antibodies are well known and anti-PGP antibodies are well known (see for example 6,479,639 Monoclonal antibody to a human MDR1 multidrug resistance gene product and uses 6,365,357 Methods and reagents for preparing and using immunological agents specific for P-glycoprotein 6,171,786 Methods for preventing multidrug resistance in cancer cells 6,030,796 Monoclonal antibody to a human MDR1 multidrug resistance gene product, and uses 5,994,088 Methods and reagents for preparing and using immunological agents specific for P-glycoprotein 5,972,598 Methods for preventing multidrug resistance in cancer cells 5,773,280 Monoclonal antibody to a human MDR1 multidrug resistance gene product, and uses 5,434,075 Monoclonal antibody to a human MDR1 multidrug resistance gene product, and uses; Chen Y et al. J Cell Biol Mar 6; 148(5):863-70; Mickisch GH et al. Cancer Res. 1992 52: 3768-3775 see also Cianfriglia M, Cenciarelli C, Barca S, Tombesi M, Flego M, Dupuis ML. Monoclonal antibodies as a tool for structure-function studies of theMDR1-P-glycoprotein.Curr Protein Pept Sci. 2002 Oct;3(5):513-30. Tang Y, Beuerlein G, Pecht G, Chilton T, Huse WD, Watkins JD. Use of a peptide mimotope to guide the humanization of MRK-16, ananti-P-glycoprotein monoclonal antibody.J Biol Chem. 1999 Sep 24;274(39):27371-8. Zhou Y, Gottesman MM, Pastan I. The extracellular loop between TM5 and TM6 of P-glycoprotein is required forreactivity with monoclonal antibody UIC2.Arch Biochem Biophys. 1999 Jul 1;367(1):74-80. Romagnoli G, Poloni F, Flego M, Moretti F, Di Modugno F, Chersi A, FalascaG, Signoretti C, Castagna M, Cianfriglia M. Epitope mapping of the monoclonal antibody MM12.10 to external MDR1P-glycoprotein domain by synthetic peptide scanning and phage displaytechnologies.Biol Chem. 1999 May;380(5):553-9.Vasudevan S, Tsuruo T, Rose DR. Mode of binding of anti-P-glycoprotein antibody MRK-16 to its antigen. Acrystallographic and molecular modeling study.J Biol Chem. 1998 Sep 25;273(39):25413-9. Naito M, Tsuruo T. Monoclonal antibodies to P-glycoprotein: preparation and applications to basicand clinical research.Methods Enzymol. 1998;292:258-65. Loo TW, Clarke DM. The minimum functional unit of human P-glycoprotein appears to be a monomer.J Biol Chem. 1996 Nov 1;271(44):27488-92.). Some anti-PGP antibodies function by by interfering with ATP utilization.

It will also be appreciated that a cell specific marker need not differentiate between sub-populations of cells that do not express the second target ligand eventhough those cells are not the targets of action of the multispecific ligand. Examples of bispecific antibody technologies are described in Appendix C.

Examples of antibodies that bind to cell specific ligands, receptors, etc. are abundant and well known in the art (see for example Biosource International 2002 Research Products Catalog e.g. pages 178-193, Upstate Cell Signalling Solutions 2002 Catalog and other catalogs of well known to those skilled in the art)

Some examples are described in our co-pending application No. 2,402,930 filed September 19, 2002 and others are mentioned or referenced throughout the disclosure. See also Appendix D

According to one embodiment of a multispecific ligand of the invention, a first ligand binding moiety which recognizes a cell specific marker on a non-diseased cell is combined with a second ligand binding moiety which binds with a lower intrinsic affinity and/or slower on-rate to a ligand which is the target of a cytotoxic molecule eg. a radiolabelled cytokine, cytokine-toxin fusion protein eg. IL-2-diptheria toxin or PE fusion, an immunotoxin etc. The underlying principle of the invention is that such a strategy can be used to protect non-target cells while minimally protecting target cells which don't express the target of the first ligand binding moiety. Following this conceptual approach the invention is more generally directed to first ligands which protect non-diseased cells bearing a corresponding target ligand from the effects of a second cytotoxic ligand (e.g an immunotoxin, cytokine-toxin fusion protein, radionuclide bearing ligand, etc.) having the same target specificity and which targets diseased cells through the instrumentality of the target ligand eg. a cancer associated marker e.g Lewis Y antigen, EPCAM, MK-1 antigen. In this case the first ligand which is intended to bind preferentially to non-diseased cells will be selected to have one or more and preferably all of the following characteristics:
1) as suggested above, a cell-specific binding moiety of higher affinity and/or on-rate which targets the non-diseased cells (e.g. through an epithelial cell marker which is expressed on normal epithelial cells);
2) increased molecular weight, for example an IgG, minibody or other format that does not as readily penetrate solid tumors;
3) through the introduction of enzyme cleavage sites which render the ligand unable to bind to the diseased cells in virtue of specific enzymes differentially expressed in the microenvironment of the diseased cells which can degrade the first ligand, for example in the case of a solid tumors, introducing a cleavage site for a matrix metalloprotease in a portion of the ligand that is essential for maintaining the binding properties of the ligand eg. the portion of the polypeptide that binds to the cell surface ligand eg. a CDR3 or a more remote location responsible for the ligand's integrity (with respect to such cleavage sites see discussion for example in US patent application 20020103133, WO02/060488, WO02/072620, WO 96/05863, USP 5,962,216, WO01/95943, WO02/00263, WO01/68145, WO01/36003, Dubois V, et al. CPI-0004Na, a new extracellularly tumor-activated prodrug of doxorubicin: invivo toxicity, activity, and tissue distribution confirm tumor cell selectivity.Cancer Res. 2002 Apr 15;62(8):2327-31.Fernandez AM, et al. N-Succinyl-(beta-alanyl-L-leucyl-L-alanyl-L-leucyl) doxorubicin: anextracellularly tumor-activated prodrug devoid of intravenous acute toxicity.J Med Chem. 2001 Oct 25;44(22):3750-3.

Denny WA. Prodrug strategies in cancer therapy.Eur J Med Chem. 2001 Jul-Aug;36(7-8):577-95. Trouet A, et al. Extracellularly tumor-activated prodrugs for the selective chemotherapy of cancer: application to doxorubicin and preliminary in vitro and in vivo studies. Cancer Res. 2001 Apr 1;61(7):2843-6.Chari RV. Targeted delivery of chemotherapeutics: tumor-activated prodrug therapy. Adv Drug Deliv Rev. 1998 Apr 6;31(1-2):89-104).

### Definitions

The term "associated" in relation to markers that are dominantly distributed on one or more particular entities is used to mean exclusively expressed, primarily expressed, or over-expressed to advantage from a targeting standpoint.

The term "receptor ligand" means a target ligand which is a ligand for a receptor, for example, a receptor on a cell or infectious agent or a receptor which circulates independently of another entity.

The term affinity is contrasted to functional affinity which may result from avidity.

The term epitope though technically understood to be specific for a given antibody, is used in a preferred embodiments to refer to antigenic determinants that are situated proximally to one another so that two antibodies will be considered to bind to the same epitope if one competively inhibits the binding of the other through any probative competitive inhibition experiment known to those skilled in the art.
The invention contemplates that two antibodies with the same epitope specificity may have substantially the same amino acid composition ie. with possible exception of one or more additions, deletions or substitutions including conservative amino acid substitutions which do not substantially affect the specificity and amino acid composition of the paratope

The term approximately in the context of orders of magnitude variations in affinity refers a variability that is up to a half an order or magnitude.

Without limiting the scope of the claims it is generally understood that biodistribution of a multispecific ligand in contrast to that of a ligand will be predicated on the bioavailability of its target ligand.

The term "overlap" and related terms connote that notwithstanding the difference in distributions of the first and second ligands the first and second ligands are bioavailable for recognition on the same entity. This term and related terms, exemplified below, are intended to exclude a situation where both ligands are preferentially expressed on substantially the same entity, for example two different tumor associated antigens associated differentially with a differentiated population of cells within a tumor , most particularly in the case where they are individually suitable targets for delivery of a toxic payload. Thu sthe terms "different" in regard to biodistributions and "heterogeneous" and "diverse" in reference to populations of entities are similarly understood to exclude such a common distribution, in the appreciation that the invention primarily represents an improved strategy for targeting two different ligands, in which one ligand has a broader distribution than the other or both have distributions that may overlap but are different from that of the target population. It will also be appreciated that the invention has particular application to a situation in which at least one of the non-target populations is one on which one of said first and second ligands is substantially represented (in contrast to one on which it simply enjoys limited expression).

The term "receptor ligand" means a target ligand which is a ligand for a receptor, for example, a receptor on a cell or infectious agent or a receptor which circulates independently of another entity.

The term "antigen binding fragment" refers to a polypeptide or a plurality of associated polypeptides comprising one or more portions of an antibody including at least one VH or VL or a functional fragment thereof.

A moiety that exerts a biologic function is understood to be a "biologic effector" in the sense that its intended interaction with an entity in the lymphatic system or elsewhere in the organism has a biological consequence.

The tern neutralizing in regard to an an immune function is used broadly to refer to any interposition, interference or impediment which affects the function of the target entity

The terms modulating, mediating, neutralizing function etc. are not intended to be mutully exclusive and are each used broadly, for example, without limiting the generality of the scope accorder herein or by those skilled in the art the term modulating preferably refers to effecting a change, and the term mediating preferably connotes an indirect effect achieved through the instrumentality of another entity, for example a cell, cytokine, chemokine etc..

The term "preferentially binds" recognizes that a given ligand binding moiety might have some non-defeating cross-reactivities.

The term biologic effector ligands is used to refer to any ligand for which there is a complementary target ligand on a target entity, and wherein binding of the biologic effector ligand to the target ligand exerts a biologic effect. For example the target ligand is typically a receptor and the biologic effector ligand may be any complementary ligand such as a cytokine, chemokine, hormone, colony stimulating factor, growth factor, receptor inhibitor, agonistor antagonist, which binds to the receptor with resulting biologic effect.

The term "pre-selected" in reference to the affinity of ligand binding moiety refers to any selection or choice of differential or cooperative affinities relative to a second ligand binding moiety which is generated as a result of a mental or physical process or both, preferably through a process of prediction or post-facto validation of the effects of the choice of the first and second affinities and/or more preferably through an empirical evaluation of different choices for at least one of the first and second affinities, and preferably both.

The term multi means at least two and the term ligand is used broadly to refer to any entity or part thereof which can participate in an intermolecular interaction that can result in specific binding of suitable affinity for the interaction in question.

The term entity includes without limitation any molecule including without limitation, antibodies, complex or association of molecules, drugs, drug carriers (eg.vesicles eg. liposomes, nanoparticles,etc.) or any cell as well as any infectious agent or parasite (including, without limitation, spores, viruses, baceria, fungi) as well as any other immune or therapeutic target.

The term "low affinity" means an affinity of approximately (this term is defined herein) 10 ⁻⁴ molar to micromolar affinity, preferably (subject to safety considerations), approximately, 10⁻⁵ molar affinity, more preferably (subject to safety considerations), approximately micromolar affinity, the term "medium affinity" means approximately 10⁻⁷ to nanomolar affinity, preferably approximately 10⁻⁸ molar affinity, more preferably approximately nanomolar affinity, and the term "high affinity" means approximately 10⁻¹⁰ affinity or greater. Thus is one embodiment the invention contemplates that the multispecific ligand comprises a "target-ligand" binding moiety which binds with low or medium affinity to a target ligand present on a diverse population of cells (preferably this moiety is an effector moiety ie. one which exerts a biological effect attributable to its binding eg. blocking or activating a receptor or blocking a cell membrane channel) and a "targeting", ligand binding moiety, which binds with medium or high affinity to a ligand associated with a sub-population of those cells so as to bias the biodistribution of the multifunctional ligand in favor of said sub-population. Preferably the multispecific ligand is adapted to be bound contemporaneously to the same cell. In another embodiment the first and second ligands binding moieties each bind to ligands present on diverse overlapping populations of entities eg. cells (ie. neither ligand being preferentially associated with a target cell population) and are adapted to be bound contemporaneously and to both bind individually with low affinity, so as to bias the distribution of the multispecific ligand to the population of cells bearing both ligands.

As discussed elsewhere the term approximately, in reference to "order of magnitude" increments in affinity, refers to up to a half order of magnitude in affinity.

According to another embodiment, the invention is directed to an antibody termed a "coybody". A "coybody" is an antibody in which the on-rate contribution to the affinity of the antibody is proportionally less than the off-rate contribution relative to a reference antibody of the same specificity and a greater affinity of up to several orders of magnitude; preferably a reference antibody of approximately one to three orders of magnitude greater instrinsic affinity, preferably a reference antibody of medium affinity or preferably high affinity, optionally a reference antibody with an optimally high on-rate, for example in which the intrinsic affinity or intrinsic on-rate has been optimized after an antibody of the desired specicity has been generated. Optionally, the reduction in on-rate is brought about by modifying amino acids that are known or engineered to so as contribute minimally to the specificity and slow off-rate of the antibody. Typically, the reference antibody may be derived from a hybridoma or from a phage, yeast bacerial or ribosome display library. As discussed above the reference antibody optionally comprises cooperating light and heavy variable regions in which 2 of 6, 3 of 6, 4 of 6 or 5 of 6 of the CDRs, optionally CDRs of both the VH and VL, including preferably at least one CDR3, optionally that of the heavy chain, are collectively made or selected to be primarily or exclusively responsible for the binding affinity of the coybody, such that alterations in the length (e.g. by additions of one or more amino acids) or amino acid composition, optionally by a parsimonious mutagenesis procedure (e.g. where the library is optionally partially biased in favour of the parental amino acid composition to introduce change more incrementally) of one or more other non-contributing or minimally contributing CDRs can be leveraged to diminish the on-rate, for example due to steric and/or electrostatic hindrance. For example, combinatorial libraries in which one more amino acids for example amino acids corresponding to locations of the greatest variability in residue identity for example by a Kabat -Wu plot (see Cellular and Molecular Immunology 4^{th} Ed. ISBN 0-7216-8233-2 page 49) or greatest probability of contact with the target ligand as determined by three dimensional modeling, alanine scanning or high throughput mutagenesis may be randomized or partially randomized in the one or both of the CDR1s and CDR2s or in one CDR1 and one CDR2 to permit selection of an antibody with a reduced intrinsic on-rate. In one embodiment, such contact or potentially affinity contributing amino acids may have been deleted or modified to residues with reduced capacity for intermolecular interaction in the initial selection of the relatively high affinity reference antibody or in a later step after generation of the parental template with the desired specificity but prior to a subsequent affinity maturation step to generate the reference antibody. This approach among others may be used to set up a scenario in which select portions of one or more CDRs are modified to organize that they contribute minimally to the specificity and affinity (slow off-rate) of the antibody. In one embodiment, in the case of a single domain antibody at least one and preferably two of the CDRs are primarily or exclusively responsible for the binding. In one embodiment the on-rate is reduced by a factor of 2 to 100x. In one embodiment the coybody binds to a ligand which is over-expressed on a target population of entities (eg. cells) relative to a non-target population of entities such that the biodistribution of the coybody to the non-target population (and target population) is diminished in a given increment of time following administration. This targeting strategy is understandably adapted to situations where the resulting delay in biodistribution is preferable for diminished toxicity attributable to reduced non-target entity binding in a given unit of time especially where the effectiveness threshold in that same amount of time is not significantly if at all compromised or is preferable due to a sustained release effect (for example using a larger antibody format that is not readily cleared). Examples of suitable targets for cobodies per se include hormones or other secreted growth regulatory factor, differentiation factor, or intercellular mediators (e.g., cytokine) such as TNF alpha, FAS L, IL-8, IL-6, IL-5, VEGFs, fibroblast growth factors. Numerous examples of antibodies which recognize such targets are described in patents classified in US Class 424/145.1 (see Appendix A). Methods of prolonging the half-life of antibodies, producing bispecifics, scfvs and dsFvsand altering Fc effector function are well known and noteworthy references include US 6,277,375, US 5,869,046, US 5,624,821, US 6096871, US 4,479,895, US 6207804, US 5,681,566, US5,864,019, US5,869,620, US6,025,165; US 6,027,725; US 6,239,259; US 6121424; WO00/09560; US 6,420,140.

As discussed below, advantages accrue when this antibody is coupled to a higher affinity antibody (in the form of a multifunctional ligand) which binds to a different ligand associated with the target population. The invention contemplates that coybodies have multiple independent applications, including tempering the effects through antibody mediated neutralization of an over-production or sensivity to biologic effector ligands (eg. cytokines eg. TNFₐₗₚₕₐ, chemokines eg. IL-16 (crohns disease) etc. which are over-produced and/or mediate or aggravate eg. a chronic medical condition (which for example is not an acute phase) by binding to such ligands, over a prolonged periods, preferably using larger antibody formats which are not readily cleared, especially where such tempering has side effects which are better spread over time and/or where effectiveness is not a limiting factor and/or where a second therapeutic with different non-cumulative side-effects shares the therapeutic burden and/or where a the same antibody with a higher on-rate is used in combination.

Accordingly, in one embodiment the affinity of the first ligand binding moiety is comparable (equal to or no greater than one order of magnitude less that than, preferably no greater than five times less, preferably no greater than four times less, preferably no greater than three fold less, preferably no greater than two fold less etc.) or preferably greater than the affinity of the second ligand binding moiety and the on-rate of the first ligand binding moiety is greater than that of the second ligand binding moiety. Testing and selection of binders with high on-rates via BIACORE or other methods of on-rate measurement are well known in the art (see particularly WO 02/36615, US 2002/098189, US 2002/164326 and WO 01/64751; see also WO 01/55217 and WO 01/27160). It will be appreciated that best safety/effectivenss profile will be achieved by selecting the relative on-rates and/or off-rates of both binding moieties. For example: in one embodiment the on-rate of the first ligand binding moiety is five to 500 fold greater that the on-rate of the second ligand binding moiety (including all the increments therebetween), optionally one to two orders of magnitude greater (including all the increments therebetween). The on-rate of the first ligand binding moiety may also be 2, 3 or 4 times greater than the on-rate of the second ligand binding moiety. In any of the preceding examples the intrinsic affinity of the first ligand binding moiety is preferably anywhere between 2 to 100,000 times, preferably 5 to 1000 times, greater that of the second ligand binding moiety.

The term "antibody" is used broadly, unless the context dictates otherwise, to refer without limitation, to a whole antibody of any class or biologic origin, or chimeric combinations of antibody regions or domains (eg. FRs and CDRs) of different origins or species eg. humanized, any combination of one or more antibody fragments or recombinant reconstructions (scFvs) of antibodies including dimers, diabodies, triabodies, a myriad of known bispecific, trispecific, tetraspecific antibody formats or monovalent, divalent, trivalent, tetravalent or other multivalent antibody formats (see for example review in Kriangkum J, et al. Bispecific and bifunctional single chain recombinant antibodies. Biomol Eng 2001 Sep;18(2):31-40 and others herein directly or otherwise referenced) or any fragment, portion, or reconstruction of one or more portions of an antibody (scFv) or any truncated form a ligand binding entity, such antibody typically comprising at least a VH or VL portion or both or a functional portion of same (eg microbodies), including single domain antibodies, F(ab')₂, Fab, Fab', Facb, Fc, etc. The term antibody also includes fusions of such an antibody so defined and other functional moieties (eg. toxins, cytokines, chemokines, streptavidin, adhesion molecules).

According to one aspect, the invention is directed to a multispecific ligand with at least two different binding specificities for different target ligands on the same target entity eg. a cell and which is preferably adapted to bind contemporaneously to (ie. there are no geometric or other constraints which preclude both moieties from functionally interacting with their respective target ligands at the same time)the different target ligands, said multispecific ligand comprising a first target binding moiety which preferentially(some cross-reactivity(s) does not preclude the utility of the invention) recognizes a first target ligand and a second target binding moiety which preferentially recognizes a second target ligand, and wherein the ability of the second target binding moiety to bind to the second target ligand is diminished relative the ability of the first target binding moiety to bind to the first target ligand, the first target binding moiety having an ability to bind to the first target ligand which is at least sufficient for the first target moiety to bind to the first target ligand independently of the second target binding moiety binding to the second target ligand and an off-rate (with respect to the first target ligand) which at least sufficiently exceeds the on-rate of the second target binding moiety for the second target ligand to at least provide opportunity for the second target moiety to bind the second target ligand when the first target binding moiety is bound to first target ligand, the second target binding moiety having a relatively diminished ability to bind and/or stay bound to the second target ligand independently of the binding of the first target binding moiety to the first target ligand (such that a plurality of the multispecific ligand will bind to a population of cells bearing both target ligands *in preference to* a population of cells bearing only the second target ligand (ie. at least in part due to the first target binding moiety assisting (ie. providing opportunity) the second target binding moiety to bind to the second target ligand and preferably out of proportion to what could be statistically attributed to the presence of two targets ligands on the target cell eg. the binding of the first target binding moiety providing necessary assistance for the second target moiety to bind is relatively increased (ie. relative to the situation where both of are of comparable affinity).

It will be appreciated that relative number of bioavailable second target ligands relative to the number of the bioavailable first target ligands will influence the selection of affinities of the first and second target binding moieties. For example, from the standpoint of safety, the affinity of the first target binding moiety for the first target ligand may well be sufficient if initially approximatingnanomolar affinity and the affinity of the second target binding moiety for the second target ligand will be selected to limit the number of effective binding events on the population of cells bearing only the second target moiety; an affinity which is inversely proportional to the number of bioavailable second target ligands on the population of cells bearing only the second target ligand ie. the non-target population (relative to the number of first target ligands on the target population of cells). For example, this may be assessed by determining the amount of labelled multispecific ligand on the target and non-target populations of cells in vivo (or in vitro where the number of bioavailable first and second target ligands can be roughly estimated). This selected affinity, from a effectiveness point of view, will then be assessed as to whether it is sufficient for the second ligand binding moiety to bind to the second target ligand on the target population of cells, with the benefit of the first ligand binding moiety bound or having been bound to first target ligand. For example, where the binding of the second target binding moiety may be assessed through an in vitro assay (eg. an assay in which the blocking or activating of a receptor is measurable eg. through inhibition of binding of the natural ligand for a target receptor or through some measurable parameter associated with effective binding for example the release of cytokines or other biologic effector ligand. The effect of binding may be also be assessed by comparing the effects over time relative to a higher affinity second binding moiety which is not associated with a first ligand binding moiety. It will be appreciated that a more ubiquitous second target ligand may require selecting a higher initial affinity of the first target binding moiety for the first target ligand eg. picomolar affinity, and selecting an affinity of the second target ligand which may for example be of micromolar affinity plus/minus approximately one order of magnitude. It will also be appreciated that the deleterious effects of non-target cell binding will vary as will the degree to which the first target ligand is uniquely found on the target population of cells. In the final analysis a suitable difference in affinity between the two binding affinities may well be at least, approximately, one, two, three, four, five, six, seven or eight orders of magnitude. In this connection the term approximately refers to +/- up to a half order of magnitude (<5x). As discussed below, the invention contemplates that variants of a dual affinity multispecific ligand may be assessed in a high throughput screen or series of such screens with a view to selecting a variant that has one or more predefined properties, alluded to above such as a) the ability to mediate a biologic effect on a target population relative to a negative control; b) the ability to mediate an improved or diminished biologic effect on a target population relative to a positive control. This ability may also be assessed in a competition experiment of any probative type well-known to those skilled in the art; c) the inability or diminished ability to mediate a biologic effect on a non-target population relative to negative and positive controls. Such diminished ability may be also assessed in a competition experiment of any probative type well known to those skilled in the art; d) the ability to target a target population through binding relative to controls and in a competition; e) the inability or diminished ability to target a non-target population relative to controls and in such competition experiement. The invention also contemplates that the multispecific ligand may bind to a ligand which is cell specific in the sense that it binds to cells to which it has been delivered by prior administration (eg an antibody or fusion protein thereof which only binds to the target cells or at least to cells which do not have the ligand recognized by the second ligand binding moiety present in any significant amount), akin to the pre-targetting strategies well known in the art. For example, this strategy could be used to increase the number of first ligands relative to second ligands, where indicated.

In one embodiment, said first target binding moiety recognizes an entity-associated ligand eg. a target cell-associated* target ligand, for example a ligand which is exclusively expressed, primarily expressed or over-expressed to advantage on the target cell population and said second target binding moiety recognizes a non-target cell-associated target ligand which is present on target cells and non-target cells, for example a receptor, including a decoy receptor eg. for TRAIL. The multispecific ligand is thereby adapted to block or activate the receptor primarily on the target population of cells. In this connection, the invention is also directed to methods of evaluating or implementing the effects of this enhanced selectivity for the receptor on the target cell population and can be employed to diminish the adverse consequences and evaluate the benefits associated with using a ligand binding moiety that would otherwise undesirably bind to receptors on non-target cells.

The invention contemplates that a variety of different strategies that can be used alone, or in any variety of compatible permutations to differentiate between target cells and/or between target and non-target cells. The choice of strategies, may depend at least in part on the circumstances, including the nature of the fluid environment in question, including the rapidity and pressure of flow and the direction(s) of this pressure, the method of delivery, the medical condition for which the molecule is being evaluated, whether the target is moving or stationary, or both, the location or various locations of the target, the targeting venue or venues that is/are most effective and the importance of the size of the molecule for reaching the target as well as bioavailablility, and the importance of creating immunoconjugates and immunofusions with other molecules (insofar as this affects the size and distribution of weight in the molecule). The invention contemplates that employing more than one than one type of construct may be desirable and the invention is therefore directed to the various combinations and permutation of constructs according to the invention, in combination with each other and other therapeutic molecules or modalities. One of constructs contemplated by the invention, is a multispecific antibody, for example a bispecific antibody having a configuration which allows for binding to two antigens on the same cell, for example a traditional four chain immunoglobulin configuration having a hinge region (including F(ab')₂ minibodies etc.), a diabody configuration (depending on the relative positions of the target ligands) and others herein referenced and known to those skilled in the art. It will also be appreciated that the mode of action of the multifunctional ligand may be contributed to by fusing or conjugating the multifunctional ligand to another functional moiety, for example, as described in the literature referenced below. These supplementary strategies are set forth below:
Additional Strategies For Modifying Targeting Capabilities
According to one embodiment, the intrinsic affinity of the first target binding moiety for the first target is greater than the intrinsic affinity of the second target binding moiety for the second target. The term "intrinsic" affinity connotes a measure of the affinity of a given target binding moiety for its target ligand which is independent of the affinity of the at least one other target binding moiety for its target ligand and as used herein could theoretically be evaluated in the context of the multispecific ligand as a whole, if the other target binding moiety had an irrelevant specificity and therefore could not bind to its target ligand. The invention contemplates that at least approximately one, two, three, four, five, six, seven or eight orders of magnitude differences in "intrinsic affinity" may be required to accomplish the targeting objectives of the invention.
According to another embodiment, the relative on-rate* of the first target binding moiety is greater than the relative on-rate of the second target binding moiety. The term relative on rate is used to connote an effective difference in on-rate that may be instrinsic to the individual target binding ligand or may attributable to its configuration or relationship vis-à-vis other parts of the molecule.

Where the intrinsic on-rate⁴ of the first target binding moiety is greater than the intrinsic on-rate of the second target binding moiety, the invention contemplates that the off-rate contribution to the affinity of the second target binding moiety may be proportionally greater than the off-rate contribution to the affinity of the first target binding moiety. The invention contemplates that the binding of the second target ligand binding moiety to its target ligand may be more effective if its lower affinity is attributable in part due its reduced on-rate. The invention contemplates methods for reducing the affinity a target binding moiety by reducing its on rate for example by mutating or adding amino acid residues in regions of the VH or VL that don't directly contribute to the off-rate (of a relatively high affinity binder for the target, for example, as determined by modeling and structural analysis, for example, by evaluating x-ray crystal structure and evaluating NMR data of the binding, or by mutagenesis, preferably by introducing a diversity of changes in a high-throughput manner (eg. phage display, ribsome display,microarray or other expression library) including substitutions, additions and deletions within various regions of the VH or VL and determining their effect. For example, the invention contemplates that the second target binding moiety is generated using a library characterized by members in which one of the regions of VH or VL, including particularly the CDR1 and CDR2, for example the CDR1 of the VH or CDR2 of the VL, is shortened and/or mutated in a manner to reduce the probability of its having any direct contribution to the affinity of the selected molecule (through molecular interaction), for example mutated to introduce amino acids that are least important for intermolecular interactions, for example by minimizing the occurrence of amino acids that are important for electrostatic interactions and optionally also hydrogen binding, generating a binder whose affinity will be postulated to be independent of the contribution of the modified CDR, and then optionally evaluating the success of this latter step through further mutagenesis (this step is most revealing if the CDR
⁴ The actual on-rate if the on-rate was to be measured independently of the on-rate of the other binding moiety
is shortened but not mutated or mutated to introduce amino acids important for intermolecular interactions) and then using the library to incrementally lengthen the region and/or introduce amino acids important for intermolecular interaction at a distance (eg. electrostatic interactions and optionally also hydrogen binding) to introduce minimal steric hindrance or intermolecular repulsion. The invention also contemplates that introducing amino acids that have the greatest potential for hydrogen bonding may introduce an aqueous cushion into the interface region with the target ligand to diminish the on-rate contribution to affinity. The invention also contemplates modifying the amino acid composition of an existing binder by introducing or one or amino acids or mutations into a framework region at a location which is proximal to the binding region or a region which borders the interface of approach to the binding region or any interface between the target binding moiety and the target ligand. The invention contemplates that the on-rate and off-rate can be routinely measured using various technologies (eg. Biacore) known to those skilled in the art, including various techniques of measuring these rates in real-time, for example those that measure the deflection pattern of an incident form of radiation (eg. Biosite). In one embodiment of the method the antibodies each have unique preferably cleavable peptide tags that are generated for example through a random or partially random insertion of nucleotides into the DNA encoding the antibody and that serve to link them to their DNA eg a phage (as per techniques known to those skilled artisans or published in the art) and the antibodies are evaluated independently of a phage (eg. they may even be cleavable from the phage) or other expression system linkage which allows a more accurate measure of their true on rates and off-rates. The invention also contemplates that FR1 could be lengthened in a relatively high affinity second target binding moiety to reduce its on rate. The cleanable peptide could be a unique identifying CDR.

In another aspect the invention contemplates that the multispecific ligand may comprise an Fc portion and a hinge portion and that one or both of a) the length, amino acid composition or* molecular weight (or various combinations of these interrelated factors) of the Fab or Fc portion; and b) the amino acid composition (including length) of the hinge portion (eg. any polypeptide segment that provides means for linking two typically heavy chains, eg. through one or more disulfide bonds, leucine zipper fos-jun, optionally a flexible hinge typical of an IgG1 or having one to several more disulfide bonds eg. IgG3) are selected to reduce the circumstantial(shear rate, presence of degrading enzymes) affinity of the second ligand binding moiety where the first ligand binding moiety is unbound relative to the circumstantial affinity of,the second ligand binding moiety where the first ligand binding moiety is bound. The term circumstantial affinity broadly contemplates that the length and molecular weight of the Fc and the flexibility of the hinge region will individually and collectively contribute to the affinity of the molecule in proportion the shear rate of the fluid environment to a degree depending on whether the target is stationary or moving, once the multispecific ligand is bound. If bound via the second target binding moiety, any increase in the molecular weight especially a distribution of the molecular weight towards the Fc or first ligand binding moiety will serve as a lever in a moving fluid environment, to favor disengagement from binding especially since the off-rate of this binding arm is relatively low to begin with. This same lever effect will impinge on the binding of the first ligand binding moiety but to a lesser functional degree due to its higher affinity. To an extent depending on the context in which binding occurs, the invention also contemplates that the high affinity ligand binding moiety will draw the multispecific ligand from the circulation into a desired target tissue and that the low affinity binding arm will then have greater opportunity to bind even if it does not bind simultaneously with the high affinity binding arm. Where the hinge region is extra flexible or has several regions of flexibility (for example where the heavy chains are linked through several disulfide bonds with regions of flexibile linker therebetween) the disengaging effect on the individual and paired binding of both the first and second ligand binding moieties will be less Similarly, using a truncated Fc portion (CH3 deleted, F(ab')₂ or minibody format) will assist the first ligand binding moiety to remain bound or foster binding of the second ligand moiety and will assist the second ligand binding moiety to remain bound. This construct may be preferred from an effectiveness standpoint (getting both ligand binding moieties bound), where the affinity of the second ligand binding moiety is low to begin with.On the other hand, decreasing the flexibility of the hinge region by alteration to its length and/or amino acid composition and increasing the molecular weight distribution towards the "free" end of the Fc will affect all binding scenarios to a greater extent. The latter strategy may be less desirable where the Fab of the first ligand binding moiety is lengthened (eg. has a longer hinge region at the N-terminus of the disulphide bond linking the heavy chains, than the low affinity binding arm) to increase its propensity for individual binding. For example, in a conventional four chain or heavy chain antibody (two heavy chains but no light chains) the hinge region could be lengthened or shortened on the amino terminus side of the disulfide bond linking the heavy chains to an extent that does interfere with the simultaneous binding to both the first and second target binding moieties. The invention also contemplates that the target cell environment, naturally or through intervention, is a fluid environment (low shear rate) or enzyme environment which will favor a greater impact on disengagement of the second ligand binding moiety, in the case of an enzyme, one which will cleave off an Fc into which a cleavage site has been introduced so that disengagement due to the lever effect will primarily impinge on binding of the second ligand moiety to the non-target cell population (eg. low shear rate or presence of MMP type enzymes in a targeted solid tumor environment).

The invention also contemplates that second ligand binding moiety may be selected in an environment in which there is a selective pressure (moderate fluid flow eg. using live cells or tissue, candidate ligand binding molecules or pairs of the target ligands on latex beads, where the substrate to which they are bound is on an incline or otherwise subject to fluid flow (optionally with rigid or high mol. weight Fc), for simultaneous binding so that the affinity of the second ligand binding moiety is selected on the basis of its ability to augment the binding affinity of a first ligand binding moiety of preselected affinity for the first target ligand (after or optionally before its affinity maturation, depending on the shear force and affinity in question) and thereby augment the affinity of the multispecific binding ligand as a whole, while the first ligand binding moiety is bound. In this way, the strength of the binding affinity of the second ligand may be predicated on the first ligand moiety being bound. The foregoing strategy may have accentuated or at least equal application where the first ligand binding moiety has a longer Fab or for example where both the first and second ligand binding moiety are devoid of a light chain ie. where having the correct binding interface for the second target binding moiety might be more acute. The invention contemplates that the individual affinity of second ligand binding moiety selected in the above manner would be tested to ensure that its individual affinity was not sufficient for substantial independent targeting.

The invention also contemplates that engineering a suitable affinity antibody for solid tumor targeting in which the on-rate contribution to affinity is reduced (according to the strategy suggested above) may assist a dose of such antibody in achieving better tumor penetration. An antibody having a reduced on rate could be fused to a toxin such as a truncated version of PE or conjugated to a radionuclide, etc. the reduced on-rate contribution ensuring that the antibody will be less likely to bind at sites proximal to the point of entry to relieve congestion in that area and better ensure its diffusion throughout a tumor. The invention contemplates that the strategies decribed above will better permit the affinity to be more suitably apportioned between the on-rate and the off-rate. The invention contemplated that a higher on-rate lower off-rate Ab could be delievered in alternating days or other cycles of treatment. Thus the invention is directed to an antibody conjugated or fused to a functional moiety, wherein the on-rate contribution to the affinity of the antibody is anywhere between 3x and two order of magnitudes less than typical molecules having suitable properties for tumor penetration through diffusion, for example molecules having anywhere (any increments) between 10⁻⁷ and 10⁻¹⁰ molar affinities (eg. 5x 10⁻⁷, 3×10⁻⁸) preferably increments between 10⁻⁸ to 10⁻¹⁰ (molecules where the on rate is normally approx. 10⁻⁵) molar affinities, more preferably increments between 5×10⁻⁸ and 5×10⁻⁹

It will be appreciated that the foregoing strategies could be employed for designing a multispecific ligand which will primarily target cells which have both the first and second target ligand (eg. where the ligands together are present primarily on the target cell population) even where neither target ligand is individually found primarily on the target cell population, by employing a multispecific ligand in which neither target ligand is of sufficient affinity in the circumstances to effectively (with effect) bind or remain bound without the other target ligand being available for simultaneous binding. As suggested above, it will be appreciated that a relatively higher affinity ligand could initially be employed on one of the ligand binding arms to select a second ligand binding arm which improves the binding properties of the multispecific ligand under a suitable biologically relevant shear stress and which is selected or later modified so that it is individually insufficient for targeting its target on non-target cells in the circumstances in which it will be employed, and that the high affinity ligand binding arm can subsequently be reduced to moderate affinity with similar lack of individual effect. In one embodiment, this construct can be employed to evaluate the effect of blocking two receptors on the same cell, for example chemokine receptors eg. CCR7 and CXCR4 on a breast cancer cell. In one embodiment, the off-rate of one or optionally both ligand binding moities is sufficient in the circumstances to permit the moiety to remain bound for a sufficient duration for the other moiety to bind ie. it exceeds its effective or intrinsic on-rate. In one embodiment, both arms of such multispecific ligand, bind to their respective ligands with low affinity. In one embodiment, one such arm is a "coybody".

In connection with the foregoing and ensuing strategies it will also be appreciated that the hinge region may be lengthened on the N-terminal side of the most N-terminus linker between the heavy chains so as to permit greater flexibility in the binding of different antigens at different possible proximities to one another.

The invention also contemplates that the two heavy chains of an IgG (with or without light chains and/or CH1/CL domains), minibody/ F(ab')₂ (with or without light chains and/or CH1/CL domains), may be linked (whether they have a full size or fully truncated Fc or elongated hinge regions) through a flexible peptide linker (such as used for making scFvs i.e. multiples of gly4ser) in order to ensure correct pairing of the heavy chains by expressing the linked heavy chains in E. Coli, for example, as inclusion bodies, which are refolded in refolding solution according to well established techniques in the art. In a construct employing light chains, the light chains may be linked through a disulphide bond linking according to well known methods of making disulphide stabilized Fvs (dsFvs) and the same light chain may be employed for both the high and low affinity arms.

With respect to each of the preceding aspects of the invention, the invention also directed to a multispecific ligand comprising a first ligand moiety which recognizes a first target ligand that is over-expressed on a disease associated entity for example a diseased or disease-causing or mediating cell or infectious agent and a second ligand binding moiety that recognizes a target ligand and wherein the first target ligand is characterized in that it does not lend itself to facilitating or permitting internalization of the second ligand binding moiety.

The invention also contemplates that a target ligand can be distributed in various concentrations for testing purposes on cell sized latex beads, columnar packing materials or flat substrates having a high density dispersion of both target ligands.

The invention is also directed to combination therapies with the foregoing multispecific ligands including, without limitation, immunotoxins, drugs, therapies with other multispecific ligands herein described and particularly for cancer therapies directed at interfering with the integrity of tumor cell vasculature.

### Delivering Biologic Effector Ligands To A Target Entity

With respect to each of the preceding aspects of the invention, the invention also contemplates that the second ligand binding moiety may be constituted in whole or in part by a ligand which binds to a biologic effector ligand (such as a cytokine, colony stimulating factor, chemokine, growth factor etc. or related extracellularly expressed regulatory molecules that control their expression such as inhibitors, agonists, antagonists of same, which may have corresponding biological receptors), the ligand optionally having a higher affinity for the biologic effector ligand than the affinity of that biologic effector ligand for its receptor, and wherein the ligand, combined with the biologic effector ligand (ie. bound thereto), has a relatively diminished ability to bind and/or stay bound to the receptor (the second target ligand) independently of the binding of the first target binding moiety to the first target ligand eg. a lower affinity of approximately one, two, three, four, five, six, seven or eight orders of magnitude. The invention contemplates that the foregoing construct can be used to deliver the biologic effector ligand more selectively to the target cell population recognized by the first ligand binding moiety. The second ligand binding moiety may be an antibody portion of a multispecific ligand of the invention and the invention contemplates that a library of second ligand binding moieties, recognizing multiple different epitopes on the biologic effector ligand, can be screened for their ability to bind to the biologic effector ligand, while it is bound in situ to its receptor, for example, using a microarrary of such antibodies, and the affinities of the binders can be evaluated. The invention also contemplates that suitable antibodies could be generated by "panning" (with an expression library, eg. phage display, ribosome display, or other similar display systems including yeast, bacterial, viral, cell based or cell-free display systems) or otherwise screening (eg. using antibody microarrays) against the biologic effector ligand while bound to its receptor and screening for their ability to bind to the biologic effector ligand independently of its receptor. Again, the affinities of the antibody coupled to the-biologic effector ligand for the target receptor could be evaluated. More generally, the invention contemplates that an array of antibodies which recognize all different epitopes on a given biologic effector ligand could be generated and tested for their ability to accommodate binding of a biologic effector ligand to a first but not a second in a related family of receptors. This could be accomplished by screening the array for one or more members that bind to the biologic effector ligand (BEL) while bound to its receptor, and testing the identified members for their ability to bind to the second receptor, preferably by loading the biologic effector ligand onto an array of those members pre-bound with BEL and detecting those BEL bound members for those which do and do not bind to the second receptor. Therefore the invention is also directed to an antibody which accommodates binding of the BEL to one receptor but hinders the binding to at least one second receptor, preferably by steric, charge or other inter-molecular hindrance, attributable to the proximity of the antibody epitope on the BEL to the BEL's receptor binding site and optionally also the amino acid composition of the antibody at that interface.

The invention contemplates that fluid flow can be simulated in a purification or immunoaffinity column packed with one or more known packing materials to simulate flow over a ligand coated substrate.

The invention also contemplates an apparatus and method for testing ligand binding in a circulating fluid environment in which the multispecific ligands of the invention can be tested and wherein a continous flow of ligands, including target ligands, ligands of the invention and/or ligand bearing entities (eg. cells or synthetic eg. latex spheres which can be adjusted to a cell size) to which one or types of ligands have been affixedly associated accordingly to known methods) can be generated. The fluid contact interface of the apparatus has a generally circular shape and is convex or otherwise capable of containing the fluid and thereby preferably permits fluid to flow around the surface continuously. For example, this surface may be enclosed with a bagel-shaped cylinder which is optionally open at a location opposite the fluid contact surface for introducing and/or removing its contents, or it may completely enclosed with the exception of an access port, from which any air may optionally be displaced or evacuated. The invention contemplates that the apparatus (at least the fluid contact vessel) can be rotated or oscillated (eg. in an elliptical, oval or similar shape well known to those skilled in the arts of fluid mechanics and related engineering arts) in a variety of different planes or with rocking-like motion in multiple planes or subject to peristaltic pressure (ie. where flexible tubing is used) to generate a continuous, optionally turbulence free fluid flow over the fluid contact surface at selected rates simulating the various shear rates of arterial, venous, intra-lymphatic flow (including different diameters of such vessels) or interstitial flow. The invention also contemplates that the fluid contact surface may be provided with a 1) substrate for linking ligands of the invention or target ligands or ligand bearing entities to permit fluid flow across the substrate in a plane substantially parallel or conforming to the axis of flow.

In another aspect the invention is directed to methods of making a multispecific antibody in which:
a) the light chains are the same for both the VL domains . For example, the light chains (assuming the construct has two light chains) are generated for a first target binding moiety eg. in one aspect of the invention, the relatively high affinity binder, optionally from a light chain germline sequence, and this light chain is then coupled with a diversity of heavy chains to select a pair of chains which bind to the second target ligand, thereby constituting the second ligand binding moiety, which may be a relatively low affinity binder. An alternate or concomitant strategy to generate a lower affinity second ligand binding moiety would simply be to substitute the light chain of the first ligand binding moiety for that of the second ligand binding moiety and to test the affinity. In the case of a multispecific which target BELs to particular target cells, where for example, two high affinity binders are preferred, the heavy chain and light chain binding to the BEL can be truncated correspondingly at the CH1/CL region so that the VH/VL interfaces and cysteines pairing these heavy and light chains are similarly spaced but spaced differently from the other VH/VL chains. By linking the heavy chains as explained above, all chains will pair correctly. It will be appreciated that the foregoing production strategies could be applied to the production of heavy chain antibodies (two chains structures without associated light chains), wherein the heavy chains are from human or other species and that production in this case could be adapted to E. Coli. It will also be appreciated that deletion of a substantial part of the CH1 and CL domains can be measured to provide a space for the BEL to sit in line with the other Fab which can be lengthened in the linker or CH1 domain, as shown in Figure C. The invention contemplates that evaluation of a diversity of the first target binding moiety can be accomplished with the BEL place to best accommodate selection in the context of the entire structure as a whole.
b) With respect to other methods to make bispecific and bispecific fusions see Antibody Fusion Proteins Wiley-Liss 1999 (infra) eg. particularly p 131 et seq., and Chapter 7 and the discussion, Methodologies improving the correct pairing of heavy chains are well-known in the art.

Such a construct could also be employed in conjunction with other functional moieties fused or conjugated thereto, for example toxins, cytokines, enzymes, prodrugs, radionuclides etc.

In one preferred embodiment, the invention is directed to a multispecific ligand* with at least two different binding specificities for different target ligands* on the same target cell* and adapted to bind contemporaneously to the different target ligands, said multispecific ligand comprising a first target binding moiety which preferentially* recognizes a first target ligand and a second target binding moiety which preferentially recognizes a second target ligand, and wherein said first target binding moiety recognizes a target cell-associated* target ligand and said second target binding moiety recognizes a non-cell-associated target ligand which is present on target cells and non-target cells; and wherein the ability of the second target binding moiety to bind to the second target is diminished relative the ability of the first target binding moiety to bind to the first target ligand, the first target binding moiety having an ability to bind to the first target ligand which is at least sufficient for the first target moiety to bind to the first target ligand independently of the second target binding moiety binding to the second target ligand and an off-rate which at least sufficiently exceeds the on-rate of the second target binding moiety for the second target ligand to provide opportunity for the second target moiety to bind the second target ligand when the first target binding moiety is bound to first target ligand, the second target binding moiety having a relatively diminished ability to bind or stay bound to the second target ligand independently of the binding of the first target binding moiety to the first target ligand, such that the multifunctional ligand will bind to the target population of cells in preference to the non-target population of cells. As suggested above, the strategy embodied in this preferred embodiment can also be employed in connection with any one or any combination of compatible strategies referred to above, to diminish in degree the requirement of using a low affinity second ligand binding moiety.

In another aspect the invention is directed to heterofunctional ligand comprising a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the relative avidity or affinity or both the affinity and avidity of said second moiety are selected or adjusted to substantially reduce the probability of the second moiety binding to the second target ligand without the first moiety, first or substantially contemporaneously, binding to the first target ligand. For example, in one embodiment the first moiety is divalent and the second moiety is monovalent. In one embodiment the affinity of the first moiety for its target ligand is for example up to several orders of magnitude greater than the affinity of the second moiety for its target ligand, as discussed below. In a preferred embodiment both moieties are capable of binding to different target ligands on the same cell, for example as hereinafter specified, although in the case of tumor cell targeting, particularly with respect to cells that are growing adjacent to another the invention contemplates that the first moiety may bind to one cell and the second moiety may bind to a neighbouring cell. Accordingly, in the case of receptors requiring cross-linking for biological activity the invention contemplates that such same cell interactions and adjacent cell interactions are optionally accomplished when the second moiety is bivalent. In one embodiment, at least one of said first and second moities comprise one or more antibody components. In another embodiment, said first moiety binds to at least one cell-surface ligand which differentiates between cells of the same population or sub-population, for example, at least one ligand which diffentiates which between populations or sub-populations of immune cells (eg. see WO 01/21641, US 6156878), for example, activated vs. non-activated, disease-associated or non-disease-associated (over-expressing or uniquely expressing certain receptors or other ligands [for example cytokine or growth factor receptors, particular immunoglobulin like molecules or MHC peptide complexes] or other differentiating markers hereinafter exemplified or apparent to those skilled in the art), and said second moiety, in virtue of its binding to the second target ligand, directly or indirectly exerts a biologic effect eg. a therapeutic effect, for example an immune modulating effect. In a further preferred embodiment said second moiety has a broader target cell population than said first moiety Eg. see Wiley H. et al. Expression of CC Chemokine Receptor-7 and Lymph Node Metastasis..., J. Natl. Cancer Inst. 93:1638-1643; Moore MA Bioessays 2001 Aug;23(8):674-6. (The invention contemplates that by targeting CCR7 receptor selectively on tumor cells, for example using a relatively high affinity binding moiety for a tumor associated antigen and a relatively low affinity moiety which binds to and blocks CCR7 receptor, eg. when combined in therapy with a chemotherapeutic agent or an immunotixun for the same tumor, metastasis can be inhibited). For example, in one embodiment said first moiety binds to a tumor associated antigen on a tumor cell and said second moiety binds to a receptor which is found on the tumor cell but also on a broader population of cells. In another embodiment said first moiety binds to an antigen associated with particular population of leukocytes and said second moiety binds to a receptor which is found on that population of cells but also on a broader population of cells (eg. apoptosis mediating receptors Journal of Immunology 1998 160:3-6, Nat Med 2001 Aug; 7(8)954-960, WO 01/85782; ICAM-R WO 00/29020; see also WO 01/85768, WO 01/85908; WO 01/83755, WO 01/83560, WO 01/29020; Vitale et al. Prpc. Nat. Acad. Sci. 2001 May 8; 98(10):5754-5769; CCR2 see also USP 6312689; USP 6,294,655 Anti-interieukin-1 receptor antagonist antibodies and uses thereof; USP 6,262,239; USP 6,268,477) . In another embodiment the second moiety does not necessarily bind with lower affinity to its target however it may bind to a first ligand which in turn binds to a second ligand on a target cell (eg. a receptor on the target cell eg. a cytokine, chemokine or growth factor receptor), for example the receptor being on the same cell to which he first moiety binds, and it binds in a manner in which it partially interferes with the binding of the first ligand to the second ligand and thereby directs or retargets that first ligand to the second ligand in a manner which accomplishes the intended interaction of the the first with the second ligand (eg a signal transduction or blocking interaction ie. the second moiety causes the eg. cytokine to bind to its receptor without engendering the biological effects attributable to receptor binding eg. signal transduction, which may be assessed by assaying for effects of eg. signal transduction according to well established techniques in the art) but less competitively relative to the first moiety so that the first moiety exerts a targeting function ie. where the first ligand bound by the second moiety binds to a broader than desired population of cells. The binding of the second moiety may also be compatible with the first ligand binding to one cell surface ligand but not another eg: see WO 00/64946 the contents of which are hereby incorporated by reference. The ability to identify ligand residues of importance to binding or residues other these, the alteration of which might interfere with binding is well established in the art. The invention contemplates varying, by high throughput techniques eg. phage display, residues of an antibody that are not involved in first ligand binding to create variants which can be tested for partial interference with first ligand binding to the second ligand eg. receptor binding.

Examples of receptors for blocking or activation by the targeting methods described herein include tyrosine kinase type recptors, serine kinase type receptors, heterotrimeric G-protein coupled receptors, receptors bound to tyrosine kinase, TNF family receptors, notch family receptors, guanylate cyclase types, tyrosine phosphatase types, adhesion receptors etc. (for example receptors see those discussed in Cancer: Principles and Practice of Oncology 6th Ed. De Vita et al. Eds Lippincott 2001, including particularly Chapter 3, 7 and 18, The Autoimmune Diseases, Academic Press Third Edition, Rose/Mackay ISBN: 0125969236, Immunology 6th Edition, Mosby 2001 Roitt et al. Eds; Molecular Mimicry, Microbes & Autoimmunity by Madeleine W. Cunningham (Editor), Robert S. Fujinami (Editor) December 2000, among other references hereininbelow identified). Further mention may also be made of interleukin and interferon type receptors, HGF receptor (see for example USP 6,214,344), CD45, CXC family receptors including CXCR1 and CXCR2 receptors including IL-8 receptor, EGFRs, receptors for molecules with functions in apoptosis or homeostasis, receptors such as FGF which sensitize tumor cells to chemotherapeutic agents, etc. It is known for example to modify receptor ligands in a way which does not interfere with a signaling function (the residues important for signaling may be known or can be readly ascertained eg. see Retargeting interleukin 13 for radioimmunodetection and radioimmunotherapy of human high-grade gliomas. Debinski W, Thompson JP.Clin Cancer Res 1999 Oct;5(10 Suppl):3143s-3147s) but reduces the affinity of the ligand for this receptor (see also WO 01/19861). Alternatively, the second moiety may be an antibody which is agonistic or antagonistic and used to block, activate, neutralize etc the receptor. With respect to EGFR family,TNF family and other receptor targeting antibodies which are capable of causing apoptosis directly or indirectly, see US 5,876,158, WO 00/20576, WO96/08515 WO 01/44808 (P75AIRM1), WO 00/29020 (ICAM-R), WO 99/12973, CA 2236913 etc. The invention also contemplates that the second moiety may also be targeted to a specific portion of a receptor which differentiates it from other receptors of its class and more generally contemplates that the second moiety may contribute to the targeting ability of the multifunctional ligand. Examples of antibodies that bind to receptors for application of the are also listed in Appendix B.

In another aspect, the invention also contemplates that the first moiety binds to a target cell and said second moiety binds to a ligand, for example a natural ligand, (eg. a cytokine or chemokine circulating at normal levels or at higher levels attributable to a disease or treatment of a disease with another therapeutic molecule) and retargets that ligand (for example, the ligand may be retargetted from circulation) to a targeted cell. For example the invention comtemplates that IL-2 may be retargeted to LAK cells or CTLs via a high affinity Leu-19 binding first moiety. For example, antibodies including fragments thereof which bind to cytokines or other natural ligands for retargeting purposes (eg. single domain antibodies) can be made by phage display against the cytokine or ligand while bound in situ to its receptor. The invention also contemplates that the affinity for the cytokine may be adjusted to regulate the degree of targeting and that serum samples may be evaluated to assess the degree of bound cytokine and the relative degree of bound and unbound cytokine. Among other methods, for example, the invention contemplates that a radiolabelled multifunctional ligand may be used assess the amount of label associated with the multifunctional ligand when bound to the cytokine, by capturing the 'complex' with an antibody that recognizes both antigenic determinants on both the cytokine and an adjacent portion of the ligand binding thereto ie. forming a composite epitope), such as may be generated by phage display and assessing the amount of label relative to the amount of captured complex.The invention also contemplates administration of supplemental amounts of natural ligand to compensate for the degree in which the ligand is retargeted insofar as such retargeting might impact negatively on immune or other physiological processes.

In another aspect the invention contemplates that patients treated with antibodies to a particular biologic effector ligand eg. a natural ligand eg. a cytokine, for example TNFα, may preferably be treated with a multifunctional ligand having a first moiety which binds to at least one cell type and a second moiety which binds to a natural ligand such as a cytokine for retargeting that cytokine to that cell type, as in a preventative method for treating a disease, eg. cancer. In this respect the invention contemplates that the antibody is capable of binding to the cytokine but once bound the cytokine, the cytokine is incapable and/or only weakly capable of binding to its receptor and/or that the multifunctional ligand also comprises a higher affinity receptor blocking moiety to minimize retargeting of the primary disease site. In one embodiment, the first moiety binds with relatively higher functional affinity (ie. avidity, affinity, and/or relatively advantageous binding capacity in virtue of multiple ligand binding arms, each binding to different ligands on the target cell) to ensure binding to the retarget cell. In another embodiment the bound cytokine is capable of binding to the cytokine receptor at the retarget site but incapable of binding to the receptor at the disease site owing to differences in the receptors at the two sites. The nvention also contemplates using antibodies which interfere but do not preclude binding of the biologic effector to provide a less toxic effect.

For example, patients with Crohn's disease that are treated with anti-TNFα (see for example, Expert Opin Pharmacother 2000 May;1(4):615-22 and references cited therein) may be treated according to the invention with a bispecific antibody having, in addition to an anti-TNFα binding moiety, Which reduces the affinity of the bound TNF for the receptor, but also an antibody moiety which binds to tumor antigen which is expressed on many different tumor types or optionally a trispecific antibody which additionally binds to a second multi-carcinomic antigen, preferably one which broadens the range of targeting against prevalent cancers. With respect to tumor antigens mention may be made of EGFR, EPCAM, MUCINs, TAG-72, CEA, H11 among other known multicarcinomic antigens (see also Cancer: Principles and Practice of Oncology 6th Ed. De Vita et al. Eds Lippincott 2001 Chapters 18 and 20.5). In another embodiment, the second moiety differentially retargets a cytokine to one receptor in preference to another, for example, to a TNF receptor over-expressed on tumor cells in preference to a TNF receptor associated with Crohns disease. In a related but also independent aspect, the invention contemplates a method of screening for an antibody which preferentially binds to a ligand when bound to a first receptor relative to another second receptor by screening for antibodies (eg. by phage display, ribosome display, etc.) which bind to the ligand eg. a cytokine, when bound in situ to the first receptor, and selecting among them those that bind to the ligand eg. cytokine but do not bind (substractive screening) or bind with lesser affinity to the cytokine when bound to the second receptor, as well as to antibodies and multifunctional ligands created by this method (see also USP 6,046,048 and WO 99/12973 and references cited therein with respect to TNF family of receptors). Variations in the extracellular domains of such receptors are known and can be ascertained by methods known to those skilled in the art.

Further with respect to multifunctional ligands having a higher affinity targeting moiety relative to the second ie. effector moiety, the second moiety may be for example an antibody or other ligand which interferes with the binding of the regular ligand for this receptor. For example, the invention comtemplates a first ligand binding moiety which recognizes activated T-cells and a second ligand binding moiety which blocks the IL-16 receptor for testing the effect on Crohns disease (or alternatively activates an IL-16 receptor on those cells eg. by using a high affinity IL-16 bound second moiety which becomes relatively low affinity IL-16 receptor ligand when bound to the antibody, again to test the effect on Crohn's disease (see Gut 2001 Dec. 49(6) 795-803) For example, in one embodiment, the invention contemplates that the second moiety blocks a receptor that are found on cells other than the target cell, the blockage of which leads to the apoptosis of or destruction of the cell eg. CD95 (eg. see Jung G. et al., Target cell-restricted triggering of the CD95 (APO-1/Fas) death receptor with bispecific antibody fragments Cancer Res 2001 Mar 1;61 (5):1846-8). With respect to blocking insulin like growth factor receptor, insulin receptor etc. see The IGF system in thyroid cancer. new concepts. Vella V., Mol Pathol 2001 Jun;54(3):121-4; Insulin receptor isoform A, a newly recognized, high-affinity insulin-like growth factor II receptor in fetal and cancer cells. Mol Cell Biol 1999 May;19(5):3278-88; Expression of the insulin-like growth factors and their receptors in adenocarcinoma of the colon. Freier S Gut 1999 May;44(5):704-8; Pandini G., Insulin and insulin-like growth factor-I (IGF-I) receptor overexpression in breast cancers leads to insulin/IGF-I hybrid receptor overexpression: evidence for a second mechanism of IGF-I signalingClin Cancer Res 1999 Jul;5(7):1935-44. With respect to targeting beta-1 integrins see eg. Masumoto A, et al Role of beta1 integrins in adhesion and invasion of hepatocellular carcinoma cells. Hepatology. 1999 Jan;29(1):68-74. Arao S, et al. Beta1 integrins play an essential role in adhesion and invasion of pancreatic carcinoma cells. Pancreas. 2000 Mar;20(2):129-37. Xie Y, Xie H. Characterization of a novel monoclonal antibody raised against human hepatocellular carcinoma. Hybridoma. 1998 Oct;17(5):437-44. Peng H, et al Production and characterization of anti-human hepatocellular carcinoma monoclonal antibodies. Hua Xi Yi Ke Da Xue Xue Bao. 1990 Sep;21(3):259-62; Whittard JD, Akiyama SK. Activation of beta1 integrins induces cell-cell adhesion. Exp Cell Res. 2001 Feb 1;263(1):65-76 Nejjari M, et al. alpha6beta1 integrin expression in hepatocarcinoma cells: regulation and role in cell adhesion and migration. Int J Cancer. 1999 Nov 12;83(4):518-25; Yao M et al Expression of the integrin alpha5 subunit and its mediated cell adhesion in hepatocellular carcinoma. J Cancer Res Clin Oncol. 1997;123(8):435-40.

The invention also contemplates a method of optimizing the cooperative affinities of respective binding ligands of a multifunctional ligand described herein and the length of a linker therebetween for the above and applications described below by phage or ribosome display etc. in which the multifunctional ligand is a single polypeptide chain, for example, two single chain Fvs or single domain antibodies linked in sequence, or a diabody (see USP 5,837,242), by varying the DNA sequence corresponding to amino acids that represent linker and/or for example CDR regions that are postulated to impact on affinity according to methods and strategies that well known in the art for affinity maturation. These same strategies can be employed for engineering lower affinity molecules. Accordingly, more generally the invention is directed to a phage display or similar library (eg. a ribosome display library or a microarray) in which the population of variants is a multispecific ligand, including a multispecific ligand according to the invention herein defined.

The term ligand binding moiety includes any ligand that can be used as a targeting arm or to bind to the second ligand for example cytokines, chemokines, etc and optionally those that can be modified, preferably by high throughput means to adjust its affinity and/or specificity, including scaffolds comprising a polypeptide, a peptide, a carbohydrate, a ribonucleic acid, a lipid and a small molecule, or a binding moiety consisting essentially of a combination of such types of molecules. Example include non-immunoglobulin protein scaffolds such affibodies (see US 5831012, US 5958736, EP 0486525) Trinectin domains (Phylos Inc.), TCRs, peptide mimetics, peptides located within a antibody variable region scaffold (eg. see
WO 02/ 44197, especially with respect to selectively targeting interferon alpha to specific cell populations) peptide fusion proteins and stabilized polypeptide loops, disulfide stabilized loops (eg. see WO 99/23222); all known in the art for such uses. These may be linked by peptide linkers of for example 10 to 50 amino acids. A variety of linkersincluding flexible linkers such as multiples of gly4ser are well known in the art.

In another embodiment blockage of a receptor does not necessarily lead to cell death but may lead only to decreased or increased release of certain cytokines etc, for example as mediated via the IL-6 receptor. In another embodiment the second moiety may achieve the desired therapeutic effect by constituting the normal ligand for that receptor or a functional substitute. The multispecific ligand may also be fused or conjugated to a toxic moiety or other effector. In another or further preferred embodiment, said first moiety comprises two binding ligands (eg. one or both of which may be an antibody) which respectively bind to two different target ligands each of which contributes to its total binding capacity and neither of which are sufficient to efficiently target the the cell, for example a ligand which binds to a specific MHC peptide complex and a second reduced affinity ligand which binds to a ligand on an APC. This approach also obviates the need to create high affinity ligand for a particular MHC petide complex, although this can been accomplished. In another or further preferred embodiment the target cell is an immune cell and the second moiety binds to a molecule involved in cellular adhesion, a cytokine receptor, a ligand which stimulates the activity of said immune cell, a ligand which inhibits the activity of said immune cell, a ligand which causes one or more cytokines to be released, a ligand which prevent one or more cytokines from being released, a ligand which causes or facilitates apoptosis of said immune cell or a ligand which permits internalization of said multispecific ligand. In another preferred embodiment the heterofunctional ligand is fused or conjugated to a therapeutic agent or a moiety that binds to a therapeutuc agent (exemplified below) or a ligand which effects binding to another immune cell, for example a T cell. In another preferred embodiment, the multispecific ligand is a bispecific antibody, a trispecfic antibody or a tetraspecific antibody. In a further preferred embodiment the first moiety binds to but is incapable of modulating the activity of said immune cell and said second moiety modulates the activity of said immune cell independently of said first moiety. In another preferred embodiment the multispecific ligand further comprise a moiety that binds to at least one ligand located on the intraluminal surface of a lymphatic vessel, preferably a lymphatic vessel associated ligand, as hereinafter defined. In other aspects the invention is directed to a pharmaceutical composition comprising such a multispecific ligand and a pharmaceutically acceptable carrier, a method of using the heterofunctional ligand in the preparation of a pharmaceutical composition for treating a disease, and to a method of treating a subject by administering same in a therapeutically effective amount.

The invention is also directed to a multispecific ligand which comprises a first ligand binding moiety which neutralizes a ligand eg. a natural ligand such as a cytokine, chemokine, colony stimulating factor or growth factor and a second ligand binding moiety which binds to a cell marker associated with a cell through which the natural ligand exerts a deleterious affect. Preferably the affinity for the first ligand binding moiety for the natural ligand will be greater than that of the second ligand binding moiety for the cell associated marker. Optionally the construct will be selected so that the binding interfaces are pointed in opposite directions and the lever effect is maximized, for example a bispecific construct where the heavy chains are joined directly or through an inflexible linker and are optionally linked to their respective (optionally common respective light chains) through a disulphide linker via framework residues as is well known in the art. Optionally, the molecular weight of the first ligand binding moiety is substantially less or more (by at least 10%, preferably at least 20%, more preferably at least 25%) and preferably less than that of the second ligand binding moiety as may be effected or maximized through mutating from higher to lower (on the first ligand binding moiety) and lower to higher mol. wt residues (on the second ligand binding moiety), the native residues which are not exposed ( to avoid immunogenicity) and not essential for proper folding and function of the VH/VL (as may be determined from the degree of conservation of such residues among immunoglobulins of the species, through % frequency tables available through the Kabat database and well known published determinations in this regard (see for example WO 02/40545). Examples include neutralizing IL-2 via a marker on activated T cells, blocking IL-15 via CD8+ T cells, blocking TNFalpha on mast cells; binding thrombin via activated endothelial cells etc.

The term heterofunctional is used broadly to refer to a ligand: 1) comprising at least two functional moieties that have different functions or different capacities to perform the same function and 2) which is typically and preferably heterospecific (having two binding specificities).

Unless the context dictates otherwise the term avidity when used in a comparative, quantifiable or controllable sense is used to refer the valency of the binding entity or moiety. The term functional affinity is used a composite term referring to a quantitative and contollable (though not necessarily quantifiable, especially when its consists of both avidity and affinity components) propensity to specific binding attributable to one or both of avidity and affinity effects.

In another aspect, the invention contemplates that cells, particularly immune cells, that are expected to be present at or proximal to a disease site (eg. at the site where an immune cell crosses the vascular endothelial cell wall), in virtue of the disease or a therapeutic modality which is employed in relation to the disease or a concurrent disease, including cells that directly mediate the disease, may be targetted in virtue of a marker associated with such cells, eg.markers associated with activated immune cells or disease mediating immune cells eg. LEU-19, a marker associated with activated or killerT-cells, etc for example with an antibody, which is linked to a moiety that is capable of exerting a therapeutic effect in relation to the disease, for example, an immunoliposome or an antibody linked to another therapeutic delivery system (for example example streptavidin or biotin fused, coated or conjugated entities or other payload carrying entities (see for example US patents 5439686, 6007845, 5879712, 5456917, 6165502, 5079005, 5888500, 5861159, 6193970, 6190692, 6,077, 499, WO 00/69413, WO 01/07084, etc.). For example, an immunoliposome may carry one of or a combination of cytokines, chemokines, toxins or other therapeutic molecules suitable for treating the disease directly or indirectly, for example by attracting or preventing the attraction, activating, anergizing or otherwise modulating the activity of immune cells for therapeutic or related purposes. Thus according to another aspect, the invention is directed to a multifunctional ligand characterized in that it exerts an independent biologic function said multifunctional ligand comprising a ligand which binds to a non-diseased disease associated cell and: a) a therapeutic entity; b) a ligand which binds to a therapeutic entity; or c) a ligand which binds to a disease mediating entity eg. a biologic effector molecul which is released by the disease mediating entity or the diseased cell eg. a cytokine or other BEL which mediates or aggravates a disease process. Preferably said multifunctional ligand comprises at least two of a), b) or c) and preferably all three.

The term "independent" refers to a function which is primarily exerted in relation to an entity other than the entity that is targeted (save for possible entity associated side effects). The invention contemplates that targeting a cell which localizes to a disease site will better localize the independent effect of the targeting ligand to that locale. For example, an antibody which binds to and neutralizes a cytokine or other BEL associated with Crohn's disease eg. TNF alpha at the disease locale if targeted to an activated CD4+ T-cell using a marker which identifies activated T-cells.

In another aspect the invention is directed to a heterofunctional ligand comprising a first moiety which specifically binds to at least a first target ligand on a first entity and a second moiety which specifically binds to at least a second target ligand on a second entity, and wherein the affinity or avidity or both the affinity and avidity of said first moiety are selected to enable the first moiety to bind to the at least one first target ligand independently of the ability of said second moiety to bind to the at least one second target ligand and wherein the avidity or affinity or both the affinity and avidity of said second moiety are selected to enable the second moiety to bind to the second entity in preference to the first moiety binding to the first entity when both first and second moieties are substantially contemporaneously bound to the respective first and second entities. In one embodiment the first moiety comprises at least one ligand preferably at least one antibody which binds to a first cell, for example an intraluminal lymphatic endothelial cell and the second moiety comprises a ligand, preferably at least one antibody which binds to a different cell, for example a disease associated cell (hereinafter exemplified and meaning, unless the context implies otherwise, diseased cells or disease causing, mediating (ie. having a role whicn is known to be intermediary or indirectly facilitating eg. antigen presenting cells) or mitigating cells (cells, typically immune cells, which directly or indirectly counteract the diseased or disease causing or mediating cells). In other aspects the invention is directed to a pharmaceutical composition comprising such a heterofunctional ligand and a pharmaceutically acceptable carrier, a method of using the heterofunctional ligand in the preparation of a pharmaceutical composition for treating a disease, and to a method of treating a subject by administering same in a therapeutically effective amount.

In another aspect the invention is directed to a multispecific ligand comprising a first moiety which specifically binds to at least one first target ligand on a first entity (eg. a lymphatic endothelial cell, a diseased cell or a cell proximal to a site of disease) and a second moiety which specifically binds to a second target ligand or site on a second entity, and wherein the second entity binds to a third target ligand, and wherein the first and third target ligands may be on the same or different entities eg. the same or different cells, and wherein preferably the affinity or avidity or both the affinity and avidity of said first moiety are selected to enable the first moiety to bind to the first target ligand independently of the ability of said second moiety to bind to the second target ligand and independently of the ability of the second moiety to bind to the third target ligand (the first moiety optionally comprising more than one ligand (which may be the same ligand or a different ligand) one or more of which are necessary for binding and optionally each of which is sufficient for specific binding) to corresponding first target ligands) and preferably wherein 1) the avidity or affinity or both the affinity and avidity of said first moiety is/are selected to enable it to bind to the at least first target ligand in preference to the second moiety binding to the third target ligand when both said first and second moieties and the second entity are substantially contemporaneously bound to their respective target ligands eg. to effect a tranfer or 2) wherein the avidity or affinity or both the affinity and avidity of said second moiety for the second entity are selected to enable the first moiety to bind to the first entity in preference to the second moiety binding to the second entity and/or 3) wherein the avidity or affinity or both the affinity and avidity of said second moiety for the second entity are selected to enable the second moiety to bind to the third target ligand in preference to the second moiety binding the second entity when both first and second moieties are substantially contemporaneously bound to the respective first and second entities and the second moiety is substantially comtemporaneously bound to the third target ligand), or 4) wherein both 1) and 2) above are both operative conditions. In one embodiment, the first entity is a diseased or disease causing, mediating or mitigating cell, for example an immune cell (the first moiety preferably binding to a particular population or sub-population of the first target entity eg. the immune cell, for example activated T cells), the first moiety optionally comprising two or more ligands which may be the same or different and which bind to two or more respective first target cell surface ligands (though not necessarily to any particular effect (and in one embodiment to no effect at all) *other than* to better bind to and thereby target the cell, preferably in competition with the second entity, which in a preferred embodiment targets a broader population of cells), and the second entity eg. a biologic effectore ligand is an entity that binds to a third target ligand, the third target ligand preferably being expressed on the surface of a cell for example the same immune cell, for example a natural cell surface ligand, to which binding yields a desired effect, for example a therapeutic advantage, the second moiety being, for example, the natural ligand for the cell surface ligand or functional mimitope or antagonist or agonist thereof, for example a cytokine, the third target ligand in this,case being a cytokine receptor on the immune cell. The invention is also directed to a method of "targeted delivery" of a therapeutic entity to a cell in need of such therapy by administering said heterofunctional ligand. In this respect numerous therapeutic entities will be apparent to those skilled in the art, only some of which are mentioned herein by referring to the therapeutic entity itself or by referring to the third target ligand for which such entity is known and available or readily made by routine skill in the art. Optionally the heterofunctional ligand (and similarly in the case of other multispecific ligands of the invention described above which are adapted to deliver a BEL) is delivered with the second entity, preferably in the same composition (preferably bound). In the case where the second entity is a natural ligand circulating in the path of delivery of the heterofunctional ligand, some proportion (0-100%) of the heterofunctional ligand may be delivered without supplied second entity, particularly if the treatment or the disease generates an abundance of the natural ligand. In another embodiment the first moiety binds to a target ligand on a stationary cell (for example a vascular endothelial cell or a lymphatic endothelial cell), preferably a tissue or cell type "associated" ligand (more abundantly expressed uniquely expressed on target cells relative to non-target cells), and the third target ligand and the second moiety are cell-suface target and ligand therefor as stated above, for example the second moiety binds to a cytokine and the third target ligand is a cytokine receptor, for example on an immune cell. In one embodiment the first moiety binds to at least one target ligand which differentiates between populations or sub-populations of immune cells and the second entityin virtue of its binding to the third target ligand, directly or indirectly exerts a therapeutic effect, for example by modulating the activity of said immune cell. In another or further preferred embodiment the first moiety is incapable of modulating the activity of said immune cell and said second entity modulates the activity of said immune cell independently of said first moiety. In another or further preferred embodiment the second entity binds to a molecule involved in cellular adhesion, a cytokine receptor, a ligand which stimulates the activity of said immune cell, a ligand which inhibits the activity of said immune cell (eg. via anergy or tolerance mechanisms), a ligand which causes one or more cytokines to be released, a ligand which prevent one or more cytokines from being released, a ligand which causes or facilitates apoptosis of said immune celll, a ligand which permits internalization of said heterofunctional ligand. In another preferred embodiment the heterofunctional ligand is fused or conjugated to a therapeutic agent or a moiety (eg. biotin, avidin) that binds to a therapeutuc agent (exemplified below) or a ligand which effects binding to another immune cell, for example a T cell. In another preferred embodiment, the heterofunctional ligand is a bispecific antibody, a trispecfic antibody or a tetraspecific antibody. In another preferred embodiment the heterofunctional ligand further comprises a moiety that binds to at least one ligand located on the intraluminal surface of a lymphatic vessel, preferably a lymphatic vessel associated ligand, as hereinafter defined.

In other aspects the invention is directed to a pharmaceutical composition comprising such aforementioned heterofunctional ligand and a pharmaceutically acceptable carrier, a method of using the heterofunctional ligand in the preparation of a pharmaceutical composition for treating a disease, and to a method of treating a subject by administering same in a therapeutically effective amount. As suggested below, the foregoing strategy could be used in combination with other targeting strategies herein mentioned or known in the art. The invention contemplates making antibodies to second entities, for example, while bound to their natural receptor, by phage or ribosome display, by methods as hereinafter disclosed.

In another aspect the invention is directed to a heterofunctional ligand comprising at least a first moiety which specifically binds to a first target ligand on a cell and a second moiety which specifically binds to at least a second target ligand on the same cell, and wherein the affinity or avidity or both the affinity and avidity of said first moiety and the affinity or avidity or both the affinity and avidity of the second moiety are selected to substantially reduce the probability of the either moiety singly binding to its respective ligand for a sufficient duration or series of durations to accomplish the function of said heterofunctional ligand unless both first and second moieties are substantially contemporaneously bound to the cell. In a preferred embodiment the first moiety binds to at least one target ligand which differentiates between populations or sub-populations of immune cells and the second moiety in virtue of its binding to the second target ligand, directly or indirectly exerts a therapeutic effect, for example by modulating the activity of said immune cell. In another or further preferred embodiment the first moiety is incapable of modulating the activity of said immune cell and said second moiety modulates the activity of said immune cell independently of said first moiety. In another or further preferred embodiment the second moiety binds to a BEL, for example a molecule involved in cellular adhesion, a cytokine receptor, a ligand which stimulates the activity of said immune cell, a ligand which inhibits the activity of said immune cell (eg. via anergy or tolerance mechanisms), a ligand which causes one or more cytokines to be released, a ligand which prevent one or more cytokines from being released, a ligand which causes or facilitates apoptosis of said immune celll, a ligand which permits internalization of said heterofunctional ligand. In another preferred embodiment the heterofunctional ligand is fused or conjugated to a therapeutic agent or a moiety (eg. biotin, avidin) that binds to a therapeutuc agent (exemplified below) or a ligand which effects binding to another immune cell, for example a T cell. In another preferred embodiment, the heterofunctional ligand is a bispecific antibody, a trispecfic antibody or a tetraspecific antibody. In another preferred embodiment the heterofunctional ligand further comprises a moiety that binds to at least one ligand located on the intraluminal surface of a lymphatic vessel, preferably a lymphatic vessel associated ligand, as hereinafter defined. In other aspects the invention is directed to a pharmaceutical composition comprising such a heterofunctional ligand and a pharmaceutically acceptable carrier, a method of using the heterofunctional ligand in the preparation of a pharmaceutical composition for treating a disease, and to a method of treating a subject by administering same in a therapeutically effective amount

In other aspects the invention is directed to a method of in vivo modeling or testing using one or more foregoing targeting strategies by administering a heterofunctional / multifunctional ligand as hereinbelow disclosed as well as a method of intra-lymphatic drug delivery employing such ligand and such strategies including adaptations thereof for such purposes, as hereinafter described. In related aspects the invention is directed to a test ligand in the form of such a heterofunctional / multifunctional ligand and compositions thereof.

In one aspect, the invention is directed to a heterofunctional ligand, comprising a first moiety which specifically binds to at least one ligand located on the intraluminal surface of a lymphatic vessel and a second moiety which specifically binds to a disease associated cell and the use of such heterofunctional ligand in treating or preparing a pharmaceutical composition for treating disease associated cells, including diseased cells or disease causing, mediating (ie. having a role whicn is known to be intermediary or indirectly facilitating eg. antigen presenting cells) or mitigating cells (cells, typically immune cells, which directly or indirectly counteract the diseased or disease causing or mediating cells), within a lymphatic vessel. Preferably, the ligand located on the intraluminal surface of a lymphatic vessel is a lymphatic vessel associated ligand.

In another aspect, the invention is directed to a pharmaceutical composition comprising a pharmaceutically acceptable carrier and a heterofunctional ligand comprising a first moiety which specifically binds to a ligand located on the intraluminal surface of a lymphatic vessel and a second moiety which specifically binds to said disease associated cell' and the use of such ligand in treating treating disease associated cells, including diseased cells or disease causing or mediating cells, within a lymphatic vessel. Preferably, the ligand located on the intraluminal surface of a lymphatic vessel is a lymphatic vessel associated ligand.

In another aspect, the invention is directed to a method of treating disease associated cells, including diseased cells or disease causing or mediating cells, within a lymphatic vessel comprising administering to a subject a heterofunctional ligand comprising a first moiety which specifically binds to a ligand located on the intraluminal surface of a lymphatic vessel and a second moiety which specifically binds to said disease associated cell.

It is to be understood that disease causing cells as used herein includes diseased cells and pathogens, including micro-organisms and viruses.

In another aspect, the invention is directed to a heterofunctional ligand, comprising a first moiety which specifically binds to at least one ligand located on the intraluminal surface of a lymphatic vessel and a second moiety which specifically binds to a therapeutic entity for example a cytotoxin or cytotoxin-linked-entity or a non-toxic entity which is present in toxic amounts and to a method of reducing the toxic effect of such entity in a subject by administering said heterofunctional ligand to said subject.

In another embodiment the invention is directed to a method of therapeutic evaluation and/or targeting / intervention in which such heterofunctional ligand is administered substantially contemporaneously with a cytotoxic substance for example a cytotoxic substance useful for treatment of cancer. The term substantially contemporaneously is used in this connection to mean in a time frame that permits both to exert their respective effects, preferably one or both exerting their respective effect optimally, or one exerting its effect dominantly. It will be appreciated that this might entail that one such entity is advanced in its delivery over the other. Optionally, one or both of these cooperating entities are delivered proximally to their respective target cells, for example by cannulating one or more blood vessels as proximally as possible to the site(s) of a tumor and/or actual or anticipated site(s) of metastases (as discerned by using one or more tumor and vascular imaging agents, for example, one or a combination two or more agents selected from a vascular opaquing agent, a radionuclide conjugated anti-angiogenic antibody, and a radionuclide conjugated anti-vascular endothelial cell marker antibody, which cannulation may occur for example in the course of initial surgical intervention with respect to the primary tumor site) and/or at the same time cannulating one or more lymphatic vessels (which may optionally be located which the help of a radionuclide conjugated anti-lymphatic vessel marker antibody) leading to or from such tumor sites or metastases. The invention contemplates that small sections of vascular prostheses, well known to those skilled the art (eg. Dacron types) may be grafted into those locations to permit a prolonged and secure attachment of such prosthesis to an intra-vascular cannula for secure delivery to such vascular or lymphatic locations for repeated and/or prolonged administration, optionally while the patient is mobile, optionally using one or more portable infusion devices, including micropumps designed for such purpose (see for example J Neurosci Methods 1997 Mar;72(1):35-8, US 5180365:)mplantable infusion device. See also Cancer: Principles and Practice of Oncology (infra). Numerous embodiments and improvements in vascular prosthesis and in such portable infusion devices and micopumps are described in the relevant scientific and patent literature known to those skilled in the art. The invention also contemplates delivering any multifunctional ligand herein disclosed in the above manner.

It is to be understood that targeting strategies employing the cooperative action of ligands with different affinities for their targets exemplified above, may preferably have affinities which differ, depending on the application and their avidity, by a factor of 20% up to a number of orders of magnitude which may one, two, three, four, five, six and even seven or eight order of magnitude, in order to achieve substantial advantage, as herefter detailed in connection with one such strategy.

In another aspect the invention is directed to a heterospecific ligand comprising a first moiety which specifically binds to at least a first disease associated ligand located on a diseased or disease causing, mediating or mitigating cell for example a cancer cell or an immune cell, as well as on non- diseased or disease causing, mediating or mitigating cells (non-target cells) and at least a second moiety which specifically binds to a second different disease associated ligand on the same cell and wherein each ligand is expressed on a substantially (see definition below) different, n-overlapping, subset of non-target cells, so that functional binding to a non-target tissue is substantially (see definition below) precluded. In another embodiment the functional affinities of the respective ligands may be selected in accordance with the strategies suggested above, to further facilitate targeting. In another embodiment, both different ligands are required for internalization. In other related embodiments, the heterofunctional ligand comprises at least two different pairs of binding moieties (eg. a trispecific or tetraspecific antibody which depending on its construction will permit 2, 3 or 4 such different pairs eg. a tetraspecific single domain type antibody (ie. consisting primarily of the heavy or light chain variable region or a functional fragment thereof) (see discussion below regarding its construction) allowing the greatest variation in such geometries and preferably simultaneous binding of more than one pair), wherein 1) at least three such ligands are expressed on a substantially (see definition below) different, preferably non-overlapping, subset of non-target cells, so as to further limit binding to non-target cells and/or 2) wherein at least two different pairs of ligands target a substantially different subset of cells within the same target population eg. different cells within the same tumor (eg. proliferating vs. non-proliferating cell - the respective amounts of the different types of cells will dictate the perecentage of the dose that will be targeted to one population or another). In other aspects the invention is directed to a pharmaceutical composition comprising such a heterospecific ligand and a pharmaceutically acceptable carrier, a method of using the heterospecific ligand in the preparation of a pharmaceutical composition for treating a disease, and to a method of treating a subject by administering same in a therapeutically effective amount. It will be appreciated that the foregoing general strategy can be accomplished with two or more different antibodies have differing and preferably non-overlapping normal ie. non-targeted cell distributions, preferably administered in the same composition and preferably cross-linked by biotin-avidin like complementary pairs to facilitate cross-linking for internalization or targeting of therapeutic agents. In a preferred embodiment each such independent antibody carries a different complimentary aspect of a toxic payload eg. a different liposome (or other payload carrying entity for example a micro or nano particle or sphere or albumin) which complement each other in virtue of their respective contents (eg. one carries the prodrug and the other the necessary converting enzyme).

In another aspect, the invention is directed to a multifunctional ("multi" meaning at least two) ligand having, at least, a first portion which binds to a lymphatic vessel associated ligand and a second portion, comprising an immune function-exerting moiety.

The term lymphatic vessel is used to facilitate broader reference to ligands (eg. antigens / receptors) present on cells bordering the intra-luminal pathway through the lymphatic system including preferably the lymphatic vessels and optionally also parts of the lymph nodes, and refers in the case of the lymphatic vessels, primarily (from a functional standpoint) to the intra-luminal cell surfaces (not necessarily to the exclusion of non-luminal surfaces) on the intra-luminal endothelial cells (not necessarily to the exclusion of non-luminal lymphatic endothelial cells) of those vessels.

The term 'associated' with reference to lymphatic vessels, is used to mean differentially expressed on the surface of endothelial cells of those vessels for targeting purposes, such as to accomplish an object of the invention, but unless otherwise expressly indicated in a particular instance, it is used limitatively, to reference ligands that are predominantly, if not exlusively, found on the aforementioned endothelial cell surface (as well as in lymph nodes), such that the first portion of the multifunctional ligand is for all intents and purposes functionally targeted to the intra-luminal surface of the lymphatic system. For instance, it is appreciated that the ligand in question may be targetted to a limited extent elsewhere eg. in the case of preferred LYVE-1 ligand discussed below, to parts of the spleen (which also provides a venue for immune cell interactions).

The invention is not concerned with imparting effects to or simply blocking a receptor on the intraluminal lymphatic endothelial cell. In this context, the multifunctional ligand of the invention is intended to exclude only, unless otherwise specifically stated in the claims, only those embodiments disclosed in WO 98/06839 or other references describing ligands, antagonists or antibodies which bind to a lymphatic vessel associated ligand or receptor (see examples of such references below), insofar as such embodiments comprise lymphatic vessel associated ligands as hereinabove limitatively defined, and to this limited extent only, the term therapeutic function exerting moiety or immune function exerting moiety preferably excludes: 1) an antibody *F_{c} receptor,* insofar as such limitation excludes from the scope of the multifunctional ligand (per se) aspects of the invention, substantially intact naked antibodies which simply bind to a lymphatic vessel associated ligands, as well as preferably excluding 2) *cytotoxins or drugs,* insofar as this excludes from the scope of the mutifunctional ligands of the invention an antibody or fragment thereof which is fused or conjugated etc. exclusively to a cytotoxic molecule (including an atom) or drug (ie. an antibody linked to a cytotoxin or drug only, which is not per se an or is not integrated with *an independent biologic* or immune function exerting component) so as to accomplish a function in relation to cells or other entities (including other multifunctional ligands) within the lymphatic system other than the cell or ligand to which the multifunctional ligand is anchored. Similarly, the invention is not concerned with multifunctional ligands which are adapted to be internalized into a lymphatic endothelial cell and the invention is specifically concerned with targeting a lymphatic vessel associated marker which does not promote internalization and/or in which the first portion has an affinity (high or medium) which limits this effect (ie. to a side effect)

In the same vein, the term immune function is broad in intent (as discussed below, and includes particularly any function, including binding, capable of being exerted by an ligand preferably an antibody (eg. multifunctional ligands which are bispecific antibodies) however it is to be understood that the invention and particularly the immune function exerting moiety does not have as an object (despite possible incidental effects) evaluating or exerting a disease responsive or immune function vis-à-vis ligands / cells lining the intra-luminal surface of the lymphatic system ie. insofar as such ligands have a role in disease (other than simple binding exclusively for anchoring purposes which is attributable not the immune funtion exerting moiety but to the first portion) *but rather,* as evident in preferred aspects of the invention, preferably an *independent biologic or* immune function which is not predicated on blocking the lymphatic endothelial receptor or treating cells bearing the receptor. ie. exerted vis-à-vis targets other than the lymphatic endothelium target, for example 1) in the case of stationary diseased cells or disease causing cells or molecules, targets at the site of the disease (which may optionally be effected, for example, in case of immunization or other immune cell stimulation, inhibition etc. in the lymphatic system); and 2) in the case of non-stationary diseased or disease causing cells or molecules, at the site of those cells / molecules including, preferably, within the lymphatic system, for example by binding to or signaling those cells in the lymphatic system.
1. In one embodiment, the first portion of the multifunctional ligand is an antibody.
2. In another embodiment, the immune function exerting moiety binds to a target ligand and thereby directly or indirectly accomplishes its effect (in whole or part). For example, the target ligand may be a cytokine, for example in order to target immune cells to the lymphatic system to assist in, diseased, disease causing or other target cell ablation or phagocytic type activity (eg. by the cytokine in turn binding to a ligand, for example on an immune cell having phagocytic activity) or exerting a chemotactic effect within the lymphatic system, or to mop up cytokines, for example, when released in toxic amounts due, for example due to effects of a disease or particular immunotherapy (such as anti-CD3 therapy; see for example USP 6193969, Kummer U. et al., Immunol Lett 2001 Jan 1; 75(2):153-158) (with respect to removing disease associated antibodies from circulation see for example a bispecific dsDNAx monoclonal antibody construct for clearance of anti-dsDNA IgG in systemic lupus erythematosus. J Immunol Methods. 2001 Feb 1; 248(1-2):125-138). (see also, for example, US 5,968,510 with respect to antibody-CTLA-4 fusion proteins for use in binding to various target ligands).
3. In another embodiment, said immune function exerting moiety comprises an antibody and optionally both the first portion and the immune function exerting moiety are antibodies (with respect to bispecific antibodies, and a recent review of some of the technologies referred to or applicable to various aspects of the invention (see particularly, Journal of Immunological Methods February 2001 Vol. 248(1-2) page 1-200)
4. In another embodiment, said immune function exerting moiety binds to an immune cell, a diseased host cell or a disease causing cell or entity (eg see US6193968). The term disease is used broadly to refer to any undesirable condition. The term diseased host cell includes but is not limited to a cancerous (in the broadest sense of that term) cell and a virally infected cell (these examples are given inasmuch as the invention in a preferred embodiment involves targeting such cells for destruction) and the term disease causing cell includes but is not limited to a virus or other infectious agent and as well as immune cell which is directly or indirectly involved in mediating or causing a undesired, deleterious or pathologic consequence, including but not limited to autoimmune disorders, transplant rejection, and other immune system linked diseases. The term disease causing entity is used to refer, without limitation, to any molecule, atom, peptide, ligand, complex, chemical, component, epitope etc. that is directly or indirectly involved or associated in mediating or causing a disease or disease causing event including an antibody. Such binding to the entity may be effected through the instrumentality of one or more (same or different) multifunctional ligands and through binding to any ligand or set of ligands, including receptors, multi-component epitopes etc, including for example, tumor *"associated"* (ie. differentially expressed to advantage for targeting purposes) epitopes which may or may not or may only be partially present on tumor associated antigens, or commonly, for example antigens / epitopes / ligands / receptors etc. which are over-expressed in association with cancer cells; or for example, antigens / epitopes / ligands / receptors etc. involved in immune signaling, stimulatory, co-stimulatory, inhibitory, adhesion or other interactions, including without limitation, cytokine receptors, ligands associated with immune cell adhesion (see for example US 5,747,035), ligands to which binding results in stimulation, activation, apoptosis, anergy or costimulation, or ligands which differentiate between different populations or subpopulations or immune cells, including sub-populations of B cells and T cells, activated versus non-activated lympocytes, diseased or disease-causing cells versus non-diseased / disease causing lymphocytes and specific immune cell clones for example those having specific Ig type and MHC-peptide type ligands / and correlative ligands. Examples of such ligands include CCR5, CTLA-4, LFA-1, LFA-3. ICAMs eg. ICAM-1, ELAM-1, CD2, CD3, CD4 (eg see US 6,136,310), CD5, CD6, CD18, CD22, CD40, CD44; CD80, CD86, CD134 and CD154, to name only a few (see also US6087475: PF4A receptor, US6135941, WO 01/13945 Such ligand may also selectively be targeted using any dual affinity strategy according to the invention..
5. The invention is also directed to a multifunctional ligand and a method which comprises using the multifunctional ligand to assess the toxicity of directly or indirectly targeting, for example, primarily within the lymphatic vessel system (see discussion below), cells having well known markers that are associated with immune cells, for example, those exclusively associated with activated immune cells, in-so-far as such targeting has a role in prolonging or counteracting the activated state, destroying the cell (eg. where the multifunctional ligand is a immunotoxin) causing the cell to be destroyed (eg. through apoptosis (eg. see WO 01/19861, fas - fasL, U.S 6,046,048) or assisting another molecule or cell for example a T-cell or other killing or immune modulating cell to do the modulation or killing (markers such as CD23, CD25, CD26, CD28, CD30, CD38, CD49a, CD69, CD70, are just some of the markers associated with activated immune cells) etc. (for a complete listing of marker associated with activated immune cells see for example Roitt I et al. Immunology, sixth edition, Mosby 2001 referenced below and Encyclopedia of Immunology (1998), Abbas et al. Cellular and Molecular Immunology 2000, Harcourt & Brace, the contents of which are incorporated by reference herein). Antibodies for many such ligands are known or could be readily made by eg. phage display (see references herein including J Immunol Methods 1999 Dec 10;231(1-2):65-81), and natural ligands for such markers or functional analogues thereof are in some cases known or could be made by recombinant DNA technologies referenced herein (see also Cellular & Molecular Immunology 4th Edition, Abbas Ak et al. WB Saunders and Company 2000, Antibody Fusion Proteins, Steven M Chamow , Avi Ashkenazi Eds. ISBN 047118358X May 1999 Wiley; Kontermann, R., et al.(Eds.) Antibody Engineering, Springer 2001. ISBN 3-540-41354-5; Antibody Engineering, Carl A. Borrebaeck Oxford University Press, 1995; Antibody Engineering:A Practical Approach David J. Chiswell, Hennie R. Hoogenboom, John McCafferty Oxford University Press,1996; Antibody Engineering Protocols, Sudhir Paul (1995) Humana Press; Antibody Expression & Engineering (1998) Henry Y. Wang, Tadayuki lmanaka, American Chemical Society). The term modulation is used broadly to refer to any change, directly or indirectly, in an immune function or effect, as broadly understood. Many such forms of modulation are well known in the art (some are exemplified herein), and therefore these need not be specifically recited (for a review of such effects see for example Roitt I et al. Immunology, sixth Edition, Mosby 2001; Encyclopedia of Immunology ; (1998) Morgan Kaufmann Publishers, ISBN:0122267656).
6. In one aspect the invention contemplates that the multifunctional ligand exerts its function substantially (ie. upon gaining entry into lymphatic system and when bound to the lymphatic endothelial cells, which is dependant on the mode of administration) within the lymphatic system, *on cells and*/*or molecules circulating through the lymphatic system,* for example with respect to some embodiments, for greatest effect, to avoid an undesired degree of immunosuppression (for example, embodiments where immune cells are targeted for ablation and/or apoptosis). Preferably, such effect, is exerted at least in part, and preferably substantially to the exclusion of regions within lymphatic system that house at the time of administration non-circulating cells (eg. thymus, bone marrow, and various parts of the secondary lymphoid tissues) or/and with respect to some embodiments (excluding for example those related to immunization or mopping up toxins or antibodies) preferably, non-activated cells. This specificity of targeting can be accomplished in part to the natural distribution of the lymphatic endothelium associated marker of choice, the mode of administration and various targeting strategies herein described.
7. For example, the invention contemplates modes of delivery that to *varying degrees* ensure a greater degree of lymphatic system targeting, for example administration directly within the lymphatics, adminstration in tissues that drain to the lymphatics or parts thereof, intravenous delivery, as are well known to those skilled in the art, preferably in each individual case at strategic sites of administration that are most pertinent or selective for the disease in question, to the extent that selectivity is desired. The invention contemplates a variety of different size multifunctional ligands (MRU, single domain, scFv, Fab, minibodies, F(ab)₂, F(ab')₂, substantially whole antibodies etc. and known or obvious multimers thereof referenced herein and in the referenced literature) that are most suitable (eg. for small enough or, for example, having longest half life in circulation) for particlar modes of administration to the extent that this is a limitation (eg. size, where drainage into the lymphatic system is sought to be increased or optimized).
8. In a preferred embodiment the invention contemplates that the immune function exerting moiety of the multifunctional ligand comprises (eg. by way of recombinant fusion, conjugation etc.), or binds to (such antibodies are known or may be made by phage, ribosome or other such 'display' methods), so as to present the functional part of an adhesion molecule (molecule involved in cellular adhesion), for example an endothelial adhesion molecule such as a selectins, ICAMs (eg. ICAM-1, ICAM-2) V-CAM, MAdCAM-1 or functional analogues or portions thereof (see for example USP6143298, 5512660, 5861151, 5489533, 5,538,725, 6037454, 5565550, Circulation 2001 Feb 27; 103(8):1128-1134, and specific examples/references recited below) in order to control cell traffic including facilitating cell anchoring within the lymphatic system, including for example to facilitate interaction with another "arm" (functional moiety) of the multifunctional ligand or a second etc.multifunctional ligand or an immune cell (or a celf-sized latex sphere as described herein - for this pupose the adhesion molecule may be on the surface of another, preferably multifunctional-ligand-anchored latex sphere or on a similarly anchored cell) as well as combination therapies, for example, with therapeutic entities that enhance or inhibit leucocyte adhesion, or multifunctional ligands or antibodies that bind to one of their corresponding ligands on immune cells (eg. intergrins) or other ligands eg. CD44, to facilitate control and/or some selectivity of cell entry into the lymphatic system, for example, for reactivity with the multifunctional ligands of the invention. The invention also contemplates that such adhesion molecules may be the subject of targeting with dual affinity ligands of the invention and that such ligands may include a moiety which binds to a lymphatic endothelial cell.
9. The invention also contemplates that one or more multifunctional ligands in which the immune function exerting moiety comprises an antibody type molecule targeted to a particular cell surface ligand may be able to mimic effect of such adhesion molecules, as discussed below (any such discussion of an antibody mimicking this function is unless otherwise stated not intended to limit the broader concept of utilizing any class of molecule that would facilitate anchoring or controlling, eg. slowing the passage of cells through the lymphatic vessels). It is to be understood that there may be limitations in the number of cells that can be targeted for ablation in the lymphatic system by slowing the passage of cells, particularly for the purpose herein specified of allowing them the requisite period of residence within the lymphatic system for immune cell targeting or interaction or prolonged interaction with multifunctional ligands of the invention for binding purposes while bound to the lymphatic system endothelium, for example, certain end stage lymphomas/leukemias. In this particular context it is to be understood that: 1) the invention may have greatest application when the multifunctional ligand is administered so as to primarily target cells within the circulatory system, or as an adjunct therapy, or for remission or near remission conditions, or when combined with hyaluronic acid therapy. For example, the invention contemplates that an effective amount of hyaluronic acid is pre-administered to tissues draining to the lymphatic system so as to initially occupy binding sites on LYVE-1 primarily in the smallest lymphatic vessels and thereby minimize excessive binding within the narrowest vessels.
10. In a preferred embodiment said first portion binds to LYVE-1 or podoplantin described below. The anti-lymphatic marker antibody may be fused for example to a liver enzyme which is known to metabolize chemotherapetic agents or an affinity matured variant thereof, so as to to degrade such agents that are not taken up by the diseased tissue.to which they are targeted.*
11. In a preferred embodiment, said first portion is fused, conjugated or otherwise linked directly or indirectly to an immunizing moiety, for example an antigen, epitope, mimotope or peptide etc. presenting/incorporating entity/scaffold that generates by itself or with the help of one or more cytokines, costimulatory molecules and/or adjuvants etc. an immune response to a desired antigenic determinant (this term is used broadly to correspond at least in scope to the overlapping groupings: antigen, epitope, mimotope or peptide), for example an anti-idiotypic antibody, an antibody component which is capable of binding to a T cell activating entity for example a cell (eg. an APC see Int Immunol 2000 Jan; 12(1):57-66 or other cell having eg. immune modulating activity eg. see USP 6,004,811 ) which is for example genetically engineered to express relevant ligands for activating (or with respect to functions not necessarily related to immunizing, anergizing, tolerizing or otherwise modulating the activity of), an immune cell for example a B cell or T-cell, for example an MHC-peptide and B7 co-stimulatory molecules for activation of T-cells ( see for example Proc Natl Acad Sci U S A 2001 Jan 2; 98(1):241-246 see also Tham EL et al. J of Immunological Methods Vol. 249(1-2)(2001) p111-119 with respect to latex spheres that can be used for this purpose), or for example a CTLA-4 scaffold, a peptide fused to an Fc domain (see WO 01/18203) a HSP-peptide complex/conjugate, an MHC protein or peptide complex etc. Antibody-MHC complex fusions and antibody-B7 costimulatory fusion molecules are known and the invention contemplates that fusion molecules with anti-lymphatic marker antibodies could be made and used together for immunization purposes. For example the invention contemplates that two Fabs (or linked Fvs, with linker extensions of suitable lengths comparable (i.e. in a range of up to two fold shorter/longer when compared to extensions used in the art see Park et al. A divalent recombinant immunotoxin formed by a disulfide bond between the extension peptide chains. Mol Cells. 2001 Dec 31;12(3): 398-402) which recognize a lymphatic endolethelial marker can be disulfide linked and fused respectively to an MHC petides and a B7 molecule) It is also contemplated that the absence of costimulatory molecules for presentation in a co-stimulatory fashion with an MHC peptide complex will cause a tolerizing effect. Accordingly the invention is also directed to a multifunctional ligand comprising an immune function exerting moiety which comprises an MHC, preferably complexed or otherwise linked to a peptide. Peptide linking may for example be effected independently, naturally or for example through causing release of peptides from an MHC peptide or HSP peptide complex by injecting a weak acidic solution into tumor eg. just prior to excision. Suitable such solutions which may for example be combined with a cytokine, eg.IL-12 and/or adjuvant are known in the art.
12. In a preferred embodiment said immune function exerting moiety comprises an anti-idiotypic antibody, for example an antibody that a) mimics, for example, a cell surface expressed tumor associated epitope, a virus or other infectious agent associated surface epitope, a toxin, an immune stimulatory, costimulatory, inhibitory, or otherwise interactive ligand; or b) serves to bind to the idiotype (ie. paratope) bearing antibody to which it binds as an anti-idiotype, for example an autoimmune antibody, etc. or an antibody bearing a toxic moiety for removing such antibody from passage into the circulation.
13. In a preferred embodiment, the invention contemplates that the first multifunctional ligand is used for development, therapeutic evaluation or combination therapy in conjunction with a second different multifunctional ligand of the invention, to achieve a cooperative effect (for example, in the same composition or substantially contemporaneously administered (ie. to reach the same or an interrelated destination in a cooperative time frame) or in necessary or desired sequence/interval, etc.). An example of such cooperative effect is an interaction (not necessarily simultaneously) with two different immune cell surface ligands (for example via an antibody binding interaction), or to deliver different payloads eg. toxins, to a diseased cell see (USP 6,077,499). The invention also contemplates a method of effecting substantially coordinated interactions of differing temporal and spatial complexities, ranging from a somewhat proximal and contemporaneous delivery (eg. in the same composition) of a first multifunctional ligand having, for example, a cancer cell binding second portion, and a second multifunctional ligand having, for example, a cytokine binding Ab, eg. to reduce any toxic effects associated with toxic levels of cytokine release, a cytokine component (for example to harness the effect of such component as a means to attract one or more immune cells to kill a diseased cell or to harness the inhibitory effect of such component (eg. using one or more cytokines employed by cancer cells to evade immune cell targeting) eg. on undesired immune cell elimination or immune cell elimination of the multifunctional ligand, or a T-cell binding component (eg. anti-CD3) to harness the effects of such component on cancer cell killing optionally with a concomitant object of assessing possible counterproductive immune cell elimination (eg. as would be enabled by using a radiolabelled multifunctional ligand and determining the disposistion of the label over time) of the multifunctional ligand.
14. Also contemplated are methods to implement more spatially and/or temporally sensitive interactions. For example, when administered in empirically determined suitable proportions and in empirically determined sufficient total amounts for, at least, partial and/or local lymphatic-vessel-associated-ligand saturation or partial saturation to achieve proximal binding of a first to second multifunctional ligand (having regard to the route of administration eg. local saturation can be more readily accomplished by administration into the lumen of the lymphatic vessel). Two different such multifunctional ligands may be used, for example, to deliver two different immune function exerting moieties in substantial proximity to one another for contemporaneous interaction with two different ligands on an immune cell (ie. when it approaches the luminal wall of a lymphatic vessel). For example, this approach may be used to implement one or more effects including increased avidity to the cell for prolonged cell anchoring, which may positively impact on desired (in some embodiments) transfer of the multifunctional ligand from the lymphatic vessel wall to the target cell eg. for achieving an inhibitory effect via ligand binding (eg. assessed via duration of multifunctional ligand binding eg. quantitative or radioimage approximated label elimination)(N.B. this effect may be assessed with multiple copies of the same multifunctional ligand), delivery of a cooperative payload eg. different entities which contribute to the same or a different mechanism of cell killing, counterparts in a two component interaction (biotin-avidin), which preferably yields evidence (preferably quantifiable evidence) of the interaction, for example an enzyme-substrate interaction to quantitatively assay the amount of an enzyme converted substrate (eg. using a conjugated prodrug and pro-drug conversion akin to ADEPT and assessing the extent of prodrug conversion eg by labeled anti-drug specific antibody). For example, the invention contemplates the use of a respectively linked catalytic antibody component (see for example US5658753:Catalytic antibody components) and labeled substrate or RNAase and labeled RNA etc.for this purpose. Another example, discussed in more detail below is the use of one multifunctional ligand for targeting (selectivity) purposes and another for implementing directly or indirectly a desired therapeutic effect, both ligands optionally being required to to give rise to a substantial probability of binding (the invention also contemplates that this strategy could be used with a single multifunctional ligand having two intra-luminally directed binding moieties).
15. The invention contemplates that such interactive entities may be conjugated fused or otherwise linked to a respective first and second multifunctional ligand for achieving a cooperative interaction between adjacently bound such ligands.
16. The invention contemplates that adjacently interacting multifunctional ligands yielding detectable evidence of the interaction, could be use in a method to assess eg. a) luminal ligand saturation for dosing, b) multiple simultaneous binding interactions, and c) perhaps most spatially sensitive, development of a process to achieve cross-linked binding with multiple eg. immune cell ligands eg. a costimulatory immune effect (ie. the effect of different simultaneous interactions eg. on stimulation, inhibition etc.of eg. an immune cell for example combining a first multifunctional ligand capable of selectively binding to, conjugated to or fused to a B7component (see J Immunother 2001 Jan-Feb; 24(1):27-36; J Immunol 2001 Feb. 15; 166(4):2505-2513; Chalitta PM et al. J. Immunol. 160:3419-3426) and a second multifunctional ligand capable of selectively binding to, conjugated to or fused to an MHC molecule delivered initially with or without peptide. For example, the invention contemplates using various amounts/proportions of multifunctional ligands having antibody components fused or conjugated to or capable of binding selectively to, for example an MHC class I or II peptide complex and recombinant B7-1-Fc and/or B7-2-Fc respectively (see Eur J Immunol 2001 Jan; 31 (1):32-38; Eur J Immunol 2001 Feb; 31(2):440-449) (for tumor reactive peptides see for example J Immunother 2001 Jan-Feb; 24(1):1-9). In this latter connection (cross-linking type interaction), and/orfor permanence of binding or ease of attaching other cooperative entities (for example biotin coated or conjugated radionuclide, liposomes or other payload carrying entities (eg. see for example US patents 5439686, 6007845, 5879712, 5456917, 6165502, 5079005, 5888500, 5861159, 6193970, 6190692, WO 00/69413, WO 01/07084) the invention contemplates biotinylating the two multifunctional ligands and linking the two biotinylated cooperative mulfunctional ligands with avidin, streptavidin (or other modified forms thereof eg. deglycosysylated avidin or using other complementary linking components- see eg. US Patent (USP) 6,077, 499).
17. The invention also contemplates enhancing the cross-linking of the multifunctional ligands of the invention through complementary components such as biotin and avidin.
18. Preferably, with respect to, for example, increasing selectivity of targeting certain cells (eg. to induce immune tolerance), the invention also contemplates that a first multifunctional ligand is used to bind to a marker specific to a particular kind of cell (eg. activated immune cells) and a second multifunctional ligand (which may not be specific for activated immune cells) is used to modulate the activity of the immune cell (for example inactivate it or reduce its disease causing capability directly or indirectly by binding to it ). For example, where the marker is used to determine the selectivity of the targeting but cannot be used for modulating its activity, it is contemplated that the functional affinity of one or both the first portion and second portions of one or both of the cooperating multifunctional ligands can be selected to at least partially control the selective modulating effect of the pair, for example both interactions would be required for the second multifunctional ligand to have an optimal opportunity to bind. For example, the functional affinity for the target cell is relatively weak for the purpose of attaching to the eg. immune cell for a sufficient duration (eg. so as to yield the effect of becoming attached to the immune cell in preference to the lymphatic vessel), compared with that of the first multifunctional ligand (ie the one that accomplishes the selective recognition through binding) to *reduce the likelihood* that the second moiety will bind in the absence of binding of the first moiety (notably a similar type of coordinated interaction ie. two binding interactions, is naturally used for cell adhesion). (NB. this type of coordination has application ie. both specificities are optimally required for binding, to a single multifunctional ligand, having a divalent immune function exerting moiety eg a triabody or tetrabody or for cross-linking and other types of coordinated interactions). In a preferred embodiment, if transfer of binding of the first multifunctional ligand to the immune cell is not desired its functional affinity of the first portion to the lymph vessel can be greater than that of its second portion, while the reverse could be true for the second multifunctional ligand. It will also be appreciated that antibodies which cross-link for example an integrin and a marker of immune cell activation could be used to limit the number of activated immune cells that migrate through the lymphatic system. For example bispecific d Abs, diabodies, etc. in which the functional affinity of each specific binding portion individually does not srongly favour binding, could be used to selectively target specific sub-populations of immune cells or even specifically activated immune cells (for example antibodies that recognize particular antigen / peptide specific T cell or B cells).
19. Accordingly, more generally speaking, the invention is directed a bispecific ligand, preferably a bispecific antibody, having a first portion which binds to a ligand which differentiates between members of the same immune cell population (eg a particular type of T cell) any a second portion which binds to a second ligand on the same cell, which binding exerts directly or indirectly a desired effect, wherein the functional affinity of said first and second portions are selected so as to substantially increase amount of immune cells in which both such portions are bound to their respective ligands relative to those which a single such portion is bound to a single ligand and preferably wherein the amount of immune cells to which the bispecific ligand is not bound is substantially greater than the number of immune cells that are not bound when compared to using a bispecific ligand having the same specificity and for example a 10¹ to 10⁷ (preferably 10¹ to 10⁶, preferably 10³ to 10⁶, preferably 10³ to 10⁵) increase in affinity of one or both portions. This invention also contemplates that binding to the ligand which differentiates between members of the same population (a particular type of T cell) does not have a negative consequence other than to cause the molecule to be ineffectual unless both of its portions are bound and that its binding is itself sufficient for binding and/or stronger relative to the second portion by two fold to 5 orders of magnitude ,preferably 1 to 3 orders of magnitude. The term substantially greater imports medical significance and may preferably be 15% - 10000% greater. The foregoing examples are not meant to be limitative.
20. In a preferred embodiment, the invention more broadly speaking contemplates a two ligand interaction (using one or more multifunctional ligands) wherein for example both are required or increase the likelihood of interaction and wherein the interaction of at least one contributes to specificity, though not necessarily to modulation, thus permitting a broader selection of modulators including those that but for the selectivity enhancing effect of the cooperating ligand and the lymphatic system venue, would be toxic in the desired therapeutic dose. Examples of markers that could assist in selectivity include those are unique to, for example, activated B cells or T cells or those having particular specificites in virtue of unique Ig type receptors. Examples of ligands on, for example immune cells, through which modulation/inhibitionlstimulation etc. (including, for example apoptosis), for example by antibody binding or supply of a natural interactive ligand, are well known. Some examples are provided herein. Combinations and permutations of markers and ligands for selectivity and exerting an immune effect such as modulation/inhibition/stimulation referred to herein or in the literature incorporated herein by reference or well known in the art are contemplated to be within the scope of the invention.
21. It will be appreciated that a combination of factors, such as dose, using additional molecules that increase or decrease migration or adhesion optionally in a tissue targeted manner, route of administration (eg within tissue that best drain to lymphatic vesssels or a portion thereof), use of cytokines, etc. and immune modulating drugs, as well combination therapies with known entities, can be employed in various combinations for strategies of harnessing the unique properties of the multifunctional ligand of the invention, to achieve a selectivity enhancing and/or modulatory/inhibitory/stimulatory etc or otherwise cooperating effects with respect to the desired target population of cells. Unless their function are self-evidently conflicting the invention contemplates all permutations of the multifunctional ligands disclosed herein or in the literature incorporated by reference hererin as well as those evident to persons skilled in the art whose mention is omited.
22. In a preferred embodiment, the immune function exerting moiety binds with greater functional affinity to its target ligand than said first portion binds to its target ligand. For example said immune function exerting moiety may bind with greater avidity (preferably at least 2 times greater (divalent vs. monovalent) and lesser or greater affinity (eg. within a range of 1×10⁻⁶ to 1×10⁶ fold) or with the same avidity and greater affinity (eg. up to 1×10⁶ fold). In applicable aspects, the invention contemplates that this increased functional affinity can be employed to effect transfer of a lymphatic vessel bound multifunctional ligand (eg. a bispecific antibody) to a cell passing through lymphatic system. The invention also contemplates a method comprising radiolabelling the mutifunctional ligand to assess, for example, the degree to which immune cells at a disease site have passed through the lymphatic system. Certain aspects of the invention, discussed herein, relate to a multifunctional ligand based system of targeting a particular immune cell ligand for stimulation, inhibition etc. predominantly within select portions of the lymphatic system that contain migrating cells (although some general targeting can controllably occur before the multifunctional ligand binds to the lymphatic system or when the multifunctional ligand releases from the lymphatic system without having found its target within the lymphatic system) will have at least a partially selective effect on targeting disease causing/mediating immune cells (eg. activated with a specificity that causes the disease) as opposed to non-disease causing/mediating cells, in the case where such ligand is also expressed on such other immune cells eg. of the same type eg. T cells. This permits targeting of immune cells primarily within the portions of the lymphatic system that contain migrating cells particularly disease causing/mediating cells while minimizing immune system dysfunction. This effect can be even more selectively accomplished, for example, by delivering the multifunctional ligand directly into the lymphatic system and within a time frame which is shorter that the normal duration of binding of the multifunctional ligand determining the degree to which the multifunctional ligand is bound to such diseased related cells at the disease site and similarly the degree to which it is bound to the cells unrelated to the same disease eg. via radiolabel. As discussed more fully below, the invention also contemplates a multifunctional ligand based system of assessing the effects of certain types of immune stimulation eg. how stimulating enhanced migration or adhesion, will differentially affect disease activated cell migration through the lymphatic system to enhance such disease cell targeting within the lymphatic system. For example, for tumor cell targeting and stimulation of disease-activated immune cells the invention contemplates evaluating cytokine (eg. TNFα) linked anti-angiogenic marker antibodies, optionally, preferably in combination with anti-tumor vaccination strategies, to direct disease activated immune cells to tumor site and the lymphatic system for further immune stimulation. Based on a "bait and trap" type approach, ligands such as OX40L and CD44 may also be assessed for this purpose.
23. In this connection and more generally the invention also contemplates using a bi-specific antibody, for example having a lymphatic endothelial binding first portion and for example a cytokine binding second portion, wherein the cytokine binding portion has a lower functional affinity for the cytokine (for example 1 x 10 -6 to 0.9 fold) compared with that of its natural receptor on an immune cell. It is contemplated that a multifunctional ligand of the invention could be used optionally in conjunction with a multifunctional ligand which displays a functional adhesion molecule (a selectin, ICAM, etc.) to assess the optimal parameters for transfer of the cytokine, for example, as is known to occur by monitoring the effects of cytokine release attributable to such cytokine transfer. It will be appreciated that this information or approach could be used to optimize the binding parameters for other ligands as well (eg. anti CD3) and could be employed not only in lymphatic system but to locally deliver inhibitory or stimulatory cytokines or other ligands to certain tissue targets, for example new blood vessels forming within tumors or other tissue specific markers.
24. The foregoing strategies could be used as part of a primary, adjunct or low disease burden therapy.
25. In a preferred embodiment, the second portion comprises a ligand which is capable of binding to an immune cell for example B cells, T cells etc, preferably in one embodiment to assist in cell killing or immune modulation of a target cell (re NK cells see for example US 5770387)(see also US6071517:Bispecific heteroantibodies with dual effector functions; Bispecific antibody-mediated destruction of Hodgkin's lymphoma cells. J Immunol Methods 2001 Feb 1; 248(1-2):113-123; Bispecific antibody-targeted phagocytosis of HER-2/neu expressing tumor cells by myeloid cells activated in vivo.J Immunol Methods. 2001 Feb 1; 248(1-2):167-182 as well as J Immunol Methods 2001 Feb 1; 248(1-2):103-111).
26. With respect to avidity, affinity and other elements of design including size, blood clearance, additional functionality etc.the multifunctional ligand may be, for example, a bispecific antibody having a monovalent first portion and a monovalent second portion, a bispecific antibody having a divalent first portion and a divalent second portion, a trivalent trispecific antibody having a monovalent first portion and a second portion comprising a monovalent immune function exerting moiety which binds, for example, to a target ligand on a target diseased, disease causing or immune cell, and for example, a monovalent portion which binds to an immune cell which assists in killing or modulation for example anti-CD3 or anti-CD28 antibody component, a tetravalent trispecific antibody having a monovalent first portion and a second portion comprising a divalent immune function exerting moiety which binds, for example, to a target ligand on a target diseased, disease causing or immune cell, and for example, a monovalent anti-CD3 or anti-CD28 antibody component (it is contemplated that this orientation might advantageously position the anti-CD3 component for interaction with a T-cell almost exclusively when the first portion is not bound to the luminal wall of a lymphatic vessel), a trivalent bispecific antibody having a monovalent first portion and a second portion comprising a divalent immune function exerting moiety, for example, one which binds, for example, to a target ligand on a target diseased, disease causing or immune cell. The antibody subunit may be for example, a Fab, a substantially intact antibody, a single domain antibody (see also Hufton SE. Dis Markers 2000;16(1,2):37 Single domain human immunoglobulin fold-based biomolecules; Antigen specificity and high affinity binding provided by one single loop of a camel single-domain antibody. J Biol Chem. 2001 Jul 13;276(28):26285-90. Optimal Design Features of Camelized Human Single-domain Antibody Libraries. J Biol Chem. 2001 Jul 6;276(27):24774-24780; Recognition of antigens by single-domain antibody fragments: the superfluous luxury of paired domains.Trends Biochem Sci. 2001 Apr;26(4):230-5; Llama heavy-chain V regions consist of at least four distinct subfamilies revealing novel sequence features. Mol Immunol. 2000 Aug;37(10):579-90) a minibody an scFv or a minimal recognition unit (MRU eg see US6174691:Minimum recognition unit of a PEM mucin tandem repeat specific monoclonal antibody).
27. In a preferred embodiment, the multifunctional ligand binds to an immune cell which is associated with an autoimmune reaction, for example a CCR5-expressing cell. (see also Apoptosis genes and autoimmunity. Curr Opin Immunol. 2000 Dec; 12(6):719-24. for application herein)
28. In a preferred embodiment, the second portion comprises a cytokine component
29. In a preferred embodiment, the second portion comprises a cytotoxic component
30. In a preferred embodiment, the second portion of the multifunctional ligand comprises an internalizing antibody and a cytotoxic component.
31. In a preferred embodiment, the second portion consists of an antibody which binds to T cells, for example, an anti-CD3 antibody or an anti-CD28 antibody.
32. In a preferred embodiment, the second portion consists of a cytokine component.
33. In a preferred embodiment, the second portion comprises an antibody which binds to a target diseased, disease causing or immune cell and further comprises one or more components selected from the group consisting of a cytokine component, a cytotoxic component and an anti-CD3/CD28 component.
34. In another aspect the invention is directed to a composition comprising a multifunctional ligand and a pharmaceutically acceptable excipient.
35. In another aspect the invention is directed to a composition comprising a plurality of different multifunctional ligands.
36. In another aspect the invention is directed to methods and compositions for developing and evaluating the therapeutic value of stimulators, mediators, inhibitors etc. of immune cell signaling (eg. stimulatory, inhibitory, costimulatory), adhesion, migration,etc. including the effects of ligand/receptor blocking and supply of specific cooperative ligands, using the multifunctional ligands of the invention.
37. In a preferred aspect, the multifunctional ligands of the invention may be used to assess the effects of such compositions on the sub-populaton of cells that migrates into lymphatic vessels. In particular, the invention is directed to assessing the expectation that some disease causing, mediating or otherwise disease active immune cells have an enhanced ability/opportunity (and/or can be enhanced in their ability/opportunity to make their way into the lymphatic system) so that targeting of relevant ligands on that sub-population of cells within the lymphatic system will cause at least a partial selective targeting effect, preferably with positive effect on the dosing capability and choice of ligands ie. in terms of limiting more universal and/or deleterious consequences. The invention is also directed to a method of reducing the toxic side effects of a pharmaceutical composition comprising a multifunctional ligand in which the immune function exerting moiety is targeted to a ligand that is not found exclusively on disease causing, mediating or otherwise disease active immune cells, by administering said composition in a manner in which it enter more directly into the lumen of a lymphatic vessel. (It contemplated that immunization within the lymphatic system can also be enhanced in virtue of such selective targeting.) In particular, the invention is directed to a multifunctional ligand, a pharmaceutically acceptable composition therof and method of using same for assessing enhanced migration or enhancing migration of disease-active immune cells, said multifunctional ligand comprising an immune function effecting moiety which has an immune effect on an immune cell surface ligand ie. effects including signaling (eg. stimulatory, inhibitory, costimulatory, antagonistic, agonistic), including for adhesion and migration effects,etc. This may be accomplised practically, for example through ligand/receptor blocking eg. via antibody, or by antibody fusions/conjugates etc. that supply the natural ligand or a functional fragment or chemical/biological mimotope thereof. In a preferred embodiment the invention is directed to a multifunctional ligand in which the immune function exerting moiety is an antibody that binds to a ligand selected, for example from the group consisting of CTLA-4, IL-2 receptor, CCR5, CD44, CD134, CD3, CD28, CD2.
38. In another aspect the invention is directed to a composition comprising a plurality of different multifunctional ligands which exert a potentially cooperative immune effect with respect to an immune cell, for example binding to two or more different ligands on an immune cell, wherein said ligands are selected, for example from the group consisting of CTLA-4, IL-2 receptor, CCR5, CD44, CD134, from any of the ligands herein mentioned or referenced or preferably CD3, CD28, CD2.
39. The invention is also directed to a method of inhibiting metastasis during the course of surgical removal of a tumor comprising administering to a patient prior to surgical treatment of the tumor site, a pharmacetical composition comprising a multifunctional ligand in which the immune function effecting moiety binds to a tumor associated epitope on a cancer cell.
40. In another aspect the invention is directed to an immunocytokine having an anti-idiotypic antibody component which recognizes the paratope of an antibody which binds to a lymphatic vessel associated ligand and a cytokine component fused therewith or conjugated thereto. For example the cytokine component comprises IL-2 or a functional fragment thereof and/or IL-12 or a functional fragment thereof. In addition to their individual use in fusion proteins for tumor cell killing, combinations of II-2 and IL-12 have been used successfully for this purpose. It is contemplated that such cytokine fusion could be used to target T-cells or phagocytic cells to a multifunctional ligand that has bound to its disease causing or diseased cell target, preferably having left the lymphatic vessel endothelium in preference for binding its target. In this connection it is contemplated that the functional affinity of the anti-idiotypic Ab for the first portion would be less than that of the first portion to the lymphatic endothelium, so as to minimize competition between the two. It is also contemplated that the delivery of the immunocytokine occur substantially contemporaneously but separately and after that of the multifunctional ligand, optionally by a different route of administration.
41. Similarly the invention contemplates for the same purpose, a bispecific antibody having an anti-idiotypic antibody component which recognizes the paratope of an antibody which binds specifically to a lymphatic vessel associated ligand (preferably with lower affinity than that of the Ab for its target) and for example an immune cell binding portion eg. an anti-CD3 antibody or an anti-CD28 antibody component.
42. Thus the invention is directed to a method of targeting a diseased or disease causing cell for destruction by the immune system comprising administering separately but substantially contemporaneously to a subject hosting the diseased or disease causing cell, preferably in sequence with an interposed interval and/or by different routes of administration, first a multifunctional ligand in which the immune function effecting moiety binds to a diseased or disease causing cell surface associated epitope, and an immunocytokine or bispecific antibody as decribed in the immediately preceding two paragraphs.
43. In a preferred embodiment the invention contemplates modification of the multi-functional ligand to substitute one or more amino acids which reduce without functional impact on the molecule the number of immunogenic MHC II class peptide sequences within the molecule. This can be accomplished through procedures available to those skilled in the art, for example through the Delmmunisation services of Biovation Limited (see also US 5821123 and related Xoma patents).
44. Inasmuch as the invention is predicated on intraluminal lymphatic system targeting such lymph associaton may be alternatively implemented, in suitable circumstances by the method of delivering the multifunctional ligand, for example into the lumen of a lymphatic system vessel or (where the multifunctional ligand is not of an unsuitable size (see for example Ikomi, F. et al. Lymphology 32 (1999) 90-122, within a portion of body that drains to the lymphatic system (ie a portion of the lymphatic system), for eventual migration to the lymphatic system. Particularly, with respect to embodiments of the invention in which the immune function exerting moiety is targeted with greater functional affinity to a therapeutic target (ie. not the lymphatic system target), such lymphatic system oriented modes of delivery coupled with preferred targeting to the therapeutic target may combine, absent saturated binding to the therapeutic target, to better accomplish functional lymphatic targeting. Accordingly, in a broader aspect the invention is directed a lymphatic system targeted multifunctional ligand in which the second portion is as described herein and in which the specificity of the first portion exclusively for a lymphatic system is inessential. In this connection, the invention contemplates targeting markers on lymphatic vessels that are also present, for example on blood vessel endothelial cells (eg. VEGF2). (with respect to lymph specific markers see also Birner P. et al. Clin Cancer Res 2001 Jan; 7(1):93-7 "Selective immunohistochemical staining of blood and lymphatic vessels reveals independent prognostic influence of blood and lymphatic vessel invasion in early-stage cervical cancer" and published references to the markers therein mentioned.)
45. In the case of purely sustained release aspects of the invention where the first portion is temporarily anchoring a second portion for eventual release back into the circulation, the use of term immune function affecting moiety with reference to the role of the second portion does not adequately accommodate the breadth of the invention since any form of disease palliating active moiety or entity which exerts its effect elsewhere than at the lymphatic endothelial cell may gain advantage from this form of delayed delivery (depot effect) or anchoring.
46. Furthermore, in another preferred aspect, the second portion is capable of binding directly or indirectly (eg. binding to an entity which in turn binds to a target entity) to a target entity, for example a therapeutic entity (for example to mop up excess such entity that does not immediately reach its target (eg. an entity that is toxic elsewhere in the body), a toxic entity including an entity which is not per se toxic but the presence of which is undesirable at a particular time or in particular amount or concentration (eg. a cytokine, for example when released as a result of anti-CD3 therapy), to *redirect* an an entity to a target, for example a therapeutic entity, for example through the instrumentality of an antibody portion that is directed to that target (eg. a multifunctional ligand in which the second portion comprises an anti-tumor antibody portion that is conjugated to streptavidin, to retarget biotin conjugated radionuclide back to the tumor (see Martin J. et al. (1997) Cancer Chemother, Pharmacol. 40:189-201), to temporarily anchor liposomes or other carriers of entities (eg. drugs) having an direct or indirect beneficial effect elsewhere.

In a preferred embodiment, the invention provides a multifunctional ligand having, at least, a first portion which binds to a lymphatic vessel associated antigen/receptor (and thereby exerts, not necessarily to the exclusion of other effects) at least an anchoring function, and a second portion having at least one independent immune function. The term "immune function" is broad in intent including but not limited to direct or indirect and primary or corollary effects related to simple targeting, tolerance, immunization, stimulation, inhibition, modulation or various other immune related effects (other than simply forming part of the entity which blocks the lymphatic endothelial associated ligand). The term independent is used to exclude only an effect specifically targeted towards the ligand (blocking) or cell bearing the ligand to which the first portion of the multifunctional ligand is bound, which is not contemplated as an object of the invention. The invention contemplates rather that the immune function is exerted, for example, vis-à-vis immune cells or molecules or against cancer or infected cells to affect an immune function that relates to assessment, diagnosis, therapeutic modeling, or treatment of various disease states such as autoimmune disease, transplant rejection, cancer and infectious disease. In a preferred embodiment, the invention contemplates that the independent immune function is exerted through a physical ligand-ligand interaction. In a preferred embodiment the multifunctional ligand has an ability to bind in the manner of an antibody in virtue of at least one of the first or second portions, and preferably at least the first portion. The lymphatic system directed first portion may in some embodiments (LYVE-1) be hyaluronic acid or analogues thereof that have the appropriate binding capacity. In a further preferred embodiment the second portion binds to a target ligand on a cell or molecule (eg. a cytokine or autoimmune antibody) which passes through the lymphatic system. In a more preferred embodiment the multifunctional ligand is a bispecifc antibody. The term antibody is used to refer to any antigen binding fragment of an antibody that substantially has the binding capability of an antibody including anti-idiotypic antibodies, and therefore the term bispecific antibody is used (unless the context implies a more specific usage) in a functional sense to refer to at least two different specificities (including trispecifc antibodies etc.) and includes well known entities which are diabodies, triabodies, tetrabodies, minibodies, scFv dimers, etc., and entities in which one or both binding moieties are scFv or single domain type antibody fragments or dimers etc of such fragments (with respect to single domain antibodies see for example Camel single-domain antibodies as modular building units in J Biol Chem. 2000 Oct 25, & Mulligan-Kehoe U.S. patents).

The term "anchoring function" is used broadly to refer to physical attachment for a period which renders the second portion of the multi-functional ligand capable of exerting its immune function. For example where the function of the second portion is to interact with a cell passing through the lymphatic vessels, for at least a period which permits sufficient interaction for the desired effect.

The term ligand is used very broadly herein to refer to any moiety, preferably in some cases, a specifically interacting moiety including binding moieties (eg antibodies, receptors etc.) and bound moieties (eg antigens, epitopes etc) and/including otherwise interacting moieties (eg. chemotactic interactions or interactions that require multiple points of interface eg. cross-linking or multi-component epitopes). In other words, the term ligand is used broadly to refer to any entity or part thereof which can be subject to an intermolecular interaction that can result in binding. The term moiety is used broadly and non-limitatively to refer primarily to a functional part of an entity, and may refer to even the whole of the entity depending on the context in light of the broadest concept of the invention.

Optionally, depending on the mode of delivery and the relative functional affinity of the respective first and second portions, the multi-functional ligands of the present invention, may exert their immune function primarily in lymphatic system and also significantly before and optionally after entry into the lymphatic system. In a preferred embodiment the multifunctional ligand is capable of simulating a depot effect by binding for a prolonged period to the intra-luminal lymphatic endothelium for later release over time back into the circulation. The choice (avidity effect resulting from multiple binding "arms") and affinity of the binding molecule as well as various, preferably controllable factors impacting on any "undulating" movements of the lymphaticvessels (eg. water consumption)or competitive binding is contemplated to impact the binding time.

With respect to the depot and delivery aspects of the invention discussed herein, it is contemplated the second portion of the multi-functional ligand of the invention may have at least primary medicinal effects that are not immune function related as broadly understood.

It is to be understood that a use of a slash (/) means the broader of "or" or "and/or" unless to the context dictates otherwise.

Some immune interactions require, prefer or are capable of being enhanced via coordinated ligand interactions, for example for optimal immune stimulation, for example, specific costimulatory ligand interactions eg. CD80/CD86 interactions with CD28, or for example, interactions aimed at tolerizing or otherwise inhibiting or reducing immune effects or preventing such inhibition (for example using anti-CTLA-4/CD152 see related U.S. patents, for example 6,051,227, 5,844,095) (see also Hodge JW et al. Ernst Schering Res Found Workshop 2000 (30): 23-52 and Immunological Reviews Vol 172 Dec 1999, Entire Issue).

The invention contemplates modeling, evaluating and/or effecting these interactions for therapeutic intervention within the lymphatic system through the substantially contemporaneous use of different multifunctional ligands of the invention. Furthermore, control of the relative proportion of each of the different ligands permits different spatial interspersion of these ligands on the intraluminal suface of the lymphatic system (primarily) so as to provide controlled variability of spatial configurations appropriate for optimizing the coordinate interaction with multiple ligands on another entity, for example immune cells or cancer cells. This strategy also permits controls on avidity that extend beyond the choice of valency for a given single multifunctional ligand for controlling the nature and duration of the coordinate interactions including the duration of temporary anchoring, for example to allow cancer cells to be killed by immune cells, as well delivery of, for example, cytokines (through cytokine antibody fusions), superantigens etc. to the site of interaction. Such coordinate interactions may be substantially contemporaneous or sequential, for example the effect of a first interaction with a first multifunctional ligand slowing the progression of a cell or infectious agent through the lymphatic system for eventual rection with another first multifunctional ligand (ie of the same type) or reaction with a second type of multifunctional ligand. The invention also contemplates as a strategy, alone or in combination with other strategies: 1) delivery of a multifunctional ligand of the invention to a particular site of action for the purpose of exerting, for example a local effect, with the result of causing the multifunctional ligand (whether or not it has exerted its effect, provided that or to the extent that it remains functional in at least one aspect) to subsequently be targeted to the lymphatic system for exerting a second effect (be it the same or a different disease counteracting effect) including simply elimination, or return back to the circulation (ie. where the ligand is selected (eg. based on size, immunogenicity etc.) to be preferably minimally eliminated (at least not maximally eliminated) by the body in the course of its circulation, having regard to competing design considerations) for example, in the case of multifunctional ligand which is an anti-tumor ligand that has some residual binding to normal tissues, to set up, in effect, a site of competitive binding that advantageously impacts ( ie.reduces) undesired binding more than desired target binding; 2) delivery of a multifunctional ligand of the invention or an entity that binds to a multifunctional ligand of the invention to a particular site of action eg. local disease mediating immune cells, for the purpose of simple binding with the expectation that a delayed immune or other effect will be exerted within the lymphatic system. Accordingly, the invention is also directed to a composition comprising at least one and optionally a plurality of different multi-functional ligands of the invention. The invention is also directed to (such a composition when combined with a pharmaceutically acceptable carrier for example those that may be suitable for one or more of the various well known and heretofore used routes of administration including intravenous, intradermal etc which (for present purposes) are preferably not incompatible with delivering a multifunctional ligand of the invention to the lymphatic system. The invention is also directed to therapeutic compositions comprising a multifunctional ligand of the invention and to methods of treatment using such compositions. The invention is also directed to method of : 1) evaluating the therapeutic effect of a particular therapeutic entity against a particular target with reduced effect on undesired targets; 2) facilitating elimination a therapeutic entity; -- by administering the therapeutic entity as part of or in circumstances which permit interaction with, a multifunctional ligand of the invention.

The invention also contemplates cannulating particular portions of the lymphatic system to localize the delivery of a multifunctional ligand (see United States Patent 4,911,690), for example 1) to accommodate or further accommodate the treatment of conditions in which the immune affecting molecule has an undesirable systemic or localized side-effect if delivered otherwise; 2) for the localized delivery, as required, of larger molecules, complexes (eg. for temporarily anchoring MHC-peptide complexes) or otherwise associated (at least temporarily) entities (ie. associated other than through complex formation) etc. and/or 3) for the localized delivery of additional compositional elements eg. adjuvants, cytokines (see Immunological Reviews 2000 Vol 177 p. 5-246; Nature Immunology Feb 2001 Vol 2 No. 2 page 89), or for affecting only subsets of populations of cells or molecules that pass through the lymphatic system or a desired portion of the lymphatic system or are found with greater concentration within the lymphatic system. The invention also contemplates methods of selective, enhanced or localized, targeting/ delivery by administering multifunctional ligands of the invention as well as methods (including methods directly or indirectly employing the multifunctional ligands of the invention) of *enhancing* / *inducing* entry of cells or molecules, particularly immune cells (ie. cells having an immune system function as broadly understood) or subsets thereof, to the lymphatic system or a portion of the lymphatic system, for example for the purpose of direct or indirect interaction with the multifunctional ligands of the invention (in order to be acted on directly or indirectly, by multifunctional ligands of the invention) or for recruiting cells that will for example kill or modulate the activity of other cells, for example kill cancer cells or infected cells that will have, are having or have had direct or indirect interaction with the multifunctional ligands of the invention, as discussed further below, for example in the case of cancer, by targeting immunocytokines to the disease affected tissue eg. using cytokines eg. TNFα fused to antibody that binds specifically to tumor cell markers or markers for angiogenesis. Similarly tissue targetted as opposed to disease targeted immunocytokines could be used selectively recruit immune cells within that tissue for example a diseased tissue to enter the lymphatic system for such purposes including for example interaction with a multifunctional ligand of the invention.

It is also contemplated that a single multifunctional ligand can have multiple requisite interactive functionalities for example to stimulate, attract, anergize (or otherwise inactivate) sub-populations of B-cells of T cells via the use, for example, of trivalent or tetravalent antibodies and antibody conjugates/fusions thereof having multiple ligand interactive capabilities (see also for example technologies being developed for selection of successful binders by phage or ribosome display (see for example WO 01/00866; Adv Protein Chem 2000; 55:367-403). A particular application of this technology for application to this invention are antibodies which retarget T-cells to tumor cells (see for example Manzke O. et al. Int. J. Cancer 82, 700-708 (1999); Br J Cancer 2000 Jan; 82(2):472-9; J Control Release 2000 Feb 14; 64(1-3):229-39 as well as related references, cited therein or citing these publications.

The present invention accommodates such technology through multispecific antibodies or alternatively obviates the need for combining a T-cell receptor type molecule with the primary immune function effecting moiety (eg. a cancer cell binding moiety) by using a separate multifunctional ligand which combines, for example, a first portion and a second portion comprising a T-cell interacting moiety (eg. anti-CD3). This is accomplished by administering in the same composition or substantially contemporaneously an amount of the second multifunctional ligand that provides, as may empirically predicted by assessing the dispersion of the marker on the endothelial cell, a strong probability (eg..001-100%, optionally 1-100%, optionally 5-100%, optionally 10-100%, etc) that the T cell will be targeted in the vicinity of a given lymphatic endothelial cell that happens be proximal to the cell sought be targeted eg the cancer cell. It is self-evident that a 50/50 proportion of the first and second multifunctional ligand will yield a strong chance that a second multifunctional ligand will be immediately adjacent on a particular given side (assuming for the sake of argument that there are sides when in reality the dispersion of the lymphatic endothelial marker is governing). It is also contemplated that adjacent multifunctional ligands may be linked for example through linkage effective pairs of ligands (avidin-biotin), the second portions having an antibody component which binds to a common ligand (eg on a liposome (see US 6197333 and refs. therein cited) or other pharmaceutically acceptable micro/nano particle/sphere of preferably selectable size for optimal spacer or endothelial cell protective purposes) and that such entities could optionally also be employed to house and deliver a payload to a given target vicinity.

In one aspect the multi-functional ligands of the present invention provide for a method and preferably a means for evaluating and/or inducing immune tolerance (with respect to B cells see strategies discussed in Immunological Reviews 2000 Vol. 176 pp. 5-247).

It is believed that immune tolerance is enhanced or prolonged through prolonged /strategic exposure to tolerance inducing and/or enhancing molecules for example prolonged antigen exposure (see Wang Y et al. Eur. J. Immunol. 2000; 30(18):2226-2234; Encyclopedia of Immunology ; (1998) Morgan Kaufmann Publishers, ISBN:0122267656; Hoyne GF et al. Immunology 2000 Jul; 100(3):281-8; Lerner CG et al. J Immunol. 2000 Apr. 15; 164(8): 3996-4002; Grossman Z. et al. Semin Immunol 2000 Jun; 12(3):197-203; discussion 257-344 Textbook of the Autoimmune Diseases by Lahita R. et al. ISBN: 0781715059 Lippincott Williams & Wilkins; Multi-Systemic Auto-Immune Diseases : An Integrated Approach Dermatological & Internal Aspects ISBN: 0444818960 Elsevier Science ; Arthritis and Allied Conditions - A Textbook of Rheumatology, Thirteenth and Fourteenth Editions, William J. Koopman, MD 14th:ISBN: 0-7817-2240-3, November 2000; Principles of Drug Development in Transplantation & Autoimmunity Landes Bioscience, ISBN:0412100614; Cancer & Autoimmunity by Gershwin M. et al.,ISBN: 0444503315 Elsevier Science ; J Autoimmun 2000 Jun; 14(4):278-82; The multi-functional ligands of the present invention, depending on their mode of administration (direct application by cannulating a lymphatic vessel or conventionally eg. intradermally or intravenously), can be advantageously employed to provide prolonged/strategic exposure to tolerance enhancing molecules (for example by employing a multivalent eg. bi-specific Ab fragment or diabody which hs a first portion which binds to a lymph associated antigen and second portion which optionally comprises anti-idiotypic Ab portion mimicking the desired Ag or the antigen itself or a suitable portion thereof fused or conjugated to the first portion) on the intra-luminal surface of the lymphatic vessels, optionally, in addition to its conventional effects, when administered intradermally or intravenously, etc.. It is anticipated that the multi-functional ligands of the present invention would be useful to assess and/or effect tolerance induction (see Bassadona GP et al. Proc Natl Acad Sci U S A 1998 Mar 31; 95(7):3821-6; USP 6,106,834; USP 6,099,838; US6010902: Antibody heteroconjugates and bispecific antibodies for use in regulation of lymphocyte activity; as well as additional examples cited below with reference to examples of suitable anti-idiotypic antibodies).

It is also contemplated that a multispecific contruct as described in WO99/37791 could be used with respect to various aspects aspects of the invention..

With respect to tetravalent constructs see also WO 02/096948

### Additional Applications of Various Aspects of the Invention

It is contemplated that the present invention could be used to strategically mediate, CD45 (or variants/other PTPs) related "cell signaling", for example through signaling molecules (eg. inhibitors) using multifunctional ligands of the invention including but not limited to bispecific antibodies, antibody fusions/conjugates eg. where the immune affecting antibody portion or other moiety is conjugated, fused etc. to an antibody or fragment that binds to an entity associatied marker eg. LYVE-1 (1999) Journal of Cell Biology Vol 144 No 4 p. 789-801) (see for example USP 5,914,111 Sievers EL, Cancer Chemother Pharmacol 2000 46 Suppl s18-22 WO9946268, Neel BG Curr Opin Inmunol 1997 Jan 9(3) 405-420; Front Biosci 1998 Nov 1 3:D-1060-96, Slifka MK et al. J. Mol. Med 2000 78(2) 74-80 Goodnow CC Ciba Found Symp 1997 204: 190-202; Mustelin T. et al. Front Biosci. 1998 Nov 1; 3: D1060-96; Gaya A, Leuk Lymphoma, 1999 Oct 35 (3-4): 237-43; Sievers EL, Curr Opin Oncol. 2000 Jan 12(1): 30-5; Thomas ML, et al. Immul. Today 1999 Sep 20(9): 406-411; Appelbaum FR, Semin. Hematol. 1999 Oct; 36 (4 suppl. 6): 2-8; Ulyanova T; Immul. Res 1997 Feb; 16(1): 101-13; re PP32 for example USP 5,846,822 and Brody JR, et al. J Biol Chem. 1999 Jul 16; 274(29):20053-5 regarding the functional moiety of PP32 which is necessary for interaction with CD45, and for example USP 5,981,251 with respect to methods of identifying such molecules).

In preferred embodiments the invention is directed to multifunctional ligands that comprise immune function exerting moieties having functionalities of molecules currently in clinical trials or proposed for clinical trials (see for example Glennie MJ et al. Aug 2000, Immunology Today 408 Vol 21(8); see also Journal of Immunological Methods 237 (2000) 131-145; Mol Immunol 2000 Jun; 37(9) 515-526; Annu Rev Med 2001; 52:125-145; Annu Rev Med 2001 52:63-78; Q J Nucl Med 2000 Sep; 44(3) 268-83) including those that have an anti-CD2 functionality (see USP 5,795,572) anti-CD4 functionality (see for example USP 6,136,310 Herzyk D, J Infect Immun 2000 Feb; 69(2): 1032-1043) anti-CD3 functionality (for example WO 00/41474; WO 98139363; USP 6,113,901; Transplantation 2000 Dec 27 70 (12) 1707-12); Anti-CD44 functionality see for example Weiss L, et al., Proc Nat Acad Sci USA 2000; Jan 4 97(1) 285-290; Sugiyama K, Immunol Invest (1999) Mar-May 28(2-3) 185-200; Brocke S. et al. Proc Nat Acad Sci USA 1999 Jun 8 96(12) 6896; Mickecz K et al. Nat Med 11995 Jun; 1(6); 558-63; Ahrens T et al., J Invest Dermatol. 2001 Jan116(1) 93-101); with respect to control of migration of T-cell lymphocytes see Nohara C, et al. J Immunol. 2001 Feb 1; 166(3) 2108-2115), anti-CD20 functionality (see Crit Rev Oncol Hematol 2001 Jan 37(1):13-25) etc. anti-CD22 functionality see for example Newton DL, et al. Blood 2001 Jan15; 97 (2): 528-535, USP 5,184,892; Anti-CD40/CTLA-4 see for example J Immunol 2000 Oct 1; 165(7):3612-9; Microsurgery 2000; 2c (8); 448-452; USP 5874082; USP 6056959; USP 5,801,227; USP 6004552; USP 5677165; USP 6087329; USP 5961974; USP 6051228; White CA,et al. Annu Rev Med. 2001; 52: 63-78 (see also reviews and specific applications referred to in Ditzel et al., Immunol Res. 2000; 21(2-3):185-93; USP 6,010,902, USP 5876950; USP 5876718; USP 5,601,819, USP 5981251, USP 5885579 and 5885796; Cancer Immuno/ Immunother 2000 Jun; 49(3):173-80; Omar K, J Neuroimmunol 2001 Feb 1, 113(1) 129-141; Bellido M, Eur J. Haematol 2001 Feb. 66(2) 100-106; Broeren et al. J Immunol (2000) Dec15 165(12) 6908-14; Alexandroff AB et al Mol Immunol 2000 June 37(9) 515-526; Werkerle T J Immunol. 2001 Feb 15 166(4) 2311-2316; Howard LM J Immunol 2001 Feb; 116(3) 1547-53 anti-CD154; J Pharmacokinet Biopharm 1999 Aug; 27(4):397-420, J Clin Oncol 2000 Apr; 18(8):1622-36, Leukemia 2000 Mar; 14(3):474-5, Clin Cancer Res 2000 Feb; 6(2):372-80, Leukemia 2000 Jan; 14(1):129-35, J Nucl Med 1999 Nov; 40(11):1935-46, Blood 1999 Nov 15; 94(10):3340-8, Blood 1999 Aug 15; 94(4):1237-47, Cancer Res 1999 May 1; 59(9):2096-101, Vaccine 1999 Apr 9; 17(15-16):1837-45, Blood 1998 Dec 1; 92(11):4066-71, J Rheumatol 1998 Nov; 25(11):2065-76, Clin Pharmacol Ther 1998 Sep; 64(3):339-46, Mult Scler 1996 Jul; 1(6):339-42, Cancer Immunol Immunother 1997 Jul; 44(5):265-72, Transplant Proc 1996 Dec; 28(6):3210-1, Arthritis Rheum. 1996 Jul; 39(7):1102-8, Immunology 1996 May; 88(1):13-9 and USP 5,876,718).

The invention contemplates assessment and therapeutic benefit of lymphatic localization in the case of antibodies and multispecific ligands which are toxic to non-target cell populations which express the targeted ligand to a limited extent or in the case of toxic cross-reactivity of the second portion eg. antibody for its desired target with an undesired target (see eg. Lancet 1999 Nov 13; 354(9191):1691-5). It is contemplated that the toxic effect of a given effector moiety of a multifunctional ligand of the invention could be alternated using an additional binding arm for a lymphatic marker.

### Antibody Structure and Function

Antibody structure and function has bee extensively described in the literatue. For example see Antibody Engineering 2nd ed. Carl A.K. Borrebaeck, Oxford University Press 1995 p 3-44.

### Production of Bispecific Antibodies

A variety of different constructs have been developed for the production of bispecific antibodies including conventional four chain antibodies (including truncated version thereof such minibodies (see USP 5,837,821), F(ab')₂ (see Antibody Fusion Proteins, Steven M Chamow , Avi Ashkenazi Eds. ISBN 047118358X May 1999 Wiley p.136-144; or using CH3-truncated heavy chains), diabodies (see USP 5,837,242 Multivalent and multispecific binding proteins, their manufacture and use) constructs in which of one or two diabody molecules are heterodimerized by creating a fusion protein with the CL and CH1 immunoglobulin constant domains (see WO 02/02781).
In recent years, a variety of chemical and recombinant methods have been developed for the production of bispecific and/or multivalent antibody fragments. For review, see: Kriangkum J, et al. Bispecific and bifunctional single chain recombinant antibodies. Biomol Eng 2001 Sep;18(2):31-40, Holliger P. and Winter, G., Curr. Opin. Biotechnol. 4, 446-499 (1993); Carter, P. et al., J. Hematotherapy 4, 463-470 (1995); Pluckthan, A. and Pack, P., Immunotechnology 3, 83-105 (1997).

Bispecificity and/or bivalency has been accomplished by fusing two scFv molecule via flexible linkers, leucine zipper motifs, C_{H}C_{L}-heterodimerization, and by association of scFv molecules to form bivalent monospecific diabodies and related structures. Multivalency has been achieved by the addition of multimerization sequences at the carboxy or amino terminus of the scFv or Fab fragments, by using for example, p53, streptavidin and helix-tum-helix motifs. For example, by dimerization via the helix-turn-helix motif of an scFv fusion protein of the form (scFv1)-hinge-helix-turn-helix-(scFv2), a tetravelent bispecific is produced having two scFv binding sites for each of two target antigens.

Production of IgG type bispecific antibodies, which resemble IgG antibodies in that they posses a more or less complete IgG constant domain structure, has been achieved by chemical cross-linking of two different IgG molecules or by co-expression of two antibodies from the same cell. Both methods result in production of significant amounts of undesired and non-functional species due to mispairing among the component heavy and light chains. Methods have been employed to reduce or eliminate mispairing.

One strategy developed to overcome unwanted pairings between two different sets of IgG heavy abd light chains co-expressed in transfected cells in modification of the Cₕ3 domains of two heavy chains to reduce homodimerization between like antibody heavy chains. Merchant, A. M., et al., (1998) Nat. Biotechnology 16, 677-681. In that method, light chain mispairing was eliminated by requiring the use of identical light chains for each binding site of those bispecific antibodies.

To produce bispecific antibodies, Kostelny et al (J. Immunology 148:1547 (1992)) fused Fab fragments of antibodies to the leucine zipper portions of fos and jun proteins in the absence of a single chain construct for the antigen combining region. These methods are well described in the literature and summarized with references in Antibody Fusion Proteins, Steven M Chamow , Avi Ashkenazi Eds. ISBN 047118358X May 1999 Wiley; Kontermann, R., et al.(Eds.) particularly at pages 139-145. Pack and Pluckthun, fused a single chain antibody to amphipathic helices from a four helix bundle or from leucine zipper proteins.

Bispecific antibodies that are in a conventional IgG-like and Fab-like format have been developed by Zhu as tetravalent or bivalent molecules, respectively with each of the chains serving to anchor a binding moiety (see WO 01/90192 and Figure 1 therein), preferably consisting of a scFv. In the bispecific IgG-like construct, each side of the molecule comprises a CH1 domain and a CL domain and each CH and CL domain is linked through its N-terminus to a scFv of different specificity. The invention herein contemplates that this construct can readily be adapted to have each each half of the molecule associated with a polypeptide eg. a scFv of the same specificity so that each half of the molecule is monospecific (or to have each half of the molecule associated with different pairings of scFvs) so that each half of the molecule is effectively monospecific. The invention herein contemplates that a bivalent relatively low affinity second ligand binding moiety is used to activate receptors that require cross-linking for activity. The invention also contemplates that numerous permutations in which the functional affinity of the first ligand binding moiety whether monospecific or bispecific can be accentuated relative the functional affinity of the second ligand binding moiety including employing a first ligand high affinity scFvs for a single antinstances in which the second ligand binding moiety is effectively monovalent (has one, or one useful binding moiety). The invention also contemplates that this construct can have a truncated Fc portion and various known methods in the art for improving the pairing efficiency of the heavy chains. The invention also contemplates that the CH1 and CL domains of the second ligand binding moiety can be truncated as in camelid antibodies for efficient delivery eg. of biologic effector ligands.

### Methods of Generating Antibodies That Bind To Selected Target Ligands

A variety of technologies for generating antibodies with desired specificity have been extensively developed and become well known to and routinely practiced by those skilled in the art including phage display (see review in Basic Methods in Antibody Production & Characteriztion G.C. Howard et al. eds. CRC Press 2001 p. 105) and other display systems (ribosome display, display on the surface of various cells), immunizing mice, including particularly mice having human Ig genes, and antibody microarray technologies. These methods have also been extended to making antibodies with dual specificites such as diabodies (USP 5,837,242 Multivalent and multispecific binding proteins, their manufacture and use) and are the subject of extensive scientific and patent literature. For example, see US patents of Winter et al. 6,291,650; 6,291,161; 6,291,158; 6,017,732; 6,225,447; 6,172,197; 6,140,471, 6,010,884, 5,969,108, 5,871,907, 5,858,657; 5,733,743, 5,723,287 and those of Dyax, Morphosys, and Cambridge Antibody Technology.

### Affinity Maturation

Methods of codon based mutagenesis have been extensively developed for engineering the antibody binding site. For example, the use of such methods in a filamentous phage display system is described in Antibody Engineering 2nd ed. Carl A.K. Borrebaeck, Oxford University Press 1995 p117-128 see also pp.53-84 with respect to techniques of phage display of antibodies (see also Kontermann, R.: Dübel, S., (Eds.): (2001)Antibody Engineering ISBN: 3-540-41354-5,

### Methods of Generating Single Domain Ligands

The ability of a single variable fragment of an antibody to bind with specificity and suitable selected affinities in the nanomolar+ range has been extensively demonstrated using camelid and human VH fragments. Methods of generating VHs with the desired specificity have been extensively described (see USP 6,248,516 Single domain ligands, receptors comprising said ligands methods for their production, and use of said ligands and receptors). (see also literature referenced herein on this subject).

### Methods of Making Antibodies In E. Coli

The expression of recombinant antibodies, including diabodies in E. Coli has become routine. General precepts, and methods are discussed in Antibody Engineering 2nd ed. Carl A.K. Borrebaeck, Oxford University Press 1995 p229-266 see also Antibody Therapeutics WJ Harris et al. eds. CRC Press 1997 p. 221; see also review in Biotechnology, Volume 5A, Recombinant Proteins, Monoclonal Antibodies, and Therapeutic Genes A. Mountain, U. Ney, Dietmar Schomburg ISBN: 3-527-28315-3, January 1999, Antibody Production: Essential Techniques Peter J. Delves ISBN: 0-471-97010-7 WileyJune 1997 and Antibody Therapeutics Production, Clinical Trials, and Strategic Issues, By Rathin C. Das, Ph.D., M.B.A. & K. John Morrow, Jr., Ph.D., D&MD Publications October 2001 Chapter 3.

### Eukaryotic & Other Expression & Production Systems

Approaches for the eukaryotic expression of antibodies and antibody fusion proteins and the preparation of vectors for use in such methods are well known and extensively described in the literature. General precepts, and methods are discussed is Antibody Engineering 2nd ed. Carl A.K. Borrebaeck, Oxford University Press 1995 p267-293 (see also Antibody Therapeutics WJ Harris et al. eds. CRC Press 1997 p. 183-220; see also review in Biotechnology, Volume 5A, Recombinant Proteins, Monoclonal Antibodies, and Therapeutic Genes A. Mountain, U. Ney, Dietmar Schomburg ISBN: 3-527-28315-3, Wiley, January 1999 and Antibody Production: Essential Techniques Peter J. Delves ISBN: 0-471-97010-7 Wiley June 1997 and Antibody Therapeutics Production, Clinical Trials, and Strategic Issues, By Rathin C. Das, Ph.D., M.B.A. & K. John Morrow, Jr., Ph.D., D&MD Publications October 2001 Chapter 3.

With respect to a review of immunotoxins see also Antibody Therapeutics WJ Harris et al. eds. CRC Press 1997 p 33

With respect to Methods for producing recombinant vectors see also 5,962,255 Methods for producing recombinant vectors

Formulation, purification and analytic methods involving antibodies are well knoen to those skilled in the art and have been extensively reviewed. With respect to formulation, purification and analytic methods see for example, reviews in Antibody Therapeutics Production, Clinical Trials, and Strategic Issues, By Rathin C. Das, Ph.D., M.B.A. & K. John Morrow, Jr., Ph.D., D&MD Publications October 2001, Chapter 4.

With respect to methods of generating antibodies against self-antibodies see USP 5,885,793 Production of anti-self antibodies from antibody segment repertoires and displayed on phage

### Antibody Conjugates

Methods of chemical manipulation of antibodies for attachment of ligands (eg.biotin), radionuclides etc. are well known in the art and have been extensively reviewed (for example see review in Basic Methods in Antibody Production & Characteriztion G.C. Howard et al. eds. CRC Press 2001, p. 199; with respect to therapeutic principles see for example, Antibody Therapeutics WJ Harris et al. eds. CRC Press 1997 p 53-88).

The applications of bispecific antibodies, including methods of making and using them have been extensively reviewed (ee for example van Spriel AB, van Ojik HH, van De Winkel JG. Immunotherapeutic perspective for bispecific antibodies. Immunol Today. 2000 Aug; 21(8):391-7; Weiner LM. Bispecific antibodies in cancer therapy. Cancer J Sci Am. 2000 May; 6 Suppl 3:S265-71. Barbet J, et al. Pretargeting with the affinity enhancement system for radioimmunotherapy. Cancer Biother Radiopharm. 1999 Jun; 14(3):153-66. de Wolf FA, Brett GM. Ligand-binding proteins: their potential for application in systems for controlled delivery and uptake of ligands. Pharmacol Rev. 2000 Jun; 52(2):207-36.: Wang H, Liu Y, Wei L, Guo Y. Bi-specific antibodies in cancer therapyAdv Exp Med Biol. 2000; 465:369-80; Staerz UD, Lee DS, Qi Y. Induction of specific immune tolerance with hybrid antibodies. Immunol Today. 2000 Apr; 21(4):172-6: 1999 Dec; 43(4):336-43. Elsasser D, Stadick H, van de Winkel JG, Valerius T. GM-CSF as adjuvant for immunotherapy with bispecific antibodies. Eur J. Cancer. 1999 Aug; 35 Suppl 3:S25-8. Molema G, Kroesen BJ, Helfrich W, Meijer DK, de Leij LF. The use of bispecific antibodies in tumor cell and tumor vasculature directed immunotherapy. J Control Release. 2000 Feb 14; 64(1-3):229-39. Bodey B, Bodey B, Siegel SE, Kaiser HE. Genetically engineered monoclonal antibodies for direct anti-neoplastic treatment and cancer cell specific delivery of chemotherapeutic agents. Curr Pharm Des. 2000 Feb; 6(3):261-76. Kudo T, Suzuki M, Katayose Y, Shinoda M, Sakurai N, Kodama H, Ichiyama M, Takemura S, Yoshida H, Saeki H, Saijyo S, Takahashi J, Tominaga T, Matsuno S. Specific targeting immunotherapy of cancer with bispecific antibodies. Tohoku J Exp Med. 1999 Aug; 188(4):275-88. Koelemij R, et al. Bispecific antibodies in cancer therapy, from the laboratory to the clinic. J Immunother. 1999 Nov; 22(6):514-24. Segal DM, Weiner GJ, Weiner LM Bispecific antibodies in cancer therapy Curr Opin Immunol. 1999 Oct; 11 (5):558-62. Hudson PJ. Recombinant antibody constructs in cancer therapy. Curr Opin Immunol. 1999 Oct; 11 (5):548-57. Barth RF et al, Boron neutron capture therapy of brain tumors: an emerging therapeutic modality. Neurosurgery. 1999 Mar; 44(3):433-50; Fleckenstein G, Osmers R, Puchta J. Monoclonal antibodies in solid tumours: approaches to therapy with emphasis on gynaecological cancer, Med Oncol. 1998 Dec; 15(4):212-21. Guyre CA, Fanger MW. Macrophage-targeted killing and vaccines. Res Immunol. 1998 Sep-Oct; 149(7-8):655-60 Cao Y, Suresh MR. Bispecific antibodies as novel bioconjugates. Bioconjug Chem. 1998 Nov-Dec; 9(6):635-44. Farah RA, et al, The development of monoclonal antibodies for the therapy of cancer. Crit Rev Eukaryot Gene Expr. 1998; 8(3-4):321-56.: Volm M. Multidrug resistance and its reversal.Anticancer Res. 1998 Jul-Aug; 18(4C):2905-17. Rouard H, et al, Fc receptors as targets for immunotherapy.Int Rev Immunol. 1997; 16(1-2):147-85. Fan Z et al. Therapeutic application of anti-growth factor receptor antibodies; Curr Opin Oncol. 1998 Jan; 10(1):67-73. de Gast GC, et al,Clinical perspectives of bispecific antibodies in cancer. Cancer Immunol Immunother. 1997 Nov-Dec; 45(3-4):121-3. Carter P, Merchant AM. Engineering antibodies for imaging and therapy.Curr Opin Biotechnol. 1997 Aug; 8(4):449-54. Pluckthun A, et al, New protein engineering approaches to multivalent and bispecific antibody fragments. Immunotechnology. 1997 Jun; 3(2):83-105. Rihova B. Targeting of drugs to cell surface receptors. Crit Rev Biotechnol. 1997; 17(2):149-69. Molema G et al,Tumor vascular endothelium: barrier or target in tumor directed drug delivery and immunotherapy. Pharm Res. 1997 Jan; 14(1):2-10. Bodey B, et al, Human cancer detection and immunotherapy with conjugated and non-conjugated monoclonal antibodies. Anticancer Res. 1996 Mar-Apr; 16(2):661-74 Hartmann F et al, Treatment of Hodgkin's disease with bispecific antibodies. Ann Oncol. 1996; 7 Suppl 4:143-6. Wels W, et al, Intervention in receptor tyrosine kinase-mediated pathways: recombinant antibody fusion proteins targeted to ErbB2. Curr Top Microbiol Immunol. 1996; 213 (Pt 3):113-28.: Kairemo KJ. Radioimmunotherapy of solid cancers: Acta Oncol. 1996; 35(3):343-55. Verhoeyen ME, et al, Antibody fragments for controlled delivery of therapeutic agents. Biochem Soc Trans. 1995 Nov; 23(4):1067-73. Haagen IA. Performance of CD3xCD19 bispecific monoclonal antibodies in B cell malignancy. Leuk Lymphoma. 1995 Nov; 19(5-6):381-93.

In another aspect the invention is directed to presenting antigen within the lymphatic system (eg. in the form of an anti-idiotype antibody) such as to facilitate a desired immune response eg. vaccination type responses). Optionally, adjuvants can be conventionally employed to assist initial immune stimulation eg. intradermally when appropriately delivered. Activating cytokines for example as specified above, can also be employed to enhance the immune response. Examples of antibodies having an anti-idiotypic counterpart or for which an anti-idiotypic counterpart could made by well known techniques in the art (and that are capably of exerting the desired anti-idiotypic effect) are numerous and numerous such antiidiotypic antibodies have application to immunization as well as applications relating to tolerance (see for example US patents: 6,146,627 Method for reducing T cell-mediated cytotoxicity in HIV using anti-idiotypic antibody; 6,063,679 Anti-idiotypic monoclonal antibodies and compositions including the anti-idiotypic monoclonal antibodies; 6,060,049 Surrogate tolerogenesis for the development of tolerance to xenografts; 6,042,827Anti-idiotypic antibody induction of anti-tumor response; 6,007,815 Anti-idiotype vaccination against diseases resulting from pathogenic responses by specific T cell populations; 5,981,502 Methods and compositions for inducing apoptosis in tumor cells; 5,766,588 Tumor immunotherapy using anti-idiotypic antibodies; 5,728,812 Anti-idiotypic antibody composition for inhibiting acute complement-mediated cytotoxicity.

According to another aspect of the invention the multi-functional ligand comprises a first portion which binds to a lymph associated antigen and a second portion which binds to a tumor cell infected cell or infectious agent. This embodiment of the invention can be used for example, to assess and affect the ability of the tumor-binding portion to more advantageously inhibit metastasis. Optionally, for example, the portion which binds to a lymph associated antigen has a lower affinity and/or avidity so that the tumor cell binding portion preferentially binds to the tumor cell and is therefore more likely to accompany its passage through the lymphatic system. This strategy also has application to bi-specific antibodies of the invention in which the second portion is for example targeted to an immune cell. Optionally, multiple such muti-functional ligands may permit sufficient tumor cell anchoring to permit the tumor cell to be killed within the lymphatic system via a toxic payload carried by the multifunctional ligand or through the recruitment of immune cells which accomplish this end (eg using the same or a different multifunctional ligand fused or conjugated to a suitable cytokine (eg IL-2, IL-12). The prolonged presence of these cells could be advantageously used to assess methods of immunization directly against the tumor cell using, for example, cytokines including cytokines fused or conjugated in whole or functional part to a lymph targeted Ab on the same, or a different multifunctional ligand delivered in a suitable dose (with respect to generation of anti-tumor antibodies and other antibody fragments for application herein as well as important related technologies see also WO 00/50008; WO 01/01137; WO 97/37791; WO 99/37791; WO 97/10003; Hoogenboom et al. Nat. Biotechnology 15(2) Feb 1997 p125-126; Fell H. et at. Journal Of Immunolgy Vol 146(7) Apr 1991 p2446-2452; Anderson D. et al Bioconjugate Chemistry 14(1) Jan 1993 p10-18; USP 6, 172,197; USP 6,171,782; Immunological Investigations 2000 29(2) entire issue). Optionally the tumor binding portion internalizes and/or delivers a toxic payload, for example a radionuclide, or other toxin, or a cytokine to the tumor cell (with respect to selection of tumor internalizing human antibodies see for example Pool M et al. J Mol Biol. 2000 Sep 1; 301(5):1149-61, see also Kohl MD et al. J Mol. Biol. Biotechniques (2000) Vol 28(1) p162 In this way the multi-functional ligands of the invention, for example, when provided in a sufficient dose to both target the tumor and line a portion of the lymphatic system to which the target tumor is likely to drain, acts as a cancer treatment as well as a sentry system for assessing / augmenting (for example as an adjunct therapy) the ability of the tumor binding portion with/without payload to inhibit metastasis. There are numerous examples of functional cytokine and toxin fusions used for example in cancer therapy that may have application to the invention herein (for examples and reviews see references herein cited as well as WO 99/37791; WO99 WO00/06605 ; WO 99/52562WO 99/37791 MULTIPURPOSE ANTIBODY; Proceeding of the IBC's 11th Annual International Conference on Antibody Engineering State of the Art Science, Technology and Applications, December 3-6, 2000; Amplification of T cell-mediated immune responses by antibody-cytokine fusion proteins. Immunol Invest. 2000 May; 29(2):117-20; Cancer Res.1999 May 1; 59(9):2159-66.; Pharmacokinetics and stability of the ch14.18-interleukin-2 fusion protein in mice. Cancer Immunol Immunother. 1999 Aug; 48(5):219-29. Phase I study of single, escalating doses of a superantigen-antibody fusion protein (PNU-214565) in patients with advanced colorectal or pancreatic carcinoma. J Immunother. 2000 Jan; 23(1):146-53. Targeted toxin therapy for malignant astrocytoma.Neurosurgery. 2000 Mar; 46(3):544-51 ; Targeting cytokines to tumors to induce active antitumor immune responses by recombinant fusion protein. Hum Antibodies. 1999; 9(1):23-36; Lode HN, et al. Tumor-targeted IL-2 amplifies T cell-mediated immune response induced by gene therapy with single-chain IL-12. Proc Natl Acad Sci USA. 1999 Jul 20; 96(15):8591-6; Cancer Vaccines and Immunotherapy 2000 (textbook) ; Immunotherapy With Intravenous Immunoglobulins P. Imbach (1991) Academic Press; Molecular Approaches to Tumor Immunotherapy (1997) World Scientific Publishing Company, Incorporated; Vaccines & Immunotherapy S. J. Cryz (1991) McGraw-Hill Ryerson, Limited

*With respect to* internalizing antibodies see eg Biological Effects of Anti-ErbB2 Single Chain Antibodies Selected for Internalizing Function.; Biochem Biophys Res Commun. 2001 Jan 12; 280(1):274-279 and references cited therein, Immunoconjugates of geldanamycin and anti-HER2 monoclonal antibodies: antiproliferative activity on human breast carcinoma cell lines J Natl Cancer Inst. 2000 Oct 4; 92(19):1573-81; Foulon CF, et al., Radioiodination via D-amino acid peptide enhances cellular retention and tumor xenograft targeting of an internalizing anti-epidermal growth factor receptor variant III monoclonal antibody. Cancer Res. 2000 Aug 15; 60(16):4453-60. Poul MA, Becerril B, Nielsen UB, Morisson P, Marks Selection of tumor-specific internalizing human antibodies from phage libraries J Mol Biol. 2000 Sep 1; 301(5):1149-61.Vrouenraets MB, et al.,Targeting of a hydrophilic photosensitizer by use of internalizing monoclonal antibodies: A new possibility for use in photodynamic therapy. Int J Cancer. 2000 Oct 1; 88(1):108-14.

In yet another aspect, the invention contemplates that the passage of tumor cells can be inhibited within the tumor vasculature using a bispecific ligand, optionally a bispecific antibody, which targets on the one hand a well known vascular endothelial marker and one the other hand binds to a ligand on the surface of the tumor. Other aspects of the invention related to tumor cell targeting are understood to described in reference to this aspect of the invention as well. It is also contemplated that markers which are present on both the lymphatic endothelium and the tumor vasculature can be simultaneously targeted with bispecific ligands of the invention to inhibit tumor metastasis and/or immunize a subject against tumor cells.

It is contemplated that the multifunctional ligands of the invention when used to inhibit metastasis, for example, in the manner described above, could be advantageously employed in combination with other well known therapies foe example cytoxic drugs, other tumor targeted antibodies and conjugate/fusions therewith used or currently being evaluate for immunotherapies, angiogenesis targeted drugs etc. (re angiogenesis see for example Angiogenesis in cancer and other diseases. Nature. 2000 Sep 14; 407(6801):249-57).

Similarly, a bi-specific antibody of the invention could be used to bind to antigens/ligands on lymphocytes which are known or become known to inhibit or enhance immune function or mediate a disease eg. CD45.

With respect to target receptors related to the inventions defined herein see also USP 6,277,962.

As discussed above, as used herein the term "lymph associated antigen" refers to antigens that are expressed significantly on lymphatic endothelial cells but not significantly expressed, if at all, on other tissues. Examples of such antigen include LYVE-1 a CD44 receptor analogue which binds to HA (February 22, 1999, Banerji et. al., Journal of Cell Biology Vol. 144, #4, p789-801) and which is expressed primarily on lymphatic endothelial cells. LYVE-1 specific antisera have been shown to inhibit binding of HA. The invention contemplates research and treatments using multi-functional ligands of the invention with respect to non-human mammals, including preferably agricultural animals, canine species, primates and mice having similar receptors/antigens. For example, a murine counterpart to LYVE-1 (published in Prevo R. et al. 2001 Feb 20, J. Biol. Chem.; Manuscript M011004200) can be employed to implement the various methods and embodiments described herein in a mouse model, for example to assess the extent of inhibition of metastasis effected by a multifunctional ligand (optionally comprising for example to a toxin, cytokine T cell receptor etc) which has a first portion which binds to LYVE-1 and a second portion which binds to, for example to GI-101, a breast tumor which is known to metastisize to the lung (see USP 6037520 and 5, 693, 533 see also US patents 5,643, 551, 5491284, 5569812, 5917124 and 6107540 and references cited in these patents, particularly with respect to other metastatic models and methods of evaluating anticancer drugs in mice). LYVE-1 counterparts in other mammals can be identified in the manner described by Prevo R. et al. (see also Skobe M. et al. Induction of tumor lymphangiogenesis by VEGF-C promotes breast cancer metastasis Nat. Med. Feb; 7(2) 192-8.)

Other models of metastasis in animals are well known in the art (see for example Chirgwin JM, Guise TA.Molecular mechanisms of tumor-bone interactions in osteolytic metastases. Crit Rev Eukaryot Gene Expr. 2000;10(2):159-78. 3: Kobaek-Larsen M, et alReview of colorectal cancer and its metastases in rodent models: comparative aspects with those in humans. Comp Med. 2000 Feb;50(1):16-26. 5: Magnano M, et al.A physical-based model for the simulation of neoplastic growth and metastasis. J Surg Oncol. 2000 Jun;74(2):122-9. 6: Hoffman RM. Orthotopic metastatic mouse models for anticancer drug discovery and evaluation:a bridge to the clinic. Invest New Drugs. 1999;17(4):343-59.

Russo J, Russo IH.The pathway of neoplastic transformation of human breast epithelial cells.Radiat Res. 2001 Jan;155(1 Pt 2):151-154. Duffy MJ, McCarthy K.Matrix metalloproteinases in cancer: prognostic markers and targets for therapy(review).Int J Oncol. 1998 Jun;12(6):1343-8. 22: Banerjee A, Quirke P.Experimental models of colorectal cancer. Dis Colon Rectum. 1998 Apr;41(4):490-505.

Wu TT et al.Establishing human prostate cancer cell xenografts in bone: induction of osteoblastic reaction by prostate-specific antigen-producing tumors in athymic and SCID/bg mice using LNCaP and lineage-derived metastatic sublines. Int J Cancer. 1998 Sep 11;77(6):887-94.61: Molpus KL, et alCharacterization of a xenograft model of human ovarian carcinoma which produces intraperitoneal carcinomatosis and metastases in mice. Int J Cancer. 1996 Nov 27;68(5):588-95.65: Pages JC, Sordat B, Bautista D, Costa J, Benhattar J. Detection of rare circulating human colon tumor cells in a nude mouse xenograft model. Cancer Lett. 1996 Aug 23;106(1):139-44.66: Sakakibara T, et al.Doxorubicin encapsulated in sterically stabilized liposomes is superior to free drug or drug-containing conventional liposomes at suppressing growth and metastases of human lung tumor xenografts. Cancer Res. 1996 Aug 15;56(16):3743-6.

With respect to modifying an antibody to increase its affinity see also Crystal structure of Fab198, an efficient protector of the acetylcholine receptor against myasthenogenic antibodies. Eur J Biochem. 2001 Jul;268(13):3685-3693.

For example, in one embodiment the invention contemplates a bispecific antibody comprising an antigen binding component specific for a tumor cell associated antigen and a relatively low affinity anti-IL-6 receptor antibody component. With respect to the anti-tumor role of IL-6 see Wei LH et al. Interleukin-6 in cervical cancer: the relationship with vascular endothelial growth factor. Gynecol Oncol. 2001 Jul;82(1):49-56.

The invention contemplates that TCRs and modified TCRs (see for example, WO 01/48145) may be used as ligands, in place of antibody fragments for binding to target ligands such as peptide/MHC ligands.

Techniques for generating antibodies, and methods, for example of subtractive screening useful to identify other lymphatic vessel associated antibodies, including those optionally having smaller scFv, Fab and dAb (single domain antibody or functional fragment thereof) component (more easily passaging to lymphatic vessels from tissues particularly when constructed in the form of bispecific antibodies eg. diabodies etc.) by phage or ribosome display are well known in the art (see for example Hoogenbom HR et al. Immuno., Today (Aug. 2000) Vol 8 p 371; Schaffitzel C. et al. J Immunol. Methods (Dec. 10, 1999) 231 (1-2) p. 119; Roberts RW et al. Curr Opin Chem Biol. 1999 Jun; 3(3):268-73; Winter G. et al. Annu Rev Immunol 1994 12:433-55; Kontermann RE et al. Nat Biotechnol. 1997 Jul; 15(7):629-31; Phage Display of Peptides and Proteins, A Laboratory Manual Kay BK et al. Eds 1996 Academic Press; Immunology Methods Manual Lefkovits, I ed. 1997 Academic Press;Hoogenboom et al. Immunotechnology 4 (1998)1-20;
**With respect to making single domain antibodies see for example USP 5,824,520, USP 5622836, USP 5,702,892, USP 5,959,087, Unique single-domain antigen binding fragments derived from naturally occurring camel heavy-chain antibodies.J Mol Recognit. 1999 Mar-Apr; 12(2):131-40. An antibody single-domain phage display library of a native heavy chain variable region: isolation of functional single-domain VH molecules with a unique interface. J Mol Biol. 1999 Jul 16; 290(3):685-98 and references cited in these references.

Methods for making antibody fusion proteins and bi-specific antibodies including diabodies etc. and fusion proteins thereof are well established in the art (for reviews and particular applications see for example Adams GP et al. Journal of Immunological Methods 231 (1999) 249-260; USP 6,121,424, 6,027,725 and 6,025,165; EP 0654085; Hudson P. Exp. Opin. Invest. Drugs (2000) 9(6): 1231-1242; Antibody Fusion Proteins Steven M Chamow , Avi Ashkenazi Eds. ISBN 047118358X May 1999 Wiley; Antibody Engineering, Carl A. Borrebaeck Oxford University Press, 1995; Antibody Engineering:A Practical Approach David J. Chiswell, Hennie R. Hoogenboom, John McCafferty OxfordUniversity Press,1996; Antibody Engineering Protocols, Sudhir Paul (1995) Humana Press; Antibody Expression & Engineering (1998) Henry Y. Wang, Tadayuki Imanaka, American Chemical Society; Zhu Z. Biotechnology (NY) 1996 Feb.; 14(2): 192-6; Nielsen UB et al. Cancer Res. 2000 Nov 15; 60(22):6434-40; Lawrence LJ. Et al Febs Lett. 1998 Apr. 3; 425(3) 479-84; Hollinger et al., Cancer Immunol Immunother 1997 Nov-Decc 45 (3-4) 128-30; Immunotargeting of tumors: state of the art and prospects in 2000 Bull Cancer. 2000 Nov; 87(11):777-91; Hellfrich Wet al Int. J. cancer 1998 Apr 13 76(2): 232-9; Wu AM, Q J Nuc Med. 2000 Sep.; 44(3):268-83 KrebsB. Et al. J Interferon cytokine Res 1998Sep 18(9): 783-91; Takemura Si, et al. Protein Eng. 2000 Aug.; 13(8): 583-8; Cochlovius B et al. J Immunol. 2000 Jul 15; 165(2):888-95; AtweIII JL et al. Protein Eng. 1999 Jul; 12(7) : 597-604; kiprivanov SM et al. J. Mol Biol. 1999 Oct 15, 293 (1): 41-56; Alt M et al FEBS LeH. Jul 2 454 ()1-2) 90-4. Hudson PJ et al. J Immunol Methods 1999 Dec 10; 231(1-2):177-89 Ardnt MA et al. Blood 1999 Oct 15 94(8): 2562-8; Lu D. et al. J Immunol. Methods 1999 Nov. 19; 230(1-2):159-171; Santos AD et al, Clin Cancer Res 1999 Oct 5 (10 suppl): 31185-31235 Kontermann RE et al. Nat Biotechnol. 1997 Jul; 15(7):629-31; Dolez et al. Protein eng. (2000) Aug 13 (8): 565-74; Adams GP et al. Nucl. Med. Biol (2000) May 27 (4); 339-46; Williams LE et al. Med phys 2000 may 27(5) 988-94; Fitzgerald K. Protein Eng 1997 oct 10(10): 1221-5 and the various references cited therein) as are various methods for identifying internalizing antibodies and creating toxin, radionuclide and cytokine fusions / conjugates (see ao Y et al Bioconj. Chem 1998 Nov-Dec; 9(6): 635-44) for fully exploiting various aspects of the invention herein defined (see for example Becerril B et al. Biochem Biophys Res Comm 1999 Feb 16; 255(2):386-93 see also additional references below.

Triabodies and other known multivalent antibodies etc. (see for example Iliades P et al. FEBS Lett. 1997 June 16; 409(3):437-41) etc. could advantageously be employed to provide additional functionalities, as well as variation in avidity etc. for the purposes of variously exploiting the invention herein.

Methods of expressing and identifying new molecules like LYVE-1 are also well known in the art (see WO 98/06839)

Technologies for rendering the multifunctional ligands of the invention less immunogenic (eg such as employed by Biovation) are preferably applied to the multifunctional ligands of the invention.

For recent progress in the treatment of lupus nephritis see Zimmerman R. Annu Rev. Med. 2001; 52:63-78.

With respect to targeting Fas-L see US6068841:Antibodies to Fas-L for treatment of hepatitis.

The invention also contemplates using chemokines and variously targeted antibodies and fragments thereof fused or conjugated to chemokines or other molecules with for example, lymphocyte or other immune cell attractant properties (see for example Sun J. et al. Lymphology 32 (1999) 166-170; and Gerard C. et al. Nature Immunology (2001, Feb.) 2(2): p108; Immunological Reviews 1999 Vol 170 p 5-197) to attract immune cells into target tissues for eventual penetration into the lymphatic vessels for activation, signalling, binding to, inhibition, etc.. For example, for cancer treatment antibodies that bind to angiogenesis markers fused to such type such molecules eg. TNF-a can be advantageously employed optionally in conjunction with various vaccination strategies (including the use of the muti-functional ligands of the present invention) to attract immune cells including, optionally, vaccination-activated tumor targeting lymphocytes to the tumor site. In an indirectly related aspect (having independent applications as well as for combination therapy with a multifuctional ligand, the invention is also directed to an antibody that targets an angiogenesis marker fused/conjugated to a cytokine or antibody (ie a bispecific antibody) which binds to a cytokine, which cytokine augments adhesion of immune cells to blood vessels and method of using same (by administration to a subject), alone, in combination with multifunctional ligands of the invention or with other vaccination strategies to increase immune cell targeting to a solid tumor. In the case of a bispecific antibody it is contemplated that the cytokine binding portion has a relatively low functional affinity to the cytokine so as to compete unfavourably for its binding to its natural receptor.

With reference to modulating binding of leucocytes to endothelial adhesion molecules see for example US Patent No. 6,123,915 and the references therein cited.

It is well known to those in the art to make bispecific antibodies which are adapted to bind two different ligands on the same cell, for example so called antigen-forks as disclosed in USP 5,705,614 (see also Shi T et al. Murine bispecific antibody 1A10 directed to human transferrin receptor and a 42-kDa tumor-associated glycoprotein also Clin Immunol Immunopathol 1996 Feb;78(2):188-95; Amoroso AR et al., Binding characteristics and antitumor properties of 1A10 bispecific antibody recognizing gp40 and human transferrin receptor Cancer Res 1996 Jan 1;56(1):113-20; Ring DB et al., Antigen forks: bispecific reagents that inhibit cell growth by binding selected pairs of tumor antigens, Cancer Immunol Immunother 1994 Jul;39(1):41-8; Lu D et al., Complete inhibition of vascular endothelial growth factor (VEGF) activities with a bifunctional diabody directed against both VEGF kinase receptors, fms-like tyrosine kinase receptor and kinase insert domain-containing receptor. Cancer Res 2001 Oct 1;61(19):7002-8; Schmiedl A, Breitling F, Dubel S. Expression of a bispecific dsFv-dsFv' antibody fragment in Escherichia coli. Protein Eng 2000 Oct;13(10):725-34 see also Park SS, et al., Generation and characterization of a novel tetravalent bispecific antibody that binds to hepatitis B virus surface antigens Mol Immunol 2000 Dec;37(18):1123-30; Kriangkum J et al., Bispecific and bifunctional single chain recombinant antibodies Biomol Eng 2001 Sep;18(2):31-40; USPs 4,474,893, 5,989,830; WO 00/29431).

With respect to antibodies to autoantigens, ADEPT, use of anti-eotaxin antibodies, Delmmunization, antibody-cytokine fusions, ribosome display, xenomouse technology; cutting edge phage display techniques, construction of *human* antibody fragment based phage display libraries, selection of internalizing antibodies by phage-display, cancer targeting antibodies, antibody arrays, plantibodies, design of mutant IGSF domains of CD2, CD58 and TCR; oligopeptide eg. paratope mimetics, diabodies, minibodies, triabodies, tetrabodies and related size/kinetics issues, caspase activatable pro-drugs, delivery of Bismuth-213 via scFv and diabodies,anti-angionenesis marker strategies, immunoenzype therapy of cancer (eg. with Rnases) pancarcinomic antigens like CEA (TAG")-72; and related technologies see the papers and references in Proceedings of IBC's 11^{th} Annual International Conference on Antibody Engineering, State of the Art, Science, Technology and Applications Dec 3-6 2000. La Jolla, CA.

With respect to biology of the lymphatic system having practical application herein see Ikomi, F. lymphology (1999) 32:90-102; Shield JW. Lymphology 1999 32: 118-122 and Lymphology 33 (2000) 144-147, as well as the references cited therein.

The invention also contemplates control of such migration by inhibition of receptors that mediate such migration (see for example Sun J. et al. Lymphology 32 (1999) 166-170) for controlled application of the multifunctional ligands of the invention.

With respect to recent developments with respect to target ligands and/or immunotherapy having application herein see also WO 01/12224, WO 01/14550, WO 01/11059, WO 01/10205, WO 01/00679, WO029445 WO 01/14885, WO/14564, WO 01/14558, WO 01/14224, WO 01/13945, WO 01/12840, WO 01/12781, Wo 01/12674, WO 01/12670, WO 01/12224, WO 01/12646; WO 01/12223, WO 01/12218, WO 01/12217, WO 01/12216, WO 01/12154, WO 01/14557, WO 01/11059, WO 01/10912, WO 01/11040, WO 01/10888, WO 01/10460, WO 01/10205, WO 01/09611, WO 01/09328, WO 01/09186, WO 01/09192, WO 01/08635, WO 01/07481, WO 01/07082, WO 01/07084, WO 01/07081, WO 01/07484, WO 01/07466, Triggering Fc alpha-receptor I (CD89) recruits neutrophils as effector cells for CD20-directed antibody therapy. J Immunol. 2000 Nov 15; 165(10):5954-61. CD47 engagement inhibits cytokine production and maturation of human dendritic cells. J Immunol. 2000 Feb 15; 164(4):2193-9.

The invention also contemplates that a multifunctional ligand that recognizes an immune cell as a target in virtue of a particular cell marker and will be able to deliver a toxic payload to the cell, for example, in virtue of its second portion comprising such toxic component fused or conjugated thereto. The invention also contemplates attracting or supplying other immune cells or molecules to kill, or otherwise inactivate the target immune cell (eg. lymphocytes eg. by TH cell modulation or CD4 cell modulation or using antibodies including anti-idiotypic antibodies. The invention therefore contemplates that treatment of such immune cells can be accomplished by a combination of different mechanisms or drugs depending on the disease so as to reduce immunosuppression due to immune cell ablation where this is the dominant consideration. Such interactions may require interaction with one or more ligands on the surface of the targeted immune cell, as facilitated via anchoring interactions of varying affinity/avidity/duration. The invention also contemplates using multifunctional ligands comprising or bound to selectins and ICAMs etc. to facilitate such targeting, for example co-adminstering same in a proportion which is for example 0.01% to 25% of the targeting multifunctional ligand. The relative amounts of the selectin/ICAM etc. (including antibody mimics) bearing multifunctional ligand as compared with the targeting multifunctional ligand can be determined empirically by varying the proportions and assessing any objective indicator of successful targeting in a disease related or purely experimental context. For example successful targeting (eg. antibody binding to eg. CD3, CD28, CD2) using multifunctional ligands of the invention could be monitored by evaluating levels of cytokines normally attributable to such binding (see for example CD8 T cell activation after intravenous administration of CD3 x CD19 bispecific antibody in patients with non-Hodgkin lymphoma. Cancer Immunol Immunother. 1995 Jun; 40(6):390-6. Definition of a lamina propria T cell responsive state. Enhanced cytokine responsiveness of T cells stimulated through the CD2 pathway. J Immunol. 1995 Jan 15; 154(2):664-75.

With respect to multifunctional ligands that are used to directly or indirectly exert an immunization function, other examples of disease associated peptides that can be presented as immunogens or inhibitor/modulators of immune activity or disease progression in one of the fashions suggested above include, examples as well as technologies referenced in, for example, Knuth A, Cancer Chemother Pharmacol (2000); 46 suppl: 546-51; Engelhard VH, Cancer J Sci Am 2000 May; 6 Suppl 3: S272-80; Pietersz GA et al, Cell Mol Life Sci. 2000 Feb; 57(2): 290-310; Algarra I et al, Hum Immunol. 2000 Jan; 61(1): 65-73; Tumour vaccines: a new immunotherapeutic approach in oncology.Ann Hematol. 2000 Dec; 79(12):651-9; Human tumor-rejection antigens and peptides from genes to clinical research Nippon Geka Gakkai Zasshi. 2000 Sep; 101(9):612-7. Pinilla-Ibarz J, et al CML vaccines as a paradigm of the specific immunotherapy of cancer. Blood Rev. 2000 Jun; 14(2):111-20).

In order to present an MHC-peptide complex in proximity to a B7 co-stimulatory molecule, the invention contemplates using, in addition to varying amounts (varying from a 50/50 proportion) of adjacent multifunctional ligands (which may be a dAb, diabody etc.) preferably cross-linked by an avidin component, - as a different strategy - cross-linking with avidin or the like adjacent arms of a single diabody, triabody or tetrabody etc. which binds to or has been fused or conjugated individually to respective B7 and MHC peptide components (with respect to recombinant B7 and MHC molecules and fusion proteins thereof including antibody fusions and related technologies see references above and EP 99/97477 WO 99/42597, WO 97 28191, US 6, 197, 302, US6015884 US6140113, US 6,045,796, US 5580756, EP0935607, WO 9806749 WO9803552, EP 1054984, US 5869270, Construction and characterization of bispecific costimulatory molecules containing a minimized CD86 (B7-2) domain and single-chain antibody fragments for tumor targeting; method is useful for cancer therapy Rohrbach F et al., Clin.Cancer Res.; (2000) 6, 11, 4314-22 WO 00/008057 17 Feb 2000; WO 9921572 6 May 1999; WO 9913095 18 Mar 1999; WO 9742329 13 Nov 1997; WO 9720048 5 Jun 1997; WO 9640915 19 Dec 1996; WO 00/023087; EP 610046 10 Aug 1994, USP 6056952 as well as references therein cited).

In a related aspect, the invention similarly contemplates using or more antibodies (optionally biotinylated and cross-linked by an avidin component) that bind to the same or different epitopes on a tumor including, where two such antibodies are used different proportions of MHC and B7 linked (ie fused, conjugated or capable of binding to) antibodies as well as different proportions of different epitope-specific antibodies to optimize the distribution of such cross-linked B7 and MHC peptide complexes for T-cell recognition. In this way any strongly immunogenic peptide may be used in conjunction with suitable vaccination strategies to create a universal cancer antigen. Using a tumor unrelated peptide is advantageous to avoid any tolerization effects resulting from T-cell binding to the MHC-peptide alone and does not preclude immune system recognition of a different epitope or other therapies. In a preferred embodiment, a single multifunctional ligand or pair of multifunctional ligands optionally biotinylated and cross-linked by an avidin (or variants), is used to bind to both the lumen of the lymphatic system and to a tumor cell. (using for example a trispecific antibody with monovalent linkage to both the cancer cell and lymphatic endothelial cell and a third antibody component having respective fusions to one of MHC-peptide and B7 on heavy and light chain, or a trispecific or tetraspecific tetrabody having an antibody component devoted to each or the B7 and MHC linkages). This permits a single molecule to be used for both the immunization within the lymphatic system and the tumor targeted antigen display. However, It will be appreciated that presentation of MHC-peptide complex on a tumor does not necessarily require costimulatory B7 presentation to induce a cytotoxic T cell response which is specific for the peptide and that multiple such presentations, preferably in a cross-linkable fashion may be preferable. Accordingly, strategies herein for costimulatory presentation of MHC-peptide and B7 may be differently applied to a lymphatic endothelial cell surface for immunization purposes and and a tumor cell surface (primarily for recognition purposes), for example by using avidin facilitated cross-linking of in the former but not the latter (tumor) context or using different sets of molecules in each case or using modularly reconstructing the tumor cell suface with a bispefic antibody that binds to a separately administered MHC and/or B7 component.

Subject to the latter proviso, in preferred embodiments, the invention contemplates using as separate counterparts 1) separate trispecific Abs, each including for example, one antibody component which binds to the each of the respective B7 and MHC molecules which are preferably together, separately administered. Such multifunctional ligands are preferably biotinylated for cross-linking - both between adjacent trispecific Abs and adjacent T-cell stimulatory/co-stimulatory arms; or 2) separate bispecific pairs of Abs each respectively having 1) either a B7 and lymphatic vessel or B7 and tumor binding portion or 2) a MHC peptide complex and a lymphatic vessel or MHC complex and tumor binding, portions which again are prefereably cross-linked by an avidin,streptavidin or a variant (ie. using biotinylated antibodys) This latter embodiment permits smaller size antibody molecules to be used for better tumor targeting. Antibody components which recognize the non-T cell interactive portion of the B7 or MHC molecule can be readily generated by phage display, for example in the case of a known peptide specific antibody to an MHC peptide complex (see Chames et al. Proc Natl Acad Sci USA 97, 7969 and Chames et. al. "Affinity Maturation of TCR-Like MHC-peptide specific antibody: peptide specificity is possible over a wide affinity range" Proceedings of IBC Conference on Antibody Engineeering Dec. 2000) eg. by first causing binding of the "peptide specific" antibody and then doing the phage display eg. using an array of multiple (eg. repeats of the same antibody) such peptide specific MHC antibodies, applying the MHC peptide complex to effect binding and then performing the phage or ribosome display. Alternatively a TCR (eg cell bound) or analogue/mimotope could be used for the orientation. Similarly antibodies could be generated which in effect do not compete with CD28 or a mimotope thereof to create suitable anti-B7 type antibodies. Anti-B7 antibodies are known in the art. The invention also contemplates that the MHC-peptide binding function may be supplied using a linked superantigen (US 6197299, WO 9601650 25 Jan 1996; Proc.Natl.Acad.Sci.U.S.A.; (1994) 91, 19, 8945-49) in both the tumor and lymphatic system binding sites. Optionally, the tumor antigen or one or both of the antigens are a pan-carcinomic antigen like TAG-72, CEA, H11 (WO 97/44461). The invention also contemplates using one or more phage display libraries to optimize the development of MHC/B7 costimulatory bispecific antibodies, by using cell sized latex spheres coated with an antigen eg. CEA in various surface dispersions (or a cell) and using a array of preferably biotinylated antibodies which recognize the antigen and have a "oppositely located"portion fused, conjugated or capable of binding to one or both of MHC and B7, the library optionally also presenting also variations and combinations of lengths (truncations) of one or more constant regions or for example the CDR2 generated by phage display, depending on the choice of antibody, and with microarray technology, using a signalling means to detect T-cell recognition and evaluating cytotoxicity with for example a Cr51 release assay. (with respect to protein chip or microarray technology see WO 00/63701 references, for example in the Proceedings of IBC's conference on Protein Microarray Technology March 19-21 Santiago California

The invention also contemplates use of recently published antibodies in the context of the invention (see WO 01/19861, WO 01/19990, WO 01/19860, WO 01/19987, WO 01/19990, WO 99/58678, WO 00/59943, WO 01/18014, WO 01/18016, WO 01/18204, WO 01/18042, WO 01/18021, WO 01/18014, WO 01/18046, WO 01/16166, WO 01/15731, WO 01/15728, WO 01/16183, WO 01/16170, WO 01/15732.

The invention is also directed to a method of evaluating dosing, ligand saturation, avidity effects of simultaneous ligand binding on prolonged anchoring and associated benefits (eg. to delay a cancer cell for targeted killing or facilitate transfer of the multifunctional ligand to the targeted cell), cooperative interactions, cross-linking interactions (see J Immunol 2001 Mar 1; 166(5):3256-3265; Nippon Rinsho. 1999 Dec; 57 Suppl:428-32; Harefuah. 2000 Jun 15; 138(12):1046-50.

Leuk Lymphoma. 1998 Mar; 29(1-2):1-15 ) and costimulatory interactions by administering to a test subject two different multifunctional ligands of the invention with cooperating second portions.

With respect to the display of functional peptides on an antibody type scaffold see Nuttal SD; et al." Proteins (1999) 36: 217-227; see also Skerra A., J. Mol. Recognition 2000 July-Aug 13(4): 167-187. The invention also contemplates bispecific multifunctional ligands in which the immune function exerting moiety exerts its function through binding to an immunogenic component or carrier for such component as discussed above, for example an Fc domain fused to a peptide, a heat shock protein (see for example Wang XY, Immunol Invest 2000 May 29(2): 131-7 and references cited therein as well as US6168793; US6071956 ; US05981706; US05948646 Methods for preparation of vaccines against cancer comprising heat shock protein-peptide complexes; US05830464 Compositions and methods for the treatment and growth inhibition of cancer using heat shock/stress protein-peptide complexes in combination with adoptive immunotherapy as well as patents, scientific atricles and patent applications referenced in these patent; with respect to MHC peptide complexes( see for example WO 99/64597, WO 98/03552, WO 98/06749 and references cited therein).

As described above, the invention also contemplates that the lower affinity ligand binding arm of the aforementioned multifunctional ligand (ie. having a high affinity targeting arm and a lower affinity effector arm) is constituted by a high affinity ligand, for example an high affinity antibody or functional fragment thereof, which binds to a target biological effector (eg. a cytokine, chemokine, growth factor, hormone or other biological response modifier or drug) with high affinity, in a manner which permits the effector to continue to bind to its desired target receptor while bound to the antibody (ie. the antibody binds to a portion of the effector which is not critically involved in the effector binding to its receptor) provided that when bound to the effector the antibody or fragment thereof has, when combined with the effector, a suitably lower affinity for the receptor than the ligand binding arm which functions as the high affinity binder has for its target cell marker. In one embodiment the binding moiety which binds to the biological effector binds to it with higher affinity than the affinity that the effector has for the effector receptor. The invention also contemplates that this binding arm can bind to biological effector in a manner which permits it to bind to one receptor but not a related receptor to which the effector would otherwise bind (see examples below). The invention also contemplates that antibody arrays are used to screen for antibodies which are capable of binding to such biological effectors, while bind in situ to their receptors. The invention also contemplates that such binders, when bound to the biological effector, can be used to test their ability to bind to related receptors, such as those within the same family eg. within the same family of TNF like receptors. With respect to antibody microarrays see for example Cahill DJ.Protein and antibody arrays and their medical applications.J Immunol Methods. 2001 Apr;250(1-2):81-91. MacBeath G. Proteomics comes to the surface.Nat Biotechnol. 2001 Sep;19(9):828-9. Clewley JP.Recombinant protein arrays.Commun Dis Public Health. 2000 Dec;3(4):311-2; Holt LJ, Enever C, de Wildt RM, Tomlinson IM. The use of recombinant antibodies in proteomics.Curr Opin Biotechnol. 2000 Oct;11(5):445-9. Walter G, et al.Protein arrays for gene expression and molecular: interaction screening.Curr Opin Microbiol. 2000 Jun;3(3):298-302. de Witdt RM, Mundy CR, Gorick BD, Tomlinson IM.Antibody arrays for high-throughput screening of antibody-antigen interactions.Nat Biotechnol. 2000 Sep;18(9):989-94.Holt LJ, et al.By-passing selection: direct screening for antibody-antigen interactions using protein arrays. Nucleic Acids Res. 2000 Aug 1;28(15):E72 and the references cited therein. The term receptor as used herein for greater certainty includes decoy receptors. Examples of decoy receptors includeTRAIL decoy receptors (APO-2L), CD44 decoy like receptors (hyaluronan), interleukin receptor like protein (IL-17) (see J Biol Chem 2001 Nov 12), CD95-Fc decoy receptor, TRAMP, IL-1 RII receptor, osteoprotegerin (OPG), IL 13Ralpha2.

### Affinity Maturation

Techniques for affinity maturation using high throughput screening techniques to evaluate mutants are well known in the art. Femtomolar affinities have been achieved and it is quite common to obtain nanomolar to picomolar affinities as a result of an affinity maturation process. For example it well known to use techniques of parsimonious mutagenesis to engineer amino acid change at selected "hotspots".With respect to affinity maturation see for example Coia G, Hudson PJ, Irving RA.Protein affinity maturation in vivo using E. coli mutator cells. J Immunol Methods. 2001 May 1;251(1-2):187-93. Manivel V, Sahoo NC, Salunke DM, Rao KV. Maturation of an antibody response is governed by modulations in flexibility of the antigen-combining site. Immunity. 2000 Nov;13(5):611-20. Boder ET, Midelfort KS, Wittrup KD. Directed evolution of antibody fragments with monovalent femtomolar antigen-binding affinity.Proc Natl Acad Sci U S A. 2000 Sep 26;97(20):10701-5. Holler PD, Holman PO, Shusta EV, O'Herrin S, Wittrup KD, Kranz DM.In vitro evolution of a T cell receptor with high affinity for peptide/MHC. Proc Natl Acad Sci U S A. 2000 May 9;97(10):5387-92. Daugherty PS, Chen G, Iverson BL, Georgiou G.Quantitative analysis of the effect of the mutation frequency on the affinity maturation of single chain Fv antibodies. Proc Natl Acad Sci U S A. 2000 Feb 29;97(5):2029-34. VanAntwerp JJ, Wittrup KD. Fine affinity discrimination by yeast surface display and flow cytometry.Biotechnol Prog. 2000 Jan-Feb;16(1):31-7. Adams GP, Schier R.Generating improved single-chain Fv molecules for tumor targeting. J Immunol Methods. 1999 Dec 10;231(1-2):249-60. Daugherty PS, Chen G, Olsen MJ, Iverson BL, Georgiou G. Antibody affinity maturation using bacterial surface display. Protein Eng. 1998 Sep;11(9):825-32. Wong YW, Kussie PH, Parhami-Seren B, Margolies MN. Modulation of antibody affinity by an engineered amino acid substitution. J Immunol. 1995 Apr 1;154(7):3351-8. Balint RF, Larrick JW.Antibody engineering by parsimonious mutagenesis. Gene. 993 Dec 27;137(1):109-18. Schillbach JF, Near RI, Bruccoleri RE, Haber E, Jeffrey PD, Novotny J,Sheriff S, Margolies MN. Modulation of antibody affinity by a non-contact residue. Protein Sci. 1993 Feb;2(2):206-14.Chames P, Baty D. Engineering of an anti-steroid antibody: amino acid substitutions changeantibody fine specificity from cortisol to estradiol.Clin Chem Lab Med. 1998 Jun;36(6):355-9. Kussie PH, Parhami-Seren B, Wysocki LJ, Margolies MN.A single engineered amino acid substitution changes antibody fine specificity. J Immunol. 1994 Jan 1;152(1):146-52, as well as references cited therein.

With respect to generation of high affinity antibodies and affinity maturation of antibodies see also Hanes J. Nat. Biotechnol. 2000 Dec; 18(12): 1287-92; references in Hudson PJ Exp. Opin. Invest. Drugs (2000) 9(6) 1231-1242; Toran JL et al Evr. J. Immunol. 2001 Jan; 31(1) 128-137. Nielson VB et al. Cancer Res 2000 Nov 15; 60 (22) 6434-40 Adams Gp, Journal of Immunological Methods (1999) 249-260; Chowdhury PS et al (June 1999) Nature Biotechnology Vol 17 p. 568With respect to strategies and recent technologies which have application to the invention see references in Hudson PJ Exp. Opin. Invest. Drugs (2000) 9(6) 1231-1242 and in particular references relating to strategies to achieve multivalency and multispecificity; recruitment of viruses, ADEPT, photoactivation of cytotoxic radionuclides; surface receptor cross-linking; (see also Eur. J. Immunol 2000 30(10) 3006), use of anti-B antibodies; immunocytokines (see also Lode HN Immul. Res. 2000, 21 (2-3) 279-88; Gillies SD Cancer Research 59 2159-2166 May 1999; Lode HN et al Drugs of Today 2000 36(5) 3221-336).

With respect to practicial size limitations and pharmacokinetics of various types of antibodies and fragments see Colcher D. et al. G.J. Nucl. Med (1999) 43: 132-139; Wu AM et al G.J. Nucl. Med 2000 Sep; 44(3): 268:83; Williams LE et al Med Phys 2000 May 27(5): 988-941 lkomi F lymphology 32 (1999) 90-102.

With respect to the construction of diabodies see also Takemura SI et al. Protein Eng. 2000 Aug;13(8) 583-8; Biomol. Eng. 2001 Sept;18(2):31-40.

With respect to anti-cancer antibodies see also 6,180,357.

### Other References and Important Antibodies:

Agonist Antibodies see USP 6342220; USP 6475487; WO 00/75348; Affinity maturation US 6,127,524; WO 01/14424; WO 01/009192; Pre-targetting WO 02/082041. US 2002/0114808; US 6,458, 933; EP 1194167; WO 0074718; WO 99/66951; US 6492496; WO 02/058717; WO01/62905: WO02/079232; WO00/78815; EP 0654085; US 6492497; EP1251695; WO 0202641; WO1/667754

With respect to technologies to produce multivalent and/or multispecific antibodies see also USP 6, 172, 197; WO 92/01047; WO 93/11161; WO 94/07921; WO 94/13804; Helfrich W. et al. Journal of Immunological Methods 237 (2000) 131-145. Proceedings of 11^{th} IBC Conference on Antibody Engineering; WO 01/85795; Monoclonal antibodies may be routinely produced as taught by Harlow, E. and D. Lane, (1988) ANTIBODIES: A Laboratory Manual, Cold Spring Harbor Laboratory, Cold Spring Harbor N.Y. Humanized antibodies may be routinely produced as taught, for example, by U.S. Pat. No. 5,585,089 and U.S. Pat. No. 5,530,101. Techniques for engineering antibodies are well known and described in Winter and Millstein (1991) Nature 349:293, and Larrich and Fry (1991) Hum. Antibod. and Hybridomas 2:17. One having ordinary skill in the art may use well known techniques and starting materials and/or commercially available expression vectors and systems that are readily available and known in the art. See e.g., Sambrook et al., Molecular Cloning a Laboratory Manual, Second Ed. Cold Spring Harbor Press (1989).

Examples of radionuclides useful as toxins in radiation therapy are well known. Some examples are referred to below. Auger emitters may be preferred for internalizing antibodies. As suggested above, the term antibody is used interchangeably with antibody fragment and antigen binding fragment and includes a whole antibody; antibody fragment a portion of an antibody such as a scFV F(ab') ₂ F(ab) ₂. Fab', Fab, dAb, microbodies (WO00 29004) or the like or multivalent such fragments, including those itemized or referenced herein. Regardless of structure, an antibody fragment can be made to bind with the same antigen that is recognized by the intact antibody. More particularly, in addition to fragments formed by enzymaic digestion of an intact Ab the term antibody or "antibody fragment" unless otherwise stated also includes any synthetic or genetically engineered protein that acts like an antibody by binding to a specific antigen to form a complex including/as applicable, cysteine noose peptides and minimal recognition units consisting of the amino acid residues that mimic the hypervariable region. Although fully human antibodies, for example, antibodies generated via human-human hybridomas or through phage display using human antibody based libraries, are preferred, the invention does not preclude other strategies to avoid a HAMA type response.

A chimeric antibody is a recombinant protein that contains the variable domains and complementary determining regions derived from, for example, a rodent antibody, while the remainder of the antibody molecule is derived from a human antibody.

With respect to stability engineering of scFv fragments for enhanced mulfunctional ligands comprising scfvs see J Mol Biol 2001 Feb 2; 305(5):989-1010.

Humanized antibodies are recombinant proteins in which murine LDR's of a monoclonal antibody have been transferred from heavy and light variable chains of the murine immunoglobulin into a human variable domain.

The term therapeutic agent as used herein, is a molecule or atom which is conjugated etc. to an antibody moiety to produce combination including a conjugate which is useful for therapy. Examples of therapeutic agents include drugs, toxins, immunomodulators, chelators, boron compounds, photoactive agents or dyes, and radioisotopes.

The term "a naked antibody" may be used to refer specifically to an entire antibody, as opposed to an antibody fragment, which is not conjugated with a therapeutic agent. Naked antibodies include both polyclonal and monoclonal antibodies, as well as certain recombinant antibodies, such as chimeric and humanized antibodies.

The term immunoconjugate may be used to refer a conjugate of an antibody component with a therapeutic agent.

As used herein, the term antibody fusion protein refers to a recombinant molecule that comprises an antibody component and a second functional component for example a therapeutic agent. Examples of therapeutic agents suitable for such fusion proteins include immunomodulators ("antibody-immunomodulator fusion protein") and toxins ("antibody-toxin fusion protein").

Production of Antigen - Specific Monoclonal Antibodies, Rodent monoclonal antibodies to antigen can be obtained by methods known to those skilled in the art. See generally, for example, Kohler and Milstein, Nature 256:495 (1975), and Coligan et al. (eds.), Current Protocols in Immunology, Vol. 1, pages 2.5.1-2.6.7 (John Wiley & Sons 1991) ["Coligan"]. Briefly, monoclonal antibodies can be obtained by injecting mice with a composition comprising the antigen in a question (Ag), verifying the presence of antibody production by removing a serum sample, removing the spleen to obtain B-lymphocytes, fusing the B-lymphocytes with myeloma cells to produce hybridomas, cloning the hybridomas, selecting positive clones which produce anti-Ag antibodies, culturing the clones that produce antibodies to the antigen, and isolating the antibodies from the hybridoma cultures. Transgenic mice having for example engineered immune systems to create human antibodies such those used by Medarex and Abgenix are also contemplated for use herein to create suitably targeted antibodies.

Monoclonal antibodies can be isolated and purified from hybridoma cultures by a variety of well-established techniques. Such isolation techniques include affinity chromatography with Protein-A Sepharose, size-exclusion chromatography, and ion-exchange chromatography. See, for example, Coligan at pages 2.7.1-2.7.12 and pages 2.9.1-2.9.3. Also, see Baines et al., "Purification of Immunoglobulin G (IgG) ," in Methods in Molecular Biology, Vol. 10, pages 79-104 (The Humana Press, Inc. 1992).

With respect to relevant molecular biology techniques See also, for example, Ausubel et al., (eds.), CURRENT PROTOCOLS IN MOLECULAR BIOLOGY, pages 8.2.8 to 8.2.13 (1990) ["Ausubel"]. Also, see Wosnick et al., Gene 60:115 (1987); and Ausubel et al. (eds.), Short Protocols in Molecular Biology, 3rd Edition, pages 8--8 to 8-9 (John Wiley & Sons, Inc. 1995). Established techniques using the polymerase chain reaction provide the ability to synthesize genes as large as 1.8 kilobases in length. Adang et al., Plant Molec. Biol. 21:1131 (1993) Bambot et al., PCR Methods and Applications 2:266 (1993); Dillon et al., "Use of the Polymerase Chain Reaction for the Rapid Construction of Synthetic Genes," in Methods in Molecular Biology, Vol. 15: PCR Protocols: Current Methods and Applications, White (ed.), pages 263-268, (Humana Press, Inc. 1993).

Techniques for constructing chimeric antibodies are well-known to those of skill in the art. As an example, Leung et al., Hybridoma 13:469 (1994).

In yet another embodiment, an antibody of the present invention is a "humanized" monoclonal antibody. That is, mouse complementarity determining regions are transferred from heavy and light variable chains of the mouse immunoglobulin into a human variable domain, followed by the replacement of some human residues in the framework regions of their murine counterparts. Humanized monoclonal antibodies in accordance with this invention are suitable for use in therapeutic methods. General techniques for cloning murine immunoglobulin variable domains are described, for example, by the publication of Orlandi et al., Proc. Nat'l Acad. Sci. USA 86: 3833 (1989). Techniques for producing humanized monoclonal antibodies are described, for example, by Jones et al., Nature 321:522 (1986), Riechmann et al., Nature 332:323 (1988), Verhoeyen et al., Science 239:1534 (1988), Carter et al., Proc. Nat'l Acad. Sci. USA 89:4285 (1992), Sandhu, Crit. Rev. Biotech. 12:437 (1992), and Singer et al., J. Immun. 150:2844 (1993). The publication of Leung et al., Mol. Immunol. 32:1413 (1995), describes the construction of humanized LL2 antibody.

In a preferred embodiment of the invention the multifunctional ligand has a unique portion which differentiates it from other antibodies and preferably other co-administered different multifunctional ligands, which unique portion, allows the multifunctional ligand to be efficiently segregated on an immunoaffinity column. In the case of differentiating a single multifunctional ligand an anti-idiotype (assuming the first portion consists of an antibody) or other antibody uniquely recognizing the first portion could be employed. Modifying a portion of the first portion, for example in the case where it is antibody component and creating a antibody thereto, for example by phage display, is a matter of routine skill in the arts of antibody engineering and phage display.

In another embodiment, an antibody of the present invention is a human monoclonal antibody. Such antibodies are obtained from transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994). Lonberg et al., Nature 368:856 (1994), and Taylor et al., Int. Immun. 6:579 (1994).

Examples of Production of Antibody Fragments

Antibody fragments can be prepared, for example, by proteolytic hydrolysis of an antibody or by expression in E. coli of the DNA coding for the fragment.

Antibody fragments can be obtained by pepsin or papain digestion of whole antibodies by conventional methods. For example, antibody fragments can be produced by enzymatic cleavage of antibodies with pepsin to provide a 5 S fragment denoted F(ab')₂. This fragment can be further cleaved using a thiol reducing agent, and optionally a blocking group for the sulfhydryl groups resulting from cleavage of disulfide linkages, to produce 3.5 S Fab' monovalent fragments. Alternatively, an enzymatic cleavage using pepsin produces two monovalent Fab fragments and an Fc fragment directly. These methods are described, for example, by Goldenberg, U.S. Pat. Nos. 4,036,945,and 4,331,647 and references contained therein. Also, see Nisonoff et al., Arch Biochem. Biophys. 89:230 (1960); Porter, Biochem: J. 73:119 (1959), Edelman et al., in Methods in Enzymology Vol 1, page 422 (Academic Press 1967), and Coligan at pages 2.8.1-2.8.10 and 2.10.-2.10.4.

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of V_{H} and V_{L} chains. This association can be noncovalent, as described in Inbar et al., Proc. Nat'l Acad. Sci. USA 69:2659 (1972). Alternatively, the variable chains can be linked by an intermolecular disulfide bond or cross-linked by chemicals such as glutaraldehyde. See, for example, Sandhu, supra.

Preferably, the Fv fragments comprise V_{H} and V_{L} chains which are connected by a peptide linker. These single-chain antigen binding proteins (scFv) are prepared by constructing a structural gene comprising DNA sequences encoding the V_{H} and V_{L} domains which are connected by an oligonucleotide. The structural gene is inserted into an expression vector which is subsequently introduced into a host cell, such as E. coli. The recombinant host cells synthesize a single polypeptide chain with a linker peptide bridging the two V domains. Methods for producing scFvs are described, for example, by Whitlow et al., Methods: A Companion to Methods in Enzymology 2:97 (1991). Also see Bird et al., Science 242:423 (1988), Ladner et al., U.S. Pat. No. 4,946,778, Pack et al., Bio/Technology 11:1271 (1993), and Sandhu, supra.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells.

See, for example, Larrick et al., Methods: A Companion to Methods in Enzymology 2:106 (1991); Courtenay-Luck, "Genetic Manipulation of Monoclonal Antibodies," in Monoclonal Antibodies: Production, Engineering and Clinical Application, Ritter et al. (eds.), pages 166-179 (Cambridge University Press 1995); and Ward et al., "Genetic Manipulation and Expression of Antibodies," in Monoclonal Antibodies: Principles and Applications, Birch et al., (eds.), pages 137-185 (Wiley-Liss, Inc. 1995).

Preparation of Immunoconjugates

The present invention contemplates immunoconjugates to assess and effect treatment of various disease conditions. Such immunoconjugates can be prepared by indirectly conjugating a therapeutic agent to an antibody component. For example, general techniques are described in Shih et al., Int. J. Cancer 41:832-839 (1988); Shih et al., Int. J. Cancer 46:1101-1106 (1990); and Shih et al., U.S. Pat. No. 5,057,313. The general method involves reacting an antibody component having an oxidized carbohydrate portion with a carrier polymer that has at least one free amine function and that is loaded with a plurality of drug, toxin, chelator, boron addends, or other therapeutic agent. This reaction results in an initial Schiff base (imine) linkage, which can be stabilized by reduction to a secondary amine to form the final conjugate.

The carrier polymer is preferably an aminodextran or polypeptide of at least 50 amino acid residues, although other substantially equivalent polymer carriers can also be used. Preferably, the final immunoconjugate is soluble in an aqueous solution, such as mammalian serum, for ease of administration and effective targeting for use in therapy. Thus, solubilizing functions on the carrier polymer will enhance the serum solubility of the final immunoconjugate. In particular, an aminodextran will be preferred.

The process for preparing an immunoconjugate with an aminodextran carrier typically begins with a dextran polymer, advantageously a dextran of average molecular weight of about 10,000-100,000. The dextran is reacted with an oxidizing agent to effect a controlled oxidation of a portion of its carbohydrate rings to generate aldehyde groups. The oxidation is conveniently effected with glycolytic chemical reagents such as NalO.sub.4, according to conventional procedures.

The oxidized dextran is then reacted with a polyamine, preferably a diamine, and more preferably, a mono- or polyhydroxy diamine. Suitable amines include ethylene diamine, propylene diamine, or other like polymethylene diamines, diethylene triamine or like polyamines, 1,3-diamino-2-hydroxypropane, or other like hydroxylated diamines or polyamines, and the like. An excess of the amine relative to the aldehyde groups of the dextran is used to insure substantially complete conversion of the aldehyde functions to Schiff base groups.

A reducing agent, such as NaBH₄, NaBH₃ CN or the like, is used to effect reductive stabilization of the resultant Schiff base intermediate. The resultant adduct can be purified by passage through a conventional sizing column to remove cross-linked dextrans.

Other conventional methods of derivatizing a dextran to introduce amine functions can also be used, e.g., reaction with cyanogen bromide, followed by reaction with a diamine.

The aminodextran is then reacted with a derivative of the particular drug, toxin, chelator, immunomodulator, boron addend, or other therapeutic agent to be loaded, in an activated form, preferably, a carboxyl-activated derivative, prepared by conventional means, e.g., using dicyclohexylcarbodiimide (DCC) or a water soluble variant thereof, to form an intermediate adduct.

Alternatively, polypeptide toxins such as pokeweed antiviral protein or ricin A-chain, and the like, can be coupled to aminodextran by glutaraldehyde condensation or by reaction of activated carboxyl groups on the protein with amines on the aminodextran.

Chelators for radiometals or magnetic resonance enhancers are well-known in the art. Typical are derivatives of ethylenediaminetetraacetic acid (EDTA) and diethylenetriaminepentaacetic acid (DTPA). These chelators typically have groups on the side chain by which the chelator can be attached to a carrier. Such groups include, e.g., benzylisothiocyanate, by which the DTPA or EDTA can be coupled to the amine group of a carrier. Alternatively, carboxyl groups or amine groups on a chelator can be coupled to a carrier by activation or prior derivatization and then coupling all by well-known means.

Boron addends, such as carboranes, can be attached to antibody components by conventional methods. For example, carboranes can be prepared with carboxyl functions on pendant side chains, as is well known in the art. Attachment of such carboranes to a carrier, e.g., aminodextran, can be achieved by activation of the carboxyl groups of the carboranes and condensation with amines on the carrier to produce an intermediate conjugate. Such intermediate conjugates are then attached to antibody components to produce therapeutically useful immunoconjugates, as described below.

A polypeptide carrier can be used instead of aminodextran, but the polypeptide carrier must have at least 50 amino acid residues in the chain, preferably 100-5000 amino acid residues. At least some of the amino acids should be lysine residues or glutamate or aspartate residues. The pendant amines of lysine residues and pendant carboxylates of glutamine and aspartate are convenient for attaching a drug, toxin, immunomodulator, chelator, boron addend or other therapeutic agent. Examples of suitable polypeptide carriers include polylysine, polyglutamic acid, polyaspartic acid, copolymers thereof, and mixed polymers of these amino acids and others, e.g., serines, to confer desirable solubility properties on the resultant loaded carrier and immunoconjugate.

Conjugation of the intermediate conjugate with the antibody component is effected by oxidizing the carbohydrate portion of the antibody component and reacting the resulting aldehyde (and ketone) carbonyls with amine groups remaining on the carrier after loading with a drug, toxin, chelator, immunomodulator, boron addend, or other therapeutic agent Alternatively, an intermediate conjugate can be attached to an oxidized antibody component via amine groups that have been introduced in the intermediate conjugate after loading with the therapeutic agent. Oxidation is conveniently effected either chemically, e.g., with NalO₄ or other glycolytic reagent, or enzymatically, e.g., with neuraminidase and galactose oxidase. In the case of an aminodextran carrier, not all of the amines of the aminodextran are typically used for loading a therapeutic agent. The remaining amines of aminodextran condense with the oxidized antibody component to form Schiff base adducts, which are then reductively stabilized, normally with a borohydride reducing agent.

Analogous procedures are used to produce other immunoconjugates according to the invention. Loaded polypeptide carriers preferably have free lysine residues remaining for condensation with the oxidized carbohydrate portion of an antibody component. Carboxyls on the polypeptide carrier can, if necessary, be converted to amines by, e.g., activation with DCC and reaction with an excess of a diamine.

The final immunoconjugate is purified using conventional techniques, such as sizing chromatography on Sephacryl S-300.

Alternatively, immunoconjugates can be prepared by directly conjugating an antibody component with a therapeutic agent. The general procedure is analogous to the indirect method of conjugation except that a therapeutic agent is directly attached to an oxidized antibody component.

For application to linking MHC I/II peptide/B7 molecules to a latex which has previously conjugated to biotin, for avidin assisted linking to a multifunctional ligand, it will be appreciated that biotin can be conjugated to a part of a latex sphere which is then linked to MHC peptide and B7 molecules by placing the spheres in a confluent layer or in the spheres in a microwells such that only part of the sphere is exposed for conjugation and then coating the spheres onto avidin coated plates for the B7 and MHC linkage.

It will be appreciated that other therapeutic agents can be substituted for the chelators described herein. Those of skill in the art will be able to devise conjugation schemes without undue experimentation.

As a further illustration, a therapeutic agent can be attached at the hinge region of a reduced antibody component via disulfide bond formation. For example, the tetanus toxoid peptides can be constructed with a single cysteine residue that is used to attach the peptide to an antibody component. As an alternative, such peptides can be attached to the antibody component using a heterobifunctional cross-linker, such as N-succinyl 3-(2-pyridyldithio)proprionate (SPDP). Yu et al., Int. J. Cancer 56:244 (1994). General techniques for such conjugation are well-known in the art. See, for example, Wong, CHEMISTRY OF PROTEIN CONJUGATION AND CROSS-LINKING (CRC Press 1991); Upeslacis et al., "Modification of Antibodies by Chemical Methods," in MONOCLONAL ANTIBODIES: PRINCIPLES AND APPLICATIONS, Birch et al. (eds.), pages 187-230 (Wiley-Liss, Inc. 1995); Price, "Production and Characterization of Synthetic Peptide-Derived Antibodies," in MONOCLONAL ANTIBODIES: PRODUCTION, ENGINEERING AND CLINICAL APPLICATION, Ritter et al. (eds.), pages 60-84 (Cambridge University Press 1995).

As described above, carbohydrate moieties in the Fc region of an antibody can be used to conjugate a therapeutic agent. However, the Fc region is absent if an antibody fragment is used as the antibody component of the immunoconjugate. Nevertheless, it is possible to introduce a carbohydrate moiety into the light chain variable region of an antibody or antibody fragment. See, for example, Leung et al., J. Immunol. 154:5919 (1995); Hansen et al., U.S. Pat. No. 5,443,953 (1995). The engineered carbohydrate moiety is then used to attach a therapeutic agent.

In addition, those of skill in the art will recognize numerous possible variations of the conjugation methods. For example, the carbohydrate moiety can be used to attach polyethyleneglycol in order to extend the half-life of an intact antibody, or antigen-binding fragment thereof, in blood, lymph, or other extracellular fluids. Moreover, it is possible to construct a "divalent immunoconjugate" by attaching therapeutic agents to a carbohydrate moiety and to a free sulfhydryl group. Such a free sulfhydryl group may be located in the hinge region of the antibody component

Methods for determining the binding specificity of an antibody are well-known to those of skill in the art. General methods are provided, for example, by Mole, "Epitope Mapping," in METHODS IN MOLECULAR BIOLOGY, VOLUME 10: IMMUNOCHEMICAL PROTOCOLS, Manson (ed.), pages 105-116 (The Humana Press, Inc. 1992). More specifically, competitive blocking assays for example to determine CD22 epitope specificity are described by Stein et al., Cancer Immunol. Immunother. 37:293 (1993), and by Tedder et al., U.S. Pat. No. 5,484,892 (1996).

In another aspect the invention is directed to a bispecifc ligand, preferably a bispecifc antibody, comprising at least a first ligand, preferably an antibody component, which binds specifically to a first cell surface associated ligand and at least a second ligand, preferably a second antibody component which binds specifically to a second cell surface associated ligand on the same cell, and wherein the functional affinity of at least one and preferably both of said antibody components is selected so as to substantially limit functional binding unless both of said first and second antibody components are substantially contemporaneously bound to said cell. It is known to provide bifunctional ligands wherein functional binding, for example, to accomplish signal transduction, is predicated on both ligands being bound or cross-linking. However this effect is not contemplated to be predicated on differentially controlling the functional affinity of the respective ligands. According to a broad aspect of this invention (in which inclusion of a ligand which binds to a lymphatic vessel associated marker is optional), the invention excludes known such bispecific ligands which inherently have a suitable differential functional affinity. Such bispecific ligand are mentioned herein. By controlling the affinity of at least one of said ligands, for example where the functional affinity of one said ligands is substantially less than that of the other ligand the invention contemplates that a substantially greater percentage of the administered dose of the bispecific ligand will affect cells in which only both ligands are present, and/or that a reduced percentage of the dose administered will functionally bind to the cells in virtue only of the reduced functional affinity ligand. The invention also contemplates that functional affinity of one ligand is greatly increased to establish the functional affinity differential and that the functional affinity of both ligands is reduced relative to that of a standard, for example relative to that of a comparable ligands in hand or known in the art or identified by phage display, ribsome display or other comparable techniques using a single such ligand. The invention also contemplates that a microarray (or library) of bispecific ligands in which for example, the bispecific ligand is "tethered" (ie. immobilized) directly or indirectly in virtue of one or more amino acid residues which are positioned within the molecule to preferably minimally interfere with any binding, and in which the signal (eg its intensity) associated with a single ligand binding interaction can be differentiated from a two or more ligand interactions, for example cell surface binding (altematively the ligands or cell may be immobilized) and that ribsome and phage display could be adapted to bispecific single domain antibodies constituting a single chain (see references herein) by elongating the end of the chain from which the molecule is tethered. The invention contemplates that the affinity of one such ligand may be fixed and that the variability in members of the library lies in the permutations of certain key residues to which binding is attributable which can readily be identified by persons skilled in the art. The invention also contemplates assessing single ligand binding capability of successful bi-ligand binders for example by blocking the other (non-assessed at that time) ligand (eg. with correlative ligand or a mimotope thereof) and for example determining limited or non-existent such binding to as well as using inclined ligand testing surfaces for washing over the correlative ligand, for example of defined surface area, including preferably defined lengths and widths and concentrations / distributions / amounts of the bound ligand, where the degree of incline is selected to roughly simulate the micro-environment of the comparable in vivo target, be it a stationary cell with a roughly defined average shear force of bathing fluids eg. within a tumor or in the lymphatic system, or a mobile cell within a vein, artery, or lymphatic vessel, including those of different sizes. The invention is also directed to a mthod of generating a target ligand or improving the target specificity of any ligand by using a population of variants of that ligand within a micro-environment simulated microarray system in which the at least one of the follwing factors is simulated: concentration or amount or distribution of correlative ligand, shear force and shape using length and width parameters to simulates intraluminal diameter and length.The invention also contemplates in the case of a multifunctional ligand or in the case of a bispecific or multispecfic ligand (as herein described) that the affinity of its component binding ligands may be selected for venous or arterial tageting or to accommodate lymphatic system targeting or targeting within or through tissues or combinations of the aforementioned eg. median, average or or weighted compromises to improve desired targeting. In a preferred embodiment the first ligand is selected on the basis of its ability to at least partially discriminate between a target population of cells (eg. a ligand that is "associated" with a target population of cells) and a non-target population of cells (in one embodiment it is selected so as to have no other effect other than binding for targeting purposes) and the second ligand is selected for its ability to modulate the activity of the targeted cell, optionally in virtue of binding alone eg. without delivering a payload (the term modulate referring broadly to any desired effect on the cell or its functionality) In this case the functional affinity for the ligand which is targeted for modulating the activity of the cell is selected so as to reduce the likelihood of binding unless binding has first or contemporaneously occurred to the first ligand targeted for selectivity (eg. the second ligand would have monovalent as opposed to divalent binding to the ligand required for selectivity and/or from 0.20 to 10⁻⁹ fold reduction in affinity (for example as measured by Biacore) relative to the binding affinity for the first ligand. This reduction in affinity is preferably greater than a 100% reduction in affinity (multiply by 0.1), preferably greater than a 200% reduction in affinity, preferably greater than a 300% reduction in affinity, preferably greater than a 400% reduction in affinity, preferably greater than a 500% reduction in affinity, preferably greater than a 600% reduction in affinity, preferably greater than a 700% reduction in affinity, preferably greater than a 800% reduction in affinity, preferably greater than a 900% reduction in affinity, preferably greater than a 1000% reduction in affinity, preferably greater than a 2000% reduction in affinity, preferably greater than a 3000% reduction in affinity, preferably greater than a 4000% reduction in affinity, preferably greater than a 5000% reduction in affinity, preferably greater than a 6000% reduction in affinity, preferably greater than a 7000% reduction in affinity, preferably greater than a 8000% reduction in affinity, preferably greater than a 9000% reduction in affinity, preferably greater than a 10000% reduction in affinity, preferably greater than a 20000% reduction in affinity, preferably greater than a 30000% reduction in affinity, preferably greater than a 40000% reduction in affinity, preferably greater than a 50000% reduction in affinity, preferably greater than a 60000% reduction in affinity, preferably greater than a 70000% reduction in affinity, preferably greater than a 80000% reduction in affinity, preferably greater than a 90000% reduction in affinity, preferably greater than a 100000% reduction in affinity, preferably greater than a 500000% reduction in affinity, preferably greater than a 1000000% reduction in affinity, preferably greater than a 10000000% reduction in affinity, preferably greater than a 20000000% reduction in affinity, preferably a greater than 3000000% reduction in affinity, preferably a greater than 40,000,000% reduction in affinity, preferably a greater than 50000000% reduction in affinity, preferably a greater than 60000000% reduction in affinity, preferably a greater than 70000000% reduction in affinity, preferably a greater than 80000000% reduction in affinity, preferably a greater than 90000000% reduction in affinity preferably a greater than 100,000,000% reduction in affinity, preferably a reduction in affinity of between one and two orders of magnitude, preferably a reduction in affinity of between two and three orders of magnitude, preferably a reduction in affinity of between three and four orders of magnitude, preferably a reduction in affinity of between four and five orders of magnitude, preferably a reduction in affinity of between five and six orders of magnitude, preferably a reduction in affinity of between six and seven orders of magnitude preferably a reduction in affinity of between seven and eight orders of magnitude, preferably a reduction in affinity of between eight and nine orders of magnitude, preferably a reduction in affinity of between nine and ten orders of magnitude.

It will be appreciated that a suitable reduction in affinity, if any, will depend on the valency of the respective first and second ligands and the selected affinity of the first ligand, which for example may have been augmented. The invention also contemplates a trispecific (and triavalent) ligand in which two ligands differently define its specificity to reduce the likelihood of an undesired effect attributable to the function exerting moiety binding alone. In terms of the physical constitution of a ligand having a trispecific binding capability, the invention also contemplates linking three monovalent dabs, MRUs or the like or mixed combinations thereof or two bivalent dabs, MRUs or the like or mixed combinations thereof (see WO 99142077, US 6174691, WO0029004, Camel single-domain antibodies as modular building units in J Biol Chem. 2000 Oct 25, & Mulligan-Kehoe U.S. patents including US 5702892, US 5824520; se also US 6040136) (in the latter case optionally one or both may be bispecific and linked by well known methods in the art (see WO 99/42077, Celltech's TFM, leucine zippers, US 5,910,573, US5892020, EP 0654085B, see also EP 0318554B). The term functional binding is used to refer to binding which yields the desired effect, for example a therapeutic effect on a target cell population attributable to the binding to one or both ligands. Using the previous example, one ligand, eg. the first ligand, may be used to target activated immune cells, and the second ligand may be different and may upon being bound to, for example result in inactivation, anergy, apoptosis or reduced capacity for endothelial adhesion of the immune cell. In this case, the invention contemplates that the functional affinity of the antibody component which binds to the second ligand is selected such that binding is unlikely to occur without binding to the specificity dictating ligand, for example the ratio of targeted relative non-targeted cells affected by the dose administered is approximately 1.10 to 1, preferably approximately 1.15 to 1, more preferably approximately 1.20 to 1, more preferably approximately 1.25 to 1, more preferably approximately 1.30 to 1, more preferably approximately 1.35 to 1, more preferably approximately 1.40 to 1, more preferably approximately 1.45 to 1, more preferably approximately 1.50 to 1, more preferably approximately 1.55 to 1, more preferably approximately 1.60 to 1, more preferably approximately 1.60 to 1, more preferably approximately 1.65 to 1, more preferably approximately 1.70 to 1, more preferably approximately 1.75 to 1, more preferably approximately 1.80 to 1, more preferably approximately 1.85 to 1, more preferably approximately 1.90 to 1, more preferably approximately 1.95 to 1, more preferably approximately 2 to 1, more preferably greater than 2 to 1, more preferably approximately greater than 3 to 1, more preferably approximately greater than 4 to 1, more preferably greater than 5 to 1, more preferably greater than 6 to 1, more preferably greater than 7 to 1, more preferably greater than 8 to 1 , more preferably greater than 9 to 1 , more preferably greater than 10 to 1 , more preferably greater than 20 to 1 , more preferably greater than 30 to 1 , more preferably greater than 40 to 1 , more preferably greater than 50 to 1 , more preferably greater than 60 to 1, more preferably greater than 70 to 1, more preferably greater than 80 to 1, more preferably greater than 90 to 1, more preferably greater than 100 to 1, more preferably greater than 500 to 1 , more preferably greater than 1000 to 1, more preferably greater than 10,000 to 1, more preferably greater than 100,000 to 1, more preferably greater than 500,000 to 1 more preferably greater than 1,000,000 to 1.

It will be appreciated by persons skilled in the art that the foregoing aspects of the invention apply to a variety of different combinations of immune function or other therapeutic function exerting ligands and specificity dictating ligands including those involved in immune signaling, stimulatory, co-stimulatory, inhibitory, adhesion or other interactions, including without limitation, cytokine receptors, ligands associated with immune cell adhesion, ligands to which binding results in stimulation, activation, apoptosis, anergy or costimulation, or ligands which differentiate between different populations or subpopulations or immune cells (see eg. US 6135941, WO 00/63251, WO 00/61132, US 6120767), including sub-populations of B cells and T cells (see for example US 6197524) activated versus non-activated lympocytes, diseased or disease-causing cells versus non-diseased / disease causing lymphocytes (see for exampleWO O1/13945A1, US 6132980,) and specific immune cell clones for example those having specific Ig type and MHC-peptide type ligands / and correlative ligands. Examples of such ligands include CCR5, CTLA-4, LFA-1, LFA-3. ICAMs eg. ICAM-1, CD2, CD3, CD4 (eg see US 6,136,310), CD18, CD22, CD40, CD44; CD80, CD86, CD134 and CD154, to name only a few (see also US6087475: PF4A receptor) (see also Glennie MJ et al. Clinical Trial of Antibody Therapy. Immunology Today Aug 2000, Vol. 21 (no. 8) p.406).

The invention also contemplates that the therapeutic function or immune function effecting ligand is also a specificity imparting ligand, which in the case of for example, an antigen presenting cell may be an antibody which recognizes and binds to a specific MHC peptide complex, as is established in the art (see pertinent Chames et al. references herein, see also WO 97/02342 , Direct selection of a human antibody fragment directed against the tumor T-cell epitope HLA-A1-MAGE-A1 from a nonimmunized phage-Fab library. Proc Natl Acad Sci U S A. 2000 Jul 5; 97(14):7969-74). In this case it will be appreciated that the APC targeting ligand assist the particular MHC peptide binding antibody to bind to its target.

See also WO 97/07819 which is hereby disclaimed with respect to all relevant aspects of the invention herein insofar as inherently disclosed therein. See also US 5,770,403 with respect to antibodies which bind to cytokines.

In one embodiment, the respective antibody components of the multispecific ligand recognize a substantially different subset of non-targetted tissues so that functional: binding to a non-targetted tissue is substantially precluded. It will be appreciated that this strategy can be accomplished with two different antibodies have differing and preferably non-overlapping normal ie. non-targeted tissue distributions. In a preferred embodiment the target cell is a cancer cell and the respective first and second cell surface associated ligands are expressed on different subsets of normal cells, which are non-overlapping subsets, so as to minimize deleterious normal cell targeting and distibute the undesired effects or normal cell targeting (eg. with a toxin), to different cell populations. For example in the case of tumor cell targeting one or both ligands may be expressed exclusively on a single tumor type (eg. a human sarcoma or carcinoma, e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell-carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, e.g., acute lymphocytic leukemia and acute myelocytic leukemia (mycloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenstrom's macroglobulinemia, and heavy chain disease) *or* a particular category of tumor types (eg. adenocarcinomas, tumors of neuroectodermal origin, or on multiple different tumor types or categories of tumor. One or both components (they may be the same or different) may be a dAb, a scFv, an Fab, a minibody moiety or a substantially intact antibody, for example both may be scFvs and the resulting product may be a diabody, triabody, or tetrabody. For example in a preferred embodiment the bispecific antibody comprises two dAb components comprising linked via a linker (see above) and having at least at least part of a constant region for fusion for example to a toxin (eg. at least a partial hinge region, and preferably also at least a partial CH2 domain (optionally also at least a partial CH3 domain). In another embodiment, a trispecific antibody or tetraspecific antibody with at least two different and preferably 3 or 4 subsets (preferably at least one or more of such subsets being non-overlapping subsets) of non-targeted cell reactivities may be employed in the form of a trispecific or tetraspecific antibody respectively whereby up to three or four different pairs of ligands are targeted, so as further minimize normal cell targeting and also preferably target a heterogenous population of cells within the same tumor. Ligands with distributions on normal tissues are well known, some being referenced herein, for example CEA, CD-20, P53, epidermal growth factor, including known multicarcinomic and pancarcinomic ligands (eg. see US5, 171,665, US 4349528.

The term functional binding is used to mean binding for the purpose of accomplishing the object of the binding, for example binding for a sufficient duration to inhibit or enhance a particular effect, such as cell killing, for example in the case where one both antibody components are selected for their ability to internalize, binding for a sufficient duration to permit internalization, for example to deliver a toxic payload. As discussed above, the term substantially in reference to therapeutic advantage is used to refer to a degree which provides a significant benefit from a therapeutic standpoint.

Examples of tumor associated antigens (eg. WO 01/21828) and targets and related antibodies are referenced throughout the disclosure and the foregoing aspect of the invention is for greater certainty directed to bispecific antibodies (including trispecific and tetraspecific antibodies, optionally including a component which also binds to a lymphatic vessel associated ligand), which target each of the combinations and permuations of the target cell (diseased, disease causing or immune) associated antigens, ligands, epitopes or receptors well known to those skilled in the art, herein directly or indirectly referenced or referenced in the materials herein incorporated by reference (ie. permutations and combinations of pairs or where a tri-or tetra- specific antibody is used possibly permutations of (3 or 4) groups of pairs including for example pairs in which one member is used for targeting and the second is used for modulation puposes such modulation including without limitation, simple binding eg. to deliver a payload, apoptosis inducing (eg. anti-fas), modified vascular adhesion properties (eg. anti-CD44), modified cytokine binding (anti-CCR5) etc.(re: relevant ligands/markers see also USP 6,010,902 and the references cited therein, Samter's Immunologic Diseases, Fifth and Sixth Edition, Lippincott, Frank Austen, MD Michael M. Frank, MD John P. Atkinson, MD Harvey I. Cantor, MD (6th-ISBN: 0-7817-2120-2); Fundamental Virology, Third and Fourth Edition, Lippincott David M. Knipe, PhD Peter M. Howley, MD Diane E. Griffin, MD, PhD Robert A. Lamb, PhD, ScD Malcolm A. Martin, MD Bernard Roizman, ScD Stephen E. Straus, MD (4th-ISBN: 0-7817-1833-3); Arthritis and Allied Conditions - A Textbook of Rheumatology, Thirteenth and Fourteenth Editions, William J. Koopman, MD 14th:ISBN: 0-7817-2240-3, November 2000; Cancer - Principles and Practice of Oncology, Fifth and Sixth Editions, Lippincott, Vincent T. DeVita, Jr., MD Samuel Hellman, MD Steven A. Rosenberg, MD, PhD ISBN: 0-7817-2229-2; Dubois' Lupus Erythematosus, Fifth Edition, Daniel J. Wallace, MD ISBN: 0-683-08665-0, December 1996; Cytokine Therapeutics in Infectious Diseases, Steven M. Holland, MD, PhD, Lippincott, ISBN: 0-7817-1625-X, US 6054561), in each of their permuatations of size/valency (ie. dabs, scFv, diabodies etc herein referenced) as applied to each of the applicable disease conditions herein referenced or otherwise known to those skilled in the art.

With respect to recombinant techniques for producing Fv fragments see also WO 88/01649, WO 88/06630, WO 88/07085, WO 88/07086, and WO 88/09344.

With respect to preparing ligands for specific MHC peptide complexes see also WO 01/22083; Direct selection of a human antibody fragment directed against the tumor T-cell epitope HLA-A1-MAGE-A1 from a nonimmunized phage-Fab library. Proc Natl Acad Sci U S A. 2000 Jul 5;97(14):7969-74.

With respect to bispecific antigen binding constructs that are suitable for for binding to more than one antigen on the same cell see also Schmiedl A et al. Protein Eng 2000 Oct 13(10):725-34.

Preferred immunoconjugates include radiolabeled antibody components and conjugates of an anti-Lyve-1 antibody component and an antbody component which comprises an immunomodulator.

A radiolabeled immunoconjugate may comprise an alpha-emitting radioisotope, a .B-emitting radioisotope, a gamma emitting radioisotope, an Auger electron emitter, a neutron capturing agent that emits alpha-particles or a radioisotope that decays by electron capture. Suitable radioisotopes include ¹⁹⁸ Au, ³² p,. ¹²⁵ I, ¹³¹ I, ⁹⁰ Y, ¹⁸⁶ Re, ¹⁸⁸ Re, ⁶⁷ Cu, ²¹¹ At, and the like.

As discussed above, a radioisotope can be attached to an antibody component directly or indirectly, via a chelating agent. For example, ⁶⁷ Cu, considered one of the more promising radioisotopes for radioimmunotherapy due to its 61.5 hour half-life and abundant supply of beta particles and gamma rays, can be conjugated to an antibody component using the chelating agent, p-bromoacetamido-benzyl-tetraethylaminetetraacetic acid (TETA). Chase, "Medical Applications of Radioisotopes," in Remington's Pharmaceutical Sciences, 18th Edition, Gennaro et al. (eds.), pages 624-652 (Mack Publishing Co. 1990) (see also 19^{th} edition of Reminton's). Alternatively, ⁹⁰ Y, which emits an energetic beta particle, can be coupled to an antibody component using diethylenetriaminepentaacetic acid (DTPA). Moreover, a method for the direct radiolabeling of the antibody component with ¹³¹ I is described by Stein et al., Antibody Immunoconj. Radiopharm. 4: 703 (1991) (see also USP 6, 080, 384).

Alternatively, boron addends such as carboranes can be attached to antibody components, as discussed above.

In addition, therapeutic immunoconjugates can comprise an immunomodulator moiety suitable for application for the purposes herein. Broadly speaking, the term "immunomodulator" includes cytokines, stem cell growth factors, lymphotoxins, such as tumor necrosis factor (TNF), and hematopoietic factors, such as interleukins (e.g., interleukin-1 (IL-1), IL-2, IL-3, IL6, IL-10 and IL-12), colony stimulating factors (e.g., granulocyte-colony stimulating factor (G-CSF) and granulocyte macrophage-colony stimulating factor (GM-CSF)), interferons (e.g., interferonsalpha, -beta and gamma.), the stem cell growth factor designated "S1 factor," erythropoietin and thrombopoietin. Examples of suitable immunomodulator moieties include IL-2, IL-6, IL-10, IL12, interferon-gamma., TNF-alpha., and the like.

A related form of therapeutic protein is a fusion protein comprising an antibody moiety and an immunomodulator moiety.

Methods of making antibody-immunomodulator fusion proteins are known to those of skill in the art as discussed herein. For example, antibody fusion proteins comprising an interleukin-2 moiety are described by Boleti et al., Ann. Oncol. 6:945 (1995), Nicolet et al., Cancer Gene Ther. 2:161 (1995), Becker et al., Proc. Nat'l Acad. Sci. USA 93:7826 (1996), Hank et al., Clin. Cancer Res. 2:1951 (1996), and Hu et al., Cancer Res. 56:4998 (1996). In addition, Yang et al., Hum. Antibodies Hybridomas 6:129 (1995), describe a fusion protein that includes an F(ab')₂ fragment and a tumor necrosis factor alpha moiety.

Such immunoconjugates and antibody-immunomodulator fusion proteins provide a means to deliver an immunomodulator to a target cell and are particularly useful against tumor cells. The cytotoxic effects of immunomodulators are well known to those of skill in the art. See, for example, Kle et at., "Lymphokines and Monokines," in Biotechnology and Pharmacy, Pessuto et al. (eds.), pages 53-70 (Chapman & Hall 1993) as well as other references herein cited. As an illustration, interferons can inhibit cell proliferation by inducing increased expression of class I histocompatibility antigens on the surface of various cells and thus, enhance the rate of destruction of cells by cytotoxic T lymphocytes. Furthermore, tumor necrosis factors, such as TNF-alpha., are believed to produce cytotoxic effects by inducing DNA fragmentation.

Moreover, therapeutically useful immunoconjugates can be prepared in which an antibody component is conjugated to a toxin or a chemotherapeutic drug. Illustrative of toxins which are suitably employed in the preparation of such conjugates are ricin, abrin, ribonuclease, DNase I, Staphylococcal enterotoxin-A, pokeweed antiviral protein, gelonin, diphtherin toxin, Pseudomonas exotoxin, and Pseudomonas endotoxin. See,references herein as well as for example, Pastan et al., Cell 47:641 (1986), and Goldenberg, CA-A Cancer Journal for Clinicians 44:43 (1994). Other suitable toxins are known to those of skill in the art.

With to respect to bispecific antibody constructs which are capable of binding simultaneously to two ligands on the same cell see also WO96/32841 Various such constructs are known in the art.An alternative approach to introducing the combination of therapeutic antibody and toxin is provided by antibody-toxin fusion proteins. An antibody-toxin fusion protein is a fusion protein that comprises an antibody moiety and a toxin moiety. Methods for making antibody-toxin fusion proteins are known to those of skill in the art (see references cited herein); antibody-Pseudomonas exotoxin A fusion proteins have been described by Chaudhary et al., Nature 339:394 (1989), Brinkmann et al., Proc. Nat'l Acad. Sci. USA 88:8616 (1991), Batra et al., Proc. Nat'l Acad. Sci. USA 89:5867 (1992), Friedman et al., J. Immunol. 150:3054 (1993), Wels et al., Int. J. Can. 60:137 (1995), Fominaya et al., J. Biol. Chem. 271:10560 (1996), Kuan et al., Biochemistry 35:2872 (1996), and Schmidt et al., Int. J. Can. 65:538 (1996). Antibody-toxin fusion proteins containing a diphtheria toxin moiety have been described by Kreitman et al., Leukemia 7:553 (1993), Nicholls et al., J. Biol. Chem. 268:5302 (1993), Thompson et al., J. Biol. Chem. 270:28037 (1995), and Vallera et al., Blood 88:2342 (1996). Deonarain et al., Tumor Targeting 1:177 (1995), have described an antibody-toxin fusion protein having an RNase moiety, while Linardou et al., Cell Biophys. 24-25:243 (1994), produced an antibody-toxin fusion protein comprising a DNase I component. Gelonin was used as the toxin moiety in the antibody-toxin fusion protein of Wang et al., Abstracts of the 209th ACS National Meeting, Anaheim, Calif., Apr. 2-6, 1995, Part 1, BIOT005. As a further example, Dohisten et al., Proc. Nat'l Acad. Sci. USA 91:8945 (1994), reported an antibody-toxin fusion protein comprising Staphylococcal enterotoxin-A. Numerous other examples have been reported in the literature.

Useful cancer chemotherapeutic drugs for the preparation of immunoconjugates include nitrogen mustards, alkyl sulfonates, nitrosoureas, triazenes, folic acid analogs, pyrimidine analogs, purine analogs, antibiotics, epipodophyllotoxins, platinum coordination complexes, hormones, and the like. Suitable chemotherapeutic agents are described in Remington's Pharmaceutical Sciences, 19th Ed. (Mack Publishing Co. 1995), and in Goodman and Gilman's The Pharmacological Basis of Therapeutics, 7th Ed. (MacMillan Publishing Co. 1985). Other suitable chemotherapeutic agents, such as experimental drugs, are known to those of skill in the art.

In addition, therapeutically useful immunoconjugates can be obtained by conjugating photoactive agents or dyes to an antibody composite. Fluorescent and other chromogens, or dyes, such as porphyrins sensitive to visible light, have been fused to detect and to treat lesions by directing the suitable light to the lesion. In therapy, this has been termed photoradiation, phototherapy, or photodynamic therapy (Jori et al. (eds.), Photodynamic Therapy of Tumors and Other Diseases (Libreria Progetto 1985); van den Burgh, Chem. Britain 22:430 (1986)). Moreover, monoclonal antibodies have been coupled with photoactivated dyes for achieving phototherapy. Mew et al., J. Immunol. 130:1473 (1983); idem., Cancer Res. 45:4380 (1985); Oseroff et al., Proc. Natl. Acad. Sci. USA 83:8744 (1986); idem., Photochem. Photobiol. 46:83 (1987); Hasan et al., Prog. Clin. Biol. Res. 288:471 (1989); Tatsuta et al., Lasers Surg. Med. 9:422 (1989); Pelegrin et al., Cancer 67:2529 (1991). However, these earlier studies did not include use of endoscopic therapy applications, especially with the use of antibody fragments or subfragments. Thus, the present invention contemplates the therapeutic use of immunoconjugates comprising photoactive agents or dyes.

With respect to a multifunctional ligand having a first portion that binds to both lymphatic endothelial cells and tumor vasculature, the invention contemplates using phage display or ribosome display to generate an antibody that binds to vefgr-3 as well as one or both of of vegfr-2 or vegfr-1, having regard to the sequences of those respective receptors (see USPs 5,776,755, 5877020, 5952199, 6107046, 6130071, 6221839, 6235713, 6245530; see also WO 00/21560, WO 95/33772, WO 97/05250, WO 98/33917). Preferably the antibody does not internalize, particularly in the case where the multifunctional ligand is fused or conjugated to a toxic moiety. The invention also contemplates, for example, fusing the binding domain of VEGF-C or VEGF-D to antitumor antibody. The invention also contemplates that the risk of retargeting cancer cells to non-tumor sites of angiogenesis, can be minimized by employing one or more of the following strategies pre- and/or co-treatment with inhibitors of angiogenesis, providing the multifunctional ligand with an effector function, such as a toxic moiety, cytokine or antibody component which retargets immune cells capable of killing such cancer cells. The invention also contemplates using in combination or alone a multifunctional ligand having a second portion that comprises an anti-VEGF antibody portion which binds to one or more of the VEGF family of ligands in order to inhibit lymphangiogenesis and/or angiogenesis. (see also for example, WO 00/37025, WO 98/33917, USP 6130071, WO 01/12669). With respect to angiogenesis and particularly lymphangiogenesis see also:1: Shibuya M. Structure and function of VEGF/VEGF-receptor system involved in angiogenesis. Cell Struct Funct. 2001 Feb;26(1):25-35: Yonemura Y, et al.Lymphangiogenesis and the vascular endothelial growth factor receptor (VEGFR)-3in gastric cancer Eur J Cancer. 2001 May;37(7):918-23.: lljin K, et aIVEGFR3 gene structure, regulatory region, and sequence polymorphisms FASEB J. 2001 Apr;15(6):1028-36.: Tang RF, et alOverexpression of lymphangiogenic growth factor VEGF-C in human pancreatic cancer. Pancreas. 2001 Apr;22(3):285-92: Kadambi A, Carreira CM, Yun CO, Padera TP, Dolmans DE, Carmeliet P, FukumuraD, Jain RK.Vascular endothelial growth factor (VEGF)-C differentially affects tumorvascular function and leukocyte recruitment: role of VEGF-receptor 2 and hostVEGF-A.Cancer Res. 2001 Mar 15;61(6):2404-8. Karpanen T, et alVascular endothelial growth factor C promotes tumor lymphangiogenesis and intralymphatic tumor growth. Cancer Res. 2001 Mar 1;61(5):1786-90: Baldwin ME, et al The Specificity of Receptor Binding by Vascular Endothelial Growth Factor-D Is Different in Mouse and Man. J Biol Chem. 2001 Jun 1;276(22):19166-19171: Niki T, et al J Pathol. 2001 Apr;193(4):450-7: Veikkola T, et al Signalling via vascular endothelial growth factor receptor-3 is sufficient forlymphangiogenesis in transgenic mice. EMBO J. 2001 Mar 15;20(6):1223-31 Achen MG, et al Localization of vascular endothelial growth factor-D in malignant melanoma suggests a role in tumour angiogenesis. J Pathol. 2001 Feb;193(2):147-54 Stacker SA, et alVEGF-D promotes the metastatic spread of tumor cells via the lymphatics. Nat Med. 2001 Feb;7(2):186-91 Plate K. From angiogenesis to lymphanglogenesis. Nat Med. 2001 Feb;7(2):151-2.. Joukov V, et al; A novel vascular endothelial growth factor, VEGF-C, is a ligand for the Flt4 (VEGFR-3) and KDR (VEGFR-2) receptor tyrosine kinases.EMBO J. 1996 Apr 1;15(7):1751. Lee J, et al, Proc Natl Acad Sci USA. 1996 Mar 5;93(5):1988-92.

Multimodal therapies are also contemplated within the present invention, including particularly for cancer, therapies which can be determined to be useful complementary therapies for the anti-metastatic embodiments of this invention such as anti-angiogenic Ab conjugates

In another form of multimodal therapy, subjects receive the multifunctional ligands of the present inventionand standard cancer chemotherapy. For example, "CVB" (1.5 g/m.sup.2 cyclophosphamide, 200-400 mg/m² etoposide, and 150-200 mg/m² carmustine) is a regimen used to treat non-Hodgkin's lymphoma. Patti et al., Eur. J. Haematol. 51:18 (1993). Other suitable combination chemotherapeutic regimens are well-known to those of skill in the art. See, for example, Freedman et al., "Non-Hodgkin's Lymphomas," in Cancer Medicine, Volume 2, 3rd Edition, Holland et al. (eds.), pages 2028-2068 (Lea & Febiger 1993). As an illustration, first generation chemotherapeutic regimens for treatment of intermediate-grade non-Hodgkin's lymphoma include C-MOPP (cyclophosphamide, vincristine, procarbazine and prednisone) and CHOP (cyclophosphamide, doxorubicin, vincristine, and prednisone). A useful second generation chemotherapeutic regimen is m-BACOD (methotrexate, bleomycin, doxorubicin, cyclophosphamide, vincristine, dexamethasone and leucovorin), while a suitable third generation regimen is MACOP-B (methotrexate, doxorubicin, cyclophosphamide, vincristine, prednisone, bleomycin and leucovorin). Additional useful drugs include phenyl butyrate and brostatin-1.

In general, the dosage of administered multifunctional ligands, immunoconjugates, and fusion proteins will vary depending upon such factors as the patient's age, weight, height, sex, general medical condition and previous medical history. Typically, it is desirable to provide the recipient with a dosage of antibody component, immunoconjugate or fusion protein which is generally at least in the range of from about 1 pg/kg to 10 mg/kg (amount of agent/body weight of patient), although a lower or higher dosage also may be administered as circumstances dictate, particularly to take advantage of the depot effect of the invention.

Administration of the invention including, immunoconjugates or fusion proteins to a patient can be intravenous, intraarterial, intraperitoneal, intramuscular, subcutaneous, intrapleural, intrathecal, by perfusion through a regional catheter, or by direct intralesional injection. When administering therapeutic proteins by injection, the administration may be by continuous infusion or by single or multiple boluses.

Those of skill in the art are aware that intravenous injection provides a useful mode of administration due to the thoroughness of the circulation in rapidly distributing antibodies. Intravenous administration, however, is subject to limitation by a vascular barrier comprising endothelial cells of the vasculature and the subendothelial matrix. Still, the vascular barrier is a more notable problem for the uptake of therapeutic antibodies by solid tumors. Lymphomas have relatively high blood flow rates, contributing to effective antibody delivery. Intralymphatic routes of administration, such as subcutaneous or intramuscular injection, or by catherization of lymphatic vessels, also provide a useful means of treating lymphomas.

With regard to "low doses" of ¹³¹I-labeled immunoconjugates, the invention includes a dosage is in the range of 15 to 40 mCi, 20 to 30 mCi. In contrast, a preferred dosage of⁹⁰ Y-labeled immunoconjugates is in the range from 10 to 30 mCi, while the more preferable range is 10 to 20 mCi.

Immunoconjugates having a boron addend-loaded carrier for thermal neutron activation therapy will normally be effected in similar ways. However, it will be advantageous to wait until non-targeted immunoconjugate clears before neutron irradiation is performed. Clearance can be accelerated using an antibody that binds to the immunoconjugate. See U.S. Pat. No. 4,624,846 for a description of this general principle.

The immunoconjugates, and fusion proteins of the present invention can be formulated according to known methods to prepare pharmaceutically useful compositions, whereby the therapeutic proteins are combined in a mixture with a pharmaceutically acceptable carrier. A composition is said to be a "pharmaceutically acceptable carrier" if its administration can be tolerated by a recipient patient. Sterile phosphate-buffered saline is one example of a pharmaceutically acceptable carrier. Other suitable carriers are well-known to those in the art. See, for example, REMINGTON'S PHARMACEUTICAL SCIENCES, 19th Ed. (1995).

For purposes of therapy, antibody components (or immunoconjugates/fusion proteins) and a pharmaceutically acceptable carrier are administered to a patient in a therapeutically effective amount. A combination of an antibody component, optionally with an immunoconjugate/fusion protein, and a pharmaceutically acceptable carrier is said to be administered in a "therapeutically effective amount" if the amount administered is physiologically significant. An agent is physiologically significant if its presence results in a detectable change in the physiology of a recipient patient. In one aspect, an agent is physiologically significant if its presence results in the inhibition of the growth of target tumor cells.

Yet another therapeutic method included in the invention is a method of treating cancer by administering to an animal suffering from cancer a pharmaceutically effective amount of one or more multifunctional ligands capable of binding to cancer cells, wherein the compound is associated with a substance capable of damaging cancer cells.

Pharmaceutical compositions herein described or alluded to include multifunctional ligands of the invention or therapeutics used in combination therapy which may be administered by a variety of routes of adminstration.

By administration of an "effective amount" is intended an amount of the compound that is sufficient to enhance or inhibit a response, is some embodiments particularly an immune response or cellular response to a multifunctional ligand. One of ordinary skill will appreciate that effective amounts of a multifunctional ligand can be determined empirically and may be employed in pure form or, where such forms exist, in pharmaceutically acceptable salt, ester or prodrug form. The multifunctional ligand may be administered in compositions in combination with one or more pharmaceutically acceptable excipients. It will be understood that, when administered to a human patient, the total daily usage of the compounds and compositions of the present invention will be decided by the attending physician within the scope of sound medical judgement. The specific therapeutically effective dose level for any particular patient will depend upon a variety of factors including the type and degree of the cellular response to be achieved; activity of the specific multifunctional ligand employed; the specific composition employed; the age, body weight, general health, sex and diet of the patient; the time of administration, route of administration, and rate of excretion of the agonist or antagonist; the duration of the treatment; drugs used in combination or coincidental with the specific agonist or antagonist; and like factors well known in the medical arts.

On administration parenterally, for example by i.v, drip or infusion, dosages optionally at leasr on the order of from 0.01 to 5 mg/kg/day, optionally 0.05 to 1.0 mg/kg/day and more preferably 0.1 to 1.0 mg/kg/day can be used. Suitable daily dosages for patients are thus on the order of from 2.5 to 500 mg p.o., optionally 5 to 250 mg p.o., optionally 5 to 100 mg p.o., or on the order of from 0.5 to 250 mg i.v., optionally 2.5 to 125 mg i.v. and optionally 2.5 to 50 mg i.v.

Dosaging may also be arranged in a patient specific manner to provide a predetermined concentration of an agonist or antagonist in the blood, as determined by the RIA technique. Thus patient dosaging may be adjusted to achieve regular on-going trough blood levels, as measured by RIA, optionally on the order of at least from 50 to 1000 ng/ml, preferably 150 to 500, ng/ml.

From above, pharmaceutical compositions are provided comprising an agonist or antagonist and a pharmaceutically acceptable carrier or excipient, which may be administered orally, rectally, parenterally, intracisternally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

Optionally a composition for for parenteral injection can comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylceuulose and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

Some compositions herein descibed may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

In some cases, in order to prolong the effect of one or therapeutic components herein described, it is desirable to slow the absorption from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are' compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

The multifunctional ligand can also be administered in the form of liposomes. As is known in the art, liposomes are generally derived from phospholipids or other lipid substances. Liposomes are formed by mono- or multi-lameflar hydrated liquid crystals that are dispersed in an aqueous medium. Any non-toxic, physiologically acceptable and metabolizable lipid capable of forming liposomes can be used. The present compositions in liposome form can contain, in addition to the agonist or antagonist, stabilizers, preservatives, excipients, and the like. The preferred lipids are the phospholipids and the phosphatidyl choaes (lecithins), both natural and synthetic. Methods to form liposomes are known in' the art. See, for example, Prescott, Ed., Methods in Cell Biology, Volume XIV, Academic Press, New York, N.Y. (1976), p. 33 et seq.

The present invention also contemplates a method of treatment in which immunomodulators are administered to prevent, mitigate or reverse radiation-induced or drug-induced toxicity of normal cells, and especially hematopoietic cells. Adjunct immunomodulator therapy allows the administration of higher doses of cytotoxic agents due to increased tolerance of the recipient mammal. Moreover, adjunct immunomodulator therapy can prevent, palliate, or reverse dose-limiting marrow toxicity. Examples of suitable immunomodulators for adjunct therapy include G-CSF, GM-CSF, thrombopoietin, IL-1, IL-3, IL-12, and the like. The method of adjunct immunomodulator therapy is disclosed by Goldenberg, U.S. Pat. No. 5,120,525.

For example, recombinant IL-2 may be administered intravenously as a bolus at 6 x 10⁵ IU/kg or as a continuous infusion at a dose of 18 x 10⁶ IU/m² /d. Weiss et al., J. Clin. Oncol. 10:275 (1992). Alternatively, recombinant IL-2 may be administered subcutaneously at a dose of 12 x 10⁶ IU. Vogelzang et al., J. Clin. Oncol. 11:1809 (1993). Moreover, INF-gamma. may be administered subcutaneously at a dose of 1.5 x10⁶ U. Lienard et al., J. Clin. Oncol. 10:52 (1992). Furthermore, Nadeau et al., J. Pharmacol. Exp. Ther. 274:78 (1995), have shown that a single intravenous dose of recombinant IL-12 (42.5 .mu.g/kilogram) elevated IFN-.gamma. levels in rhesus monkeys.

Suitable IL-2 formulations include PROLEUKIN (Chiron Corp./Cetus Oncology Corp.; Emeryville, Calif.) and TECELEUKIN (Hoffmann-La Roche, Inc.; Nutley, N.J.). ACTIMMUNE (Genentech, Inc.; South San Francisco, Calif.) is a suitable INF-.gamma. preparation.

With respect to anti-CCR5 antibodies used to kill CCR5-expressing cells, with for example, bi-specific antibody chemokine fusions see Bruhl H. et al. J Immunol. 2001 Feb 15166(4): 2420-2426.

With respect to targeting IKAP proteins see for example US 6172195.

With respect to pertinent diseased cells, disease causing cells and other suitable targets for immunotoxins, as well as optional toxins and methods of making and using immunotoxins and related technologies see for example US05980895 Immunotoxin containing a disulfide-stabilized antibody fragment joined to a Pseudomonas exotoxin that does not require proteolytic activation; US05686072 Epitope-specific monoclonal antibodies and immunotoxins and uses thereof; US04956453 Antihuman ovarian cancer immunotoxins and methods of thereof; US06146631 Immunotoxins comprising ribosome-inactivating proteins; US05756699 Immunotoxins comprising ribosome-inactivating Proteins; US05744580 Immunotoxins comprising ribosome-inactivating Proteins; US06146850 Proteins encoding gelonin sequences; US05837491 Polynucleotides encoding gelonin sequences; US05578706 Methods and compositions concerning homogenous immunotoxin preparations; US05185434 Prolonged-action immunotoxins containing a glycopeptide constituent which inactivates ribosomes, modified on its polysaccharide units; US04958009 Anti-human ovarian cancer immunotoxins and methods of use thereof; US05980896 Antibodies reactive with human carcinomas; US06074644 Nucleic acids encoding immunotoxins containing a disulfide-stabilized antibody fragment replacing half or more of domain IB of pseudomonas exotoxin, and methods of use of the encoded immunotoxins; US04981953 Immunotoxins, process for their preparation and pharmaceutical compositions in which they are present; US04980457 Cytotoxic conjugates which can be used in therapy and process or their preparation; US04545985 Pseudomonas exotoxin conjugate immunotoxins; US06020145 Methods for determining the presence of carcinoma using the antigen binding region of monoclonal antibody BR96; US05792458 Mutant diphtheria toxin conjugates; US05338542; US06051230 Compositions for targeting the vasculature of solid tumors; B3 antibody fusion proteins and their uses; US05990275 Linker and linked fusion polypeptides; US05981726 Chimeric and mutationally stabilized tumor-specific B1, B3 and B5 antibody fragments; immunotoxic fusion proteins; and uses thereof; US05965132 Methods and compositions for targeting the vasculature of solid tumors; US05889157 Humanized B3 antibody fragments, fusion proteins, and uses thereof US06027725 Multivalent antigen-binding proteins; EP00861091A1 IMMUNOTOXIN CONTAINING A DISULFIDE-STABILIZED ANTIBODY FRAGMENT US05776427 Methods for targeting the vasculature of solid tumors; US05772997 Monoclonal antibodies directed to the HER2 receptor US05665357 Antibodies recognizing tumor associated antigen CA 55.1; US05660827 Friedrich K, et al. A two-step selection approach for the identification of ligand-binding determinants in cytokine receptors. Anal Biochem. 1999 Mar 15; 268(2):179-86. Krebs B, et al. Recombinant human single chain Fv antibodies recognizing human interleukin-6. Specific targeting of cytokine-secreting cells. J Biol Chem. 1998 Jan 30; 273(5):2858-65; Wilbur DS, et al. Related Articles Biotin reagents' for antibody pretargeting. 2. Synthesis and in vitro evaluation of biotin dimers and trimers for cross-linking of streptavidin. Bioconjug Chem. 1997 Nov-Dec; 8(6):819-32.Ring DB, et al Antigen forks: bispecific reagents that inhibit cell growth by binding selected pairs of tumor antigens. Cancer Immunol Immunother. 1994 Jul; 39(1):41-8. WO09942597A1 MONOVALENT, MULTIVALENT, AND MULTIMERIC MHC BINDING DOMAIN FUSION PROTEINS AND CONJUGATES, AND USES THEREFOR; EP00935607A2 SOLUBLE MONOVALENT AND MULTIVALENT MHC CLASS II FUSION PROTEINS, AND USES THEREFOR; WO09811914A1 TARGETING ANTIGENS TO THE MHC CLASS IPROCESSING PATHWAY WITH ANTHRAX TOXIN FUSION PROTEIN WO09728191A1 MHC COMPLEXES AND USES THEREOF; US05580756 B71G fusion protein; US06143298 Soluble truncated forms of ICAM-1; US05852175 P-selectin glycoprotein ligand blocking antibodiesUS05800815Antibodies to P-selectin and their uses; US06037454 Humanized anti-CD11a antibodies; US06020152 Lymphocyte-associated cell surface proteinUS05807734 Monoclonal antibodies and FV specific for CD2antigenUS05622701 Cross-reacting monoclonal antibodies specificfor E- and P-selectin US05622700 Method for treating a LFA-1-mediated disorder; JP06209788A2 IMMUNOASSAY OF HUMAN SOLUBLE ICAM-1. ANTIBODYAND KIT FOR MEASUREMENT THEREOF; JP03072430A2 ANTIVIRAL AGENT BY USING FUNCTIONALDERIVATIVE OF INTERCELLULAR ADHESIVE MOLECULE; JP01135724A2 TREATMENT FOR NONSPECIFIC INFLAMMATION; US06123915 Methods for using agents that bind to VCAM-1; WO09929706A2 DISALICYLATE ANALOG BASED SIALYL LEWISxMIMETICS; WO09918442A1 DIAGNOSIS OF THROMBOTIC EVENTS BY DETECTINGP-SELECTIN; US05877295 Antibodies which bind a subpopulation of Mac-1 (CD11 b/CD18) molecules which mediate neutrophil adhesion to ICAM-1 and fibrinogen; US05869460 Sulfated and phosphated saccharide derivatives,process for the preparation of the same and use thereof; US05858994 Carbohydrate conjugates as inhibitors of celladhesion; US05811405 Multiply fucosylated dicarboxylic acidspossessing antiadhesive properties; US05654282 Selectin binding glycopeptides; US05632991 Antibodies specific for E-selectin and the usesthereof; US05599676 Method for isolating a novel receptor for.alpha.4 integrins; US05580862 Sulfate ligands for L-selectins and methods ofpreventing sulfate addition; US05508387 Selectin binding glycopeptides; US06177547 Antibodies to P-selectin glycoprotein ligandUS05827670 Methods of isolating and detecting bone marrow stromal cells with VCAM-1-specific antibodies; US05756095 Antibodies with specificity for a common epitope on E-selectin and L-selectin; US05565550 Antibodies to ICAM-2, and fragments thereof; US06099838 Pharmaceutical compositions comprising anti-CD45RB antibodies for the inhibition of T-cell mediated immune responses; US05595737 Methods for using monoclonal antibodies specific for cell-surface bound LAM-1; US05324510, US06183988 Leukocyte-specific protein and gene, andmethods of use thereof; US05998598; US05997865 Agonist antibodies against the flk2/flt3 receptor and uses thereof; US05993816 Methods to inhibit humoral immune responses, immunoglobulin production and B cell activation with 5c8-specific antibodies; US05869453 Cytotoxic T-cell epitopes; US05861151 Soluble fusion molecules with binding specificity for cell adhesion molecules; US05843441 Use of endothelial-leukocyte adhesion molecule-1 specific antibodies in the treatment of asthma US05821332 Receptor on the surface of activated CD4+ T-cells: ACT-4; EP00868197A1 ANTI-SELECTIN ANTIBODIES FOR PREVENTION OFMULTIPLE ORGAN FAILURE AND ACUTE ORGAN DAMAGE; US05817515 Human B2 integrin alpha subunit antibodies; EP00528931 B1 HUMANIZED CHIMERIC ANTI-ICAM-1 ANTIBODIES, METHODS OF PREPARATION AND USE; US05776775 Anti-LAM 1-3 antibody and hybridoma; US06063906 Antibodies to integrin alpha subunit; US05997865 Agonist antibodies against the flk2/flt3 receptor and uses thereof; US05993816 Methods to inhibit humoral immune responses, immunoglobulin production and B cell activation with 5c8-specific antibodies US05951982 Methods to suppress an immune response with variant CD44-specific antibodies US05843441 Use of endothelial-leukocyte adhesion molecule-1 specific antibodies in the treatment of asthma US05821332 Receptor on the surface of activated CD4+T-cells: ACT-4; US05821123 Modified antibody variable domains; EP00868197A1 ANTI-SELECTIN ANTIBODIES FOR PREVENTION OF MULTIPLE ORGAN FAILURE AND ACUTE ORGAN DAMAGE; US05817515 Human B2 integrin alpha subunit antibodies; EP00528931B1 HUMANIZED CHIMERIC ANTI-ICAM-1 ANTIBODIES, METHODS OF PREPARATION AND USE; US05776775 Anti-LAM 1-3 antibody and hybridoma; US05776755 FLT4, a receptor tyrosine kinase; US05730978 Inhibition of lymphocyte adherence with alpha.4ࢷ -specific antibodies.

Examples of tumor specific antigens are numerous and are referred to in the hereinabove cited references and as well as the in the following references: US06132980 10/17/2000 Antibodies specific for TRP-2 a human tumor antigen recognized by cytotoxic T lymphocytes US06165464Monoclonal antibodies directed to the HER2 receptor, US05824311 Treatment of tumors with monoclonal antibodiesagainst oncogene antigens.US0614005010131/2000 Methods for determining breast cancer and melanoma by assaying for a plurality of antigens associated herewith; US06051226 MN-specific antibodies and their use in cancer treatment; US06020145Methods for determining the presence ofcarcinoma using the antigen binding region of monoclonal antibody BR96.; US05980896 Antibodies reactive with human carcinomas US05955075 Method of inhibiting tumor growth using antibodies to MN protein US05917124 Transgenic mouse model of prostate cancer; US05914389 06/22/1999 E6 associated protein US05912143 06/15/1999 Polynucleotides encoding a human mage protein homolog US05910626 06/08/1999 Acetyl-CoA carboxylase compositions and methods of use US0587456002/23/1999 Melanoma antigens and their use in diagnostic and therapeutic methods US05872217 02/16/1999 Antibodies which specifically bind a cancerrelated antigen US05869636 02/09/1999 Immunoreactive peptide sequence from a 43 kDhuman cancer antigen US05869045 02/09/1999 Antibody conjugates reactive with human carcinomas US05866124 02/02/1999 Antiidiotypic antibodies for high molecularweight-melanoma associated by sameUS0584708312/08/1998 Modified p53 US05844075 Melanoma antigens and their use in diagnosticand therapeutic methods US0584368512/01/1998 Production of chimeric mouse-human antibodies with specificity to human tumor antigensUS05843648 P15 and tyrosinase melanoma antigens and their use in diagnostic and therapeutic methods US05840854US0583047011/03/1998 Humanized antibodies to ganglioside GM2US0583046411/03/1998 US05808005Human carcinoma Bispecific molecules recognizing lymphocyte antigen CD2 and tumor antigens US05792456 Mutant BR96 antibodies reactive with humancarcinomas US0578368US05773579 Lung cancer marker US05772997 Monoclonal antibodies directed to the HER2 receptor US05770374; US05705157 Methods of treating cancerous cells with anti-receptor antibodies US0569599412/09/1997 Isolated cytolytic T cells specific forcomplexes of MAGE related peptides and HLA molecules US05693763 12/02/1997 Antibodies to human carcinoma antigen.

Tumor rejection antigens which correspond to amino acid sequences in tumor rejection antigen precursor bage, and uses thereof US05681701 Immortalized human fetal osteoblastic cellsUS0568156210/28/1997 US0567717110/14/1997 Monoclonal antibodies directed to the HER2receptorUS0567448610/07/1997US0566535709/09/1997 Antibodies recognizing tumor associated antigen CA 55.1; Fonsatti E, et al Emerging role of protectin (CD59) in humoral immunotherapy of solid malignancies. Clin Ter. 2000 May-Jun; 151 (3):187-93 Knuth A, et alCancer immunotherapy in clinical oncology. Cancer Chemother Pharmacol. 2000; 46 Supp/:S46-51 : Sievers EL.Targeted therapy of acute myeloid leukemia with monoclonal antibodies andimmunoconjugates. Cancer Chemother Pharmacol. 2000; 46 Supp/:S18-22. 172 van Spriel AB,et allmmunotherapeutic perspective for bispecific antibodies.lmmunol Today. 2000 Aug; 21(8):391-7 273: Green MC, et alMonoclonal antibody therapy for solid tumors. Cancer Treat Rev. 2000 Aug; 26(4):269-86; Xiang J Targeting cytokines to tumors to induce active antitumor immune responses by recombinant fusion proteins. Hum Antibodies. 1999; 9(1):23-Engberg J, et alRecombinant antibodies with the antigen-specific, MHC restricted specificity of T cells: novel reagents for basic and clinical investigations and immunotherapy. Immunotechnology. 1999 Mar; 4(3-4):273-8. O'Brien TJ, Tet alMore than 15 years of CA 125: what is known about the antigen, its structure andits function.Int J Biol Markers. 1998 Oct-Dec; 13(4):188-95. Sharifi J, et allmproving monoclonal antibody pharmacokinetics via chemical modification.Q J Nucl Med. 1998 Dec; 42(4): 242-9.

Ligands on immune or other cells which may be targeted with bispecific ligands in which one ligand of the pair dictates specificity for a population of cells or particular sub-population of those cells and a second ligand with reduced functional affinity is used to effect a specific immune function include those referenced in the following patents and publications therein referenced: US06132992 Expression vectors encoding bispecific fusionproteins and methods of producing biologically active bispecific fusion proteins in a mammalian cell; Antibody heteroconjugates and bispecificantibodies for use in regulation of lymphocyte activity; WO09942077 COMPOSITIONS AND METHODS FOR REGULATINGLYMPHOCYTE ACTIVATION; US059165600 Methods for inhibiting an immune response byblocking the GP39/CD40 and CTLA4/CD28/B7 pathways and compositionsfor use therewith; US05876718 Methods of inducing T cell non-responsiveness to transplanted tissues and of treating graft-versus-host-disease with anti-gp39 antibodies EP00445228B IMMUNOTHERAPY INVOLVING CD28 STIMULATION; US05709859 Mixed specificity fusion proteins; US05637481 Expression vectors encoding bispecific fusion proteins and methods of producing biologically active bispecific fusion proteins in a mammalian cell; WO09720048 MODIFIED SFV MOLECULES WHICH MEDIATE ADHESIONBETWEEN CELLS AND USES THEREOF; EP00336379 Antibody heteroconjugates for use in regulation of lymphocyte activity; EP00537293 LIGAND FOR CD28 RECEPTOR ON B CELLS ANDMETHODS; US05182368 Ligands and methods for augmenting B-cell proliferation; WO09300431 CTL4A RECEPTOR, FUSION PROTEINS CONTAINING IT AND USES THEREOF; EP00445228 IMMUNOTHERAPY INVOLVING CD28 STIMULATION; Role of cellular adhesion molecules in HIV type 1 infection and their impact onvirus neutralization. AIDS Res Hum Retroviruses. 1998 Oct; 14 Suppl 3:S247-54 Cavenagh JD,et alAdhesion molecules in clinical medicine. Crit Rev Clin Lab Sci. 1998 Sep; 35(5):415-59 Viney JL, Fong S. Beta 7 integrins and their ligands in lymphocyte migration to the gut. Chem Immunol. 1998; 71:64-76 Aplin AE, et alSignal transduction and signal modulation by cell adhesion receptors: the roleof integrins, cadherins, immunoglobulin-cell adhesion molecules, and selectins. Pharmacol Rev. 1998 Jun; 50(2):197-263

With respect to ascertaining important amino acid residues for receptor activation or binding see also Zang, Q., Springer, T. A. (2001). Amino Acid Residues in the PSI Domain and Cysteine-rich Repeats of the Integrin beta 2 Subunit That Restrain Activation of the Integrin alpha xbeta 2. J. Biol. Chem. 276: 6922-6929; Binding site on the murine IFN-gamma receptor for IFN-gamma has been identified using the synthetic peptide approach,The Journal of Immunology, Vol 151, Issue 11 6206-6213; The Journal of Immunology, Vol 143, Issue 11 3568-3579 The main immunogenic region of the nicotinic acetylcholine receptor. Identification of amino acid residues interacting with different antibodies, M Bellone et al.; Arend, W. P., Malyak, M., Guthridge, C. J., Gabay, C. (1998). INTERLEUKIN-1 RECEPTOR ANTAGONIST: Role in Biology. Annu. Rev. Immunol. 16: 27-55; The Journal of Immunology, Vol 155, Issue 10 4719-4725, Mapping of receptor binding sites on IL-1 beta by reconstruction of IL- 1ra-like domains; The Journal of Immunology, 2000, 165: 6966-6974 Identification of Fetal Liver Tyrosine Kinase 3 (Flt3) Ligand Domain Required for Receptor Binding and Function Using Naturally Occurring Ligand Isoforms Waithaka Mwangi³, Wendy C. Brown and Guy H. Palmer.

The invention also contemplates multifunctional ligands comprising various combinations and permutations of such ligands including pairs and three different such ligands including multifunctional ligands including such combinations and a ligand which binding to a lymphatic vessel associated ligand.Additional pertinent references pertaining to formation of antibody dimers, microarrays of (and tissue microarrays) proteins including heterofunctional proteins and recombinant, ligands having application to the invention, and phage or ribosome display strategies having relevance herein include Zhu H. et al. Protein arrays and microarrays.Curr Opin Chem Biol. 2001 Feb; 5(1):40-5, references in IBC's conference on Protein Microarray Technology March 19-21 Santiago California; WO 99/06834, WO 99/19506; WO 97/02342, WO 00/63701; WO 99/40434 ; US 6,127,127; US6146830, WO 00/075298, US6165709 US0620402303/20/2001 Modular assembly of antibody genes, antibodies prepared thereby and use; US0584681812/08/1998 Pectate lyase signal sequence; US0569843512/16/1997 Modular assembly of antibody genes, antibodies prepared thereby and use; US0569841712/16/1997 Modular assembly of antibody genes, antibodies prepared thereby and use; US0569349312/02/1997 Modular assembly of antibody genes, antibodies prepared thereby and use; US0551454805/07/1996 Method for in vivo selection of ligand-binding proteins; US0564823707/15/1997 Expression of functional antibody fragments; US0618034 In vitro scanning saturation mutagenesis of proteins; US06027933 Surface expression libraries of heteromeric receptors; US0591057306/08/1999 Monomeric and dimeric antibody-fragment fusion proteins; US0615058311Transgenic animals expressing artificial epitope-tagged proteins; US06132992 Expression vectors encoding bispecific fusion proteins and methods of producing biologically active bispecific fusion proteins in a mammalian cell; US06127524Binding molecules and computer-based methods of increasing the binding affinity thereof;; US06071515 Dimer and multimer forms of single chain polypeptides; US06054297 Humanized antibodies and methods for makingthem; WO00004382 ARRAYS OF PROTEINS AND METHODS OF USE THEREOF; US06008023 Cytoplasmic expression of antibodies, antibody fragments and antibody fragment fusion proteins in E. coli; Phagemid for antibody screening; US0598089511/09/1999 Immunotoxin containing a disulfide-stabilized antibody fragment joined to a Pseudomonas exotoxin that does not require proteolytic activation; US05962255 Methods for producing recombinant vectors; US05955341Heterodimeric receptor libraries using phagemids; WO09939210A1 HIGH DENSITY ARRAYS FOR PROTEOME ANALYSIS AND METHODS AND COMPOSITIONS THEREFOR; WO09931267 METHODS FOR THE SIMULTANEOUS IDENTIFICATIONOF NOVEL BIOLOGICAL TARGETS AND LEAD STRUCTURES FOR DRUGDEVELOPMENT; US05869619 Modified antibody variable domains; US05855885Isolation and production of catalytic antibodies using phage technology; US0585180112/22/1998 Method of preparing polypeptide binding compositions derived from immunoglobulin variable regions; US05849500 Phagemid for antibody screening;binding composition; US0583784611/17/1998 Biosynthetic binding proteins for immuno-targeting; US0582133710/13/1998 Immunoglobulin variants; US05821123Modified antibody variable domains; US0578965508/04/1998 Transgenic animals expressing artificialepitope-tagged proteins; US05783384 Selection of binding-molecules; US05780225 Method for generating libaries of antibodygenes comprising amplification of diverse antibody DNAs and methodsfor using these libraries for the production of diverse antigen combining molecules; US05770356 Phagemids coexpressing a surface receptor and a surface heterologous protein; WO09808603 ISOLATION OF IMMUNOGLOBULINS; US05716805 Methods of preparing soluble, oligomeric proteins; US05595898 Modular assembly of antibody genes, antibodiesprepared thereby and use; US05582996 Bifunctional antibodies and method of preparing same; US05580717Recombinant library screening methods; Haab BB, Dunham MJ, Brown PO Protein microarrays for highly parallel detection and quantitation of specific proteins and antibodies in complex solutions.Genome Biol. 2001; 2(2):: Moch H, Kononen T, Kallioniemi OP, Sauter G. Tissue microarrays Borrebaeck CA.Antibodies in diagnostics - from immunoassays to protein chips.Immunol Today. 2000 Aug; 21 (8):379-82

Mendoza LG, et alHigh-throughput microarray-based enzyme-linked immunosorbent assay (ELISA). Biotechniques. 1999 Oct; 27(4):778-80, 782-6, 788. Morozov VN, Morozova TYa Electrospray deposition as a method for mass fabrication of mono- andmulticomponent microarrays of biological and biologically active substances.Anal Chem. 1999 Aug 1; 71(15):3110-7.619: Lueking A, et alProtein microarrays for gene expression and antibody screening. Anal Biochem. 1999 May 15; 270(1):103-11. Silzel JW, et alMass-sensing, multianalyte microarray immunoassay with imaging detection. Clin Chem. 1998 Sep; 44(9):2036-43 Ekins RP.Ligand assays: from electrophoresis to miniaturized microarrays.Clin Chem. 1998 Sep; 44(9):2015-30.

All publications referred to herein are indicative of the level of skill of those in the art to which the invention pertains.

With respect to lymphatic vessel associated ligands see also US 5, 776,755 (flt4), Mod Pathol 2000 Feb;13(2):180-5; EMBO J 2001 Mar 15;20(6):1223-1231, Nat Med 2001 Feb;7(2):199-205 Inhibition of lymphangiogenesis with resulting lymphedema in transgenic mice expressing soluble VEGF receptor-3 (VEGFR-3), J Pathol 2001 Feb;193(2):147-54 Localization of vascular endothelial growth factor-D in malignant melanoma suggests a role in tumour angiogenesis.

With respect to technologies having application herein see also Immunity 2001 Apr;14(4):437-46 The immunological barrier to xenotransplantation.Cascalho M, Platt JL.; WO 01/43779; WO 01/42285; WO 98/10795; WO 01/40803; WO 00/14212; Gastroenterology 2001 May;120(6):1330-8 An engineered human antibody to TNF (CDP571) for active Crohn's disease: a randomized double-blind placebo-controlled trial. Sandborn WJ; WO 01/44282; WO 01/40309; WO 01/40274; WO 01/44300; Ann Rheum Dis 2001 May;60(5):433 Cancer and autoimmunity: autoimmune and rheumatic features in patients with malignancies, Abu-Shakra M, et al.; WO 01/40468; WO 01/40307; WO 01/42297; WO 01/42294; WO 01/42296; WO 01/40456; WO 01/40308; WO 01/42306; Curr Opin Immunol 2001 Apr;13(2):134-40 Immunity against cancer: lessons learned from melanoma. Houghton AN, Gold JS, Blachere NE.; WO 01/42288; WO 01/42288; WO 01/43771; WO 01/42308; WO 01/41804; WO 01/39722; WO 01/44808; WO 01/43770; WO 01/16166; WO 01/41803; WO 01/13110; WO 00/32752; WO 98/33528; WO 01/43695; J Am Pharm Assoc (Wash) 2001 May-Jun;41 (3):383-91 Magic bullets finally find their mark.; Leukemia 2001 Apr;15(4):675-6; WO 01/44301; Anticancer Res 2001 Jan-Feb;21(1B):621-7 Immunotherapy for recurrent colorectal cancers with human monoclonal antibody SK-1. Koda K et al.; WO 01/10911; WO 01/42506; Int J Clin Pract 2001 Apr;55(3):211-6 Tumour necrosis factor as a therapeutic target in rheumatoid arthritis and other chronic inflammatory diseases: the clinical experience with infliximab; WO 01/44472; WO 01/40302; WO 01/40305)

With respect to surface plasmon resonance measurements of affinity see US6111652:High throughput surface plasmon resonance analysis system US06208422 Surface plasmon sensor EP01080365 SURFACE PLASMON RESONANCE SENSOR FOR THE SIMULTANEOUS MEASUREMENT OF A PLURALITY OF SAMPLES IN FLUID FORM WO00106236A HIGH THROUGHPUT ANALYSIS OF MOLECULAR INTERACTION USING SURFACE PLASMON RESONANCE High throughput surface plasmon resonance analysis system as well as Dimensions of antigen recognition and levels of immunological specificity. Adv Cancer Res. 2001;80:147-87. Use of optical biosensors for the study of mechanistically concerted surface adsorption processes. Anal Biochem. 2001 Jan 15;288(2):109-25 Experimental design for analysis of complex kinetics using surface plasmon resonance. Methods. 2000 Mar;20(3):310-8. and references cited in the foregoing references.

27: Skeie GO, Lunde PK, Sejersted OM, Mygland A, Aarli JA, Gilhus NE. Autoimmunity against the ryanodine receptor in myasthenia gravis. Acta Physiol Scand. 2001 Mar;171(3):379-84. 28: Haufs MG, Haneke E. Epidermolysis bullosa acquisita treated with basiliximab, an interleukin-2 receptor antibody. Acta Derm Venereol. 2001 Jan-Feb;81(1):72. Woo EY, et al Regulatory CD4(+)CD25(+) T cells in tumors from patients with early-stage non-small cell lung cancer and late-stage ovarian cancer. Cancer Res. 2001 Jun 15;61 (12):4766-72. Barrera P, Joosten LA, den Broeder AA, van De Putte LB, van Riel PL, van Den Berg WB. Effects of treatment with a fully human anti-tumour necrosis factor alpha monoclonal antibody on the local and systemic homeostasis of interleukin 1 and TNFalpha in patients with rheumatoid arthritis. Ann Rheum Dis. 2001 Jul;60(7):660-9. Nicholson JK, Browning SW, Hengel RL, Lew E, Gallagher LE, Rimland D, McDougal JS. CCR5 and CXCR4 expression on memory and naive T cells in HIV-1 infection and response to highly active antiretroviral therapy. J Acquir Immune Defic Syndr. 2001 Jun 1;27(2):105-15. Kung SK, Su RC, Shannon J, Miller RG. Characterization of four new monoclonal antibodies that recognize mouse natural killer activation receptors. Hybridoma. 2001 Apr;20(2):91-101. 43: Bank I, Dardik R, Levy V, Goldstein I, Shoham J. Differential expression and regulation of CD6 on T-cell subsets revealed by monoclonal antibody (MAb) CH11. Hybridoma. 2001 Apr;20(2):75-84.

With respect to making multifunctional ligands see also USP 5,731,168 and 5,821,333.

With respect to TNF and TNFR variants, and functional fragments thereof, for use as antibody targets and binding moieties with respect to various aspects of the invention herein see WO 00/67793, WO 01/30300, WO 01/49321, WO 00/62790, WO 01/03720, WO 00/60079, WO 97/46686, WO 01/41803, WO 01/38526, WO 01/37874, WO 01/12812, WO 01/12671, WO 01/05834, WO 01/03720, WO 00/77191 WO 00/73321, WO 00/71150, WO 00/67793, WO 00/67034, WO 00/66608, WO 00/66156., WO 01/24811, as well as references cited therein. Many other TNFR variants and TNF analogs are known in the art.

With respect to cytokines and cytokine receptors see also the latest editions of Cytokine Reference: A Compendium of Cytokines and Other Mediators of Host Defense by Joost J. Oppenheim (Editor), Jan Vilcek, Nicos A. Nicola (Editor); Cytokine Molecular Biology: A Practical Approach by Frances R. Balkwill (Editor), Fran Balkwill (Editor); Guidebook to Cytokines and Their Receptors by Nicos Nicola (Editor); The Cytokine Network and Immune Functions by Jacques Theze; Novel Cytokine Inhibitors by Gerry A. Higgs (Editor), Brian Henderson (Editor); Homology Folding of Proteins : Application to Cytokine Engineering by Subhashini Srinivasan; Cytokines and Cytokine Receptors (2001); International Review of Experimental Pathology : Cytokine-Induced Pathology, Part B : Inflammatory Cytokines, Receptors, and Disease by G.W. Richter, Kim Solez (Editor).

With respect to antibodies that bind to CCR5 see Mol Biol Cell 2002 Feb; 13(2) : 723-737.

With respect to variations in chemokine receptors, cytokine and other receptors that can be exploited according to one or more aspects of the invention herein see 1: Csaszar A, Abel T.Receptor polymorphisms and diseases.Eur J Pharmacol. 2001 Feb 23;414(1):9-22. 2: Gibejova A.Chemokine receptors.Acta Univ Palacki Olomuc Fac Med. 2000;143:9-18. 3: Nishimoto N, Kishimoto T, Yoshizaki K.Anti-interleukin 6 receptor antibody treatment in rheumatic disease.Ann Rheum Dis. 2000 Nov;59 Suppl 1:i21-7. 4: Aggarwal BB.Tumour necrosis factors receptor associated signalling molecules and their rolein activation of apoptosis, JNK and NF-kappaB.Ann Rheum Dis. 2000 Nov;59 Suppl 1:i6-16. 5: Grignani G, Maiolo A.Cytokines and hemostasis.Haematologica. 2000 Sep;85(9):967-72. 6: Idriss HT, Naismith JH.TNF alpha and the TNF receptor superfamily: structure-function relationship(s).Microsc Res Tech. 2000 Aug 1;50(3):184-95. 7: van Deventer SJ.Cytokine and cytokine receptor polymorphisms in infectious disease.lntensive Care Med. 2000;26 Suppl 1:S98-102. 8: Gessner A, Rollinghoff M.Biologic functions and signaling of the interleukin-4 receptor complexes.Immunobiology. 2000 Jan;201 (3-4):285-307. 9: Platanias LC, Fish EN.Signaling pathways activated by interferons.Exp Hematol. 1999 Nov;27(11):1583-92. 10: Schwertschlag US, Trepicchio WL, Dykstra KH, Keith JC, Turner KJ, Dorner AJ.Hematopoietic, immunomodulatory and epithelial effects of interleukin-11.Leukemia. 1999 Sep;13(9):1307-15. 11: Blasi F.The urokinase receptor. A cell surface, regulated chemokine.APMIS. 1999 Jan;107(1):96-101. 12: Izuhara K, Shirakawa T.Signal transduction via the interleukin-4 receptor and its correlation with atopy.Int J Mol Med. 1999 Jan;3(1):3-10. 13: Tsokos GC, Liossis SN.Lymphocytes, cytokines, inflammation, and immune trafficking.Curr Opin Rheumatol. 1998 Sep;10(5):417-25. 14: Morishita R, Nakamura S, Hayashi S, Aoki M, Matsushita H, Tomita N, YamamotoK, Moriguchi A, Higaki J, Ogihara T.Contribution of a vascular modulator, hepatocyte growth factor (HGF), to thepathogenesis of cardiovascular disease.J Atheroscler Thromb. 1998;4(3):128-34. 15: Kashiwamura S, Okamura H.[IL-18 and IL-18 receptor].Nippon Rinsho. 1998 Jul;56(7):1798-806. Japanese.16: Paxton WA, Kang S.Chemokine receptor allelic polymorphisms: relationships to HIV resistance anddisease progression.Semin Immunol. 1998 Jun;10(3):187-94. 17: Arend WP, Malyak M, Guthridge CJ, Gabay C.Interleukin-1 receptor antagonist: role in biology.Annu Rev Immunol. 1998;16:27-55. 18: Camussi G, Lupia E.The future role of anti-tumour necrosis factor (TNF) product in the treatmentof rheumatoid arthritis.Drugs. 1998 May;55(5):613-20. 19: Taga T, Kishimoto T.Gp130 and the interleukin-6 family of cytokines.Annu Rev Immunol. 1997;15:797-819. 20: Paul WE.Interleukin 4: signalling mechanisms and control of T cell differentiation.Ciba Found Symp. 1997;204:208-16; discussion 216-9; 1.(WO 01/49321) TNF INHIBITORS FOR THE TREATMENT OF NEUROLOGICAL, RETINAL AND MUSCULAR DISORDERS2.(WO 01/46261) METHOD FOR TREATING INFLAMMATION3.(WO 01/40464) INTERLEUKIN-1-RECEPTOR ASSOCIATED KINASE-3 (IRAK3) AND ITS USE IN PROMOTION OR INHIBITION OF ANGIOGENESIS AND CARDIOVASCULARIZATION4.(WO 01/40464) INTERLEUKIN-1-RECEPTOR ASSOCIATED KINASE-3 (IRAK3) AND ITS USE IN PROMOTION OR INHIBITION OF ANGIOGENESIS AND CARDIOVASCULARIZATION5.(WO 01/30850) UMLR POLYPEPTIDES6.(WO 00/77195) NUCLEIC ACID ENCODING NOVEL EGF-LIKE GROWTH FACTORS7.(WO 00/74719) METHOD OF TREATING CARCINOMA USING ANTIBODY THERAPY AND AMELIORATING SIDE EFFECTS ASSOCIATED WITH SUCH THERAPY8.(WO 00/02582) TREATMENT OF CELIAC DISEASE WITH INTERLEUKIN-15 ANTAGONISTS9.(WO 99/47170) PREVENTIVES OR REMEDIES FOR INFLAMMATORY INTESTINAL DISEASES CONTAINING AS THE ACTIVE INGREDIENT IL-6 ANTAGONISTS10.(WO 99/46376) RECEPTOR FROM THE SUPERFAMILY OF TNT-RECEPTORS FROM THE HUMAN LUNG11.(WO 99/43809) PROTEASE-ACTIVATED RECEPTOR 4 AND USES THEREOF12.(WO 98/48017) FAMILY OF IMMUNOREGULATORS DESIGNATED LEUKOCYTE IMMUNOGLOBULIN-LIKE RECEPTORS (LIR)13.(WO 98/47923) IL-5R ANTAGONISTS FOR TREATMENT OF INFLAMMATION, ASTHMA AND OTHER ALLERGIC DISEASES14.(WO 98/46620) A NOVEL HUMAN G-PROTEIN COUPLED RECEPTOR15.(WO 98/46265) METHODS FOR USING ANTAGONISTIC ANTI-AVB3 INTEGRIN ANTIBODIES16.(WO 98/36767) MODULATION OF THE HYPOTHALAMIC-PITUITARY-ADRENAL-ADIPOSE AXIS WITH LEPTIN RECEPTOR LIGANDS17.(WO 98/31809) HUMAN CC CHEMOKINE SLC18.(WO 98/30706) COMPOUNDS, COMPOSITIONS AND METHODS FOR THE ENDOCYTIC PRESENTATION OF IMMUNOSUPPRESSIVE FACTORS19.(WO 98/24817) NOVEL DNA, NOVEL PROTEIN, AND NOVEL ANTIBODY20.(WO 98/22499) NEURON AND NEURAL TUMOUR GROWTH REGULATORY SYSTEM, ANTIBODIES THERETO AND USES THEREOF21.(WO 98/19706) IDENTIFICATION OF UNIQUE BINDING INTERACTIONS BETWEEN CERTAIN ANTIBODIES AND THE HUMAN B7.1 AND B7.2 CO-STIMULATORY ANTIGENS22.(WO 98/18456) PROTEASE-ACTIVATED RECEPTOR 3 AND USES THEREOF23.(WO 98/14480) G PROTEIN-COUPLED RECEPTOR ANTAGONISTS 24.(WO 98/02541 ) GAMMA-HEREGULIN25.(WO 97/49818) G-BETA-GAMMA REGULATED PHOSPHATIDYLINOSITOL-3' KINASE26.(WO 97/48804) TIE-2 RECEPTOR LIGANDS (TIE LIGAND-3; TIE LIGAND-4) AND THEIR USES27.(WO 97/41225) MAMMALIAN MIXED LYMPHOCYTE RECEPTORS, CHEMOKINE RECEPTORS [MMLR-CCR]28.(WO 97/24373) MONOCLONAL ANTIBODY ANTAGONISTS TO HAEMOPOIETIC GROWTH FACTORS29.(WO 97/21732) DESIGN OF HORMONE-LIKE ANTIBODIES WITH AGONISTIC AND ANTAGONISTIC FUNCTIONS, 6,235,880 Human sulfonylurea receptor 6,221,660 DNA encoding SNORF25 receptor 6,214,797 Urocortin peptides, nucleic acid encoding same methods for usingsame6,214,344 Hepatocyte growth factor receptor antagonists and uses thereof 6,210,904 Anticoagulant test6,207,152 Hepatocyte growth factor receptor antagonists and uses thereof6,204,017 Polynucleotide encoding a histamine receptor 6,197,541 Recombinant thrombin receptors and assays using them6,184,358 IP-10/Mig receptor designated CXCR3, antibodies, nucleic acids,and methods of use therefor6,177,079 Antagonists of interleukin-156,177,078 Monoclonal antibody antagonists to IL-36,177,077 TNT inhibitors for the treatment of neurological disorders 6,171,815 Human sulfonylurea receptor6,168,783 Antagonists of interleukin-156,166,185 Antibodies to human TIE-2 ligands6,165,466 Antagonists of interleukin-156,162,431 Serine/threonine protein kinase6,143,870 Thrombin receptor homolog6,136,957 Antibodies which bind granulocyte-macrophane colony-stimulatingfactor receptor6,124,101 Recombinant thrombin receptor and related pharmaceuticals6,111,075 Protese-activated receptor PAR4 (ZCHEMR2) 6,103,874 Human KDEL receptor 6,096,873 Gamma-heregulin 6,086,874 Antitumor agent effect enhancer containing IL-6 antagonist56,084,075 Agonist and antagonist antibodies to the chemokine receptor-2(CCR2) 6,063,596 G-protein coupled receptors associated with immune response6,054,292 T-cell receptor protein6,043,212 Recombinant C140 receptor, its agonists and antagonists, andnucleic acids encoding the receptor6,033,869 Polynucleotide encoding a novel human cytokine/steroid receptor 6,024,936 Antibody-based method of localizing activated thrombinreceptors 6,017,763 G-beta-gamma regulated phosphatidylinositol-3' kinase6,013,480 Antagonists of interleukin-156,013,479 Human Emr1-like G protein coupled receptor 5,994,097 Polynucleotide encoding human G-protein coupled receptor5,985,828 Mammalian receptors for interleukin-10 (IL-10) 5,985,583 Cloning and expression of gonad otropin-releasing hormonereceptor5,977,072 High affinity immunoglobulin E receptor-like protein5,976,852 K.kappa./.mu.-like protein tyrosine phosphatase, PTP .lambda.5,976,815 Bioassay using ALK-7, a novel serine threonine kinase receptor 5,972,621 Methods of identifying compounds that modulate body weightusing the OB receptor 5,965,709 IgE antagonists5,965,365 Serine/threonine protein kinase5,955,303 Human chemokine receptor-like protein 5,952,175 DNA encoding a human progesterone receptor complex p23-likeproteins,945,308 Human oxidized LDL receptor5,942,606 Viral receptor protein 5,928,887 .kappa./.mu.-Like protein tyrosine phosphatase, PTP .lambda.5,912,144 Edg-1-receptor homolog5,902,585 Methods of inducing T cell unresponsiveness to donor tissue ororgan in a recipient with GP39 antagonists 5,892;014 DNA encoding a protease-activated receptor 35,891,720 Isolated DNA encoding a novel human G-protein coupled receptor5,891,674 Insulin receptor tyrosine kinase substrate 5,891,638 Serine threonine kinase receptor, alk-75,888,811 Corticotropin-releasing hormone receptor5,888,510 Chronic rheumatoid arthritis therapy containing IL-6 antagonistas effective component 5,886,148 Parathyroid hormone receptor5,874,400 Recombinant C140 receptor, its agonists and antagonists, andnucleic acids encoding the receptor 5,874,273 G-beta-gamma regulated phosphatidylinositol-3' Winase5,874,224 Growth factor receptor binding protein 5,871,930 High affinity immunoglobulin E receptor-like protein 5,869,633 Thrombin receptor homolog polynucleotide5,869,609 G protein coupled glutamate receptors5,869,271 G-beta-gamma regulated phosphatidylinositol-3' kinase5,869,049 Methods of inducing T cell unresponsiveness to bone marrow withgp39 antagonists 5,863,796 Antibodies which specifically bind mammalian receptors forinterieukin-10 (IL-10)5,863,766 Human sigma receptor 5,859,201 G-beta-gamma regulated phosphatidylinositol-3' kinase5,856,448 Antibodies specifically reactive with thrombin receptor and itscomponents 5,856,133 G-beta-gamma regulated phosphatidylinositol-3'kinase5,856,132 G-beta-gamma regulated phosphatidylinositol-3' kinase5,851,797Tie ligand-3, methods of making and uses thereof5,840,853 Parathyroid hormone receptor and DNA encoding same5,837,499 DNA encoding C5A receptor antagonists having substantially noagonist activity, and methods of expressing same5,834,240 DNA encoding a transforming growth factor-.beta. receptor associated protein 5,833,987 Treatment of T cell mediated autoimmune disorders5,831,047 Oligonucleotide probes to LAP4 sensitive glutamate receptorsequences 5,830,678 Method for identifying a target peptide that modulates thebinding of epinectin ligand to integrin receptors5,824,500 Nucleic acid encoding novel human KDEL receptor 5,817,480 DNA encoding a histamine H2 receptor5,814,507 .kappa./.mu.-like protein tyrosine phosphatase, PTP .lambda.5,814,464 Nucleic acids encoding TIE-2 ligand-25,811,245 Antibodies that specifically bind to ALK-7, a novel serinethreonine kinase receptor5,807,824C5 A receptor antagonists having substantially no agonistactivity5,795,966 Antagonists of interleukin-155,789,565 Serine threonine kinase receptor, ALK-7 5,789,192 Mammalian receptors for interleukin-10 (IL-10)5,763,575 Agonist and antagonist peptides of the C140 receptor 5,759,994 Recombinant thrombin receptor and related pharmaceuticals5,750,366 Cloning and expression of gonadotropin-releasing hormonereceptor 5,747,279 Nucleic acid molecules encoding kappa.sub.3 opioid receptors,receptors encoded thereby, and uses thereof5,747,267 Method for identifying a G protein coupled glutamate receptoragonist and antagonist5,738,999 L-AP4 sensitive glutamate receptors5,730,976 Method for treating macrophage pathogen infections by TGF-Bantagonists5,726,036 Granulocyte-macrophage colony-stimulating factor receptor andderivatives thereof5,721,107 Antibodies to G protein coupled glutamate receptors5,716,804 Mammalian interleukin-10 (IL-10) -super-activating receptors;and variants5,716,789 Method to determine ligands, agonist and antagonist of C140receptor 5,707,632 Receptors for fibroblast growth factors5,688,768 Recombinant thrombin receptor, and related pharmaceuticals5,686,597 Thrombin receptor homolog 5,686,292 Hepatocyte growth factor receptor antagonist antibodies anduses thereof5,683,884 Methods for identifying modulators of human calcitoninmediated metabolism5,683,693 Method for inducing T cell unresponsiveness to a tissue ororgan graft with anti-CD40 ligand antibody or soluble CD405,674,981 Human calcitonin receptor polypeptides5,674,689 Human calcitonin receptor polypeptides and methods of use5,646,036 Nucleic acids encoding hepatocyte growth factor receptorantagonist antibodies5,629,283 Granulocyte-macrophage colony-stimulating factor receptor andderivatives thereof5,622,839 Recombinant production of human calcitonin receptorpolypeptides5,614,609 Serine threonine kinase receptor5,556,780 CDNAS encoding mouse and rat type-2 angiotensin II receptorsand their expression in host cells5,543,143 Method for activating macrophages/monocytes5,516,894 A.sub.2b-adenosine receptors5,514,555 Assays and therapeutic methods based on lymphocytechemoattractants 5,506,107 Selecting ligand agonists and antagonists5,494,806 DNA and vectors encoding the parathyroid hormone receptor,transformed cells, and recombinant production of PTHR proteins andpeptides5,451,658 Antagonists of human gamma interferon5,441,935 Growth factor receptors5,385,831 Method for producing a mammalian G protein coupled glutamatereceptor5,334,380 Anti-endotoxin, interleukin-1 receptor antagonist andanti-tumor necrosis factor antibody with arginine-free formulations forthe treatment of hypotension5,256,766 Recombinant thrombin receptor and related pharmaceuticals5,177,190 Purified C5a receptor from human polymorphonuclear leukocytes4,857,637 Methods and compositions for immunologically modulating growthhormone receptor activity; and references cited therein. (see also 1.(WO 01/49744) MOUSE G-PROTEIN COUPLED RECEPTOR MAS 2.(WO 01/49726) A NOVEL POLYPEPTIDE-HUMAN NATRIURETIC PEPTIDE RECEPTOR 18 AND THE POLYNUCLEOTIDE ENCODING SAID POLYPEPTIDE 3.(WO 01/49321) TNF INHIBITORS FOR THE TREATMENT OF NEUROLOGICAL, RETINAL AND MUSCULAR DISORDERS 4.(WO 01/00657) NOVEL INDOLE PEPTIDOMIMETICS AS THROMBIN RECEPTOR ANTAGONISTS 5.(WO 00/62790) SOLUBLE TUMOR NECROSIS FACTOR RECEPTOR TREATMENT OF MEDICAL DISORDERS 6.(WO 01/03720) PROMOTION OR INHIBITION OF ANGIOGENESIS AND CARDIOVASCULARIZATION BY TUMOR NECROSIS FACTOR LIGAND/RECEPTOR HOMOLOGS 7.(WO 01/46261) METHOD FOR TREATING INFLAMMATION 8.(WO 01/46191) 4-[ARYL(8-AZABICYCLO[3.2.1]OCTAN-3-YL)]AMINOBENZOIC ACID DERIVATIVES 9.(WO 01/46176) NON PEPTIDE TACHYKININ RECEPTOR ANTAGONISTS 10.(WO 01/45730) TWEAK RECEPTOR 11.(WO 01/45703) NITROSATED AND NITROSYLATED CYCLOOXYGENASE-2 INHIBITORS, COMPOSITIONS AND METHODS OF USE 12.(WO 01/40464) INTERLEUKIN-1-RECEPTOR ASSOCIATED KINASE-3 (IRAK3) AND ITS USE IN PROMOTION OR INHIBITION OF ANGIOGENESIS AND CARDIOVASCULARIZATION 13.(WO 01/44213) NEW P2X7 RECEPTOR ANTAGONISTS FOR USE IN THE TREATMENT OF INFLAMMATORY, IMMUNE OR CARDIOVASCULAR DISEASES 14.(WO 01/42268) DOG OREXIN 1 RECEPTOR 15.(WO 01/42208) CYCLOAMINE CCR5 RECEPTOR ANTAGONISTS 16.(WO 01/41752) ISOFORM SPECIFIC INHIBITION FOR TREATMENT OF PAIN AND REDUCTION OF ANESTHETIC THRESHOLD 17.(WO 01/03720) PROMOTION OR INHIBITION OF ANGIOGENESIS AND CARDIOVASCULARIZATION BY TUMOR NECROSIS FACTOR LIGAND/RECEPTOR HOMOLOGS 18.(WO 01/40464) INTERLEUKIN-1-RECEPTOR ASSOCIATED KINASE-3 (IRAK3) AND ITS USE IN PROMOTION OR INHIBITION OF ANGIOGENESIS AND CARDIOVASCULARIZATION 19.(WO 01/40259) MONKEY OREXIN 1 RECEPTOR 20.(WO 01/40252) MONKEY CALCIUM SENSING RECEPTOR 21.(WO 01/04139) HUMAN AXOR29 RECEPTOR 22.(WO 01/36480) MOUSE 7-TRANSMEMBRANE RECEPTOR, AXOR45 23.(WO 01/00656) NOVEL INDAZOLE PEPTIDOMIMETICS AS THROMBIN RECEPTOR ANTAGONISTS 24.(WO 00/67793) DEATH DOMAIN CONTAINING RECEPTOR 4 25.(WO 01/34645) MODULATING IL-13 ACTIVITY USING MUTATED IL-13 MOLECULES THAT ARE ANTAGONISTS OR AGONISTS OF IL-13 26.(WO 01/34138) COMPOSITIONS AND METHODS FOR TREATMENT OF NEUROLOGICAL DISORDERS AND NEURODEGENERATIVE DISEASES 27.(WO 01/32656) POLYMORPHIC FORM OF A TACHYKININ RECEPTOR ANTAGONIST 28.(WO 01/32166) NEW COMBINATION COMPRISING A β2-ADRENORECEPTOR AGONIST AND A LEUKOTRIENE RECEPTOR ANTAGONIST 29.(WO 01/32163) NEW COMBINATION COMPRISING A BETA 2 (β)2 ADRENO RECEPTOR AGONIST AND A LENKOTRIENE RECEPTOR ANTAGONIST 30.(WO 01/01922) USE OF SUBSTANCE P ANTAGONISTS FOR THE TREATMENT OF ADENOCARCINOMA 31.(WO 01/30850) UMLR POLYPEPTIDES 32.(WO 01/27153) A MURINE SEVEN-TRANSMEMBRANE RECEPTOR, MUS MUSCULUS MHNEAA81 33.(WO 01/25269) NOVEL HUMAN G-PROTEIN COUPLED RECEPTOR 34.(WO 01/24828) MODULATORS OF CYTOKINE MEDIATED SIGNALLING PATHWAYS AND INTEGRIN αVβ3 RECEPTOR ANTAGONISTS FOR COMBINATION THERAPY 35.(WO 01/24798) USE OF CENTRAL CANNABINOID RECEPTOR ANTAGONIST FOR PREPARING MEDICINES 36.(WO 01/24797) INTEGRIN RECEPTOR ANTAGONISTS 37.(WO 00/68250) 7TM RECEPTOR RAT APJ 38.(WO 01/16121) HETEROCYCLIC COMPOUNDS AND METHODS OF USE THEREOF 39.(WO 01/14406) ANTIANDROGEN AGENTS 40.(WO 01/12671) HUMAN TUMOR NECROSIS FACTOR RECEPTOR TR16 41.(WO 01/10891) IL-16 ANTAGONISTS 42.(WO 01/10889) RAT-G-PROTEIN COUPLED RECEPTOR BRS3 43.(WO 01/10423) USE OF 5-HT3 RECEPTOR ANTAGONISTS FOR THE TREATMENT OF INFLAMMATIONS OF THE RESPIRATORY TRACT 44.(WO 01/07028) THE USE OF RETINOID RECEPTOR ANTAGONISTS IN THE TREATMENT OF PROSTATE CARCINOMA 45.(WO 01/05834) HUMAN TUMOR NECROSIS FACTOR RECEPTORS TR13 AND TR14 46.(WO 01/05783) BRADYKININ B1 RECEPTOR ANTAGONISTS 47.(WO 01/04139) POLYNUCLEOTIDE AND POLYPEPTIDE SEQUENCES OF HUMAN AXOR29 RECEPTOR AND METHODS OF SCREENING FOR AGONISTS AND ANTAGONISTS OF THE INTERACTION BETWEEN HUMAN AXOR29 RECEPTOR AND ITS LIGANDS 48.(WO 01/03720) PROMOTION OR INHIBITION OF ANGIOGENESIS AND CARDIOVASCULARIZATION BY TUMOR NECROSIS FACTOR LIGANDIRECEPTOR HOMOLOGS 49.(WO 01/01922) USE OF SUBSTANCE P ANTAGONISTS IN THE TREATMENT OF THE ADENOCARCINOMAS 50.(WO 01/00659) BENZIMIDAZOLONE PEPTIDOMIMETICS AS THROMBIN RECEPTOR ANTAGONISTS 51.(WO 01/00657) NOVEL INDOLE PEPTIDOMIMETICS AS THROMBIN RECEPTOR ANTAGONISTS 52.(WO 01/00656) NOVEL INDAZOLE PEPTIDOMIMETICS AS THROMBIN RECEPTOR ANTAGONISTS 53.(WO 01/00576) INDOLE AND INDAZOLE UREA-PEPTOIDS AS THROMBIN RECEPTOR ANTAGONISTS 54.(WO 01/00198) COMPOSITIONS AND METHODS OF TREATING CANCER USING COMPOSITIONS COMPRISING AN INHIBITOR OF ENDOTHELIN RECEPTOR ACTIVITY 55.(WO 00/78317) INTEGRIN RECEPTOR ANTAGONISTS 56.(WO 00/77195) NUCLEIC ACID ENCODING NOVEL EGF-LIKE GROWTH FACTORS 57.(WO 00/76502) METHODS AND COMPOSITIONS FOR TREATING RAYNAUD'S PHENOMENON AND SCLERODERMA 58.(WO 00/74719) METHOD OF TREATING CARCINOMA USING ANTIBODY THERAPY AND AMELIORATING SIDE EFFECTS ASSOCIATED WITH SUCH THERAPY 59.(WO 00/73321) HUMAN TUMOR NECROSIS FACTOR RECEPTOR TR10 60.(WO 00/72801) ALPHA V INTEGRIN RECEPTOR ANTAGONISTS 61.(WO 00/71150) TUMOR NECROSIS FACTOR RECEPTOR 5 62.(WO 00/69831) SPIROIMIDAZOLIDINE DERIVATIVES, THEIR PREPARATION, THEIR USE AND PHARMACEUTICAL PREPARATIONS COMPRISING THEM 63.(WO 00/69820) CYCLIC AMINE DERIVATIVES AND THEIR USES 64.(WO 00/69463) COMPOSITIONS AND METHODS FOR TREATING CELL PROLIFERATION DISORDERS 65.(WO 00/69459) TREATMENT OF REFRACTORY HUMAN TUMORS WITH EPIDERMAL GROWTH FACTOR RECEPTOR ANTAGONISTS 66.(WO 00/68250) 7TM RECEPTOR RAT APJ 67.(WO 00/68244) 7TM , RECEPTOR MOUSE APJ 68.(WO 00/67793) DEATH DOMAIN CONTAINING RECEPTOR 4 69.(WO 00/67034) METHODS OF USE OF THE TACI/TACI-L INTERACTION 70.(WO 00/67024) CANCER TREATMENT WITH ENDOTHELIN RECEPTOR ANTAGONISTS 71.(WO 00/66632) AGONISTS OR ANTAGONISTS FOR HAEMOPOIETIC GROWTH FACTORS 72.(WO 00/66522) GLUCOCORTICOID RECEPTOR MODULATORS 73.(WO 00/66156) DEATH DOMAIN CONTAINING RECEPTOR 5 74.(WO 00/64465) DEATH DOMAIN CONTAINING RECEPTORS 75.(WO 00/62790) SOLUBLE TUMOR NECROSIS FACTOR RECEPTOR TREATMENT OF MEDICAL DESORDERS 76.(WO 00/59532) THE USE OF DOMAINS OF TYPE IV COLLAGEN T INHIBIT ANGIOGENESIS AN TUMOUR GROWTH 77.(WO 00/56862) HUMAN TUMOR NECROSIS FACTOR RECEPTOR TR9 78.(WO 00/56405) HUMAN TUMOR NECROSIS FACTOR RECEPTOR-LIKE 2 79.(WO 00/54772) AMYOTROPIC LATERAL SCLEROSIS TREATMENT WITH A COMBINATION OF RILUZOLE AND AN AMPA RECEPTOR ANTAGONIST 80.(WO 00/53596) IMIDAZOLE COMPOUNDS SUBSTITUTED WITH A SIX OR SEVEN MEMBERED HETEROCYCLIC RING CONTAINING TWO NITROGEN ATOMS 81.(WO 00/53175) COMPOUNDS AND METHODS 82.(WO 00/52028) TUMOR NECROSIS FACTOR RECEPTORS 6&agr; and 6&bgr; 83.(WO 00/51974) ALPHA-AMINOACETIC ACID DERIVATIVES USEFUL AS ALPHA 4 BETA 7 - RECEPTOR ANTAGONISTS 84.(WO 00/50459) HUMAN TUMOR NECROSIS FACTOR RECEPTOR-LIKE PROTEINS TR11, TR11SV1, AND TR11SV2 85.(WO 00/49170) MURINE 11cby RECEPTOR 86.(WO 00/48603) DIBENZO-AZEPINE DERIVATIVES AS &agr;V INTEGRIN RECEPTOR ANTAGONISTS 87.(WO 00/48597) SYSTEMIC USE OF 5-HT 3 RECEPTOR ANTAGONISTS AGAINST RHEUMATIC INFLAMMATORY PROCESSES 88.(WO 00/48581) USE OF 5-HT3 RECEPTOR ANTAGONISTS 89.(WO 00/46343) SCREENING ASSAY FOR ANTAGONISTS OF FGFR-MEDIATED MALIGNANT CELL TRANSFORMATION AND TUMOR FORMATION 90.(WO 00/46215) BENZAZEPINE DERIVATIVES AS ALPHA-V INTEGRIN RECEPTOR ANTAGONISTS 91.(WO 00/46197) INDOLE DERIVATIVES AND THEIR USE AS MCP-1 RECEPTOR ANTAGONISTS 92.(WO 00/44763) COMPOSITIONS FOR TREATING INFLAMMATORY RESPONSE 93.(WO 00/43031) TUMOR NECROSIS FACTOR ANTAGONISTS AND THEIR USE IN ENDOMETRIOSIS 94.(WO 00/42852) COMPOUNDS AND METHODS 95.(WO 00/40716) SOLUBLE RECEPTOR BR43x2 AND METHODS OF USING 96.(WO 00/40239) COMPOUNDS AND METHODS 97.(WO 00/39166) NOVEL HYALURONAN-BINDING PROTEINS AND ENCODING GENES 98.(WO 00/37462) NON-PEPTIDE NK 1 RECEPTORS ANTAGONISTS 99.(WO 00/35887) VITRONECTIN RECEPTOR ANTAGONIST PHARMACEUTICALS 100.(WO 00/35492) VITRONECTIN RECEPTOR ANTAGONIST PHARMACEUTICALS 51.(WO 01/00657) NOVEL INDOLE PEPTIDOMIMETICS AS THROMBIN RECEPTOR ANTAGONISTS 52.(WO 01/00656) NOVEL INDAZOLE PEPTIDOMIMETICS AS THROMBIN RECEPTOR ANTAGONISTS 53.(WO 01/00576) INDOLE AND INDAZOLE UREA-PEPTOIDS AS THROMBIN RECEPTOR ANTAGONISTS 54.(WO 01/00198) COMPOSITIONS AND METHODS OF TREATING CANCER USING COMPOSITIONS COMPRISING AN INHIBITOR OF ENDOTHELIN RECEPTOR ACTIVITY 55.(WO 00/78317) INTEGRIN RECEPTOR ANTAGONISTS 56.(WO 00/77195) NUCLEIC ACID ENCODING NOVEL EGF-LIKE GROWTH FACTORS 57.(WO 00/76502) METHODS AND COMPOSITIONS FOR TREATING RAYNAUD'S PHENOMENON AND SCLERODERMA 58.(WO 00/74719) METHOD OF TREATING CARCINOMA USING ANTIBODY THERAPY AND AMELIORATING SIDE EFFECTS ASSOCIATED WITH SUCH THERAPY 59.(WO 00/73321) HUMAN TUMOR NECROSIS FACTOR RECEPTOR TR10 60.(WO 00/72801) ALPHA V INTEGRIN RECEPTOR ANTAGONISTS 61.(WO 00/71150) TUMOR NECROSIS FACTOR RECEPTOR 5 62.(WO 00/69831) SPIROIMIDAZOLIDINE DERIVATIVES, THEIR PREPARATION, THEIR USE AND PHARMACEUTICAL PREPARATIONS COMPRISING THEM 63.(WO 00/69820) CYCLIC AMINE DERIVATIVES AND THEIR USES 64.(WO 00/69463) COMPOSITIONS AND METHODS FOR TREATING CELL PROLIFERATION DISORDERS 65.(WO 00/69459) TREATMENT OF REFRACTORY HUMAN TUMORS WITH EPIDERMAL GROWTH FACTOR RECEPTOR ANTAGONISTS 66.(WO 00/68250) 7TM RECEPTOR RAT APJ 67.(WO 00/68244) 7TM RECEPTOR MOUSE APJ 68.(WO 00/67793) DEATH DOMAIN CONTAINING RECEPTOR 4 69.(WO 00/67034) METHODS OF USE OF THE TACI/TACI-L INTERACTION 70.(WO 00/67024) CANCER TREATMENT WITH ENDOTHELIN RECEPTOR ANTAGONISTS 71.(WO 00/66632) AGONISTS OR ANTAGONISTS FOR HAEMOPOIETIC GROWTH FACTORS 72.(WO 00/66522) GLUCOCORTICOID RECEPTOR MODULATORS 73.(WO 00/66156) DEATH DOMAIN CONTAINING RECEPTOR 5 74.(WO 00/64465) DEATH DOMAIN CONTAINING RECEPTORS 75.(WO 00/62790) SOLUBLE TUMOR NECROSIS FACTOR RECEPTOR TREATMENT OF MEDICAL DESORDERS 76.(WO 00/59532) THE USE OF DOMAINS OF TYPE IV COLLAGEN T INHIBIT ANGIOGENESIS AN TUMOUR GROWTH 77.(WO 00/56862) HUMAN TUMOR NECROSIS FACTOR RECEPTOR TR9 78.(WO 00/56405) HUMAN TUMOR NECROSIS FACTOR RECEPTOR-LIKE 2 79.(WO 00/54772) AMYOTROPIC LATERAL SCLEROSIS TREATMENT WITH A COMBINATION OF RILUZOLE AND AN AMPA RECEPTOR ANTAGONIST 80.(WO 00/53596) IMIDAZOLE COMPOUNDS SUBSTITUTED WITH A SIX OR SEVEN MEMBERED HETEROCYCLIC RING CONTAINING TWO NITROGEN ATOMS 81.(WO 00/53175) COMPOUNDS AND METHODS 82.(WO 00/52028) TUMOR NECROSIS FACTOR RECEPTORS 6&agr; and 6&bgr; 83.(WO 00/51974) ALPHA-AMINOACETIC ACID DERIVATIVES USEFUL AS ALPHA 4 BETA 7 - RECEPTOR ANTAGONISTS 84.(WO 00/50459) HUMAN TUMOR NECROSIS FACTOR RECEPTOR-LIKE PROTEINS TR11, TR11SV1, AND TR11SV2 85.(WO 00/49170) MURINE 11cby RECEPTOR 86.(WO 00/48603) DIBENZO-AZEPINE DERIVATIVES AS &agr;V INTEGRIN RECEPTOR ANTAGONISTS 87.(WO 00/48597) SYSTEMIC USE OF 5-HT 3 RECEPTOR ANTAGONISTS AGAINST RHEUMATIC INFLAMMATORY PROCESSES 88.(WO 00/48581) USE OF 5-HT3 RECEPTOR ANTAGONISTS 89.(WO 00/46343) SCREENING ASSAY FOR ANTAGONISTS OF FGFR-MEDIATED MALIGNANT CELL TRANSFORMATION AND TUMOR FORMATION 90.(WO 00/46215) BENZAZEPINE DERIVATIVES AS ALPHA-V INTEGRIN RECEPTOR ANTAGONISTS 91.(WO 00/46197) INDOLE DERIVATIVES AND THEIR USE AS MCP-1 RECEPTOR ANTAGONISTS 92.(WO 00/44763) COMPOSITIONS FOR TREATING INFLAMMATORY RESPONSE 93.(WO 00/43031) TUMOR NECROSIS FACTOR ANTAGONISTS AND THEIR USE IN ENDOMETRIOSIS 94.(WO 00/42852) COMPOUNDS AND METHODS 95.(WO 00/40716) SOLUBLE RECEPTOR BR43x2 AND METHODS OF USING 96.(WO 00/40239) COMPOUNDS AND METHODS 97.(WO 00/39166) NOVEL HYALURONAN-BINDING PROTEINS AND ENCODING GENES 98.(WO 00/37462) NON-PEPTIDE NK 1 RECEPTORS ANTAGONISTS 99.(WO 00/35887) VITRONECTIN RECEPTOR ANTAGONIST PHARMACEUTICALS 100.(WO 00/35492) VITRONECTIN RECEPTOR ANTAGONIST PHARMACEUTICALS 101.(WO 00/35488) VITRONECTIN RECEPTOR ANTAGONIST PHARMACEUTICALS 102.(WO 00/35455) HETEROARYL-ARYL UREAS AS IGF-1 RECEPTOR ANTAGONISTS 103.(WO 00/32578) BENZIMIDAZOLE COMPOUNDS THAT ARE VITRONECTIN RECEPTOR ANTAGONISTS 104.(WO 00/28988) NITROSATED AND NITROSYLATED H2 RECEPTOR ANTAGONIST COMPOUNDS, COMPOSITIONS AND METHODS OF USE 105.(WO 00/27421) LOCAL USE OF SOLUBLE TUMOR NECROSIS RECEPTOR I (sTNFRI) FOR PROPHYLAXIS AND TREATMENT OF CORNEAL TRANSPLANT REJECTION AND OTHER DISORDERS OF THE EYE 106.(WO 00/25805) VASCULAR ENDOTHELIAL GROWTH FACTOR-LIKE PROTEIN FROM ORF VIRUS NZ2 BINDS AND ACTIVATES MAMMALIAN VEGF RECEPTOR-2 107.(WO 00/25745) IRRIGATION SOLUTION AND METHOD FOR INHIBITION OF PAIN AND INFLAMMATION 108.(WO 00/24395) NEW USE OF GLUTAMATE ANTAGONISTS FOR THE TREATMENT OF CANCER 109.(WO 00/23471) USE OF A CYTOKINE-PRODUCING LACTOCOCCUS STRAIN TO TREAT COLITIS 110.(WO 00/23469) FRAGMENTS OF INSULIN-LIKE GROWTH FACTOR BINDING PROTEIN AND INSULIN-LIKE GROWTH FACTOR, AND USES THEREOF 111.(WO 00/23438) N-(IMIDAZOLYLALKYL)SUBSTITUTED CYCLIC AMINES AS HISTAMINE-H 3 AGONISTS OR ANTAGONISTS 112.(WO 00/23113) PEPTIDE-BASED CARRIER DEVICES FOR STELLATE CELLS 113.(WO 00/23066) IRRIGATION SOLUTION AND METHOD FOR INHIBITION OF PAIN AND INFLAMMATION 114.(WO 00/23062) IRRIGATION SOLUTION AND METHOD FOR INHIBITION OF PAIN AND INFLAMMATION 115.(WO 00/20578) A METHOD OF MODULATING CELL SURVIVAL AND REAGENTS USEFUL FOR SAME 116.(WO 00/20389) NAPHTHALENECARBOXAMIDES AS TACHYKININ RECEPTOR ANTAGONISTS 117.(WO 00/20371) PROSTAGLANDIN RECEPTOR LIGANDS 118.(WO 00/20003) NAPHTHALENECARBOXAMIDES AS TACHYKININ RECEPTOR ANTAGONISTS 119.(WO 00/14109) BASIC PRODUCTS HAVING ANTAGONISTIC ACTIVITY ON THE NK-1 RECEPTOR AND THEIR USE IN PHARMACEUTICAL COMPOSITIONS 120.(WO 00/10391) THE USE OF ADENOSINE A3 RECEPTOR ANTAGONISTS TO INHIBIT TUMOR GROWTH 121.(WO 00/09503) INTEGRIN RECEPTOR ANTAGONISTS 122.(WO 00/09152) THERAPEUTIC CHEMOKINE RECEPTOR ANTAGONISTS 123.(WO 00/08001) SUBSTITUTED ISOXAZOLE AS ESTROGEN RECEPTOR MODULATORS 124.(WO 00/06169) INTEGRIN RECEPTOR ANTAGONISTS 125.(WO 00/03716) TOPICAL COMPOSITIONS COMPRISING AN OPIOID ANALGESIC AND AN NMDA ANTAGONIST 126.(WO 00/02859) N-SUBSTITUTED NAPHTHALENE CARBOXAMIDES AS NEUROKININ-RECEPTOR ANTAGONISTS 127.(WO 00/02582) TREATMENT OF CELIAC DISEASE WITH INTERLEUKIN-15 ANTAGONISTS 128.(WO 00/01802) PEPTIDE ANTAGONISTS OF THE HUMAN UROKINASE RECEPTOR AND METHOD FOR SELECTING THEM 129.(WO 00/00194) OPHTHALMIC USES OF PPARGAMMA AGONISTS AND PPARGAMMA ANTAGONISTS 130.(WO 99/65944) PEPTIDE INHIBITORS OF &agr;V&bgr;3 AND &agr;V&bgr;5 131.(WO 99/62955) METHOD OF DESIGNING AGONISTS AND ANTAGONISTS TO EGF RECEPTOR FAMILY 132.(WO 99/60015) IMIDAZOLIDINE DERIVATIVES, THE PRODUCTION THEREOF, THEIR USE AND PHARMACEUTICAL PREPARATIONS CONTAINING THE SAME 133.(WO 99/59635) USE OF A COX-2 INHIBITOR AND A NK-1 RECEPTOR ANTAGONIST FOR TREATING INFLAMMATION 134.(WO 99/58142) USE OF ANTI-PROLACTIN AGENTS TO TREAT PROLIFERATIVE CONDITIONS 135.(WO 99/58097) USE OF ANTI-PROLACTIN AGENTS TO TREAT PROLIFERATIVE CONDITIONS 136.(WO 99/57245) METHODS OF SCREENING FOR AGONISTS AND ANTAGONISTS OF THE INTERACTION BETWEEN THE HUMAN KIAA0001 RECEPTOR AND LIGANDS THEREOF 137.(WO 99/51245) NON-PEPTIDE BRADYKININ RECEPTOR ANTAGONISTS FOR USE IN TREATING OPHTHALMIC DISEASES AND DISORDERS 138.(WO 99150249) INTEGRIN ANTAGONISTS 139.(WO 99/49856) ANTAGONISTS FOR TREATMENT OF CD11/CD18 ADHESION RECEPTOR MEDIATED DISORDERS 140.(WO 99/47170) PREVENTIVES OR REMEDIES FOR INFLAMMATORY INTESTINAL DISEASES CONTAINING AS THE ACTIVE INGREDIENT IL-6 ANTAGONISTS 141.(WO 99/47158) THERAPEUTIC CHEMOKINE RECEPTOR ANTAGONISTS 142.(WO 99/46376) RECEPTOR FROM THE SUPERFAMILY OF TNT-RECEPTORS FROM THE HUMAN LUNG 143.(WO 99/45927) VITRONECTIN RECEPTOR ANTAGONISTS 144.(WO 99/45905) PROPHYLAXIS AND TREATMENT OF MIGRAINE HEADACHES WITH THROMBOXANE SYNTHETASE INHIBITORS AND/OR RECEPTOR ANTAGONISTS 145.(WO 99/44612) SUBSTITUTED QUINAZOLINES AND ANALOGS AND THE USE THEREOF 146.(WO 99/43809) PROTEASE-ACTIVATED RECEPTOR 4 AND USES THEREOF 147.(WO 99/42464) SUBSTITUTED IMIDAZO[1,2-a;3,4-a']DIQUINOLINYLIUM INTERLEUKIN-8 RECEPTOR ANTAGONISTS 148.(WO 99/42463) SUBSTITUTED QUINOXALINE DERIVATIVES AS INTERLEUKIN-8 RECEPTOR ANTAGONISTS 149.(WO 99/42461) SUBSTITUTED QUINOXALINE DERIVATIVES AS INTERLEUKIN-8 RECEPTOR ANTAGONISTS 150.(WO 99/41257) GLUCOCORTICOID-SELECTIVE ANTIINFLAMMATORY AGENTS 151.(WO 99/41256) GLUCOCORTICOID-SELECTIVE ANTI-INFLAMMATORY AGENTS 152.(WO 99/40192) HUMAN RECEPTOR GPR14, AND A METHOD OF FINDING AGONIST AND ANTAGONIST TO HUMAN AND RAT GPR14 153.(WO 99/40091) BICYCLIC PYRIDINE AND PYRIMIDINE DERIVATIVES AS NEUROPEPTIDE Y RECEPTOR ANTAGONISTS 154.(WO 99/38532) METHODS FOR THE PREVENTION AND TREATMENT OF FIBROSIS AND SCLEROSIS 155.(WO 99/36541) INTERLEUKIN-1 RECEPTOR ANTAGONIST BETA (IL-1RA&bgr;) 156.(WO 99/33806) 4-[ARYL(PIPERIDIN-4-YL)] AMINOBENZAMIDES WHICH BIND TO THE DELTA-OPIOID RECEPTOR 157.(WO 99/31099) INTEGRIN RECEPTOR ANTAGONISTS 158.(WO 99/31061) INTEGRIN RECEPTOR ANTAGONISTS 159.(WO 99/30713) INTEGRIN RECEPTOR ANTAGONISTS 160.(WO 99/30709) INTEGRIN RECEPTOR ANTAGONISTS 161.(WO 99/29729) ANTAGONISTS OF NEUROPILIN RECEPTOR FUNCTIONAL AND USE THEREOF 162.(WO 99/27962) USE OF A FIBRINOGEN RECEPTORANTAGONIST FOR PREVENTING DISSEMINATED INTRAVASCULAR. COAGULATION 163.(WO 99/26945) 1,3,4-THIADIAZOLES AND 1,3,4-OXADIAZOLES AS &agr; v &bgr; 3 ANTAGONISTS 164.(WO 99/26943) THROMBIN RECEPTOR ANTAGONISTS 165.(WO 99/25857) TRANSGENIC MODELS OF INFLAMMATORY DISEASE 166.(WO 99/24471) OPIATE, CANNABINOID, AND ESTROGEN RECEPTORS 167.(WO 99/24423) PIPERIDINE DERIVATIVES AND THEIR USE AS TACHYKININ ANTAGONISTS 168.(WO 99/24421) IMIDAZOYLALKYL SUBSTITUTED WITH A FIVE, SIX OR SEVEN MEMBERED HETEROCYCLIC RING CONTAINING ONE NITROGEN ATOM 169.(WO 99/24406) PHENYL-ALKYL-IMIDAZOLES AS H3 RECEPTOR ANTAGONISTS 170.(WO 99/24405) H 3 RECEPTOR LIGANDS OF THE PHENYL-ALKYL-IMIDAZOLES TYPE 171.(WO 99/21555) ADENOSINE A3 RECEPTOR ANTAGONISTS 172.(WO 99/20758) HUMAN TUMOR NECROSIS FACTOR RECEPTOR-LIKE PROTEINS TR11, TR11SV1, AND TR11SV2 173.(WO 99/19462) ENHANCED IMMUNOGENIC CELL POPULATIONS PREPARED USING H2 RECEPTOR ANTAGONISTS 174.(WO 99/17773) COMPOUNDS AND METHODS 175.(WO 99/16465) METHOD FOR INHIBITING TUMOR ANGIOGENESIS IN A LIVING SUBJECT 176.(WO 99/11790) TUMOR NECROSIS FACTOR RECEPTOR ZTNFR-6 177.(WO 99/06049) INTEGRIN RECEPTOR ANTAGONISTS 178.(WO 99/04001) TUMOR NECROSIS FACTOR RECEPTOR ZTNFR-5 179.(WO 99/02499) QUINOLINE COMPOUNDS AND MEDICINAL USES THEREOF 180.(WO 99/01764) METHOD FOR RECOGNIZING AND DETERMINING GNRH RECEPTORS AND THE USE OF GNRH AGONISTS AND GNRH ANTAGONISTS AND OTHER GNRH RECEPTOR LIGANDS FOR THE TREATMENT WITH GNRH RECEPTORS OF TUMOURS ORIGINATING IN THE BRAIN AND/OR NERVOUS SYSTEM AND/OR MENINGES AND/OR OF KAPOSI SARCOMA 181.(WO 99/01444) POLYMORPHIC FORM OF THE TACHYKININ RECEPTOR ANTAGONIST 2-(R)-(1-(R) -(3,5-BIS(TRIFLUOROMETHYL) PHENYL)ETHOXY)-3-(S)-(4-FLUORO) PHENYL-4-(3-5 (-OXO-1H,4H-1,2,4,-TRIAZOLO) METHYLMORPHOLINE 182.(WO 99/01127) COMPOUNDS AND METHODS 183.(WO 99/00406) CYCLIC AGONISTS AND ANTAGONISTS OF C5a RECEPTORS AND G PROTEIN-COUPLED RECEPTORS 184.(WO 98/58674) ANTI-TUMOUR PHARMACEUTICAL COMPOSITIONS CAPABLE OF REDUCING DRUG RESISTANCE IN TUMOUR CELLS 185.(WO 98/57647) COUP-TFII: AN ORPHAN NUCLEAR RECEPTOR REQUIRED FOR ANGIOGENESIS 186.(WO 98/56892) HUMAN TUMOR NECROSIS FACTOR RECEPTOR TR9 187.(WO 98/56779) 4-SULFINYL BENZAMIDES AS CALCITONIN GENE-RELATED PEPTIDE RECEPTOR ANTAGONISTS 188.(WO 98/55153) NON-STEROIDAL RADIOLABELED AGONIST/ANTAGONIST COMPOUNDS AND THEIR USE IN PROSTATE CANCER IMAGING 189.(WO 98/54325) HUMAN FRP AND FRAGMENTS THEREOF INCLUDING METHODS FOR USING THEM 190.(WO 98/54202) HUMAN TUMOR NECROSIS FACTOR RECEPTOR TR10 191.(WO 98/54201) HUMAN TUMOR NECROSIS FACTOR RECEPTOR-LIKE PROTEIN 8 192.(WO 98/54187) SPIRO-AZACYCLIC DERIVATIVES AND THEIR USE AS THERAPEUTIC AGENTS 193.(WO 98/53069) GDNF RECEPTORS 194.(WO 98/49170) SPIRO-AZACYCLIC DERIVATIVES AND THEIR USE AS THERAPEUTIC AGENTS 195.(WO 98/48017) FAMILY OF IMMUNOREGULATORS DESIGNATED LEUKOCYTE IMMUNOGLOBULIN-LIKE RECEPTORS (LIR) 196.(WO 98/47923) IL-5R ANTAGONISTS FOR TREATMENT OF INFLAMMATION, ASTHMA AND OTHER ALLERGIC DISEASES 197.(WO 98/46751) OSTEOPROTEGERIN BINDING PROTEINS AND RECEPTORS 198.(WO 98/46620) A NOVEL HUMAN G-PROTEIN COUPLED RECEPTOR 199.(WO 98/46265) METHODS FOR USING ANTAGONISTIC ANTI-AVB3 INTEGRIN ANTIBODIES 200.(WO 98/43962 ) HETEROCYCLIC INTEGRIN INHIBITOR PRODRUGS 251.(WO 97/44333) 1,2,4-OXADIAZOLES AS ADHESION-RECEPTOR ANTAGONISTS 252.(WO 97/44329) DIARYLALKYL CYCLIC DIAMINE DERIVATIVES AS CHEMOKINE RECEPTOR ANTAGONISTS 253.(WO 97/41225) MAMMALIAN MIXED LYMPHOCYTE RECEPTORS, CHEMOKINE RECEPTORS [MMLR-CCR] 254.(WO 97/37655) &agr;v&bgr;3 ANTAGONISTS 255.(WO 97/35969) PEPTIDE LIGANDS OF THE UROKINASE RECEPTOR 256.(WO 97/34878) SUBSTITUTED 2,3-BENZODIAZEPIN-4-ONES AND THE USE THEREOF 257.(WO 97/33904) DEATH DOMAIN CONTAINING RECEPTORS 258.(WO 97/33887) SPIROCYCLE INTEGRIN INHIBITORS 259.(WO 97/33613) PARASITE-DERIVED ANTI-INFLAMMATORY IMMUNOMODULATORY PROTEIN 260.(WO 97/30991) NOVEL SUBSTITUTED N-METHYL-N-(4-(4-(1 H-BENZIMIDAZOL-2-YL)[1,4]DIAZEPAN-1-YL)-2-(ARYL)BUTYL)BENZAMIDES USEFUL FOR THE TREATMENT OF ALLERGIC DISEASES 261.(WO 97/30990) NOVEL SUBSTITUTED N-METHYL-N-(4-(PIPERIDIN-1-YL)-2-(ARYL)BUTYL)BENZAMIDES USEFUL FOR THE TREATMENT OF ALLERGIC DISEASES 262.(WO 97/30989) NOVEL SUBSTITUTED N-METHYL-N-(4-(4-(1H-BENZIMIDAZOL-2-YL-AMINO)P IPERIDIN-1-YL)-2-(ARYL)BUTYL)BENZAMIDES USEFUL FOR THE TREATMENT OF ALLERGIC DISEASE 263.(WO 97/30079) PEPTIDE ANTAGONISTS OF CELLULAR MITOGENESIS AND MOTOGENESIS AND THEIR THERAPEUTIC USE 264.(WO 97/30069) 17-BETA-CYCLOPROPYL(AMINO/OXY) 4-AZA STEROIDS AS ACTIVE INHIBITORS OF TESTOSTERONE 5-ALPHA-REDUCTASE AND C17-20-LYASE 265.(WO 97/29775) COMPOSITIONS COMPRISING A CYCLOOXYGENASE-2 INHIBITOR AND A LEUKOTRIENE B 4 RECEPTOR ANTAGONIST 266.(WO 97/29079) NOVEL COMPOUNDS AND PHARMACEUTICAL USE THEREOF 267.(WO 97/28190) CYTOKINE ANTAGONISTS AND AGONISTS 268.(WO 97/24373) MONOCLONAL ANTIBODY ANTAGONISTS TO HAEMOPOIETIC GROWTH FACTORS 269.(WO 97/23480) NOVEL INTEGRIN RECEPTOR ANTAGONISTS 270.(WO 97/22604) NOVEL SUBSTITUTED 4-(1H-BENZIMIDAZOL-2-YL)[1,4]DIAZEPANES USEFUL FOR THE TREATMENT OF ALLERGIC DISEASES 271.(WO 97/21732) DESIGN OF HORMONE-LIKE ANTIBODIES WITH AGONISTIC AND ANTAGONISTIC FUNCTIONS 272.(WO 97/21702) 3-BENZYLAMINOPYRROLIDINES AND -PIPERIDINES AS TACHYKININ RECEPTOR ANTAGONISTS 273.(WO 97/21445) VASCULAR IRRIGATION SOLUTION AND METHOD FOR INHIBITION OF PAIN, INFLAMMATION, SPASM AND RESTENOSIS 274.(WO 97/20062) IL-12 P40 SUBUNIT FUSION POLYPEPTIDES AND USES THEREOF 275.(WO 97/19074 ) SUBSTITUTED 4-(1H-BENZIMIDAZOL-2-YL-AMINO)PIPERIDINES USEFUL FOR THE TREATMENT OF ALLERGIC DISEASES 276.(WO 97/19059) NOVEL SUBSTITUTED ARYL COMPOUNDS USEFUL AS MODULATORS OF ACETYLCHOLINE RECEPTORS 277.(WO 97/16442) SUBSTITUTED PYRIDYL PYRROLES, COMPOSITIONS CONTAINING SUCH COMPOUNDS AND METHODS OF USE 278.(WO 97/16202) CYTOKINES AND THEIR USE IN TREATMENT AND/OR PROPHYLAXIS OF BREAST CANCER 279.(WO 97/16159) ENHANCED ANTI-INFLAMMATORY ORAL COMPOSITION CONTAINING H 2 RECEPTOR ANTAGONIST AND ANTIMICROBIAL OILS 280.(WO 97/15298) COMBINATION OF LTD, RECEPTOR ANTAGONISTS WITH GLUCOCORTICOSTEROIDS 281.(WO 97/14671) CYCLOPENTYL TACHYKININ RECEPTOR ANTAGONISTS 282.(WO 97/13751) INDOLE CARBAMATES AS LEUKOTRIENE ANTAGONISTS 283.(WO 97/13514) NK-1 RECEPTOR ANTAGONISTS FOR PREVENTION OF NEUROGENIC INFLAMMATION IN GENE THERAPY 284.(WO 97/09046) COMPOUNDS AND METHODS 285.(WO 97/07135) BINDING OF OSTEOGENIC PROTEIN-1 (OP-1) AND ANALOGS THEREOF TO THE CELL SURFACE RECEPTOR ALK-1 AND ANALOGS THEREOF)

With respect to clinical and pre-clinical trial development see Antibody Therapeutics Production, Clinical Trials, and Strategic Issues, By Rathin C. Das, Ph.D., M.B.A. & K. John Morrow, Jr., Ph.D., D&MD Publications October 2001, Chapter 6.

### Entity Associated and Entity Specific Markers

The term marker is used broadly to refer to any ligand or binding site for a "targeting" or an "effector" moiety of a multispecifc ligand or antibody of the invention and is primarily used herein to refer to ligands which are the target of a "targeting" moiety (most often though not exclusively referred to herein as a first ligand binding moiety).

The literature is replete with examples of such markers, as well as antibodies which recognize them. With respect to cancer markers and immune cell markers, etc. many of these are referred to in Cancer: Principles and Practice of Oncology 6th Ed. De Vita et al. Eds Lippincott 2001 and some are summarized at pages 309-311, 3197.

With respect to markers for osteoclasts and antibodies that bind thereto see for example Endocrinology 1989 Aug; 125(2):630-7; Endocrinology 1990 Dec: 127(6): 3215-21; Lab Invest Apr; 60(4):532-8; Calcif Tissue Int 1998 Aug; 63(2): 148-53. Such markers could be used for example to target RANK (associated with bone resorption etc.) on osteoclasts using a relatively low affinity second ligand binding moiety.

Other target applications of multispecific ligands of the invention include particularly receptors associated with angiogenesis (eg.VEGFRs 1,2,3) such as KDR, FLK-1 and FLT-1, and various cancers cell types eg. HER-2 and EGF-R, including FGF-R, PDGF-R, Tek and Tie2. Numerous other examples are referred to specifically and through references to the literature herein cited. Suitable markers for many types of target entities eg. cells bering such receptors are referred to or referenced herein or described in various subject reviews and texts herein cited, in connection with one or more aspects and embodiments of the invention described in this application, and many others are known to those skilled in the art and desribed in the literature including antibodies.

With respect to binding to biologic effector ligands the multispecific ligand may comprise a recombinatly produced receptor for such ligand.

As the invention contemplates that the multispecfic ligands herein may be used for cancer, it is contemplated that combination therapies with chemotherapeutic and biotherapeutic agents may be used to advantage. Such agents are well known to those skilled in the art and include for example, alkylating agents, cisplatin and its analogues, antimetabolites, topoisomerase interactive agents, antimicrotubule agents, interferons, interleukins, hormonal therapeutics, differentiation agents, antiangiogenesis agents (see Cancer: Principles and Practice of Oncology 6th Ed. De Vita et al. Eds Lippincott 2001 pp.335-517).

With respect to ligands involved in mediating apoptosis see also WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONISTS COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211. Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION WO0144808 METHODS OF DIAGNOSIS AND TREATMENT BY BINDING P75/AIRM1 WO0144282 BCL-G POLYPEPTIDES, ENCODING NUCLEIC ACIDS AND METHODS OF USE US6242569 Regulators of apoptosis EP1106183 Antibodies to erbB2 and their therapeutic uses WO0136594 Mcl-1 GENE REGULATORY ELEMENTS AND A PRO-APOPTOTIC Mcl-1 VARIANT US2001001712 Monoclonal antibodies having property of causing apoptosis WO0134798 CLONING AND CHARACTERIZATION OF VIRAL IAP ASSOCIATED FACTOR (VIAF) IN SEVERAL ORGANISMS CZ20000907 Monoclonal antibody inducing apoptosis HU0003513 MONOCLONAL ANTIBODY INDUCING APOPTOSIS EP1094316 Method for the detection of DNA replicating cells US6207452 Antibody of the anti-proliferation domain of human Bcl-2 WO0123568 NOVEL MEMBERS OF THE IAP GENE FAMILY US6190661 Methods and compositions for the use of apurinic/apyrimidinic endonucleases EP1087993 FAS PEPTIDES AND ANTIBODIES FOR MODULATING APOPTOSIS WO0119861 APO-2 RECEPTOR ANTIBODIES US6184034 Deoxyribonuclease II proteins and cDNAS US6172211 Nucleic acid encoding tag7 polypeptide WO0118042 APOPTOSIS PROTEINS WO0116180 CD40 LIGAND AND CD40 AGONIST COMPOSITIONS AND METHODS OF USE WO0116170 NOVEL CARD PROTEINS INVOLVED IN CELL DEATH REGULATION

As stated above, in a related but also independent aspect, the invention contemplates a method of screening for an antibody which preferentially binds to a ligand when bound to a first receptor relative to another second receptor by screening for antibodies (eg. by phage display, ribosome display, etc.) which bind to the ligand eg. a cytokine, when bound in situ to the first receptor, and selecting among them those that bind to the ligand eg. cytokine but do not bind (substractive screening) or bind with lesser affinity when bound to the cytokine to the second receptor, as well as to antibodies and multifunctional ligands created by this method (see also USP 6,046,048 and WO 99/12973 and references cited therein with respect to TNF family of receptors). Variations in the extracellular domains of such receptors are known and can be ascertained by methods known to those skilled in the art. Accordingly the invention is directed to an antibody characterized in that it binds to an epitope on the ligand which permits the ligand to bind, while the antibody is bound to it, to a first receptor but not a second receptor. In a preferred embodiment both are cell surface receptors. In a preferred embodiment the ligand is a natural ligand, preferably a growth factor, cytokine or chemokine. In another embodiment one of the receptors is a soluble receptor. The invention is also directed to a method of evaluating the pleitropic effects of a natural ligand by administering the said antibody Including antigen binding fragments thereof and MRUs) and monitoring its effects. The invention contemplates that this antibody is a first or second moiety of a multifunctional ligand disclosed herein. Examples of receptors include the classes of VEGF receptors (see also 1:Sheppard D.Integrin-mediated activation of transforming growth factor-beta(1) in pulmonary fibrosis.Chest. 2001 Jul;120(1 Suppl):S49-53. Chow D, Ho J, Nguyen Pham TL, Rose-John S, Garcia KC.In vitro reconstitution of recognition and activation complexes between interleukin-6 and gp130.Biochemistry. 2001. Jun 26;40(25):7593-603. Kotenko SV, Izotova LS, Mirochnitchenko OV, Esterova E, Dickensheets H, Donnelly RP, Pestka S.Identification, cloning, and characterization of a novel soluble receptor that binds IL-22 and neutralizes its activity.J Immunol. 2001 Jun 15;166(12):7096-103. Gustin SE, Church AP, Ford SC, Mann DA, Carr PD, Ollis DL, Young IG.Expression, crystallization and derivatization of the complete extracellular domain of the beta(c) subunit of the human IL-5, IL-3 and GM-CSF receptors.Eur J Biochem. 2001 May;268(10):2905-11. McCall AM, Shahied L, Amoroso AR, Horak EM, Simmons HH, Nielson U, Adams GP, Schier R, Marks JD, Weiner LM.Increasing the affinity for tumor antigen enhances bispecific antibody cytotoxicity.J Immunol. 2001 May 15;166(10):6112-7. Piehler J, Roisman LC, Schreiber G.New structural and functional aspects of the type I interferon-receptor interaction revealed by comprehensive mutational analysis of the binding interface.J Biol Chem. 2000 Dec 22;275(51):40425-33.DLINE]7: Wiesmann C, Muller YA, de Vos AM.Ligand-binding sites in Ig-like domains of receptor tyrosine kinases.J Mol Med. 2000;78(5):247-60. Review.DLINE]8: Born TL, Smith DE, Garka KE, Renshaw BR, Bertles JS, Sims JE., Protein, Nucleotide Identification and characterization of two members of a novel class of the interleukin-1 receptor (IL-1R) family. Delineation of a new class of IL-1R-related proteins based on signaling.J Biol Chem. 2000 Sep 29;275(39):29946-54.DLINE]9: Xia XZ, Treanor J, Senaldi G, Khare SD, Boone T, Kelley M, Theill LE, Colombero A, Solovyev I, Lee F, McCabe S, Elliott R, Miner K, Hawkins N, Guo J, Stolina M, Yu G, Wang J, Delaney J, Meng SY, Boyle WJ, Hsu H., Protein, Nucleotide TACI is a TRAF-interacting receptor for TALL-1, a tumor necrosis factor family member involved in B cell regulation.J Exp Med. 2000 Jul 3;192(1):137-43.DLINE]10: Kumaran J, Colamonici OR, Fish EN.Structure-function study of the extracellular domain of the human type I interferon receptor (IFNAR)-1 subunit.J Interferon Cytokine Res. 2000 May;20(5):479-85.DLINE]11: Lu D, Kussie P, Pytowski B, Persaud K, Bohlen P, Witte L, Zhu Z.Identification of the residues in the extracellular region of KDR important for interaction with vascular endothelial growth factor and neutralizing anti-KDR antibodies.J Biol Chem. 2000 May 12;275(19):14321-30.DLINE]12: Bowie A, O'Neill LA.The interleukin-1 receptor/Toll-like receptor superfamily: signal generators for pro-inflammatory interleukins and microbial products.J Leukoc Biol. 2000 Apr;67(4):508-14. Review.DLINE]13: Kumar S, McDonnell PC, Lehr R, Tierney L, Tzimas MN, Griswold DE, Capper EA, Tal-Singer R, Wells GI, Doyle ML, Young PR., Protein, Nucleotide, OMIM Identification and initial characterization of four novel members of the interleukin-1 family.J Biol Chem. 2000 Apr 7;275(14):10308-14.DLINE]14: Fujio K, Nosaka T, Kojima T, Kawashima T, Yahata T, Copeland NG, Gilbert DJ, Jenkins NA, Yamamoto K, Nishimura T, Kitamura T., Protein, Nucleotide Molecular cloning of a novel type 1 cytokine receptor similar to the common gamma chain.Blood. 2000 Apr 1;95(7):2204-10.DLINE]15: Touw IP, De Koning JP, Ward AC, Hermans MH.Signaling mechanisms of cytokine receptors and their perturbances in disease.Mol Cell Endocrinol. 2000 Feb 25;160(1-2):1-9. Review.DLINE]16: Chritton SL, Sheng M.CYRL, a novel cytokine receptor-like protein expressed in testis, lung, and spleen.Biochem Biophys Res Commun. 2000 Jan 27;267(3):697-702.DLINE]17: Li H, Chen J, Huang A, Stinson J, Heldens S, Foster J, Dowd P, Gurney AL, Wood WI. Free in PMC , , Protein, Nucleotide, OMIM Cloning and characterization of IL-17B and IL-17C, two new members of the IL-17 cytokine family.Proc Natl Acad Sci U S A. 2000 Jan 18;97(2):773-8.DLINE]18: Gary-Gouy H, Bruhns P, Schmitt C, Dalloul A, Daeron M, Bismuth G.The pseudo-immunoreceptor tyrosine-based activation motif of CD5 mediates its inhibitory action on B-cell receptor signaling.J Biol Chem. 2000 Jan 7;275(1):548-56.DLINE]19: Wiesmann C, Ultsch MH, Bass SH, de Vos AM., Protein, Structure Crystal structure of nerve growth factor in complex with the ligand-binding domain of the TrkA receptor.Nature. 1999 Sep 9;401(6749):184-8.DLINE]20: Liu Y, Cruikshank WW, O'Loughlin T, O'Reilly P, Center DM, Kornfeld H.Identification of a CD4 domain required for interleukin-16 binding and lymphocyte activation.J Biol Chem. 1999 Aug 13;274(33):23387-95.DLINE]21: Donnelly RP, Dickensheets H, Finbloom DS.The interleukin-10 signal transduction pathway and regulation of gene expression in mononuclear phagocytes.J Interferon Cytokine Res. 1999 Jun;19(6):563-73. Review.DUNE]22: Kim H, Baumann H.Dual signaling role of the protein tyrosine phosphatase SHP-2 in regulating expression of acute-phase plasma proteins by interleukin-6 cytokine receptors in hepatic cells.Mol Cell Biol. 1999 Aug;19(8):5326-38.DLINE]23: Kemebeck T, Pflanz S, Muller-Newen G, Kurapkat G, Scheek RM, Dijkstra K, Heinrich PC, Wollmer A, Grzesiek S, Grotzinger J., Protein, Structure The signal transducer gp130: solution structure of the carboxy-terminal domain of the cytokine receptor homology region.Protein Sci. 1999 Jan;8(1):5-12.DLINE]24: Ernst M, Novak U, Nicholson SE, Layton JE, Dunn AR.The carboxyl-terminal domains of gp130-related cytokine receptors are necessary for suppressing embryonic stem cell differentiation. Involvement of STAT3.J Biol Chem. 1999 Apr 2;274(14):972 Hibi M, Hirano T.Signal transduction through cytokine receptors.Int Rev Immunol. 1998;17(1-4):75-102. Review.26: Staunton D, Hudson KR, Heath JK.The interactions of the cytokine-binding homology region and immunoglobulin-like domains of gp130 with oncostatin M: implications for receptor complex formation.Protein Eng. 1998 Nov;11(11):1093-102).

With respect to markers that are useful for differentiating between various populations and sub-populations of cells, see also Human IL-18 Receptor and ST2L Are Stable and Selective Markers for the Respective Type 1 and Type 2 Circulating Lymphocytes, Woon Ling Chan, Nada Pejnovic, Christine A. Lee, and Nadia A. Al-Ali, J Immunol 2001;167 1238-1244; CD4+CD25 high Regulatory Cells in Human Peripheral Blood, Clare Baecher-Allan, Julia A. Brown, Gordon J. Freeman, and David A. Hafler, J Immunol 2001;1671245-1253.

In the preceding detailed description, reference was made to various methodologies known to those of skill in the art of molecular biology and immunology. Publications and other materials setting forth such known methodologies to which reference was made or is made below are incorporated herein by reference in their entireties along with references cited therein as though set forth in full .

Standard reference works setting forth the general principles of recombinant DNA technology include Watson, J. D. et al, Molecular Biology of the Gene, Volumes I and II, the Benjamin/Cummings Publishing Company, Inc., publisher, Menlo Park, Calif. (1987), Darnell, J. E. et al., Molecular Cell Biology, Scientific American Books, Inc., Publisher, New York, N.Y. (1986); Lewin, B. M. Genes II, John Wiley & Sons, publishers, New York, N.Y. (1985); Old, R. W., et al., Principles of Gene Manipulation: An Introduction to Genetic Engineering, 2d edition, University of California Press, publisher, Berkeley, Calif. (1981); Maniatis, T., et al., Molecular Cloning: A Laboratory Manual, 2nd Ed. Cold Spring Harbor Laboratory, publisher, Cold Spring Harbor, N.Y. (1989), and Current Protocols in Molecular Biology, Ausubel et al., Wiley Press, New York, N.Y. (1989). Standard reference works setting forth general principles and techniques of immunology include Handbook of Experimental Immunology Blackwell Science, Incorporated, ISBN:0632009756; Antibody Engineering Blackwell Science, Incorporated, ISBN:0632009756; Therapeutic Immunology ISBN: 086542375X Blackwell Science, Incorporated ; Encyclopedia of Immunology (1998) Morgan Kaufmann Publishers, ISBN:0122267656; Immunology Mosby, Incorporated, ISBN:0723429189; Abbas AK. et al. Cellular & Molecular Immunology 4th Ed. 2000 ISBN 0721650023; Breitling F. et al. Recombinant Antibodies 1999 ISBN 0-471-17847-0; Masseyeff R. et al. Methods of Immunological Analysis Wiley-VCH ISBN 3-527-27906-7, 1992; Mountain et at Eds, Biotechnology 2nd ed. Vol 5A 1998 ISBN 3-527-28315-3 Wiley-VCH; Campbell, A. , "Monoclonal Antibody Technology," in, Burdon, R., et al., eds, Laboratory Techniques in Biochemistry and Molecular Biology, Volume 13, Elsevier, Publisher, Amsterdam (1984);

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All publications referred to herein are indicative of the level of skill of those in the art to which the invention pertains. All publications are herein (as well as references cited therein)are incorporated by reference to the same extent as if each individual publications were specifically and individdually indicated to be incorporated by reference in its entirety.

The present invention, thus generally described, will be understood more readily by reference to the preceding and following examples, which are provided by way of illustration and are not intended to be limiting of the present invention.

In another aspect the invention is directed to multifunctional ligand comprising at least a first moiety which specifically binds to a ligand on the surface of a virus particle that is capable of infecting a mammalian and particularly a human cell including a cancer cell (excluding viruses which are known for use in gene therapy) and is preferably selected from the group consisting viruses which infect substantial populations of individuals including for example influenza virus and at least a second moiety which specifically recognizes a cancer cell, in one embodiment preferably a marker present on multiple different cancer types, especially cancer types that are individually or collectively most prevalent in the general population. In one embodiment such multifunctional ligand is a bispecific, trispecific or tetraspecific antibody. The invention contemplates that such a multifunctional ligand may be used to target such viruses to tumors in a manner which preferentially kills the cancer cells either through the action of the virus and/or by causing the immune response to the virus or virus infected cell to preferentially (relative to non-cancer cells) target the cancer cell for ablation. The invention is also directed to a method of treating cancer by retargeting virus with which an idividual is otherwise infected to the cancer cell eg. influenza. The invention also contemplates that the multifunctional ligand includes one or more effector moieties which assist in killing the virus and/or cancer cell or directing immune cells to the virus and/or cancer cell, if and when present in the individual, for example a moiety which specifically binds to such immune cell eg. a T cell, as discussed above. Accordingly, the invention also contemplates that such multifunctional ligand may be used to treat influenza virus infections and secondarily to act prophylactically as a sentinel against any cancer cells which might develop during the course of the viral infection or a period of immune suppression or increased succeptibility to infection or cancer, including for example, as experienced by individuals with a particular immune suppressive disorder or condition or under treatment with immune suppressive drugs, individuals at risk for cancer or recurrence of a cancer, individuals of a particular age group, individuals experiencing a period of unusual stress which increases their succeptibility to disease or infection. The invention also contemplates that such a moiety is used in concert with prior immunization against the virus, so that the augemented immune response to the virus benefits the treatment of the cancer cells (see for example USP 6169175 and the art cited therein). The invention also directed to such a virus (excluding viruses known for use in gene therapy applications eg. adenovirus) which is engineered to expresses on its surface a cancer targeting moiety such as a scFv (see for example EP 1038967, WO 94/10323 and the art cited therein). The invention is also directed to a method of identifying the expression or over-expression of cell surface markers associated with infection by such a virus, by substractive screening relative to markers also expressed on non-infected such cells, for example using phage display or the like. Such markers may be used for vaccine-type or other immunotherapeutic strategies. Anti-virus markers including influenza virus markers and methods of identifying new such markers are well known in the art (see for example USP 5589174) (see also The role of the antibody response in influenza virus infection., Gerhard W.,Curr Top Microbiol Immunol 2001;260:171-90, Femandez-Sesma A, Schulman JL, Moran TM.A bispecific antibody recognizing influenza A virus M2 protein redirects effector cells to inhibit virus replication in vitro.J Virol. 1996 Jul;70(7):4800-4; Todorovska A, Roovers RC, Dolezal O, Kortt AA, Hoogenboom HR, Hudson PJ. Design and application of diabodies, triabodies and tetrabodies for cancer targeting. J Immunol Methods. 2001 Feb 1;248(1-2):47-66., Staerz UD, Yewdell JW, Bevan MJ. Hybrid antibody-mediated lysis of virus-infected cells. Eur J Immunol. 1987 Apr;17(4):571-4; Fernandez-Sesma A, Schulman JL, Moran TM.A bispecific antibody recognizing influenza A virus M2 protein redirects effector cells to inhibit virus replication in vitro. J Virol. 1996 Jul;70(7):4800-4.)

The invention is also directed to a multifunctional ligand having at least a tumor cell targeting moiety and a moiety which binds to a tumor antigen which is shed from a cancer cell. In a preferred embodiment, the tumor antigen binding moiety preferably does not recognize the portion of the antigen which is most immunogenic and leaves that portion exposed for recognition by the immune system. The invention contemplates generating such preferred antibody or fragment thereof by using an an immune complex between an antibody that binds to such immunogenic portion and the antigen as a target for phage display or generation or polyclonal sera. The invention also contemplates identifying antibodies which recognize immunogenic portions of the antigen by screening patient sera for antibodies which recognize the antigen. The invention also contemplates that such multifunctional ligand includes one or more effector moieties which assist in killing the cancer cell or directing immune cells to the cancer cell, for example a moiety which specifically binds to such immune cell eg. a T cell receptor, as discussed above.

Without limiting the generality of or applicability of the foregoing, and without being limited by or limiting the scope of the claims, various embodiments of the invention may be summarized for ease of reference as follows: 1. A multispecific ligand comprising at least a first ligand binding moiety which preferentially binds with a first affinity⁵ to a first ligand having a first biodistribution* and at least a second ligand binding moiety which preferentially binds with a second affinity to a second ligand having a second biodistribution which is different⁶ from that of the first ligand, and wherein the affinity of first and second ligand binding moieties are selected to bias the biodistribution of the multispecific ligand. 2. A multispecific ligand according to paragraph 1, wherein said multifunctional ligand comprises one or more ligand binding moieties which are antibodies. 3. A multispecfic ligand according to paragraph 1 or 2, wherein the affinity of said first ligand binding moiety for the first ligand is higher than the affinity of the second ligand binding moiety for the second ligand so as to bias the biodistribution of the multispecific ligand in favor of the the first ligand. 4. A multispecific ligand according to paragraph 3, wherein the first and second ligands have overlapping biodistributions.⁷ 5. A multispecfic
⁵ Contrasted to functional affinity which may result from avidity
⁶see fn 8
⁷The term epitope though technically understood to be specific for a given antibody, is used to refer to antigenic determinants that are situated proximally to one another so that two antibodies will be considered to bind to the same epitope if one competively inhibits the binding of the other through any probative competitive inhibition experiment known to those skilled in the art.
ligand according to paragraph 4, wherein the first ligand is a target cell population associated ligand and wherein said second ligand is present on a broader population of cells and wherein the biodistribution of the multispecific ligand is skewed in favour of the target cell population. 6. A multispecfic ligand according to paragraph 1 or 5, wherein said first ligand is a marker associated with* one or more specific cell populations, infectious or parasitic agents, diseased cells, or disease associated* cells, optionally one of specific ligands herein mentioned or referenced or known to those skilled in the art. 7. A multispecific ligand according to paragraph 6, wherein said marker is a specific biological structure. 8. A multispecific ligand according to paragraph 6, wherein said marker is a specific receptor or receptor ligand. 9. A multispecific ligand according to paragraph 6, wherein said marker is a specific antigen. 10. A multispecific ligand according to paragraph 6, wherein said marker is a specific epitope. 11. A multispecific ligand according to paragraph 6, wherein said marker is a CD marker. 12. A multispecific ligand according to paragraph 6, wherein said marker is associated with a cancer cell or pre-cancerous cell. 13. A multispecific ligand according to paragraph 6, wherein said marker is associated with an autoimmune disorder or rheumatic disease. 14. A multispecific ligand according to paragraph 6, wherein said marker is associated with a specific tissue type. 15. A multispecific ligand according to paragraph 6, wherein said marker is associated with a specific organ. 16. A multispecific ligand according to paragraph 6, wherein said marker is associated with a cell or tissue of specific origin or class. 17. A multispecific ligand according to paragraph 6, wherein said marker is an MHC-peptide complex. 18. A multispecific ligand according to paragraph 6, wherein said marker is associated with a cell surface immunoglobulin. 19. A multispecific ligand according to paragraph 5 or 6, wherein said second ligand is a receptor, family of receptors or one or more particular receptor family members, optionally one of those specific receptors herein mentioned or referenced or known to those skilled in the art. The invention contemplates targeting any receptor present on any population of entities for which there is an entity associated marker. 20. A multispecific ligand according to paragraph 19, wherein said second ligand is a cell surface receptor chosen from a group comprising tyrosine kinase type receptors, serine kinase type receptors, heterotrimeric G-protein coupled receptors, receptors bound to tyrosine kinase, TNF family receptors, notch family receptors, guanylate cyclase types, tyrosine phosphatase types, decoy receptors, and adhesion receptors, optionally one of the specific receptors herein mentioned or referenced or known to those skilled in the art. 21. A multispecific ligand according to paragraph 19, wherein said receptor requires cross-linking for biological activity. 22. A multispecific ligand according to paragraph 5 or 6, wherein said second ligand is a cell surface receptor and wherein said second ligand binding moiety blocks said receptor. 23. A multispecific ligand according to paragraph 1, 5 or 6, wherein said second ligand is a receptor ligand and wherein said second ligand binding moiety blocks interaction with the corresponding receptor. 24. A multispecific ligand according to paragraph 5 or 6, wherein said second ligand is a cell surface receptor which initiates a signal transduction and wherein said second ligand binding moiety effects a signal transduction. 25. A multispecific ligand according to paragraph 5, 6 or 19, wherein said antibody comprises a first VH* which preferentially recognizes said first ligand and a second VH which preferentially recognizes said second ligand. 26. A multispecific ligand according to paragraph 25, wherein at least one of said first and second VHs require the cooperation of a VL for binding to their respective ligands. 27. A multispecific ligand according to paragraph 25, comprising a first VL associated with said first VH and a second VL associated with said second VH and wherein both said first and second VHs require the cooperation of a VL for binding to the first and second ligands, respectively, and wherein said first and second VLs are the same⁸ or functionally interchangeable*. 28. A multispecific ligand according to paragraph 25 or 27, wherein said bispecific antibody is a four chain antibody. 29. A multispecific ligand according to paragraph 28, wherein said bispecfic antibody is a minibody, F(ab')₂ or antibody devoid of a CH3 domain. 30. A multispecific ligand according to paragraph 25, wherein said bispecific antibody is a diabody. 31. A multispecific ligand according to paragraph 25 or 27, wherein said bispecific antibody is devoid of light chains. 32. A multispecific ligand according to paragraph 31, wherein said bispecific antibody comprises a pair of disulfide linked heavy chains or heavy chain portions each comprising at least a VH region, a hinge region and preferably, at least a portion of an Fc region at the carboxy terminus of the hinge region. 33. A multispecific ligand according to paragraph 31, wherein said bispecific antibody comprises a pair of VHs linked via a polypeptide linker. 34. A multispecific ligand according to paragraph 3, 5, 6 or 19 wherein the affinity of the first ligand binding moiety for the first ligand is at least approximately⁹, one, two,
⁸have substantially the same amino acid composition ie. with possible exception of one or more additions, deletions or substitutions including conservative amino acid substitutions which do not substantially affect the specificity and amino acid composition of the paratope
⁹ the term approximately in the context of orders of magnitude variations in affinity refers a variability that is up to a half an order or magnitude.
three, four, five, six, seven or eight orders of magnitude greater than the affinity of said second ligand binding moiety for the second ligand. 35. A composition comprising a multispecific ligand in a pre-determined dosage, said mulfispecific ligand comprising a first ligand binding moiety which preferentially binds with a pre-selected first affinity to a first ligand having a first biodistribution and a second ligand binding moiety which preferentially binds with a pre-selected affinity to a second ligand having a second biodistribution, which is different from that of the first ligand, and wherein the affinity of the first and second ligand binding moieties are selected to bias the biodistribution of the multispecific ligand in favor of a selected location of one or both of the ligands¹⁰ such that a desired proportion of the dosage is delivered to the selected location. 36. A multispecific ligand according to paragraph 1 or 35, wherein the biodistributions of said first and second ligands overlap" and wherein the affinities of the first and second ligand binding moieties are selected to bias the biodistribution of the multispecfic ligand in favour of a target cell population on which both first and second ligands are bioavailable for recognition by the first and second ligand binding moieties, relative to one or more non-target cell populations. 37. A multispecific ligand according to paragraph 36, wherein the affinity of first ligand binding moiety for the first ligand is at least, approximately, one, two, three, four, five, six, seven or eight orders of magnitude greater than the affinity of the second ligand binding moiety for the second ligand. 38. A multispecific ligand according to paragraph 36 or 37, wherein first and second ligands are bioavailable for contemporanous* recognition by the first and second ligand binding moieties. 39. A method of controlling the biodistribution of a ligand which interacts with a target ligand present on a heterogenous population of ligand bearing entities, said method comprising using a multispecific ligand comprising at least a first ligand binding moiety which preferentially* binds with a pre-selected*
¹⁰ having regard to their respective bioavailabilities
¹¹ The term "overlap" connotes that notwithstanding the difference in distributions of the first and second ligands the first and second ligands are bioavailable for recognition on the same entity. This term and related terms, exemplified below, are intended to exclude a situation where both ligands are preferentially expressed on substantially the same entity, for example two different tumor associated antigens associated differentially with a differentiated population of cells within a tumor, most particularly in the case where they are individually suitable targets for delivery of a toxic payload, and the terms "different" distributions and "heterogeneous" population are similarly understood to exclude such a common distribution, in the appreciation that the invention primarily represents an improved strategy for targeting two different ligands, in which one ligand has a broader distribution than the other or both have distributions that may overlap but are different from that of the target population. It will also be appreciated that the invention has particular application to a situation in which at least one of the non-target populations is one on which one of said first and second ligands is substantially represented (in contrast to one on which it simply enjoys limited expression).
first affinity¹² to at least a first ligand associated with a target sub-population of said heterogeneous population on which said first ligand and target (second) ligand are bioavaible for contemporaneous recognition and a second ligand binding moiety which preferentially binds with a pre-selected lesser affinity to said target ligand, and wherein the affinity of first and second ligand binding moieties are selected to bias the biodistribution of the multispecific ligand in favor of said target sub-population of ligand bearing entities. 40. A method of testing the biological effects of limiting the biodistribution of a ligand which interacts with a target ligand present on a heterogenous population of ligand bearing entities, said method comprising the step of administering a multispecific ligand comprising at least a first ligand binding moiety which preferentially* binds with a pre-selected* first affinity¹³ to at least a first ligand associated with a target sub-population of said population of ligand bearing entities on which said first ligand and target (second) ligand are bioavaible for contemporanous recognition and a second ligand binding moiety which preferentially binds with a pre-selected lesser affinity to said target ligand, and wherein the affinity of first and second ligand binding moieties are selected to bias the biodistribution of the multispecific ligand in favor of said target sub-population of ligand bearing entities. 41. A multispecific ligand which preferentially binds to a target ligand on a selected sub-population of a heterogeneous population of cells bearing the target-ligand, the multispecific ligand comprising a first ligand binding moiety which preferentially binds to a cell sub-population associated ligand and a second ligand binding moiety which binds to the target-ligand, said first ligand binding moiety having an affinity for the sub-population associated ligand that is higher than the affinity of the second ligand binding moiety for the target ligand. 42. A multispecific ligand according to paragraph 41, wherein the affinity of said first ligand binding moiety for the cell sub-population associated ligand is approximately, one, two, three, four, five, six, seven or eight orders of magnitude greater than the affinity of said second ligand binding moiety for said target ligand. 43. A multispecfic ligand according to paragraph 42, wherein said target ligand is a receptor or a receptor ligand¹⁴. 44. A multispecific ligand according to paragraph 42, wherein at least one of said first or second ligand binding moieties comprises an antibody heavy chain or functional portion(s) thereof including a VH or fragment thereof and an antibody light chain or functional
¹² Contrasted to functional affinity which may result from avidity
¹³ Contrasted to functional affinity which may result from avidity
portion(s) thereof including a VH or fragment thereof. 45. A method of selectively exerting a biological effect mediated through binding a target-ligand on a selected sub-population of a population of cells bearing the target-ligand, the method comprising the step of exposing the cells to a multispecific ligand comprising a first ligand binding moiety which preferentially binds to a cell sub-population associated ligand and a second ligand binding moiety which binds to the target ligand, said first ligand moiety having an affinity for the sub-population associated ligand that is higher than the affinity of the second ligand binding moiety for the target ligand. 46. A method according to paragraph 45, wherein the affinity of said first ligand binding moiety for the cell sub-population associated ligand is at least approximately, one, two, three, four, five, six, seven or eight orders of magnitude greater than the affinity of said second ligand binding moiety for said target ligand. 47. A method according to paragraph 45, wherein said target ligand is a receptor or a receptor ligand. 48. A method according to paragraph 45 wherein at least one of said first or second ligand binding moieties is an antigen binding fragment¹⁵ of an antibody. 49. A method of testing or controlling the biological effects of a ligand binding molecule by circumscribing its ability to bind to a diverse population of cells bearing a complementary target ligand, said method comprising using said ligand binding molecule together with a different ligand binding molecule which preferentially binds to another target ligand which is exclusively or preferentially associated with one or more sub-population(s) of said population of cells, and wherein the biological effects of said ligand binding molecule are controlled through prior association of said ligand binding molecule with said other ligand binding molecule to form a multispecific ligand and through the affinity of at least one of said ligand binding molecules being pre-selected to limit the propensity of said ligand binding molecule to bind to cells within said population of cells which do not preferentially express said other target ligand. 50. A method according to paragraph 49, wherein the affinity of said ligand binding molecule for said complementary target ligand is less than the affinity of the other ligand binding molecule for the other target ligand. 51. A method according to paragraph 49 or 50, wherein at least one of said first and second ligand binding molecules is an antibody*. 52. A method according to paragraph 49 wherein said first ligand binding molecule is an entity which exerts a
¹⁴The term "receptor ligand" means a target ligand which is a ligand for a receptor, for example, a receptor on a cell or infectious agent or a receptor which circulates independently of another entity.
¹⁵ The term "antigen binding fragment" refers to a polypeptide or a plurality of associated polypeptides comprising one or more portions of an antibody including at least one VH or VL or a functional fragment thereof.
biologic effect and said second ligand binding molecule is a multispecific ligand comprising a first ligand binding moiety that binds to a cell sub-population. associated ligand and a second ligand binding moiety which binds to said entity. 53. A method according to paragraph 52 wherein said second ligand binging moiety is an antibody which binds to a pre-selected epitope on said entity and wherein the epitope of said second ligand binding moiety is selected on the basis of its proximity to a ligand binding portion of said entity such that the entity when bound to said multifunctional ligand has an affinity for said ligand which is less than the affinity of said first ligand binding moiety for said cell sub-population associated ligand. 54. A multispecific ligand according to paragraph 12, wherein said second ligand is a IL-8 receptor, a CCR7 receptor, a FAS receptor, or a CXCR4 receptor. 55. A multispecific ligand according to paragraph 6, wherein said marker is associated with an immune cell that is succeptible to viral infection. 56. A multispecific ligand according to paragraph 55, wherein said marker is CD4. 57. A multispecific ligand according to paragraph 55 or 56, wherein said second ligand is a CCR5 or CXCR4 receptor. 58. A multifunctional ligand according to paragraph 52, wherein said entity is a biologic effector ligand. 59. A multispecific ligand according to paragraph 36, wherein the affinities of said first and second ligand binding moieties are both selected to limit their individual ability to bind to the first and second ligands, respectively, and wherein their combined functional affinity biases the distribution of the multifunctional in favour of said target cell population. 60. An antibody which binds to an epitope on an entity which exerts a biologic effect via a binding interaction with a target ligand, said epitope being proximal to the binding site of said entity for the target ligand, such that the antibody bound to the entity reduces the affinity of the entity for its ligand without precluding its functional binding activity vis-à-vis said ligand. 61. A multispecific ligand comprising a first ligand binding moiety which binds with a pre-selected affinity to a target entity associated ligand and a second ligand binding moiety which binds with pre-selected affinity to an epitope on a biologic effector ligand which exerts a biologic effect via a binding interaction with a target ligand, said epitope being proximal to the binding site of said biologic effector ligand for the target ligand, such that the second ligand moiety bound to the biologic effector ligand reduces the affinity of the molecule for the target ligand without precluding its functional binding activity vis-à-vis said ligand and wherein said target ligand is present on a diverse population of entities consisting of the target entity and one or more non-target entities and wherein the affinity of the first ligand binding moiety for the target entity associated ligand is greater than that of the biologic effoctor ligand for the target ligand when bound to second ligand molecule, and wherein the affinity of the first ligand binding moiety is selected to bias the biodistribution of said biologic effector ligand in favour of the target entity relative to the non-target entit(ies). 62. A multispecific ligand according to paragraph 61, wherein first ligand binding moiety comprises a light chain linked to an antibody heavy chain portion comprising at least a VH, CH1 domain, hinge region and preferably at least a truncated Fc portion and said second ligand binding moiety comprises at least a VL linked to a heavy chain portion, optionally through a disulfidebond¹⁶ and wherein the heavy chain portion of said second ligand binding moiety is devoid of CH1 domain, and comprises a hinge region and preferably at least a truncated Fc portion, and wherein said heavy chain portions are linked via their respective hinge regions and optionally wherein the respective hinge regions are wholly or partially substituted or supplemented by a another linkage pair¹⁷ eg. a leucine zipper. 63. A multispecfic ligand according to paragraph 12, wherein said second ligand is a marker associated with a lymphatic endothelial cell. 64. A multispecific ligand comprising a first ligand binding moiety which preferentially binds to a lymphatic endothelial cell associated marker and a second moiety which exerts a biologic, function¹⁸, optionally a therapeutic function, optionally an immune function*, optionally at least one of an immunizing¹⁹ function, a tolerizing function, a neutralizing²⁰ function, an immune mediating function, and immune modulating function²¹, in relation* to an independent ²²entity, preferably within the lymphatic system. 65. A multispecific ligand according to paragraph 62, wherein the marker is selected to limit the ability of said endothelial cell to internalize said multispecific ligand. 66. A multifunctional ligand having, at least, a first portion which binds to a lymphatic vessel associated
¹⁶ For example of the disulfide stabilized type developed by the NCI
¹⁷ eg. fos-jun
¹⁸ The moiety that exerts a biologic function is understood to be a biologic effector in the sense that its intended interaction with an entity in the lymphatic system or elsewhere in the organism has a biological consequence.
¹⁹ For example using a toxin or immunogen fused or conjugated to (or having a corresponding ligand on the second binding moiety to which it binds) to an antibody which recognizes a lymphatic endothelial marker, for example an anthrax toxin fusion
²⁰ The tern neutralizing is used broadly to refer to any interposition, interference or impediment which affects the function of the target entity
²¹ the terms modulating, mediating, neutralizing function etc. are not intended to be mutully exclusive and are each used broadly, for example the term modulating referring to effecting a change, and the term mediating preferably connoting an indirect effect achieved through the instrumentality of another entity, for example a cell, cytokine, chemokine etc..
²² Ie. an entity other than the lymphatic endothelial cell and other than any cell to which the first moiety is anchored.
ligand and a second portion comprising an immune function exerting moiety. 67. A multifunctional ligand as defined in paragraph 66, wherein said first portion is an antibody. 68. A multifunctional ligand as defined in paragraph 66, wherein said immune function exerting moiety binds to a target ligand. 69. A multifunctional ligand as defined in paragraph 67, wherein said immune function exerting moiety comprises an antibody. 70. A multifunctional ligand as defined in paragraph 68, wherein said immune function exerting moiety comprises an antibody. 71. A multifunctional ligand as defined in paragraph 68, wherein said immune function exerting moiety binds to a ligand selected form the group consisting of CCR5, CTLA-4, LFA-1, ICAM-1, CD2, CD3, CD4, CD22, CD40, CD44; CD80, CD86, CD134 and CD154. 72. A multifunctional ligand as defined in paragraph 70, wherein said first portion binds to LYVE-1 or podoplantin. 73. A multifunctional ligand as defined in paragraph 70, wherein said immune function exerting moiety comprises an anti-idiotypic antibody. 74. A multifunctional ligand as defined in paragraph 73, wherein said anti-idiotypic antibody binds to an autoimmune antibody. 75. A multifunctional ligand as defined in paragraph 73, wherein said anti-idiotypic antibody mimics a cell surface expressed tumor antigen or a viral antigen. 76. A multifunctional ligand as defined in paragraph 70, wherein said immune function exerting moiety binds to a diseased cell. 77. A multifunctional ligand as defined in paragraph 70, wherein said immune function exerting moiety binds to an infectious agent or parasite. 78. A multifunctional ligand as defined in paragraph 76, wherein said diseased cell is a cancer cell. 79. A multifunctional ligand as defined in paragraph 76, wherein said diseased cell is a virally infected cell. 80. A multifunctional ligand as defined in paragraph 68, wherein said immune function exerting moiety binds to an immune cell. 81. A multifunctional ligand as defined in paragraph 69, 76 or 80 wherein said immune function exerting moiety binds with greater functional affinity to its target ligand than said first portion binds to its target ligand. 82. A multifunctional ligand as defined in paragraph 69, 76 or 80 wherein said immune function exerting moiety binds with greater affinity to its target ligand than said first portion binds to its target ligand. 83. A multifunctional ligand as defined in paragraph 69, 76 or 80, wherein said binds with greater avidity to its target ligand than said first portion binds to its target ligand. 84. A multifunctional ligand as defined in paragraph 80, wherein immune cell is associated with an autoimmune reaction. 85. A multifunctional ligand as defined in paragraph 80, wherein said immune cell is a CCR5-expressing cell. 86. A multifunctional ligand as defined in paragraph 78 or 80, wherein said second portion comprises an internalizing antibody and a cytotoxic component. 87. A multifunctional ligand as defined in paragraph 78 or 80, which is a bispecific antibody having a monovalent first portion and a monovalent second portion. 88. A multifunctional ligand as defined in paragraph 78 or 80, which is a bispecific antibody having a divalent first portion and a divalent second portion. 89. A multifunctional ligand as defined in paragraph 78 or 80, which is a trispecific antibody having a monovalent first portion and a second portion comprising a divalent immune function exerting moiety which binds to one or more target ligands on a target diseased cell or immune cell and a movovalent anti-CD3 or anti-CD28 antibody. 90. A multifunctional ligand as defined in paragraph 78 or 80, which is a trivalent trispecific antibody having a monovalent first portion and a second portion comprising a divalent immune function exerting moiety which binds to a target ligand on a target diseased or immune cell. 91. A multifunctional ligand as defined in paragraph 78, wherein said second portion comprises a cytokine component. 92. A multifunctional ligand as defined in paragraph 78, wherein said second portion comprises a cytotoxic component. 93. A multifunctional ligand as defined in paragraph 78, wherein said second portion comprises a ligand which is capable of binding to T cells. 94. A multifunctional ligand as defined in paragraph 87, wherein said ligand is an antibody which binds to T cells. 95. A multifunctional ligand as defined in paragraph 78, wherein said second portion comprises an anti-CD3 antibody or anti-CD28 antibody. 96. A multifunctional ligand as defined in paragraph 67, wherein second portion is a cytokine component. 97. A multifunctional ligand as defined in paragraph 67, wherein second portion is an anti-CD3 antibody or an anti-CD28 antibody. 98. A multifunctional ligand as defined in paragraph 13, wherein said second portion further comprises one or more components selected from the group consisting of a cytokine component, a cytotoxic component and an anti-CD3/CD28 component. 99. A multifunctional ligand as defined in paragraph 14, wherein said second portion further comprises one or more components selected from the group consisting of a cytokine component, a cytotoxic component and an anti-CD3/CD28 component. 100. A pharmaceutical composition comprising a multifunctional ligand as defined in paragraph 101. A pharmaceutical composition comprising a plurality of different multifunctional ligands. 102. A pharmaceutical composition as defined in paragraph 101, wherein said plurality of different multifunctional ligands exert a cooperative immune effect. 103. A pharmaceutical composition as defined in paragraph 101, wherein said plurality of different multifunctional ligands comprise a multifunctional ligand as described in paragraph 76 and at least one or both of the multifunctional ligands described in paragraph 95 or 96. 104. A method of inhibiting the formation of metastasis during the course of surgical removal of a tumor comprising administering to a patient prior to surgical treatment of the tumor site, a pharmacetical composition comprising a multifunctional ligand as described in paragraph 78. 105. An immunocytokine comprising an anti-idiotypic antibody which recognizes the paratope of an antibody which binds to a lymphatic vessel associated ligand and a cytokine fused therewith or conjugated thereto. 106. An immunocytokine as defined in paragraph 105, wherein said cytokine component comprises IL-2 or a functional fragment thereof and/or IL-12 or a functional fragment thereof. 107. An immunocytokine as defined in paragraph 42, wherein said cytokine component comprises TNF-α or a functional fragment thereof. 108. A bispecific antibody comprising an anti-idiotypic antibody which recognizes the paratope of an antibody which binds specifically to a lymphatic vessel associated ligand and an anti-CD3 antibody or an anti-CD28 antibody. 109. A multifunctional ligand according to paragraph 66 comprising one or more amino acids that are substituted for amino acids that contribute to an immunogenic epitope. 110. A multifunctional ligand having, at least, a first portion which binds to a lymphatic vessel associated ligand and a second portion comprising an independent therapeutic function exerting moiety. 111. A bispecific ligand comprising a first ligand which binds to a first target ligand and a second ligand which binds to a second target ligand, and wherein the affinity of said first ligand is selected to enable binding to the first target ligand independently of the ability of said second ligand to bind to the second target ligand and wherein the affinity of said second ligand is selected to substantially reduce the probability of its binding to the second target ligand without the first ligand binding first or substantially contemporaneously to the first target ligand. 112. A bispecific antibody comprising a first antibody component which binds to a first target ligand and a second antibody component which binds to a second target ligand, and wherein the affinity or avidity or both the affinity and avidity of said first antibody component are selected to enable binding to the first target ligand independently of the ability of said second antibody component to bind to the second target ligand and wherein the avidity or affinity or both the affinity and avidity of said second ligand are selected to substantially reduce the probability of its binding to the second target ligand without the first ligand binding first or substantially contemporaneously to the first target ligand. 113. A multispecific ligand comprising a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety are selected to substantially reduce the probability of its binding to the second target ligand without the first moiety, first or substantially contemporaneously, binding to the first target ligand. 114. A multispecific ligand according to paragraph 113, wherein both moieties bind to different target ligands on the same cell. 115. A multispecific ligand comprising a first moiety which binds to a first target ligand and a second moiety which binds to a second target ligand, and wherein the affinity or avidity or both the affinity and avidity of said first moiety and the avidity or affinity or both the affinity and avidity of said second moiety are selected to substantially reduce the probability of either moiety binding for a sufficient duration or series of durations to its respective target ligand to a accomplish a therapeutic function without the other moiety, first or substantially contemporaneously, binding to its respective target ligand 116. A multispecific ligand comprising a first moiety which specifically binds to a first target ligand on a first entity and a second moiety which specifically binds to a second target ligand on a second entity, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety are selected to enable the second moiety to bind to the second entity in preference to the first moiety binding to the first entity when both first and second moieties are substantially contemporaneously bound to the respective first and second entities. 117. A multispecific ligand comprising a first moiety which specifically binds to a first target ligand on a first entity and a second moiety which specifically binds to a second target ligand on a second entity, and wherein the second entity binds to a third target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said first moiety are selected to enable the first moiety to bind to the first entity in preference to the second moiety binding to the second entity when both first and second moieties are substantially contemporaneously bound to the respective first and second entities, and wherein the avidity or affinity or both the affinity and avidity of said second moiety are selected to enable the third target ligand to bind to the second entity in preference to the second moiety binding to the second entity when both said third target ligand and the second moiety are substantially contemporaneously bound to the second entity. 118. A multispecfic ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the multispecific ligand, and wherein the affinity of the first ligand binding moiety for the first ligand is at least, approximately, one order of magnitude greater than that of the second ligand binding moiety for the second ligand. The affinity of the first ligand binding moiety for the first ligand is optionally at least, approximately, two orders of magnitude greater than that of the second ligand binding moiety for the second ligand. The affinity of the first ligand binding moiety for the first ligand is optionally at least, approximately, three orders of magnitude greater than that of the second ligand binding moiety for the second ligand. The affinity of the first ligand binding moiety for the first ligand is optionally at least, approximately, four orders of magnitude greater than that of the second ligand binding moiety for the second ligand. The affinity of the first ligand binding moiety for the first ligand is optionally at least, approximately, five orders of magnitude greater than that of the second ligand binding moiety for the second ligand. The affinity of the first ligand binding moiety for the first ligand is optionally at least, approximately, six orders of magnitude greater than that of the second ligand binding moiety for the second ligand. The affinity of the first ligand binding moiety for the first ligand is optionally at least, approximately, seven orders of magnitude greater than that of the second ligand binding moiety for the second ligand The affinity of the first ligand binding moiety for the first ligand is optionally at least, approximately, eight orders of magnitude greater than that of the second ligand binding moiety for the second ligand. 119. A multispecific ligand according to paragraph 118, wherein the first ligand is present on a first target cell population and wherein said second ligand is present on a second target cell population comprising the first target cell population and wherein the biodistribution of the multispecfic ligand favours the first target cell population. 120. A multispecific ligand according to paragraph 119, wherein said multispecific ligand is capable of contemporaneously binding the first and second ligands on said target population. 121. A host cell or cell free expression medium comprising one or more polynucleotides, said one or more polynucleotides comprising one or more DNA sequences, said one or more DNA sequences comprising one or more polypeptides which are sufficient to constitute a multispecific ligand as defined in any of the preceding paragraphs. 122. A kit comprising one or more polynucleotides, said one or more polynucleotides comprising one or more DNA sequences, said one or more DNA sequences encoding one or more polypeptides which are sufficient to constitute a multispecific ligand as defined in any of the preceding paragraphs. 123. A liquid medium comprising comprising one or more polypeptides which are sufficeint to constitute a multispecific ligand as defined in any of the preceding paragraphs. 124. A liquid medium comprising one or more host cells, said one or more host cells comprising one or more polynucleotides, said one or more polynucleotides comprising one or more DNA sequences, said one or more DNA sequences encoding one or more polypeptides which are sufficeint to constitute a multispecific ligand as defined in any of the preceding paragraphs. 125. A substantially isolated polynuceotide comprising a DNA sequence encoding a polypeptide portion of a second ligand binding moiety as defined in any of the preceding claims, said polypeptide portion comprising a VH or VL, said second ligand binding moiety having a low affinity for said second ligand. 126. A substantially isolated polynucleotide according to paragraph 125, wherein said polynucleotide is a substantially isolated expression or cloning vector. 127. A method of making a multispecific ligand as defined in any of the preceding paragraphs comprising expressing at least one polynucleotide as defined in paragraph 122 or 125. 128. A pharmaceutical composition comprising a multispecific ligand as defined in any of the preceding paragraphs and a pharmaceutically acceptable excipient. 129. A therapeutic composition comprising a multispecific ligand as defined in any of the preceding paragraphs and a pharmaceutically acceptable excipient. 130. A method of treating a disease in a mammal comprising administering a therapeutically effective amount of a multispecific ligand according to any of the preceding claims. 131. A kit comprising a plurality of different multispecfic ligands as defined herein.

Notwithstanding any indication to the contrary, it will be appreciated that the references herein cited have application to multiple different subjects and any qualifying remarks as to the applicability of the references is to be understood as relating to each of the subjects for which references are herein provided, as limited only by the title and subject matter of the reference.

All publications and references therein cited are herein incorporated by reference to the same extent as if each of the individual publications were specifically and individually indicated to be incorporated by reference in its entirety. 
Appendix A

6,103,234 Composition comprising an antibody to macrophage-derived
inflammatory mediator (MIP-2) 6,090,383 Monoclonal antibodies to transforming
growth factor-.beta. and methods of use 6,090,382 Human antibodies that bind
human TNF.alpha. 6,086,874 Antitumor agent effect enhancer containing IL-6
antagonist 6,084,075 Agonist and antagonist antibodies to the chemokine
receptor-2 (CCR2) 6,074,642 Use of antibodies specific to human complement
component C5 for the treatment of glomerulonephritis 6,068,841 Antibodies to
Fas-L for treatment of hepatitis 6,068,840 Recombinant CDR-grafted and chimeric
anti-IL-8 antibodies containing CDRs or the variable region from the mouse
monoclonal antibody, WS-4 6,057,427 Antibody to cytokine response gene
2(CR2) polypeptide 6,056,957 Humanized monoclonal antibodies against human
interleukin-5 6,036,956 Method and dosage form using an antagonist to gamma
interferon to control MHC-associated autoimmune disease 6,024,956 Method to
treat inflammation with humanized anti-IL-8-antibodies 6,024,955 Peptides and
monoclonal antibodies 6,019,969 Composition comprising antibody to
macrophage-derived inflammatory mediator (MIP-.alpha. 1 and MIP-1.beta.)
6,001,357 Method of enhancing wound healing with anti-IL-5 antibody 5,998,586
Interleukin-5 specific recombinant antibodies 5,994,511 Anti-IgE antibodies and
methods of improving polypeptides 5,989,584 5,980,894 Antibodies to leukemia
inhibitory factor 5,976,534 Inhibition of intimal hyperplasia using antibodies to
PDGF receptors and heparin 5,959,086 Antibodies specific for human pancreatitis
associated protein 5,955,076 Immunoactive peptides and antibodies and their use
in anti-allergy treatment 5,925,352 Method of treating inflammation with antibodies
to neutrophil chemotactic factor 5,925,351 Soluble lymphotoxin-.beta. receptors
and anti-lymphotoxin receptor and ligand antibodies as therapeutic agents for the
treatment of immunological disease 5,919,452 Methods of treating TNF.alpha.-
mediated disease using chimeric anti-TNF antibodies 5,916,805 Antithrombotic
agent and anti-von Willebrand factor monoclonal antibody 5,911,988 Method for
treating asthma using SCF antibody 5,888,510 Chronic rheumatoid arthritis
therapy containing IL-6 antagonist as effective component 5,876,947
Monospecific antibody reactive with Fibrinogen and fibrinopeptide B 5,874,080
Anti-IL-8 monoclonal antibodies for treatment of asthma 5,863,537 Humanized
monoclonal antibodies against human interleukin-4 5,853,722 Use of C5-specific
 
antibodies for reducing immune and hemostatic dysfunctions during extracorporeal
circulation 5,837,239 Physiologically active substance designated as epimorphin
genes encoding the same and antibodies thereto 5,837,232 Use of an interleukin-
10 antagonist to treat a B cell mediated autoimmune disorder 5,833,986 Methods
of inhibiting the growth of neoplasia using a monoclonal antibody against .alpha.
platelet derived growth factor receptor 5,827,517 CCK antibodies used to improve
feed efficiency 5,807,550 Antibodies specifically recognizing somatotropin binding
proteins 5,783,187 Treatment of cell proliferative disorders using antibodies which
bind connective tissue growth factor 5,783,185 Monoclonal antibodies to
transforming growth factor-beta and methods of use 5,772,998 Monoclonal
antibodies to transforming growth factor-beta and methods of use 5,741,488
Treatment of rheumatoid arthritis with anti-CD4 antibodies in conjunction with anti-
TNF antibodies 5,730,975 Treatment of insulin resistance in obesity linked type II
diabetes using antagonist to TNF-alpha function 5,723,126 Methods and
compositions for therapeutic uses of a fibrin-specific monoclonal antibody
5,712,370 Erythropoietin (EPO) peptides and antibodies directed against these
5,705,154 Humanized monoclonal antibodies against human interleukin-4
5,702,705 Antibody methods for the treatment of a hormone-mediated disease
5,700,466 Method of ameliorating or preventing septic shock using a monoclonal
antibody specific to cachectin/tumor necrosis factor 5,698,197 Monoclonal
antibody specific for advanced glycosylation endproducts in biological samples
5,698,196 Antibodies specific for neutrophic chemotactic factor 6,498,237 Tumor
necrosis factor antibodies 6,486,304 Antibodies and methods of making antibodies
to human thyroid protein zsig45 6,482,408 Fibroblast growth factor 15 antibodies
6,468,532 Methods of treating inflammatory diseases with anti-IL-8 antibody
fragment-polymer conjugates 6,458,355 Methods of treating inflammatory disease
with anti-IL-8 antibody fragment-polymer conjugates 6,451,983 Tumor necrosis
factor antibodies 6,451,545 Monoclonal antibody specific to anti-human fibrin
monomer, process for producing the same, hybridomas, and immunoassay method
6,451,311 Anti-procalcitonin antibodies and the preparation and use thereof
6,448,380 Tumor necrosis factor antibodies 6,440,417 Antibodies to argatroban
derivatives and their use in therapeutic and diagnostic treatments 6,436,401
Methods for alleviating symptoms associated with diabetes and diabetic
neuropathy comprising administration of low levels of antibodies 6,416,758
Antibody conjugate kits for selectively inhibiting VEGF 6,416,757 Tumor necrosis
factor antibodies 6,413,726 Antibodies that specifically bind Cytostatin III
 
6,395,274 Monoclonal antibodies specific for human interleukin-5 6,383,484
Antibodies to truncated VEGF-D and thereof 6,375,949 Monoclonal antibody
recognizing serum amyloid A 6,365,155 Ancrod-specific monoclonal antibodies,
antibody fragments, mixtures or derivatives thereof and use of the same 6,355,245
C5-specific antibodies for the treatment of inflammatory diseases 6,346,246
Antibodies that bind human haemopoietic maturation factor 6,342,219 Antibody
compositions for selectively inhibiting VEGF 6,331,301 Antibodies specific for
vascular endothelial growth factor-B 6,328,963 Anti-human MP52 monoclonal
antibody 6,300,477 Antibodies to human cystatin E 6,294,655 Anti-interleukin-1
receptor antagonist antibodies and uses thereof 6,280,731 Antithrombotic agent
and anti-von willebrand factor monoclonal antibody 6,280,730 Antibodies that bind
to .alpha.2-antiplasmin crosslinked to fibrin which do not inhibit plasma .alpha.2-
antiplasmin 6,274,142 Methods of neutralizing coagulation with anti-tissue factor
antibodies 6,270,766 Anti-TNF antibodies and methotrexate in the treatment of
arthritis and crohn's disease 6,261,790 Monoclonal antibodies and antibody
cocktail for detection of prion protein as an indication of transmissible spongiform
encephalopathies 6,261,559 Methods of treating asthma with interleukin-9
antibodies 6,251,393 Conformation-specific anti-von Willebrand Factor antibodies
6,238,666 RANTES homolog antibody 6,235,281 Use of anti-TNF antibodies as
drugs for the treatment of disorders with an elevated serum level of interleukin-6
6,232,447 Antibody immunoreactive with a human cytokine designated LERK-6
6,228,360 Antithrombotic agent and humanized anti-von Willebrand factor
monoclonal antibody 6,218,516 Antibodies specific for the extracellular domain of
NYK/flk-1 protein and uses thereof 6,200,559 Use of antibodies against MxA or
MxB to determine levels of type I interferons in vivo 6,193,969 Antibody fragments
in therapy 6,184,359 Antibodies to epithelium-derived T-cell factor 6,180,102
Monoclonal antibody to human Mx protein MxA 6,168,791 Antibodies that bind
immunomodulators 6,165,465 Immunologic enhancement of the somatogenic
effect of somatotropin with an antibody 6,143,874 Antibodies to the neurotrophic
factor NNT-1 6,133,426 Humanized anti-IL-8 monoclonal antibodies 6,124,439
OB polypeptide antibodies and method of making 6,117,985 Antibody
compositions for preparing enriched cell preparations 6,117,980 Humanized anti-
IL-8 monoclonal antibodies 6,114,507 Anti-Fas ligand antibody and assay method
using the anti-Fas ligand antibody 6,113,897 Antibodies and their use
20020187150 Preventive and/or therapeutic agent for systemic lupus
erythematosus comprising anti-IL-6 receptor antibody as an active ingredient
 
20020176858 Monoclonal antibodies to TGF-BETA 20020172677 Method to
inhibit airway hyperresponsiveness using aerosolized T cell receptor antibodies
20020164327 Pharmaceutical composition of F(ab)2 antibody fragments and a
process for the preparation thereof 20020160010 USE OF PREPARATIONS
CONTAINING ANTI-CD44 ANTIBODIES IN THE TREATMENT OF CERTAIN
TUMOURS AND THE SUPPRESSION OF IMMUNE REACTIONS 20020150882
Antibody specific to CD14 and uses thereof 20020146419 Methods of treating
ankylosis with chimeric anti-TNF antibodies 20020141996 Methods of treating
infections with chimeric anti-TNF antibodies 20020136723 Anti-TNF antibodies
and methotrexate in the treatment of autoimmune disease 20020122797
ANTIBODIES TO VASCULAR ENDOTHELIAL CELL GROWTH FACTOR
20020119153 Antibody conjugate formulations for selectively inhibiting VEGF
20020119152 Anti-TNFalpha antibodies in therapy of asthma 20020114805
Methods of treating myelodysplastic syndrome with anti-TNF antibodies
20020106372 Anti-TNF antibodies and peptides of human tumor necrosis factor
20020098527 Gene recombinant antibody and antibody fragment thereof
20020091240 Humanized antibodies to gamma-interferon 20020082396
Reshaped human antibody to human interleukin-8 20020081300 METHODS FOR
ALLEVIATING SYMPTOMS ASSOCIATED WITH DIABETES AND DIABETIC
NEUROPATHY COMPRISING ADMINISTRATION OF LOW LEVELS OF
ANTIBODIES 20020064528 Antibodies specific to KDR and uses thereof
20020051782 Anti-CCR2 antibodies and methods of use therefor 20020028204
Antithrombotic agent and anti-von willebrand factor monoclonal antibody
20020025317 Bispecific monoclonal antibodies to IL-12 and IL-18 20020022720
Anti-TNF antibodies and peptides of human tumor necrosis factor 20020015700
Anti-CCR2 antibodies and methods of use therefor 20020012909 Small functional
units of antibody heavy chain variable regions 20020012665 Combined use of
anti-cytokine antibodies or antagonists and anti-CD20 for treatment of B cell
lymphoma 20020006405 SEPSIS REMEDY COMPRISING ANTI-IL-8 ANTIBODY
AS ACTIVE INGREDIENT 20010019833 TNF receptor-specific antibodies
20010018052 Use of anti-coagulation factor antibodies as long-lasting protective
agents 20010010819 Use of anti-TNF antibodies as drugs for the treatment of
disorders with an elevated serum level of interleukin-6 20010006637
RHEUMATOID ARTHRITIS REMEDY CONTAINING ANTI-IL-8 ANTIBODY AS
ACTIVE INGREDIENT 5,698,195 Methods of treating rheumatoid arthritis using
chimeric anti-TNF antibodies 5,688,505 Method for treating cancer with
 
monoclonal antibodies to oncofetal protein 5,670,150 Non-depleting CD4-specific
monoclonal antibodies for the treatment of insulin-dependent diabetes mellitus
(IDDM) 5,662,904 Anti-scarring compositions comprising growth factor
neutralizing antibodies 5,656,272 Methods of treating TNF-.alpha.-mediated
Crohn's disease using chimeric anti-TNF antibodies 5,654,407 Human anti-TNF
antibodies 5,635,178 Inhibition of complement mediated inflammatory response
using monoclonal antibodies specific for a component forming the C56-9 complex
which inhibit the platelet or endothelial cell activating function of the C56-9 complex
5,627,073 Hybridomas producing antibodies to cardiac hypertrophy factor
5,616,321 Method of treating bacterial meningitis with anti-tumor necrosis factor
antibody 5,610,281 Antibodies for modulating heterotypic E-cadherin interactions
with human T lymphocytes 5,601,821 Immunoactive peptides and antibodies and
their use in anti-allergy treatment 5,599,708 Osteoclast growth regulating factors
and antibodies 5,582,862 Antibodies that bind to .alpha.2-antiplasmin crosslinked
to fibrin which do not inhibit plasma .alpha.2-antiplasmin 5,580,561 Methods and
pharmaceutical compositions for blocking suppression of immune defense
mechanisms using an antibody, a factor, or an antisense peptide 5,573,762 Use
of leukemia inhibitory factor specific antibodies and endothelin antagonists for
treatment of cardiac hypertrophy 5,571,714 Monoclonal antibodies which bind
both transforming growth factors .beta.1 and .beta.2 and methods of use
5,559,012 Therapeutic, IL-6 antibody kits, and process for their preparation
5,543,144 Treating hypersensitivities with anti-IGE monoclonal antibodies which
bind to IGE-expressing B cells but not basophils 5,520,914 Antibodies to
angiogenin: immunotherapeutic agents 5,496,549 Bispecific monoclonal
antibodies, thrombolytic agent and method of cell lysis 5,487,892 Method for
treating thrombotic disease using a fibrin specific monoclonal antibody 5,478,926
Monoclonal antibody against human IgE 5,334,380 Anti-endotoxin, interleukin-1
receptor antagonist and anti-tumor necrosis factor antibody with arginine-free
formulations for the treatment of hypotension 5,279,956 Activated protein C
polypeptides and anti-peptide antibodies, diagnostic methods and systems for
inhibiting activated protein C 5,237,054 Stabilized aqueous composition
containing antibodies 5,225,540 Monoclonal antibodies to tissue plasminogen
activator (T-PA) which prolong its functional half-life 5,196,324 Monoclonal
antibodies reactive with a human atheroma associated antigen 5,183,657
Antibodies for use in antilymphocyte antibody therapy 5,149,786 Dopamine
releasing protein and antibody 5,124,439 Antibodies against fibrin; immunogenic
 
peptides suitable for preparing the antibodies, method for determining fibrin and
pharmaceutical preparation based on the antibodies   5,120,834   Fibrin-specific
monoclonal antibody   5,116,613   Antibody-thrombolytic agent product and method
of use   5,109,115   Monoclonal antibody specific for bombesin   5,055,289
Interferon antibody therapeutic compositions having an extended serum half-life
4,963,657   Monoclonal antibodies to the light chain region of human factor XII and
methods of preparing and using the same   4,940,782   Monoclonal antibodies
against IgE-associated determinants, hybrid cell lines producing these antibodies,
and use therefore   4,927,916   Method of producing fibrin-specific monoclonal
antibodies lacking fibrinogen-cross-reactivity using fibrin-specific peptides
4,916,070   Fibrin-specific antibodies and method of screening for the antibodies
4,912,201   Monoclonal antibodies and antisera to intragonadal regulatory protein
4,879,112   Monoclonal antibodies agaisnt GnRH   4,851,334   Monoclonal
antibodies specific to in vivo fragments derived from human fibrinogen, human
fibrin I or human fibrin II   4,780,401   Novel monoclonal antibodies to human renin
and hybridoma cells, processes for their preparation and their applications
4,764,475   Pancreas dependant immunoassay for determining subpopulations of
monoclonal antibodies to somatostatin.   4,676,981   Monoclonal antibodies against
GnRH   4,599,229   Method of promoting animal growth using antibodies against
somatostatin   4,565,687   Monoclonal antibodies specific for the unbound .beta.
subunit of human chorionic gonadotropin
 
Appendix B

20020028436 Anti-CCR2 antibodies and methods of use therefor 20020028178
Treatment of B cell malignancies using combination of B cell depleting antibody
and immune modulating antibody related applications 20020018775
MONOCLONAL ANTIBODIES TO STEM CELL FACTOR RECEPTORS
20020015703 Antagonists of tweak and of tweak receptor and their use to treat
immunological disorders 20020004587 Multivalent antibodies and uses therefor
20010041178 Preventing airway mucus production by administration of EGF-R
antagonists 20010036919 Preventing airway mucus production by administration of
EGF-R antagonists 20010036459 Enhancement of antibody-mediated immune
responses 20020028436 Anti-CCR2 antibodies and methods of use therefor
20020028178 Treatment of B cell malignancies using combination of B cell
depleting antibody and immune modulating antibody related applications
20020018775 MONOCLONAL ANTIBODIES TO STEM CELL FACTOR
RECEPTORS 20020015703 Antagonists of tweak and of tweak receptor and their
use to treat immunological disorders 20020004587 Multivalent antibodies and uses
therefor 20010041178 Preventing airway mucus production by administration of
EGF-R antagonists 20010036919 Preventing airway mucus production by
administration of EGF-R antagonists 20010036459 Enhancement of antibody-
mediated immune responses 5,736,138 Monoclonal antibodies with specific
binding against membrane proteins on human cells, and pharmaceutical
compositions containing them 5,736,137 Therapeutic application of chimeric and
radiolabeled antibodies to human B lymphocyte restricted differentiation antigen for
treatment of B cell lymphoma 5,730,979 LO-CD2a antibody and uses thereof for
inhibiting T cell activation and proliferation 5,730,978 Inhibition of lymphocyte
adherence with .alpha.4.beta.1-specific antibodies 5,730,975 Treatment of insulin
resistance in obesity linked type II diabetes using antagonist to TNF-alpha function
5,725,856 Monoclonal antibodies directed to the HER2 receptor 5,720,954
Monoclonal antibodies directed to the HER2 receptor 5,717,073 Anti-gp130
monoclonal antibodies 5,717,072 Antibodies that are immunoreactive with
interleukin-4 receptors 5,714,146 IL-4 bone therapy 5,709,858 Antibodies specific
for Rse receptor protein tyrosine kinase 5,705,157 Methods of treating cancerous
cells with anti-receptor antibodies 5,693,322 Enhanced intercellular interaction by
associational antibody molecules 5,690,933 Monoclonal antibodies for inducing
tolerance 5,688,504 Anti-receptor and growth blocking antibodies to the vitamin
 
B.sub.12 /transcobalamin II receptor and binding sites 5,686,292 Hepatocyte
growth factor receptor antagonist antibodies and uses thereof 5,683,693 Method
for inducing T cell unresponsiveness to a tissue or organ graft with anti-CD40
ligand antibody or soluble CD40 5,679,346 Methods of blocking adhesion with anti-
lami-3 antibody 5,670,150 Non-depleting CD4-specific monoclonal antibodies for
the treatment of insulin-dependent diabetes mellitus (IDDM) 5,667,978 Antibody to
the neural cell adhesion molecule and methods of use 5,667,781 Enhanced
inhibition of tumor cell proliferation using a combination of two monoclonal
antibodies to the human transferrin receptor 5,652,110 Antibodies to
.alpha.v.beta.3 integrin 5,652;109 Antibodies to .alpha.v.beta.3 integrin 5,651,969
Antibody to the neural cell adhesion molecule and methods of use 5,635,601 Beta-
8 integrin subunit antibodies 5,635,177 Protein tyrosine kinase agonist antibodies
5,632,991 Antibodies specific for E-selectin and the uses thereof 5,622,701 Cross-
reacting monoclonal antibodies specific for E- and P-selectin 5,620,889 Human
anti-Fas IgG1 monoclonal antibodies 5,620,687 Inhibition of intimal hyperplasia
using antibodies to PDGF beta receptors 5,582,826 Monoclonal antibodies which
bind the gamma chain of human interleukin-2 receptor 5,578,704 Antibody to
osteoclast alphavbeta3 ntegrin 5,571,513 Anti-gp130 monoclonal antibodies
5,558,864 Humanized and chimeric anti-epidermal growth factor receptor
monoclonal antibodies 5,545,405 Method for treating a mammal suffering from
cancer with a cho-glycosylated antibody 5,545,404 Method for treating a mammal
suffering from a T-cell medicated disorder with a CHO-Glycosylated antibody
5,545,403 Method for treating a mammal by administering a CHO-glycosylated
antibody 5,498,499 Peptides and antibodies that inhibit platelet adhesion 5,486,361
Hybridomas and monoclonal antibodies that speifically bind to GPIB on platelets
and inhibit the binding of thrombin to platelets 5,474,771 Murine monoclonal
antibody (5c8) recognizes a human glycoprotein on the surface of T-lymphocytes,
compositions containing same 5,424,066 Method for increasing CD4+ cell numbers
through the use of monoclonal antibodies directed against self-reactive, CD4
specific cytotoxic T-cells 5,418,135 Method of inhibiting binding of PDGF to a
PDGF receptor by biosynthetic PDGF antagonists 5,378,464 Modulation of
inflammatory responses by administration of GMP-140 or antibody to GMP-140
5,359,037 Antibodies to TNF binding protein I 5,273,743 Trifunctional antibody-like
compounds as a combined diagnostic and therapeutic agent 5,223,426 Monoclonal
antibodies reactive with defined regions of the T-cell antigen receptor 5,182,107
Transferrin receptor specific antibody-neuropharmaceutical or diagnostic agent
 
conjugates 5,101,017 Antibodies for providing protection against P. vivax malaria
infection 5,084,391 Monoclonal antibody to the interleukin-2-receptor and its use
5,028,424 Antibodies to receptor and antigen for natural killer and non-specific
cytotoxic cells 6,056,956 Non-depleting anti-CD4 monoclonal antibodies and
tolerance induction 6,051,228 Antibodies against human CD40 6,048,526
Monoclonal antibodies reactive with defined regions of the T cell antigen receptor
6,033,876 Anti-CD30 antibodies preventing proteolytic cleavage and release of
membrane-bound CD30 antigen 6,030,796 Monoclonal antibody to a human MDR1
multidrug resistance gene product, and uses 6,015,560 Antibody recognizing
endothelial cell ligand for leukocyte CR3 6,015,559 Fas antagonists 6,015,555
Transferrin receptor specific antibody-neuropharmaceutical or diagnostic agent
conjugates 6,011,138 Gamma-1 anti-human CD23 monoclonal antibodies
6,007,816 Methods of using CD44-specific antibodies 6,004,552 Methods of
blocking B cell proliferation using anti-CD40 monoclonal antibodies 6,001,358
Humanized antibodies to human gp39, compositions containing thereof 6,001,356
Method of inhibiting tissue destruction in autoimmune disease using anti-CD44
antibodies 5,997,867 Method of using humanized antibody against CD18
5,997,866 Panel of antibodies for detecting cadherins, catenins and plaque
proteins in tissues and method of using the panel 5,997,865 Agonist antibodies
against the flk2/flt3 receptor and uses thereof 5,994,515 Antibodies directed
against cellular coreceptors for human immunodeficiency virus and methods of
using the same 5,993,816 Methods to inhibit humoral immune responses,
immunoglobulin production and B cell activation with 5c8-specific antibodies
5,985,280 Diagnosis and/or therapy of tumours using monoclonal antibodies
specific for the human IL-4 receptor 5,985,279 Humanized antibody against CD18
5,985,278 Anti-.alpha.V-integrin monoclonal antibody 5,985,276 Destruction of
contaminating tumor cells in stem cell transplants using bispecific antibodies
5,980,893 Agonist murine monoclonal antibody as a stimulant for
megakaryocytopoiesis 5,980,892 Monoclonal antibodies reactive with defined
regions of the T cell antigen receptor 5,976,533 Monoclonal antibodies reactive
with defined regions of the T cell antigen receptor 5,976,532 Method of
antithrombotic therapy using anti-GPIIb/IIIa antibodies or fragments thereof,
including c7E3 5,968,512 Antibody recognizing endothelial cell ligand for leukocyte
CR3 5,968,511 ErbB3 antibodies 5,961,974 Monoclonal antibodies to CD40 ligand,
pharmaceutical composition comprising the same and hybridomas producing the
same 5,959,084 Bispecific antibodies, methods of production and uses thereof
 
5,958,410 Therapy of sarcoidosis 5,951,983 Methods of inhibiting T cell mediated.
immune responses with humanized LO-CD2A-specific antibodies 5,951,982
Methods to suppress an immune response with variant CD44-specific antibodies.
5,942,229 Method for prolonged suppression of humoral immune response to a
thymus-dependent antigen therapeutic agent 5,925,351 Soluble lymphotoxin-.beta.
receptors and anti-lymphotoxin receptor and ligand antibodies as therapeutic
agents for the treatment of immunological disease 5,922,847 Methods of purifying
hematopoietic cells using an antibody to a stem cell factor receptor 5,919,911
Monoclonal antibodies to stem cell factor receptors 5,919,453 Monoclonal
antibodies against the interferon receptor, with neutralizing activity against type I
interferon 5,916,561 Monoclonal antibody against CD44v6 5,914,112 Anti-CD18
antibodies in stroke 5,912,172 Endowing lymphocytes with antibody specificity
5,906,936 Endowing lymphocytes with antibody specificity 5,902,585 Methods of
inducing T cell unresponsiveness to donor tissue or organ in a recipient with GP39
antagonists 5,902,584 Antibodies which bind the G-CSF receptor extracelluar
domain and methods of treatment 5,897,861 Bispecific reagents for AIDS therapy
5,891,996 Humanized and chimeric monoclonal antibodies that recognize
epidermal growth factor receptor (EGF-R); diagnostic and therapeutic use
5,891,434 Monoclonal antibodies to the APO-1 antigen 5,889,160 Human IL-2
receptor .gamma.-chain molecule antibody 5,888,508 Monoclonal antibodies
against leukocyte adhesion receptor .beta.-chain methods of producing these
antibodies and use therefore 5,885,575 Antibodies that react with variant CD44
surface proteins 5,885,574 Antibodies which activate an erythropoietin receptor
5,885,573 Methods and materials for modulation of the immunosuppressive activity
and toxicity of monoclonal antibodies 5,882,644 Monoclonal antibodies specific for
the platelet derived growth factor .beta. receptor and methods of use thereof
5,880,267 Trophinin and trophinin-assisting protein agonists, antibodies and
antagonists 5,876,950 Monoclonal antibodies specific for different epitopes of
human GP39 and methods for their use in diagnosis and therapy 5,876,718
Methods of inducing T cell non-responsiveness to transplanted tissues and of
treating graft-versus-host-disease with anti-gp39 antibodies 5,874,082 Humanized
anti-CD40 monoclonal antibodies and fragments capable of blocking B cell
proliferation 5,871,732 Anti-CD4 antibody homologs useful in prophylaxis and
treatment of AIDS, ARC and HIV infection 5,869,049 Methods of inducing T cell
unresponsiveness to bone marrow with gp39 antagonists 5,863,796 Antibodies
which specifically bind mammalian receptors for interleukin-10 (IL-10) 5,856,448
 
Antibodies specifically reactive with thrombin receptor and its components
5,853,721 Antibody to interleukin-12 receptor 5,852,176 Antibodies to receptors for
human interleukin-12 5,846,535 Methods for reducing tumor cell growth by using
antibodies with broad tumor reactivity and limited normal tissue reactivity 5,844,093
Anti-EGFR single-chain Fvs and anti-EGFR antibodies 5,843,441 Use of
endothelial-leukocyte adhesion molecule-1 specific antibodies in the treatment of
asthma 5,843,439 Therapeutic application of chimeric and radiolabeled antibodies
to human B lymphocyte restricted differentiation antigen for treatment of B cell
lymphoma 5,843,398 Radioimmunotherapy of lymphoma using anti-CD20
antibodies 5,840,301 Methods of use of chimerized, humanized, and single chain
antibodies specific to VEGF receptors 5,840,299 Humanized antibodies against
leukocyte adhesion molecule VLA-4 5,833,988 Transferrin receptor specific
antibody-neuropharmaceutical or diagnostic agent conjugates 5,833,986 Methods
of inhibiting the growth of neoplasia using a monoclonal antibody against .alpha.
platelet derived growth factor receptor 5,830,471 Methods and compositions
comprising anti-lam-1 antibodies 5,830,469 Fas antagonists and uses thereof
5,824,311 Treatment of tumors with monoclonal antibodies against oncogene
antigens 5,821,123 Modified antibody variable domains 5,820,859 Method of
targeting a therapeutic agent to cells expressing the erb B-3 receptor 5,817,311
Methods of inhibiting T-cell medicated immune responses with LO-CD2a-specific
antibodies 5,808,002 Stem cell factor receptor-(c-kit)-specific monoclonal antibody
A3C6E2 5,800,815 Antibodies to P-selectin and their uses 5,795,572 Monoclonal
antibodies and FV specific for CD2 antigen 5,788,966 Antibody which is directed
against and inhibits collagen binding to a VLA-1 epitope and uses thereof
5,785,967 Antibodies immunoreactive with leukemia inhibitory factor receptors
5,783,186 Antibody-induced apoptosis 5,780,027 Methods of treatment of down
syndrome by interferon antagonists 5,777,084 Antibody BV10A4H2 specific for
human FLT3/FLK2 receptor and mybridoma 5,776,457 Antibodies to human PF4A
receptor and compositions thereof 5,776,456 Therapeutic application of chimeric
and radiolabeled antibodies to human B lymphocyte restricted differentiation
antigen for treatment of B cell lymphoma 5,772,997 Monoclonal antibodies directed
to the HER2 receptor 5,770,196 Modified antibody variable domains and
therapeutic uses thereof 5,770,195 Monoclonal antibodies directed to the her2
receptor 5,766,947 Monoclonal antibodies reactive with an epitope of a V.beta.3.1
variable region of a T cell receptor 5,762,933 Method for preventing and treating
graft failure in a human patient using a monoclonal antibody specific for leucocyte
 
functional antigen LFA-1 5,762,932 Combined treatment of iron depletion and IgG
antibody 5,756,095 Antibodies with specificity for a common epitope on E-selectin
and L-selectin 5,753,225 Antibodies that mimic actions of neurotrophins 5,747,037
Anti-GP39 antibodies 5,747,032 Monoclonal antibody to human granulocyte
macrophage colony stimulating factor 5,741,488 Treatment of rheumatoid arthritis
with anti-CD4 antibodies in conjunction with anti-TNF antibodies 5,739,289
Monoclonal antibody to human cell adhesion molecule 4,954,617 Monoclonal
antibodies to FC receptors for immunoglobulin G on human mononuclear
phagocytes 4,943,533 Hybrid cell lines that produce monoclonal antibodies to
epidermal growth factor receptor 4,935,234 Method of reducing tissue damage at
an inflammatory site using a monoclonal antibody 4,908,203 Method for inducing
HIV neutralizing antibodies using an internal image idiotope 4,863,726 Combination
therapy using immunotoxins with interleukin-2 4,859,449 Modified antibodies for
enhanced hepatocyte clearance 4,840,793 Method of reducing tissue damage at
an inflammatory site using a monoclonal antibody 4,797,277 Method for
reperfusion therapy 4,786,590 Diagnostic and therapeutic aspects of receptor-
mediated leukemogenesis 4,550,086 Monoclonal antibodies that recognize human
T cells 4,434,156 Monoclonal antibodies specific for the human transferrin receptor
glycoprotein 6,291,239 Monoclonal antibody 6,287,537 Radioimmunotherapy of
lymphoma using anti-CD20 antibodies 6,274,143 Methods of delaying development
of HMFG-associated tumors using anti-idiotype antibody 11D10 6,261,560 Method
for inhibiting muscle protein proteolysis with antibodies to interleukin-6 receptor
6,252,050 Method for making monoclonal antibodies and cross-reactive antibodies
obtainable by the method 6,251,395 Methods of inhibiting inflammation at the site
of a central nervous system injury with alphaD-specific antibodies 6,232,123
Monoclonal antibodies against leucocyte-specific G protein-coupled receptors
6,217,866 Monoclonal antibodies specific to human epidermal growth factor
receptor and therapeutic methods employing same 6,214,344 Hepatocyte growth
factor receptor antagonists and uses thereof 6,214,342 Method for increasing
mean survival times of transplants with LFA-1-specific antibodies 6,210,671
Humanized antibodies reactive with L-selectin 6,210,670 Cross-reacting
monoclonal antibodies specific for E-selectin and P-selectin 6,210,668 Destruction
of contaminating tumor cells in stem cell transplants using bispecific antibodies
6,207,156 Specific antibodies and antibody fragments 6,207,155 Method of
eosinophil depletion with antibody to CCR 3 receptor 6,207,152 Hepatocyte growth
factor receptor antagonists and uses thereof 6,200,567 Therapeutic agents as
 
cytokine antagonists and agonists 6,200,559 Use of antibodies against MxA or
MxB to determine levels of type I interferons in vivo 6,197,297 Monoclonal antibody
specific to polypeptide which induces interferon-gamma production 6,187,308
Monoclonal antibodies against leukocyte adhesion receptor .beta.-chain, methods
of producing these antibodies and use therefor 6,183,744 Immunotherapy of B-cell
malignancies using anti-CD22 antibodies 6,177,078 Monoclonal antibody
antagonists to IL-3 6,171,588 Anti-.alpha.v.beta.3 integrin antibody antagonists
6,165,464 Monoclonal antibodies directed to the HER2 receptor 6,160,099 Anti-
human .alpha..sub.v .beta..sub.3 and .alpha..sub.v .beta..sub.5 antibodies
6,143,873 Monoclonal anti-multiple drug resistance antibody, methods of
production and uses thereof 6,143,559 Methods for the production of chicken
monoclonal antibodies 6,143,297 Methods of promoting immunopotentiation and
preparing antibodies with anti-CD3 antibodies 6,143,292 Allogeneic cell therapy for
cancer following allogeneic stem cell transplantation 6,143,273 Therapeutic
composition containing antibodies to soluble polypeptide fractions of LAG-3 protein
6,136,309 Antibodies against the interferon (IFN) .alpha./.beta. receptor (IFNAR2)
that preferentially block the activity of IFN-.alpha. 6,129,915 Epidermal growth
factor receptor antibodies 6,123,939 Anti-neoplastic drugs in cancer therapy
6,117,985 Antibody compositions for preparing enriched cell preparations
6,113,901 Methods of stimulating or enhancing the immune system with anti-CD3
antibodies 6,113,900 Use of IL-2 receptor-targeted therapeutics to inhibit allograft
rejection and treat autoimmune disorders 6,113,898 Human B7.1-specific
primatized antibodies and transfectomas expressing said antibodies 6,106,834 Use
of anti-CD45 leukocyte antigen antibodies for immunomodulation 6,106,833
Bispecific antibodies, methods of production and use thereof 6,099,841 Hepatocyte
growth factor receptor agonists and uses thereof 6,099,838 Pharmaceutical
compositions comprising anti-CD45RB antibodies for the inhibition of T-cell
mediated immune responses 6,096,311 Methods for use of monoclonal antibodies
specific for the high affinity Fc receptor for immunoglobulin G 6,090,365
Radioimmunotherapy of lymphoma using anti-CD20 antibodies 6,086,877
Therapeutic agent for rheumatic disease 6,086,874 Antitumor agent effect
enhancer containing IL-6 antagonist 6,084,075 Agonist and antagonist antibodies
to the chemokine receptor-2 (CCR2) 6,083,904 Therapeutic and diagnostic
methods and compositions based on notch proteins and nucleic acids 6,072,037
Antibodies to the IL-17 receptor 6,066,719 Antibody fragments 6,056,959 CD40
antigen antibody complex 6,506,383 Methods of suppressing immune responses to
 
transplanted tissues and organs with gp39-specific antibodies 6,506,382 Method
for inhibiting reperfusion injury using antibodies to P-selectin glycoprotein ligand
6,500,428 Antibodies to a lymphocyte surface receptor that binds CAML and
methods of use thereof 6,491,916 Methods and materials for modulation of the
immunosuppresive activity and toxicity of monoclonal antibodies 6,491,915 Anti-
CCR2 antibodies and methods of use therefor 6,488,930 Anti-CCR4 antibodies and
methods of use therefor 6,461,612 Anti-idiotypic antibody and its use in diagnosis
and therapy in HIV-related disease 6,458,933 Therapeutic using a bispecific
antibody 6,458,356 Antibody-induced apoptosis 6,458,353 Anti-CCR2 antibodies
and methods of use therefor 6,455,043 Combination therapies for B-cell
lymphomas comprising administration of anti-CD20 antibody 6,455,042 Method of
treating ulcerative colitis or crohn's disease by administering an antibody
to.alpha.E.beta.7 integrin 6,451,981 Antibodies against lymphocyte-associated cell
surface protein LAM-1 6,451,310 Method for inhibiting an allergic response with a
5c8-specific antibody 6,440,418 Methods of treating autoimmune diseases with
gp39-specific antibodies 6,440,417 Antibodies to argatroban derivatives and their
use in therapeutic and diagnostic treatments 6,432,405 Method of inhibiting HIV
infection with CD44 and anti-CD44 antibodies 6,432,404 Methods of inhibiting
locomotor damage following spinal cord injury with .alpha. D-specific antibodies
6,420,126 Tumor specific internalizing antigens and methods for targeting
therapeutic agents 6,413,514 Methods of using antibodies against human CD40
6,406,695 Treatment of hepatitis C by administration of anti-IL-2 receptor
monoclonal antibody 6,406,694 Anti-CCR2 antibodies 6,403,091 Methods for
inhibiting the rejection of a transplant organ in a subject with 5C8-specific
antibodies 6,399,063 Monoclonal antibodies directed to the HER2 receptor
6,399,061 Chimeric and radiolabelled antibodies specific to human CD20 antigen
and use thereof for treatment of B-cell lymphoma 6,395,272 Therapeutic
compounds comprised of anti-Fc receptor antibodies 6,392,020 Monoclonal
antibody to the clonotypic structure of a T-cell receptor, a pharmaceutical
compostion and a diagnostic reagent comprising the same 6,387,371 Monoclonal
antibodies directed to the HER2 receptor 6,380,363 Antibodies to the Ob receptor
6,379,668 Use of anti-CD45 leukocyte antigen antibodies for immunomodulation
6,375,950 Methods for inducing T cell unresponsiveness to donor tissue or organ in
a recipient with gp39 antagonists 6,365,157 Monoclonal antibodies specific to
VEGF receptors and uses thereof 6,355,780 Antibodies to the death domain motifs
of regulatory proteins 6,348,194 Tumor specific internalizing antigens and methods
 
for targeting therapeutic agents 6,342,219 Antibody compositions for selectively
inhibiting VEGF 6,340,459 Therapeutic applications for the anti-T-BAM (CD40-L)
monoclonal antibody 5C8 in the treatment of reperfusion injury in non-transplant
recipients 6,331,302 Protein tyrosine kinase agonist antibodies 6,329,510 Anti-
CCR1 antibodies and methods of use therefor 6,329,159 Anti-GPR-9-6 antibodies
and methods of identifying agents which modulate GPR-9-6 function 6,328,964
Method to treat multiple sclerosis with GP39-specific antibodies 6,319,499 Methods
for activating an erythropoietin receptor using antibodies 6,315,998 Methods of
blocking B-cell activation using anti-CD40 monoclonal antibodies 6,312,694 Cancer
treatment methods using therapeutic conjugates that bind to aminophospholipids
6,312,693 Antibodies against human CD40 6,312,692 Method of treating graft-
versus-host disease with anti-GP39 antibodies and bone marrow cells 6,312,691
Lymphotoxin-.alpha./.beta. complexes and anti-lympotoxin-.beta. receptor
antibodies as anti-tumor agents 6,312,690 Monoclonal recombinant anti-rhesus D
(D7C2) antibody 6,312,689 Anti-CCR2 antibodies and methods of use therefore
6,309,639 Method for inhibiting an inflammatory response using antibodies to P-
selectin glycoprotein ligand 6,306,393 Immunotherapy of B-cell malignancies using
anti-CD22 antibodies
 
Appendix C

Bruhl H, et alDepletion of CCR5-Expressing Cells with Bispecific Antibodies and
Chemokine Toxins: A New Strategy in the Treatment of Chronic Inflammatory
Diseases and HIV. J Immunol. 2001 Feb 15; 166(4):2420-2426; Tesch H, et
alTreatment of patients with malignant lymphomas with monoclonal antibodies.
Bone Marrow Transplant. 2000 May; 26 Suppl 2:S50-3; Jung G, Brandl M, Eisner
W, Fraunberger P, Reifenberger G,Schlegel U, Wiestler OD, Reulen HJ, Wilmanns
W. Local immunotherapy of glioma patients with a combination of 2 bispecific
antibody fragments and resting autologous lymphocytes: Evidence for in situ t-cell
activation and therapeutic efficacy. Int J Cancer. 2001 Jan 15; 91(2):225-230; Kan
KS, Anderson VA, Leong WS, Smith AM, Worth AT, Stevenson GT. Thioether-
Bonded Constructs of Fab'gamma and Fcgamma Modules Utilizing Differential
Reduction of Interchain Disulfide Bonds J Immunol. 2001 Jan 15; 166(2):1320-
1326; Cao Y, Suresh MR. Bispecific MAb aided liposomal drug delivery. J Drug
Target. 2000; 8(4):257-66; Schoonjans R, et al Mertens N. Fab chains As an
efficient heterodimerization scaffold for the production of recombinant bispecific
and trispecific antibody derivatives J Immunol. 2000 Dec 15; 165(12):7050-7. ;
Schmiedl A, et al Expression of a bispecific dsFv-dsFv' antibody fragment in
escherichia coli Protein Eng. 2000 Oct; 13(10):725-34. Rohrbach F, et al
Construction and characterization of bispecific costimulatory molecules containing
a minimized CD86 (B7-2) domain and single-chain antibody fragments for tumor
targeting Clin Cancer Res. 2000 Nov; 6(11):4314-22. Schoonjans R, Efficient
heterodimerization of recombinant bi- and trispecific antibodies Bioseparation.
2000; 9(3):179-83. Wallace PK, etal Production of macrophage-activated killer
cells for targeting of glioblastoma cells with bispecific antibody to FcgammaRI and
the epidermal growth factor receptor Cancer Immunol Immunother. 2000 Nov;
49(9):493-503.Bioconjug Chem. 2000 Nov; 11(6):842-54. Conrath KE, et al
Camel single-domain antibodies as modular building units in. J Biol Chem. 2000
Oct 25 [Cancer Immunol Immunother. 2000 Oct; 49(8):441-8. Talac R, Nelson H.
Current perspectives of bispecific antibody-based immunotherapy J Biol Regul
Homeost Agents. 2000 Jul; 14(3):175-81. Tomlinson I, Holliger P. Methods for
generating multivalent and bispecific antibody fragments Methods Enzymol. 2000;
326:461-79 Morimoto K, Inouye K. Application of bispecific F(ab') fragments
prepared from IgMs against carcinoembryonic antigen and alkaline phosphatase
 
Clin Chem. 2000 Sep; 46(9):1492-3. Cochlovius B, et al Cure of Burkitt's
lymphoma in severe combined immunodeficiency mice by T cells, tetravalent CD3
x CD19 tandem diabody, and CD28 costimulation. Cancer Res. 2000 Aug 15;
60(16):4336-41. Takemura Si, et al. Construction of a diabody (small recombinant
bispecific antibody) using a refolding system Protein Eng. 2000 Aug; 13(8):583-
8. Katzenwadel A, et alConstruction and in vivo evaluation of an anti-PSA x anti-
CD3 bispecific antibody for the immunotherapy of prostate cancer. Anticancer
Res. 2000 May-Jun; 20(3A):1551-5. Yoshida H, Katayose Y, Suzuki M, Matsuno
S, Kudo T, Kumagai I. Functional Fv fragment of an antibody specific for CD28:
Fv-mediated co-stimulation of T cells. FEBS Lett. 2000 Jul 7; 476(3):266-71. 6
38: Zeidler R, et The Fc-region of a new class of intact bispecific antibody mediates
activation of accessory cells and NK cells and induces direct phagocytosis of
tumour cells. Br J Cancer. 2000 Jul; 83(2):261-6. 0 39: Hillairet et al Enhanced
targeting specificity to tumor cells by simultaneous recognition of two antigens.
Bioconjug Chem. 2000 Jul-Aug; 11(4):452-60. Weiner LM. Bispecific antibodies in
cancer therapy. Cancer J Sci Am. 2000 May; 6 Suppl 3:S265-71 Zangemeister-
Wittke U, Leech SH, Olie RA, Zuo Z, et al An efficient route to the production of an
IgG-like bispecific antibody. Protein Eng. 2000 May; 13(5):361-7 Helfrich W, et al
A rapid and versatile method for harnessing scFv antibody fragments with various
biological effector functions. J Immunol Methods. 2000 Apr 3; 237(1-2):131-45Lu
D, et al Acquired antagonistic activity of a bispecific diabody directed against two
different epitopes on vascular endothelial growth factor receptor 2. J Immunol
Methods. 1999 Nov 19; 230(1-2):159-71. Ohmi Y, et al Tumor-specific targeting of
T helper type 1 (Th1) cells by anti-CD3 x anti-c-ErbB-2 bispecific antibody.
Cancer Immunol Immunother. 1999 Nov; 48(8):456-62. Kipriyanov SM, et al G,
Schuhmacher J, Cochlovius B, Von der Lieth CW, Matys ER, Little M. Bispecific
tandem diabody for tumor therapy with improved antigen binding and
pharmacokinetics. J Mol Biol. 1999 Oct 15; 293(1):41-56.Segal DM, Weiner GJ,
Weiner LM. Bispecific antibodies in cancer therapy. Curr Opin Immunol. 1999
Oct; 11(5):558-62 4 88: Hudson PJ. Recombinant antibody constructs in cancer
therapy. Curr Opin Immunol. 1999 Oct; 11(5):548-57 Alt M, et al Novel
tetravalent and bispecific IgG-like antibody molecules combining single-chain
diabodies with the immunoglobulin gamma1 Fc or CH3 region. FEBS Lett. 1999
Jul 2; 454(1-2):90-4. Kontermann RE, Muller R. Intracellular and cell surface
displayed single-chain diabodies. J Immunol Methods. 1999 Jun 24; 226(1-2):179-
88. Morimoto K, Inouye K. Method for the preparation of bispecific F(ab')2mu
 
fragments from mouse monoclonal antibodies of the immunoglobulin M class and
characterization of the fragments. J Immunol Methods. 1999 Apr 22; 224(1-
2):43-50. Chaudri ZN, et al Dual specificity antibodies using a double-stranded
oligonucleotide bridge. FEBS Lett. 1999 Apr 30; 450(1-2):23-6. : Somasundaram
C, et al Development of a trispecific antibody conjugate that directs two distinct
tumor-associated antigens to CD64 on myeloid effector cells. Hum Antibodies.
1999; 9(1):47-54.Robert B, et al Tumor targeting with newly designed biparatopic
antibodies directed against two different epitopes of the carcinoembryonic antigen
(CEA). Int J Cancer. 1999 Apr 12; 81(2):285-91. Kreutz FT, et al Efficient
bispecific monoclonal antibody purification using gradient thiophilic affinity
chromatography. J Chromatogr B Biomed Sci Appl. 1998 Sep 4; 714(2):161-70.
Muller KM, et al A dimeric bispecific miniantibody combines two specificities with
avidity. FEBS Lett. 1998 Jul 31; 432(1-2):45-9. Kreutz FT, et al A new method
to generate quadromas by electrofusion and FACS sorting. Hybridoma. 1998 Jun;
17(3):267-73Production of bispecific and trispecific F(ab)2 and F(ab)3 antibody
derivatives. Methods Mol Biol. 1998; 80:121-34. Merchant AM, et Protein,
Nucleotide An efficient route to human bispecific IgG. Nat Biotechnol. 1998 Jul;
16(7):677-81. Zhu Z, et al High level secretion of a humanized bispecific diabody
from Escherichia coli. Biotechnology (N Y). 1996 Feb; 14(2):192-6. Rapid assay
of phage-derived recombinant human fabs as bispecific antibodies.
Biotechnology (N Y). 1995 Nov; 13(11):1221-4. McGuinness BT, et Phage
diabody repertoires for selection of large numbers of bispecific antibody
fragments. Nat Biotechnol. 1996 Sep; 14(9):1149-54Helfrich W, et al
Construction and characterization of a bispecific diabody for retargeting T cells to
human carcinomas. Int J Cancer. 1998 Apr 13; 76(2):232-9. Muller KM, et al The
first constant domain CH1 and CL of an antibody used as heterodimerization
domain for bispecific miniantibodies. FEBS Lett. 1998 Jan 30; 422(2):259-64.
223: FitzGerald K, et al Improved tumour targeting by disulphide stabilized
diabodies expressed in Pichia pastoris. Protein Eng. 1997 Oct; 10(10):1221-5.
Coloma MJ, Morrison SL. Design and production of novel tetravalent bispecific
antibodies. Nat Biotechnol. 1997 Feb; 15(2):159-63. Hoogenboom HR. Mix and
match: building manifold binding sites. Nat Biotechnol. 1997 Feb; 15(2):125-6.
Ridgway JB, et al 'Knobs-into-holes' engineering of antibody CH3 domains for
heavy chain heterodimerization. Protein Eng. 1996 Jul; 9(7):617-21. Reinartz
HW, et al Bispecific multivalent antibody studied by real-time interaction analysis
for the development of an antigen-inhibition enzyme-linked immunosorbent assay.
 
Analyst. 1996 Jun; 121(6):767-71. Tibben JG, et al Pharmacokinetics,
biodistribution and biological effects of intravenously administered bispecific
monoclonal antibody OC/TR F(ab')2 in ovarian carcinoma patients. Int J Cancer.
1996 May 16; 66(4):477-83. de Kruif J, Logtenberg T. Leucine zipper dimerized
bivalent and bispecific scFv antibodies from a semi-synthetic antibody phage
display library. J Biol Chem. 1996 Mar 29; 271(13):7630-4. Ruf J, et al The
molecular recognition theory applied to bispecific antibodies... Nat Med. 1995
Dec; 1(12):1222. 447: Grant SD, et al Expression of monovalent and bivalent
antibody fragments in Escherichia coli. J Hematother. 1995 Oct; 4(5):383-8.
Dubel S, et al Protein, Nucleotide Bifunctional and multimeric complexes of
streptavidin fused to single chain antibodies (scFv). J Immunol Methods. 1995
Jan 27; 178(2):201-9. Kostelny SA, et al Protein, Nucleotide Formation of a
bispecific antibody by the use of leucine zippers. J Immunol. 1992 Mar 1;
148(5):1547-53 Pack P, Pluckthun A. Miniantibodies: use of amphipathic helices
to produce functional, flexibly linked dimeric FV fragments with high avidity in
Escherichia coli. Biochemistry. 1992 Feb 18; 31(6):1579-84 Smith W, et
alImmunoglobulins secreted by a hybrid-hybridoma: analysis of chain assemblies.
Hybridoma. 1992 Feb; 11(1):87-98. Suresh MR,et al Bispecific monoclonal
antibodies from hybrid hybridomas. Methods Enzymol. 1986; 121:210-28.
Paulus H. Preparation and biomedical applications of bispecific antibodies.
Behring Inst Mitt. 1985 Dec; (78):118-32. Brennan M, et al Preparation of
bispecific antibodies by chemical recombination of monoclonal immunoglobulin
G1 fragments. Science. 1985 Jul 5; 229(4708):81-3.
 
Appendix D

Target cell-associated markers and their tissue distribution and antibodies thereto
are well known to those skilled in the art and described in standard reference works
in immunology, rheumatology, pathology, internal medicine, experimental medicine,
cell biology and clinical and experimental laboratory manuals, and source works on
CD antigens. For example with respect to cells of hemapoietic origin see Cell and
Molecular Immunology Fourth Ed. (supra) Appendix II, Manual Of Clinical Chemical
Immunology Sixth Ed. Rose NR et al Eds. Antibodies to CD antigens are described
at the following exemplary websites (most of whom also publish product catalogs
and lists):

http://www.biosource.com/
http://www1.amershambiosciences.com/aptrix/upp01077.nsf/Content/Products
http://www.sigmaaldrich.com/
http://www.upstatebiotech.com/
http://www.promega.com/Default.htm
http://www.accuratechemical.com/accuratechemical/
www.researchd.com/rdicdabs/cdindex.htm
http://home.labvelocity.com/scientists/index.jhtml
http://www.mh-hannover.de/aktuelles/projekte/hlda7/main/
http://southernbiotech.com/catalog.html
http://www.prosci-inc.com/
http://www.abcam.com/
http://www.usbio.net/
http://www.mabtech.com/
http://www.antibodies-probes.com/
http://www.labvision.com/
http://www.crpinc.com/products/index.html

With respect to first and second target ligands see also US Application
20020197655; US 20020168362; Methods Mol Biol 2001; 166:177-92; USP
6,071,517; USP 5,897,861; USP 6,096,311; USP 5,922,845, Journal of
Immunological Methods February 2001 Vol. 248(1-2) page 1-200; Mol Immunol
1999 May; 36(7):433-45; US6051227 US06143297 US05977318
 
US05968510 US05885796 US05885579 US05869050 Methods of blocking T-
cell activation using anti-B7 monoclonal antibodies; US05851795 US05747034
US06113901 US05844095 US06090914 US05718883 Transgenic animal
model for autoimmune diseases; . US05855887 US05811097 US06084067
EP01073741A2 US06130316 US06068984 Antibodies to lymphocyte
activation antigens uses therefor; US05766570 Lymphocyte activation antigens
and thereto; US05434131 US05316920 Lymphocyte activation antigen HB15,
a member of the immunoglobulin superfamily; US06111090 Mammalian cell
surface antigens; US06083751 independent cytotoxic T cells; US05977303
Mammalian cell surface antigens; US05738852 Methods of enhancing antigen-
specific T cell responses US05714667, 6,500,674 Method for the diagnosis of
brain/neurological disease using monoclonal antibodies specific for PHF-tau,
hybridomas secreting them, and antigen recognition by these antibodies and their
applications 6,294,167 Pharmaceutical compositions for immunotherapy
containing antibodies which specifically recognize the MHCII antigen of a patient to
be treated 6,258,564 Antibodies, production method of the antibodies,
hybridomas which produce the antibodies, production method the hybirdomas and
antigen proteins recognized by the antibodies 6,255,107 Antibodies, production
method of the antibodies, hybridomas which produce the antibodies, production
method of the hybridomas and antigen proteins recognized by the antibodies
6,245,897 Monoclonal antibody recognizing cell surface antigen CD14 6,193,948
Antigen recognized by patients with antibody associated paraneoplastic sensory
neuronopathy, DNA encoding same and uses thereof 6,132,980 Antibodies
specific for TRP-2 a human tumor antigen recognized by cytotoxic T lymphocytes
6,074,833 Osteoblast and fibroblast antigen and antibodies recognizing it
6,008,024 Monoclonal antibodies specific for PHF-tau, hybridomas secreting them,
antigen recognition by these antibodies and their applications 5,985,541 Peptide
capable of being recognized by antibodies recognizing the C33 antigen of hepatitis
C virus 5,925,526 Antigen recognized by patients with antibody associated
cerebellar degeneration, DNA encoding same and uses thereof 5,861,257
Monoclonal antibodies directed against the microtubule-associated protein tau,
hybridomas secreting these antibodies, antigen recognition by these monoclonal
antibodies and their applications 5,807,705 Antigen recognized by patients with
antibody associated paraneoplastic sensory neuronopathy, DNA encoding same
and uses thereof 5,773,292 Antibodies binding portions, and probes recognizing
an antigen of prostate epithelial cells but not antigens circulating in the blood
 
5,703,213 Monoclonal antibodies which recognize an adenocarcinoma cell antigen
5,668,013 Antigen recognized by patients with antibody associated paraneoplastic
cerebellar degeneration, DNA encoding same and uses thereof 5,665,357
Antibodies recognizing tumor associated antigen CA 55.1 5,614,371 Ri fusion
antigen recognized by antibodies associated with paraneoplastic opsoclonus and
methods of use thereof 5,603,934 Antigen recognized by patients with antibody
associated paraneoplastic sensory neuronopathy 5,597,707 Tumor associated
antigen recognized by the murine monoclonal antibody L6, its oligonucleotide
sequence and methods for their use 5,595,738 CTAA 81AV78, the antigen
recognized by human monoclonal antibody 81AV78 5,556,947 Monoclonal
antibody recognizing a surface molecule on a subset of antigen-stimulated T cells
and on certain malignancies of T and B cell origin 5,411,884 Monoclonal antibody
L53 which recognizes a human tumor-associated antigen 5,212,085 SF-25 Colon
adenocarcinoma antigen, and antibodies with recognize this antigen 5,075,219
Monoclonal antibody which recognizes a specific glycoprotein of a human milk-fat
globule membrane mucin antigen and said mucin antigen 4,772,552 Monoclonal
antibody which recognizes a 200-220 KD antigen on natural killer cells
 [0340] The present invention furthermore relates to the following items:
1. A composition comprising a multispecific ligand comprising at least a first ligand binding moiety which specifically binds to a first ligand having a first biodistribution and a second ligand binding moiety which specifically binds to a second ligand having a second biodistribution different from that of the first ligand, and wherein the affinity of the first and second ligand binding moieties are different and selected to bias the biodistribution of the multispecific ligand.
2. The composition according to item 1, further comprising a physiologically acceptable excipient
3. The composition according to item 2, wherein the multispecific ligand comprises a bispecific antibody.
4. The composition according to item 3, wherein the affinity of said first ligand binding moiety for the first ligand is higher than the affinity of the second ligand binding moiety for the second ligand and wherein the biodistribution of the multispecific ligand favours the first ligand.
5. The composition according to item 4, wherein the first and second ligands have overlapping biodistributions.
6. The composition according to item 5, wherein the first ligand is present on a first target cell population and wherein said second ligand is present on a second target cell population comprising the first target cell population and wherein the biodistribution of the multispecific ligand favours the first target cell population.
7. The composition according to item 1 or 3, wherein said first ligand is a cell surface marker associated with one or more specific cell populations, infectious or parasitic agents, diseased cells, or disease-associated cells.
8. The composition according to item 7, wherein said marker is an antigen.
9. The composition according to item 7, wherein said marker is an epitope.
10. The composition according to item 7, wherein said marker is a CD marker.
11. The composition according to item 10, wherein said marker is CD4.
12. The composition according to item 7, wherein said marker is specifically associated with a cancer cell or pre-cancerous cell.
13. The composition according to item 11 or 12, wherein said second ligand is a CCR5 or CXCR4 receptor.
14. The composition according to item 7, wherein said marker is: associated with an immune cell that is susceptible to viral infection.
15. The composition according to item 7, wherein said marker is specifically associated with an autoimmune disorder or rheumatic disease.
16. The composition according to item 7, wherein said marker is associated with a specific tissue type.
17. The composition according to item 7, wherein said marker is associated with a specific organ.
18. The composition according to item 7, wherein said marker is associated with a cell or tissue of specific origin or class.
19. The composition according to item 7, wherein said marker is an MHC-peptide complex.
20. The composition according to item 7, wherein said marker is a cell surface immunoglobulin.
21. The composition according to item 6 or 7, wherein said second ligand is a cell surface receptor, a family of cell surface receptors or one or more particular cell surface receptor family members.
22. The composition according to item 21, wherein said second ligand is a cell surface receptor.
23. The composition according to item 22, wherein said second ligand is a marker associated with a lymphatic endothelial cell.
24. The composition according to item 22, wherein said second ligand is a cell surface receptor is selected from the group consisting of tyrosine kinase type receptors, serine kinase type receptors, heterotrimeric G-protein coupled receptors, receptors bound to tyrosine kinase, TNF family receptors, notch family receptors, guanylate cyclase types, tyrosine phosphatase types, decoy receptors, and adhesion receptors.
25. The composition according to item 22, wherein said second ligand is an IL-8 receptor, a CCR7 receptor, a FAS receptor, or a CXCR4 receptor.
26. The composition according to item 22, wherein said receptor requires cross-linking for biological activity.
27. The composition according to item 22, wherein binding of said second ligand binding moiety to said cell surface receptor blocks said receptor.
28. The composition d according to item 22, wherein binding of said second ligand binding moiety to said cell surface receptor activates said receptor.
29. The composition according to item 22, wherein said cell surface receptor initiates a signal transduction and wherein binding of said second ligand binding moiety to said cell surface receptor effects a signal transduction.
30. The composition according to item 6, 7 or 22, wherein said antibody comprises a first VH which specifically recognizes said first ligand and a second VH which specifically recognizes said second ligand.
31. The composition according to item 30, wherein at least one of said first and second VHs require a VL for binding to its ligand.
32. The composition according to item 31, comprising a first VL in functional association with said first VH and a second VL in functional association with said second VH and wherein both said first and second functional associations are required for binding to the first and second ligands, respectively, and wherein said first and second VLs are the same or functionally interchangeable.
33. The composition according to item 31 or 32, wherein said antibody is a four chain antibody.
34. The composition according to item 33, wherein said antibody is a minibody or antibody lacking a CH3 domain.
35. The composition according to item 33, wherein said antibody is a diabody.
36. The composition according to item 31 or 32, wherein said antibody lacks antibody light chains.
37. The composition according to item 32, wherein said antibody comprises a pair of disulfide linked heavy chains or heavy chain portions each comprising at least a VH region, a hinge region and at least a portion of an Fc region at the carboxy terminus of the hinge region.
38. The composition according to item 37, wherein said bispecific antibody comprises a pair of VHs linked through a flexible linker.
39. The composition according to item 4, 6, 7 or 22 wherein the affinity of the first ligand binding moiety for the first ligand is at least approximately, one, two, three, four, five, six, seven or eight orders of magnitude greater than the affinity of said second ligand binding moiety for the second ligand.
40. A composition comprising a multispecific ligand comprising a first ligand binding moiety which specifically binds with a pre-selected first affinity to a first ligand having a first biodistribution and a second ligand binding moietv which specifically binds with a pre-selected affinity to a second ligand having a second biodistribution, and wherein the affinity of first and second ligand binding moieties are selected to bias the biodistribution of the multispecific ligand.
41. The composition according to item 40, further comprising a physiologically acceptable excipient.
42. The composition according to item 1 or 40, wherein the biodistributions of said first and second ligands overlap and wherein the affinities of the first and second ligand binding moieties are selected to bias the biodistribution of the multispecific ligand in favour of a target cell population on which both first and second biodistributions occur relative to one or more non-target cell populations.
43. The composition according to item 42, wherein the affinities of said first and second ligand binding moieties are both selected to limit their individual ability to bind to the first and second ligands, respectively, and wherein their combined functional affinity biases the distribution of the multispecific ligand towards said target cell population.
44. The composition according to item 42, wherein the affinity of first ligand binding moiety for the first ligand is at least, approximately, one, two, three, four, five, six, seven or eight orders of magnitude greater than the affinity of the second ligand binding moiety for the second ligand.
45. The composition according to item 42 or 44, wherein first and second ligands are recognized contemporaneously by the first and second ligand binding moieties.
46. A composition comprising a multispecific ligand which specifically binds to a target ligand on a selected sub-population of a heterogeneous cell population bearing the target ligand, the multispecific ligand comprising a first ligand binding moiety which specifically binds to a cell sub-population associated ligand and a second ligand binding moiety which binds to the target ligand, said first ligand binding moiety having an affinity for the sub-population associated ligand that is higher than the affinity of the second ligand binding moiety for the target ligand.
47. The composition according to item 46, further comprising a physiologically acceptable excipient.
48. The composition according to item 46, wherein the affinity of said first ligand binding moiety for the cell sub-population associated ligand is approximately, one, two, three, four, five, six, seven or eight orders of magnitude greater than the affinity of said second ligand binding moiety for said target ligand.
49. The composition according to item 48, wherein said target ligand is a receptor.
50. The composition according to item 46, wherein at least one of said first or second ligand binding moieties comprises an antibody heavy chain or functional portion(s) thereof including a VH or fragment thereof and an antibody light chain or functional portion(s) thereof including a VH or fragment thereof.
51. A composition comprising an antibody which specifically binds to an epitope on a ligand wherein said ligand exerts a biologic effect by binding to a target site on a target ligand through an affinity for said target ligand, said epitope being proximal to the binding site of said ligand for the target ligand, such that the antibody reduces but does not prevent the affinity of the ligand for its target ligand.
52. The composition according to item 51, further comprising a physiologically acceptable excipient.
53. A composition comprising a multispecific ligand comprising a first ligand binding moiety which specifically binds to a lymphatic endothelial cell associated marker and a second moiety comprising a therapeutic moiety.
54. The composition according to item 53, further comprising a physiologically acceptable excipient.
55. The composition according to item 53, wherein the therapeutic moiety provides an immune function.
56. The composition according to item 53, wherein the marker is selected to limit the ability of said endothelial cell to internalize said multispecific ligand.
57. The composition according to item 53, wherein said first portion is an antibody.
58. The composition according to item 54, wherein said second portion moiety binds to a target ligand.
59. The composition according to item 53, wherein said therapeutic moiety comprises an antibody moiety.
60. The composition according to item 53, wherein said ligand is selected from the group consisting of CCR5, CTLA-4, LFA-1, ICAM-1, CD2, CD3, CD4, CD22, CD40, CD44; CD80, CD86, CD134 and CD154.
61. The composition according to item 53, wherein said first portion binds to LYVE-1 or podoplantin.
62. The composition according to item 53, wherein said second portion comprises an anti-idiotypic antibody.
63. The composition according to item 62, wherein said anti-idiotypic antibody binds to an autoimmune antibody.
64. The composition according to item 63, wherein said anti-idiotypic antibody mimics a cell surface expressed tumour antigen.
65. The composition according to item 53, wherein said second portion binds to a diseased cell.
66. The composition according to item 65, wherein said diseased cell is a cancer cell.
67. The composition according to item 53, wherein said second portion binds to an infectious agent or parasite.
68. The composition according to item 67, wherein said diseased cell is a virally infected cell.
69. The composition according to item 53, wherein said second portion binds to a cell of the immune system.
70. The composition according to item 69, wherein immune cell is associated with an autoimmune reaction.
71. The composition according to item 69, wherein said immune cell is a CCR5-expressing cell.
72. The composition according to item 64 or 69, wherein said second portion binds with greater functional affinity to its target ligand than said first portion binds to its target ligand.
73. The composition according to item 64 or 69, wherein said second portion binds with greater affinity to its target ligand than said first portion binds to its target ligand.
74. The composition according to item 53, wherein said second portion binds with greater avidity to its target ligand than said first portion binds to its target ligand.
75. The composition according to item 53, wherein said second portion comprises an internalizing antibody and a cytotoxic component
76. The composition according to item 53, wherein said multispecific ligand is a bispecific antibody having a monovalent first portion and a monovalent second portion.
77. The composition according to item 53, wherein said multispecific ligand is a bispecific antibody having a divalent first portion and a divalent second portion.
78. The composition according to item 53, wherein said multispecific ligand is a trispecific antibody having a monovalent first portion and a second portion comprising a divalent immune function exerting moiety which binds to one or more target ligands on a target diseased cell or immune cell and a monovalent anti-CD3 or anti-CD28 antibody.
79. The composition according to item 53, wherein said multispecific ligand is a trivalent trispecific antibody having a monovalent first portion and a second portion comprising a monovalent immune function exerting moiety which binds to a target ligand on a target diseased or immune cell and a monovalent anti-CD3 or anti-CD28 antibody.
80. The composition according to item 53, wherein said mulfispecific ligand is a trivalent trispecific antibody having a monovalent first portion and a second portion comprising a divalent immune function exerting moiety which binds to a target ligand on a target diseased or immune cell.
81. The composition according to item 53, wherein said second portion comprise a cytokine component.
82. The composition according to item 53, wherein said second portion comprises a cytotoxic component.
83. The composition according to item 53, wherein said second portion comprises a ligand capable of binding to T cells.
84. The composition according to item 83, wherein said ligand is an antibody which binds to T cells.
85. The composition according to item 53, wherein said second portion comprises an anti-CD3 antibody or anti-CD28 antibody.
86. The composition according to item 53, wherein second portion is a cytokine component.
87. The composition according to item 53, wherein second portion is an anti-CD3 antibody or an anti-CD28 antibody.
88. The composition according to item 53, wherein said second portion further comprises one or more components selected from the group consisting of a cytokine component, a cytotoxic component and an anti-CD3/CD28 component.
89. A composition comprising an immunocytokine having an anti-idiotypic antibody component which recognizes the paratope of an antibody which binds to a lymphatic vessel associated ligand and a cytokine component.
90. The composition according to item 89, wherein the cytokine component is fused with or.conjugated to the lymphatic vessel associated ligand.
91. An immunocytokine as claimed in item 89, wherein said cytokine component comprises IL-2 or a functional fragment thereof and/or IL-12 or a functional fragment thereof.
92. An immunocytokine as claimed in item 89, wherein said cytokine component comprises TNF-α or a functional fragment thereof.
93. A composition comprising a bispecific antibody having an anti-idiotypic antibody component which recognizes the paratope of an antibody which binds specifically to a lymphatic vessel associated ligand and an anti-CD3 antibody or an anti-CD28 antibody component.
94. The composition according to item 90 or 91, wherein said anti-idiotypic antibody component has a lower functional affinity for the paratope of the antibody which binds specifically to the lymphatic vessel associated ligand than the later antibody has for the lymphatic vessel associated ligand.
95. A composition comprising a bispecific ligand comprising a first ligand which binds to a first target ligand and a second ligand which binds to a second target ligand, and wherein the affinity of said first ligand is selected to enable binding to the first target ligand independently of the ability of said second ligand to bind to the second target ligand and wherein the affinity of said second ligand is selected to substantially reduce the probability of its binding to the second, target ligand without the first ligand binding first or substantially contemporaneously to the first target ligand.
96. A composition comprising a bispecific antibody comprising a first antibody component which binds to a first target ligand and a second antibody component which binds to a second target ligand, and wherein the affinity or avidity or both the affinity and avidity of said first antibody component are selected to enable binding to the first target ligand independently of the ability of said second antibody component to bind to the second target ligand and wherein the avidity or affinity or both the affinity and avidity of said second ligand are selected to substantially reduce the probability of its binding to the second target ligand without the first ligand binding first or substantially contemporaneously to the first target ligand.
97. A composition comprising a multispecific ligand comprising a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety are selected to substantially reduce the probability of its binding to the second target ligand without the first moiety, first or substantially contemporaneously, binding to the first target ligand.
98. The composition according to item 97, wherein both moieties bind to different target ligands on the same cell.
99. A composition comprising a multispecific ligand comprising a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety are selected to substantially reduce the probability of either moiety binding for a sufficient duration or series of durations to its respective target ligand to accomplish a therapeutic function without the other moiety, first or substantially contemporaneously, binding to its respective target ligand.
100. The composition according to item 99, wherein both moieties bind to different target ligands on the same cell.
101. A composition comprising a multispecific ligand comprising a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety are selected to enable the second moiety to bind to the second entity in preference to the first moiety binding to the first entity when both first and second moieties are substantially contemporaneously bound to the respective first and second entities.
102. The composition according to item 101, wherein the first moiety comprises at least one antibody component which binds to a first cell and the second moiety comprises at least one antibody component which binds to a second different cell.
103. A composition comprising a multispecific ligand comprising; a first moiety which binds to a first target ligand and a second moiety which binds to a second target ligand, and wherein the affinity or avidity or both the affinity and avidity of said first moiety are selected to enable the first moiety to bind to the first target ligand independently of the ability of said second moiety to bind to the second target ligand and wherein the avidity or affinity or both the affinity and avidity of said second moiety to bind to the second target ligand and wherein the avidity or affinity or both the affinity and avidity of said first moiety are selected to enable the first moiety to bind to the first entity in preference to the second moiety binding to the second entity when both first and second moieties are substantially contemporaneously bound to the respective first and second entities, and wherein the avidity or affinity or both the affinity and avidity of said second moiety are selected to enable the third target ligand to bind to the second entity in preference to the second moiety binding to the second entity when both said third target ligand and the second moiety are substantially contemporaneously bound to the second entity.
104. A composition comprising a multispecific ligands comprising at least a first ligand binding moiety which specifically binds with a pre-selected first affinity to at least a first ligand having a first biodistribution and a second ligand binding moiety which specifically binds with a pre-selected affinity to at least a second ligand having a second biodistribution, and wherein the affinity of first and second ligand binding moieties are selected to bias the biodistribution of the multispecific ligand in favour of a selected location of one or both of the ligands.
105. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the.first and second ligand binding moieties are different and selected to bias the biodistribution of the multispecific ligand, and wherein the affinity of the first ligand binding moiety for the first ligand is at least, approximately, one order of magnitude greater than that of the second ligand binding moiety for the second ligand.
106. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the muftispecific ligand, and wherein the affinity of the first ligand binding moiety for the first ligand is at least, approximately, two orders of magnitude greater than that of the second ligand binding moiety for the second ligand.
107. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the multispecific ligand, and wherein the affinity of the first ligand binding moiety for the first ligand is at least, approximately, three orders of magnitude greater than that of the second ligand binding moiety for the second ligand.
108. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the multispecific ligand, and wherein the affinity of the first ligand binding moiety for the first ligand is at least, approximately, four orders of magnitude greater than that of the second ligand binding moiety for the second ligand.
109. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the multispecific ligand, and wherein the affinity of the first ligand binding moiety for the first ligand is at least, approximately, five orders of magnitude greater than that of the second ligand binding moiety for the second ligand.
110. A composition comprising a multispecific ligand comprising at least a first ligand binding moiety which specifically binds to a first ligand having a first biodistribution and a second ligand binding moiety which specifically binds to a second ligand having a second biodistribution, and wherein the affinity of the first and second ligand binding moieties are different and selected to bias the biodistribution of the multispecific ligand, and wherein the affinity of the first ligand binding moiety for the first ligand is at least, approximately, six orders of magnitude greater than that of the second ligand binding moiety for the second ligand.
111. A composition according to any one of items 105 to 110, wherein the biodistributions of said first and second ligands comprise a target population of cells and at least one non-target population of cells and wherein said first and second ligands are present only on said target population and wherein the biodistribution of the multispecific ligand is biased in favor of the target population of cells.
112. A composition according to item 111, wherein said multispecific ligand is adapted to bind to two ligands on the same cell.
113. A composition according to item 112, wherein said multispecific ligand comprises at least two full length heavy chains or heavy chain fragments, having differing specificities, or is chosen from a F(ab')₂, a minibody, a diabody, a four chain immunoglobulin having a truncated Fc portion, a tetravalent antibody having a four chain framework and a divalent Fab.
114. A host cell or cell free expression medium comprising one or more polynucleotides, said one or more polynucleotides comprising one or more DNA sequences, said one or more DNA sequences comprising one or more polypeptides which are sufficient to constitute a multispecific ligand as defined in any of the preceding items
115. A kit comprising one or more polynucleotides, said one or more polynucleotides comprising one or more DNA sequences, said one or more DNA sequences encoding one or more polypeptides which are sufficient to constitute a multispecific ligand as defined in any of the preceding items
116. A liquid medium comprising comprising one or more polypeptides which are sufficient to constitute a multispecific ligand as defined in any of the preceding items.
117. A liquid medium comprising one or more host cells, said one or more host cells comprising one or more polynucleotides, said one or more polynucleotides comprising one or more DNA sequences, said one or more DNA sequences encoding one or more polypeptides which are sufficient to constitute a multispecific ligand as defined in any of the preceding items
118. A substantially isolated polynucelotide comprising one or more DNA sequences, said , said one or more DNA sequences encoding one or more polypeptides which are sufficient to constitute a multispecific ligand as defined in any of the preceding items.
119. A substantially isolated polynuceotide comprising a DNA sequence encoding a polypeptide portion of a second ligand binding moiety as defined in any of the preceding items, said polypeptide portion comprising a VH or VL, said second ligand binding moiety having a low affinity for said second ligand.
120. A substantially isolated polynucleotide according to paragraph 119, wherein said polynucleotide is a substantially isolated expression or cloning vector.
121. A method of making a multispecific ligand as defined in any of the preceding paragraphs comprising expressing at least one polynucleotide as defined in item 115, 118, 119 or 120.
122. A pharmaceutical composition comprising a multispecific ligand as defined in any of the preceding items and a pharmaceutically acceptable excipient.
123. A therapeutic composition comprising a multispecific ligand as defined in any of the preceding paragraphs and a pharmaceutically acceptable excipient.
124. A method of treating a disease in a mammal comprising administering a therapeutically effective amount of a multispecific ligand according to any of the preceding items.
125. A kit comprising a plurality of different multispecific ligands as defined herein.
126. A composition according to item 1, 111 or 112, wherein at least one of said first and second ligand binding moieties, comprises human sequences.
127. A composition according to item 1 or 111, wherein at least one of said first and second ligand binding moieties comprises human framework sequences.
128. A combinatorial library comprising a diverse population of multispecific ligands according to item 1, 111 or 112, chararacterized by members of said population having a diversity of affinities for at least one of said first and second ligands.
129. A diverse population of nucleic acids which encode a combinatorial library as defined in item 28.
130. A multispecific ligand according to any of items 1 to 10, wherein said first ligand is on a cell population which circulates in the blood.

## Claims

1. A multispecific ligand comprising:
(i) at least a first ligand binding moiety which preferentially binds with a first affinity to a first ligand having a first biodistribution associated with a target cell population; and
(ii) at least a second ligand binding moiety which preferentially binds with a second affinity to a second ligand having a second biodistribution which is different from that of the first ligand and overlaps therewith, the second ligand being present on a broader population of cells;
wherein the affinity of said first and second ligand binding moieties are selected to bias the biodistribution of the multispecific ligand; and
wherein the affinity of said first ligand binding moiety for the first ligand is higher than the affinity of the second ligand binding moiety for the second ligand so as to bias the biodistribution of the multispecific ligand in favour of the first ligand; and
wherein the first and second ligands are present on the same target cell population and bioavailable for contemporaneous recognition by the first and second ligand binding moieties; and
wherein the multispecific ligand is adapted to bind contemporaneously to first and second ligands; and
wherein the affinity of the first ligand binding moiety for the first ligand:
(a) is pre-selected to serve a targeting function, and is at least sufficient for the first ligand binding moiety to bind to the first ligand independently of the second ligand binding moiety binding to the second ligand; and
(b) is pre-selected to at least provide opportunity for the second ligand binding moiety to bind the second ligand when the first ligand binding moiety is bound to the first ligand; and
wherein the affinity of the second ligand binding moiety for the second ligand:
(a) is pre-selected to have a diminished ability to bind and/or stay bound to the second ligand independently of the binding of the first ligand binding moiety to the first ligand; and
(b) is at least approximately one order of magnitude lower than the affinity of the first ligand binding moiety for the first ligand;
and wherein the first ligand binding moiety and the second ligand binding moiety are each respectively, an antibody, an antigen binding fragment of said antibody, a natural ligand for the first or second ligand or mutated forms of the natural ligand with an optimized affinity of said ligand for the target ligand; and
wherein the second ligand binding moiety is an effector having the ability to effect a biologic consequence, wherein said biological consequence is selected from:
(i) blockade of CD95L on activated T cells;
(ii) blockade of IFNγ on endothelial cells;
(iii) blockade of IL-15 receptor on CD8+ T cells;
(iv) blockade of CTLA-4 on CD8+ T cells;
(v) blockade of VCAM-1 on activated endothelial cells;
(vi) blockade of VCAM-1 on endothelial cells or activated endothelial cells;
(vii) blockade of ICAM-1 on endothelial cells or activated endothelial cells;
(viii) blockade of TGF-β on CD4+ or CD8+ T cells;
(ix) blockade of CD80/86 on dendritic cells;
(x) cross-linking of CD3 on CD45RO cells; and
(xi) enhancing association of Fc gamma RII and Fc epsilon RI on mast cells.

2. The multispecific ligand according to claim 1, wherein the first ligand binding moiety and the second ligand binding moiety are antibodies including antigen binding fragments thereof.

3. The multispecific ligand according to claim 1 or 2 wherein the second ligand binding moiety directly exerts a biological effect by directly enhancing or inhibiting a cellular response of the target population to the multispecific ligand when the multispecific ligand is administered in an effective amount.

4. The multispecific ligand according to any one of claims 1 to 3, wherein the affinity of the first ligand binding moiety for the first ligand is at least approximately nanomolar affinity.

5. The multispecific ligand according to any one of claims 1 to 3, wherein the affinity of the first ligand binding moiety for the first ligand is at least approximately 0.1 nanomolar affinity.

6. The multispecific ligand according to any one of claims 1 to 3, wherein the affinity of the first ligand binding moiety for the first ligand is at least approximately two orders of magnitude higher than the affinity of the second ligand binding moiety for the second ligand.

7. The multispecific ligand according to any one of claims 1 to 3, wherein the affinity of the first ligand binding moiety for the first ligand is at least approximately three orders of magnitude higher than the affinity of the second ligand binding moiety for the second ligand.

8. The multispecific ligand according to any one of claims 1 to 3, wherein the affinity of the first ligand binding moiety for the first ligand is between one and three orders magnitude higher than the affinity of the second ligand binding moiety for the second ligand.

9. A composition comprising the multispecific ligand of any one of the preceding claims and a pharmaceutically acceptable excipient or carrier, wherein the amount of the multispecific ligand in the composition is effective for the multispecific ligand to enhance or inhibit a cellular response of the target cell population.

10. The multispecific ligand of any of claims 1 to 8 for treating a condition in which the effector function of the second ligand binding moiety is indicated.

11. A method of identifying a multispecific ligand as defined in any of claims 1 to 8, comprising the steps of (1) screening variants of a dual affinity multispecific ligand; and (2) selecting variants having an improved or diminished ability, relative to a control ligand, to bind to, block or effect a signal transduction or otherwise enhance or diminish a cellular response of cells of a heterogeneous population or target cell-population.

12. A method according to claim 11, wherein the improved or diminished ability is demonstrated in a competition binding experiment.

13. A method according to claim 11, wherein the first ligand binding moiety or the second ligand binding moiety is selected from a library of variants having differing affinities.

14. The multispecific ligand of any one of claims 1 to 8 for
(1) facilitating the evaluation of a more targeted biodistribution of the second ligand binding moiety;
(2) assessing the safety of administering a second ligand binding moiety that would be inappropriate for therapeutic use in the absence of a first ligand binding moiety; or
(3) providing a vehicle to preferentially target, on a sub-population of cells for which there is a first ligand, a receptor or receptor ligand which is present on a more heterogeneous population of cells.
